# EUROPÄISCHE PATENTSCHRIFT

(11) **EP 2 144 879 B1**
(45) Veröffentlichungstag und Bekanntmachung des Hinweises auf die Patenterteilung: **08.04.2015**
(21) Anmeldenummer: 08735266.2
(22) Anmeldetag: 16.04.2008
(51) Int. Cl.: C07D 213/82, C07D 401/06, C07D 413/06, C07D 417/06, C07D 211/76, A01N 25/32

(54) **PYRIDONCARBOXAMIDE, DIESE ENTHALTENDE NUTZPFLANZENSCHÜTZENDE MITTEL UND VERFAHREN ZU IHRER HERSTELLUNG UND DEREN VERWENDUNG**
PYRIDON CARBOXAMIDES, AGENTS CONTAINING THESE BUT NOT IMPACTING USEFUL PLANTS AND METHOD FOR THEIR MANUFACTURE AND APPLICATION
PYRIDINECARBOXAMIDE, AGENT PHYTOPROTECTEUR LA COMPORTANT, SON PROCÉDÉ DE FABRICATION ET SON UTILISATION

(30) Priorität: 30.04.2007 EP 07400013
(43) Veröffentlichungstag der Anmeldung: 20.01.2010
(73) Patentinhaber: Bayer Intellectual Property GmbH, 40789 Monheim am Rhein (DE)
(72) Erfinder: ZIEMER, Frank, 65830 Kriftel (DE); WILLMS, Lothar, 65719 Hofheim (DE); ROSINGER, Christopher.H., 65719 Hofheim (DE); AULER, Thomas, 42799 Leichlingen (DE); HACKER, Erwin, 65329 Hochheim (DE); BICKERS, Udo, 65779 Kelkheim (DE)
(74) Vertreter: BIP Patents
(86) Internationale Anmeldenummer: PCT/EP2008/003016
(87) Internationale Veröffentlichungsnummer: WO 2008/131860

(56) Entgegenhaltungen:
- EP-A- 0 298 134
- EP-A- 0 544 151
- WO-A-2006/007981
- GB-A- 2 305 174
- LANG R W ET AL: "SYNTHESIS OF SELECTIVELY TRIFLUOROMETHYLATED PYRIDINE DERIVATIVES AS POTENTIAL ANTIHYPERTENSIVES" HELVETICA CHIMICA ACTA, VERLAG HELVETICA CHIMICA ACTA. BASEL, CH, Bd. 71, 4. Mai 1988 (1988-05-04), Seiten 596-601, XP000601402 ISSN: 0018-019X in der Anmeldung erwähnt

## Beschreibung

Die vorliegende Erfindung betrifft nutzpflanzenschützende Verbindungen und Mittel, welche spezielle Verbindungen als Safener zur Reduktion phytotoxischer Wirkungen von Agrochemikalien, insbesondere von Herbiziden, enthalten. Insbesondere betrifft die Erfindung Pyridonderivate als Safener und Verfahren zu ihrer Herstellung.

Bei der Bekämpfung unerwünschter Organismen in land- und forstwirtschaftlichen Nutzpflanzenkulturen mit Pestiziden werden häufig auch die Nutzpflanzen durch die verwendeten Pestizide mehr oder weniger stark geschädigt. Dieser unerwünschte phytotoxische Nebeneffekt tritt in besonderem Maße bei der Verwendung von zahlreichen Herbiziden - und dort in erster Linie bei der Applikation im Nachauflauf - in Nutzpflanzenkulturen wie beispielsweise Mais, Reis oder Getreide auf. Durch den Einsatz sogenannter "Safener" oder "Antidots" können in manchen Fällen die Nutzpflanzen gegen die phytotoxischen Eigenschaften der Pestizide geschützt werden, ohne daß die pestizide Wirkung gegenüber den Schadorganismen geschmälert oder wesentlich beeinträchtigt wird. In manchen Fällen ist sogar eine verbesserte pestizide Wirkung gegen Schadorganismen wie Unkräutern beobachtet worden.

Die bislang als Safener bekannt gewordenen Verbindungen gehören zu einer großen Zahl unterschiedlicher chemischer Strukturklassen, wobei deren Eignung für die Safeneranwendung in der Regel auch von den chemischen Strukturen der Pestizide und von den Nutzpflanzenkulturen abhängig ist.

Lange bekannt sind Safenerwirkungen von Verbindungen aus der Gruppe der Derivate von Phenoxy- oder Heteroaryloxyalkancarbonsäuren, wenn diese Verbindungen in Kombination mit Herbiziden angewandt werden. Beispiele für solche Verbindungen sind MCPA und ähnliche Verbindungen, welche zugleich gegen Schadpflanzen noch herbizid wirksam sind, oder Cloquintocet-mexyl. Weiterhin bekannt sind Safener aus der Gruppe der Derivate von N-phenylsubstituierten Heteroaromat-carbonsäureestem mit mehreren Heteroatomen im Heterocyclus. Beispiele für solche Safener sind die in Handelsprodukten verwendeten Safener Mefenpyr-diethyl und Isoxadifen-ethyl.

Aus WO 2004/084631 ist die Verwendung von hydroxy-substituierten aromatischen Carbonsäurederivaten bekannt. In WO 2005/015994 sind speziell Derivate der Salizylsäure als Safener beschrieben. Diese eignen sich besonders für die Anwendung als Safener in Mais- und Sojakulturen.

Weiterhin sind aus WO 2005/112630 1,2-Dihydrochinoxalin-2-on-derivate als Safener bekannt.

Wirkstoffe aus der chemischen Klasse der Pyridone mit pestiziden Eigenschaften sind aus der Literatur bekannt. Es werden unterschiedliche biologische Wirkungen beschrieben; so ist z. B. in WO 2001/014339 die fungizide Wirkung bestimmter substituierter Pyridoncarboxamide erwähnt, WO 2005/042492 und WO 2005/042493 beschreiben u.a. die fungizide Wirkung von Heterocyclylcarboxaniliden. EP-A- 544151 beschreibt die Wirkung von hydroxy-substituierten Pyridoncarboxamiden als Herbizide.

Weiterhin sind Vertreter mit pharmakologischen Eingenschaften bekannt. So werden in WO 2001/055115 Nicotinanilide als Induktoren der Apoptose sowie in US 2004/0116479 Dialkylnicotinamide als Inhibitoren der Angiogenese beschrieben.

Ferner beschreibt EP-A-522392 6-Trifluormethyl-substituierte Pyridoncarboxamide als Vorprodukte zur Synthese herbizid wirksamer Sulfonylharnstoffe. In Helv. Chim. Acta 71 (1988) 596-601 und GB 2305174 werden 1,2-Dihydro-2-oxo-6-trifluormethylpyridin-3-carboxamid, 6-Chlor(difluor)methyl-1,2-dihydro-2-oxo-pyridin-3-carboxamid sowie 6-Difluormethyl-1,2-dihydro-2-oxo-pyridin-3-carboxamid als Zwischenprodukte in der Synthese von Pyranopyridinen erwähnt. In der WO 2007/041052 wird 1,2-Dihydro-2-oxo-6-trifluormethylpyridin-3-carboxamid als Zwischenprodukt in der Synthese von pharmakologisch wirksamen Spiropiperidinen erwähnt.

Eine Verwendung derartiger Verbindungen als Safener in Kombination mit bestimmten Pestiziden ist noch nicht bekannt.

WO 2006/007981 beschreibt ein Verfahren zum Auffinden von die Pathogenabwehr von Pflanzen induzierenden Verbindungen, wobei die Steigerung der Expression pflanzenendogener Gene als Indiz für die Induktion bewertet wird.

Dabei wird 1,2-Dihydro-2-oxo-6-trifluormethylpyridin-3-carboxamid innerhalb einer Gruppe von sechs Verbindungen genannt, die als Safener bezeichnet werden. Eine durch biologische Tests an Pflanzen belegte Safenerwirkung für diese Verbindung ist bisher noch nicht bekannt gewesen und wird auch durch WO 2006/007981 nicht hinreichend offenbart.

Insbesondere die Expressionswerte, die gemäß WO 2006/007981 mit der Verbindung erhalten wurden und die teilweise auf einem deutlich niedrigeren Niveau liegen als für ebenfalls genannte kommerziell erhältliche Safener, lassen allenfalls erwarten, dass sie als Safener, wenn überhaupt, deutlich weniger geeignet sein sollten.

Bei der Anwendung von Safenern zum Schutz von Nutzpflanzen vor Schädigungen von Pestiziden hat sich gezeigt, daß die bekannten Safener in vielen Fällen Nachteile aufweisen können. Dazu zählen:
- der Safener vermindert die Wirkung der Pestizide, insbesondere die von Herbiziden, gegen die Schadpflanzen,
- die nutzpflanzenschützenden Eigenschaften sind nicht ausreichend,
- in Kombination mit einem bestimmten Herbizid ist das Spektrum der Nutzpflanzen, in denen der Safener/Herbizid-Einsatz erfolgen soll, nicht ausreichend groß,
- ein bestimmter Safener ist nur mit wenigen Herbiziden kombinierbar,
- Die Verwendung von Safenem erhöht die zu applizierende Aufwandmenge und Menge an Formulierung und kann damit anwendungstechnische Probleme verursachen.

Aus den genannten Gründen besteht ein Bedarf an der Bereitstellung alternativer Verbindungen mit Safener-Wirkung.

Gegenstand der Erfindung ist die Verwendung von Verbindungen der Formel (I), oder deren Salzen, 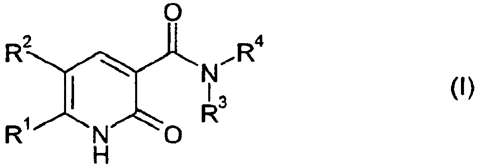 worin
- R¹: einen (C₁-C₆)Haloalkylrest, vorzugsweise einen Rest der Formel CF₃, CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃, oder C(CH₃)₂F bedeutet und
- R²: Wasserstoff oder Halogen bedeutet und
- R³: Wasserstoff, (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonykl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet und
- R⁴: (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
- R³: (C₁-C₄)-Alkoxy, (C₂-C₄)Alkenyloxy, (C₂-C₆)Alkinyloxy oder (C₂-C₄)Haloalkoxy bedeutet und
- R⁴: Wasserstoff oder (C₁-C₄)-Alkyl bedeutet oder
- R³ und R⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet oder
- R³ und R⁴: zusammen mit dem direkt gebundenen N-Atom die Gruppe -N=CR⁵-NR⁶R⁷ bedeutet, worin
R⁵ für Wasserstoff oder (C₁-C₆)Alkyl steht, wobei Wasserstoff bevorzugt ist, und
R⁶, R⁷ unabhängig voneinander für Wasserstoff oder (C₁-C₄)Alkyl, vorzugsweise (C₁-C₂)Alkyl stehen oder R⁶ und R⁷ zusammen mit dem direkt gebundenen N-Atom einen fünf- bis siebengliedrigen, vorzugsweise gesättigten heterocyclischen Ring, wie beispielsweise Piperidinyl, Pyrrolidinyl oder Morpholinyl bilden, oder
- R¹: einen (C₁-C₆)Haloalkylrest, vorzugsweise einen Rest der Formel CF₃, CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃ oder C(CH₃)₂F, weiter bevorzugt einen Rest der Formel CF₃, CF₂Cl, CF₂H, CF₂CF₃, CF₂CF₂H, CF₂CF₂Cl, CF₂CF₂CF₃ oder C(CH₃)₂F, insbesondere CF₂Cl, CF₂H, CF₂CF₃, CF₂CF₂H, CF₂CF₂Cl, CF₂CF₂CF₃ oder C(CH₃)₂F, noch weiter bevorzugt CF₂Cl, CF₂H, CF₂CF₃, CF₂CF₂H, CF₂CF₂Cl oder CF₂CF₂CF₃ bedeutet,
- R²: Halogen bedeutet,
- R³: Wasserstoff bedeutet und
- R⁴: Wasserstoff bedeutet oder
- R¹: einen Rest der Formel CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃ oder C(CH₃)₂F, weiter bevorzugt CF₂Cl, CF₂H, CF₂CF₃, CF₂CF₂H, CF₂CF₂Cl, CF₂CF₂CF₃ oder C(CH₃)₂F, noch weiter bevorzugt CF₂Cl, CF₂H, CF₂CF₃, CF₂CF₂H, CF₂CF₂Cl oder CF₂CF₂CF₃ bedeutet,
- R²: Wasserstoff bedeutet,
- R³: Wasserstoff bedeutet und
- R⁴: Wasserstoff bedeutet,

als nutzpflanzenschützendes Mittel zum Reduzieren oder Verhindern von Schadwirkungen von Agrochemikalien, vorzugsweise Pestiziden, insbesondere Herbiziden, an den Nutzpflanzen.

Im Folgenden werden die Verbindungen der Formel (I) und ihre Salze in einigen Fällen auch kurz als erfindungsgemäß verwendete oder erfindungsgemäße "Verbindungen (I)" bezeichnet.

Die Verbindungen der Formel (I) umfassen auch Tautomere, welche durch Wasserstoffverschiebung gebildet werden können und welche strukturell formal nicht unter die Formel (I) fallen. Gleichwohl gelten diese Tautomere als von der Definition der erfindungsgemäßen Verbindungen der Formel (I) umfasst. Insbesondere umfasst sind von der Definition der Verbindungen der Formel (I) die tautomeren Strukturen der Formel (la) (2-Hydroxy-pyridin-3-carboxamide) oder deren Salze, 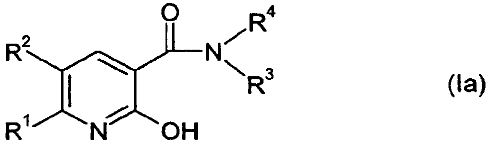 worin
R¹, R², R³ und R⁴ wie in Formel (I) definiert sind.

Einige erfindungsgemäße Verbindungen der Formel (I) oder deren Salze sind neu und ebenfalls Gegenstand der Erfindung.

Gegenstand der Erfindung sind auch nutzpflanzenschützende Mittel, welche Verbindungen der Formel (I) oder deren Salze und Formulierungshilfsmittel enthalten. Gegenstand der Erfindung sind auch nutzpflanzenschützende Mittel, welche Verbindungen der Formel (I) oder deren Salze in Kombination mit weiteren Agrochemikalien, vorzugsweise Pestiziden, insbesondere Herbiziden, und gegebenenfalls Formulierungshilfsmitteln enthalten.

Einige Verbindungen der Formel (I) sind bereits als Zwischenprodukte zur Herstellung von Wirkstoffen beschrieben; siehe die bereits erwähnte GB-A-2305174 (Verb. (I), worin R¹ = CF₃, CF₂Cl oder CF₂H und R³ = R⁴ = H). In der bereits erwähnten EP-A-522392 sind unter anderem Verbindungen (I) als Zwischenprodukte zur Herstellung von Sulfonylharnstoffen allgemein beschrieben worden. Die Safenerwirkungen der Verbindungen sind nicht beschrieben worden.

Gegenstand der Erfindung sind auch Verbindungen der Formel (I) oder deren Salze, 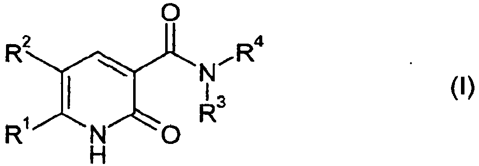 worin
- R¹: einen (C₁-C₆)Haloalkylrest, vorzugsweise einen Rest der Formel CF₃, CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃ oder C(CH₃)₂F, insbesondere einen Rest der Formel CF₃, CF₂Cl, CF₂H, CF₂CF₃, CF₂CF₂H, CF₂CF₂Cl, CF₂CF₂CF₃ oder C(CH₃)₂F, weiter bevorzugt CF₃, CF₂Cl, CF₂H, CF₂CF₃, CF₂CF₂H, CF₂CF₂Cl oder CF₂CF₂CF₃ bedeutet und
- R²: Wasserstoff oder Halogen bedeutet und
- R³: Wasserstoff, (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl und [(C₁-C₄)Haloalkoxy]-carbonyl, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet und
- R⁴: (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl und [(C₁-C₄)Haloalkoxy]-carbonyl, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
- R³: (C₁-C₄)-Alkoxy, (C₂-C₄)Alkenyloxy, (C₂-C₆)Alkinyloxy oder (C₂-C₄)Haloalkoxy bedeutet und
- R⁴: Wasserstoff oder (C₁-C₄)-Alkyl bedeutet oder
- R³ und R⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet oder
- R³ und R⁴: zusammen mit dem direkt gebundenen N-Atom die Gruppe -N=CR⁵-NR⁶R⁷ bedeutet, worin
R⁵ für Wasserstoff oder (C₁-C₆)Alkyl steht, wobei Wasserstoff bevorzugt ist, und
R⁶, R⁷ unabhängig voneinander für Wasserstoff oder (C₁-C₄)Alkyl, vorzugsweise (C₁-C₂)Alkyl stehen oder R⁶ und R⁷ zusammen mit dem direkt gebundenen N-Atom einen fünf- bis siebengliedrigen, vorzugsweise gesättigten heterocyclischen Ring, wie beispielsweise Piperidinyl, Pyrrolidinyl oder Morpholinyl bilden, oder
- R¹: einen (C₁-C₆)Haloalkylrest, vorzugsweise einen Rest der Formel CF₃, CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃ oder C(CH₃)₂F, weiter bevorzugt CF₃, CF₂Cl, CF₂H, CF₂CF₃, CF₂CF₂H, CF₂CF₂Cl, CF₂CF₂CF₃ oder C(CH₃)₂F, insbesondere CF₂Cl, CF₂H, CF₂CF₃, CF₂CF₂H, CF₂CF₂Cl, CF₂CF₂CF₃ oder C(CH₃)₂F, noch weiter bevorzugt CF₂Cl, CF₂CF₃, CF₂CF₂Cl oder CF₂CF₂CF₃ bedeutet,
- R²: Halogen bedeutet,
- R³: Wasserstoff bedeutet und
- R⁴: Wasserstoff bedeutet oder
- R¹: einen Rest der Formel CF₂CF₃, CF₂CF₂H, CF₂CF₂Cl, CFClCF₃, CFHCF₃, CF(CF₃)₂, CH(CF₃)₂, CF₂CF₂CF₃ oder C(CH₃)₂F, vorzugsweise CF₂CF₃, CF₂CF₂H, CF₂CF₂Cl, CFClCF₃, CF₂CF₂CF₃ oder C(CH₃)₂F, insbesondere CF₂CF₃, CF₂CF₂H, CF₂CF₂Cl, CFClCF₃ oder CF₂CF₂CF₃, noch weiter bevorzugt CF₂CF₃, CF₂CF₂H, CF₂CF₂Cl oder CF₂CF₂CF₃ bedeutet,
- R²: Wasserstoff bedeutet,
- R³: Wasserstoff bedeutet und
- R⁴: Wasserstoff bedeutet.

Vorzugsweise ausgenommen sind dabei Verbindungen der Formel (I) oder deren Salze, worin
- R¹: (C₁-C₃)Alkyl, das mit ein bis drei Fluoratomen substituiert ist, bedeutet,
- R²: Wasserstoff bedeutet,
- R³: (C₁-C₂)Alkyl bedeutet und
- R⁴: (C₁-C₂)Alkyl bedeutet.

Die letztgenannten vorzugsweise ausgenommenen Verbindungen sind allgemein in der genannten EP-A-0522392 als Zwischenprodukte zur Herstellung von Sulfonylharnstoffen beschrieben.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der Formel (I) umfaßt.

Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfaßt, jedoch nicht mit ihrer spezifischen Stereoform angegeben sind, und deren Gemische.

Die Kombinationsmöglichkeiten der verschiedenen Substituenten der allgemeinen Formel (I) sind so zu verstehen, daß die allgemeinen Grundsätze des Aufbaus chemischer Verbindungen zu beachten sind, d.h. die Formel (I) nicht Verbindungen umfasst, von denen der Fachmann weiß, daß sie chemisch nicht möglich sind.

Die vorstehend und weiter unten verwendeten Bezeichnungen sind dem Fachmann geläufig und haben insbesondere die im Folgenden erläuterten Bedeutungen:

Der Ausdruck "(C₁-C₄)Alkyl" bedeutet eine Kurzschreibweise für offenkettiges Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. (C₂-C₆)-Alkynyl bedeutet beispielsweise Ethinyl, Propargyl, 1-Methyl-2-propinyl, 2-Methyl-2-propinyl, 2-Butinyl, 2-Pentinyl oder 2-Hexinyl, vorzugsweise Propargyl, But-2-in-1-yl, But-3-in-1-yl oder 1-Methyl-but-3-in-1-yl.

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Cycloalkyl bedeutet ein carbocyclisches, gesättigtes Ringsystem mit vorzugsweise 3-8 C-Atomen, z.B. Cyclopropyl, Cyclobutyl, Cyclopentyl oder Cyclohexyl. Im Falle von substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Adamantan-1-yl und Adamantan-2-yl.

Cycloalkenyl bedeutet ein carbocyclisches; nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend.

Die Bezeichnung "Halogen" bedeutet beispielsweise Fluor, Chlor, Brom oder Iod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CH₂ClCH₃, CH₂FCH₃, CH₂Cl, CH₂F; Perhaloalkyl wie CCl₃ oder CF₃ oder CF₃CF₂; Polyhaloalkyl wie CHF₂, CH₂F, CH₂FCHCl, CHCl₂, CF₂CF₂H, CH₂CF₃, CH₂ClCH₃, CH₂FCH₃; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Cycloalkenyl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono-und Dialkylaminocarbonyl, substituiertes Amino, wie Acylamino, Mono- und Dialkylamino, Trialkylsilyl und gegebenenfalls substituiertes Cycloalkyl, gegebenenfalls substituiertes Phenyl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, Alkylsulfonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise

Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkyl-aminocarbonyl, N,N-Dialkylaminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie gesättigte N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Fluorphenyl, 2-, 3-und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist,

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren, Phosphinsäuren.

Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl, N-Alkyl- und N,N-Dialkylcarbamoyl und andere Reste von organischen Säuren. Dabei können die Reste jeweils im Alkyl- oder Phenylteil noch weiter substituiert sein, beispielsweise im Alkylteil durch ein oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Alkanoyl, wie Formyl und Acetyl, Aroyl wie Phenylcarbonyl, und andere Reste von gesättigten oder ungesättigten organischen Säuren.

"Aroyl" bedeutet einen wie vorstehend definierter Arylrest, der über eine CarbonylGruppe gebunden ist, z.B. die Benzoyl-Gruppe.

Wenn ein allgemeiner Rest mit "Wasserstoff" definiert ist, bedeutet dies ein Wasserstoffatom.

Mit "yl-Position" eines Restes ist dessen Bindungstelle bezeichnet.

Entsprechend den allgemeinen Definitionen bedeutet:
"(C₁-C₆)Alkyl" Methyl-, Ethyl-, Propyl-, Isopropyl-, 1-Butyl-, 2-Butyl-, 2-Methylpropyl-oder tert.-Butylrest;
"(C₁-C₁₀)Alkyl" umfaßt demnach die vorgenannten Alkylreste, sowie isomere Pentylreste, wie n-Pentyl, 1,1-Dimethylpropyl- oder 2-Methylbutyl-, isomere Hexyl-, Heptyl-, Octyl-, Nonyl- oder Decyl-Reste.
"(C₂-C₄)-Alkenyl" steht demnach z.B. für die Vinyl-, Allyl-, 2-Methyl-2-propen-1-yl-, 2-oder 3-Buten-1-yl-,
"(C₃-C₁₀)-Alkenyl" steht demnach z.B. für die Allyl-, 2-Methyl-2-propen-1-yl-, 2- oder 3-Buten-1-yl-, Pentenyl-, 2-Methylpentenyl-, Hexenyl-, Heptenyl-, Octenyl-, Nonenyl-oder Decenyl-Gruppe.
"(C₂-C₄)-Alkinyl" steht z.B. für die Ethinyl-, Propargyl oder 2-Butin-1-yl-Gruppe, "(C₃-C₁₀)-Alkinyl" steht z.B. für die Propargyl-, 2-Butin-1-yl-,
2-Pentin-1-yl-, 2-Methylpentin-3-yl-, Hexinyl-, Heptinyl-, Octinyl- Noninyl- oder die Decinyl- Gruppe.

Ist die Kohlenstoffkette eines Alkylrests mehrfach durch Sauerstoffatome unterbrochen, so bedeutet dies, daß zwei Sauerstoffatome nicht direkt benachbart sein sollen.

"(C₃-C₆)-Cycloalkyl" steht für den Cyclopropyl-, Cyclobutyl-, Cyclopentyl- oder Cyclohexyl-Rest,
"(C₃-C₁₀)-Cycloalkyl" steht für monocyclische Alkylreste, wie den Cyclopropyl-, Cyclobutyl-, Cyclopentyl-, Cyclohexyl-, Cycloheptyl-, Cyclooctyl- oder Cyclodecyl-Rest, für bicyclische Alkylreste, wie den Norbornyl- oder Bicyclo[2.2.2]octyl-Rest, oder für kondensierte Systeme, wie den Decahydronaphthyl-Rest. "(C₄-C₁₀)-Cycloalkenyl" steht für monocyclische Cycloalkylenreste, wie den Cyclobutenyl-, Cyclopentenyl-, Cyclohexenyl-, Cycloheptenyl- Cyclooctenyl- oder Cyclodecenyl-Rest, für bicyclische Alkylreste, wie den Norbornenyl- oder Bicyclo[2,2,2]octenyl-Rest, oder für kondensierte Systeme, wie den Tetra-, Hexa- oder Octahydronaphthyl-Rest.

"(C₁-C₄)-Alkoxy" und "(C₁-C₁₀)-Alkoxy" sind Alkoxygruppen, deren Kohlenwasserstoffreste die unter den Ausdrücken "(C₁-C₄)-Alkyl" und "(C₁-C₁₀)-Alkyl" angegebenen Bedeutungen haben.

Vor allem aus den Gründen der höheren nutzpflanzenschützenden oder kulturpflanzenschützenden Wirkung (Safenerwirkung), besseren Selektivität und/oder besseren Herstellbarkeit sind erfindungsgemäße Verwendungen von Verbindungen der genannten Formel (I) oder deren Salzen von besonderem Interesse, worin einzelne Reste eine der bereits genannten oder im folgenden genannten bevorzugten Bedeutungen haben, oder insbesondere solche, worin eine oder mehrere der bereits genannten oder im folgenden genannten bevorzugten Bedeutungen kombiniert auftreten.

Vorzugsweise bedeutet, sofern im Rahmen der obengenannten allgemeinen Definition von Formel (I) umfasst,
- R¹: einen (C₁-C₄)Haloalkylrest, weiter bevorzugt CF₃, CF₂Cl, CF₂H, CF₂CF₃, CF₂CF₂H, CF₂CF₂Cl, C(CH₃)₂F oder CF₂CF₂CF₃, weiter bevorzugt CF₃, CF₂Cl, CF₂H, CF₂CF₂CF₃ oder CF₂CF₃, weiter bevorzugt CF₃, CF₂Cl, CF₂CF₂CF₃ oder CF₂CF₃, insbesondere CF₃, CF₂Cl oder CF₂CF₃.

Vorzugsweise bedeutet
- R²: Wasserstoff oder Halogen. Halogen ist dabei vorzugsweise Fluor, Chlor, Brom oder Iod, insbesondere Chlor, Brom oder Iod, ganz besonders Chlor oder Brom.

Vorzugsweise bedeuten
- R³: Wasserstoff, (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, vorzugsweise unsubstituiert oder durch (C₁-C₄)Alkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, vorzugsweise unsubstituiert oder durch einen oder mehrere Reste aus der Gruppe (C₁-C₄)Alkyl, (C₁-C₄)Haloalkyl und (C₁-C₄)Alkoxy substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, vorzugsweise unsubstituiert oder durch (C₁-C₄)Alkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, vorzugsweise unsubstituiert oder durch einen oder mehrere Reste aus der Gruppe (C₁-C₄)Alkyl, (C₁-C₄)Haloalkyl und (C₁-C₄)Alkoxy substituiert ist, substituiert ist, weiter bevorzugt Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist, weiter bevorzugt Wasserstoff oder (C₁-C₄)Alkyl, insbesondere Wasserstoff,
und
- R⁴: (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, vorzugsweise unsubstituiert oder durch (C₁-C₄)Alkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, vorzugsweise unsubstituiert oder durch einen oder mehrere Reste aus der Gruppe (C₁-C₄)Alkyl, (C₁-C₄)Haloalkyl und (C₁-C₄)Alkoxy substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C4)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, vorzugsweise unsubstituiert oder durch (C₁-C₄)Alkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, vorzugsweise unsubstituiert oder durch einen oder mehrere Reste aus der Gruppe (C₁-C₄)Alkyl, (C₁-C₄)Haloalkyl und (C₁-C₄)Alkoxy substituiert ist, substituiert ist.

Dabei bedeutet Heterocyclyl vorzugsweise einen heterocyclischen 3- bis 9-gliedrigen, insbesondere 5- oder 6-gliedrigen Ring mit 1 bis 3 Heteroringatomen aus der Gruppe N, O und S.

Weiter bevorzugt bedeuten
- R³: Wasserstoff, (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C4)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, vorzugsweise unsubstituiert oder durch (C₁-C₄)Alkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy; (C₁-C₄)Alkylthio und Oxo substituiert ist, substituiert ist, oder (C₃-C₆)Cycloalkyl oder (C₃-C₆)Cycloalkyl, das an einer Seite des Rings mit einem 4 bis 6-gliedrigen gesättigten oder ungesättigten carbocyclischen Ring kondensiert ist, wobei jeder der letztgenannten 2 Reste unsubstituiert oder durch einen oder mehrere Reste aus der Gruppe (C₁-C₄)Alkyl substituiert ist, weiter bevorzugt Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist, weiter bevorzugt Wasserstoff oder (C₁-C₄)Alkyl, insbesondere Wasserstoff, und R⁴ wie oben bereits für R⁴ definiert oder vorzugsweise (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C4)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, vorzugsweise unsubstituiert oder durch (C₁-C₄)Alkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, oder (C₃-C₆)Cycloalkyl oder (C₃-C₆)Cycloalkyl, das an einer Seite des Rings mit einem 4 bis 6-gliedrigen gesättigten oder ungesättigten carbocyclischen Ring kondensiert ist, wobei jeder der letztgenannten 2 Reste unsubstituiert oder durch einen oder mehrere Reste aus der Gruppe (C₁-C₄)Alkyl substituiert ist.

Weiter bevorzugt bedeuten
- R³: Wasserstoff, (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und [(C₁-C₄)Alkoxy]-carbonyl, substituiert ist, vorzugsweise Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist, weiter bevorzugt Wasserstoff oder (C₁-C₄)Alkyl, insbesondere Wasserstoff, und
- R⁴: wie oben bereits für R⁴ definiert oder vorzugsweise (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und [(C₁-C₄)Alkoxy]-carbonyl, substituiert ist, vorzugsweise (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist.

Besonders bevorzugt ist die erfindungsgemäße Verwendung mit Verbindungen der Formel (I) oder deren Salzen, worin
- R¹: CF₃, CF₂Cl, CF₂H, CF₂CF₂CF₃ oder CF₂CF₃, vorzugsweise- CF₃, CF₂Cl, CF₂CF₂CF₃ oder CF₂CF₃, insbesondere CF₃, CF₂Cl oder CF₂CF₃ und
- R²: Wasserstoff oder Halogen, vorzugsweise Wasserstoff, und
- R³: Wasserstoff, (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist, vorzugsweise Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist, insbesondere Wasserstoff oder (C₁-C₄)Alkyl, und
- R⁴: (C₁-C₁₀)Alkyl, (C₃-C₁₀)Alkenyl oder (C₃-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C4)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, vorzugsweise unsubstituiert oder durch (C₁-C₄)Alkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, oder (C₃-C₆)Cycloalkyl oder (C₃-C₆)Cycloalkyl, das an einer Seite des Rings mit einem 4 bis 6-gliedrigen gesättigten oder ungesättigten carbocyclischen Ring kondensiert ist, wobei jeder der letztgenannten 2 Reste unsubstituiert oder durch einen oder mehrere Reste aus der Gruppe (C₁-C₄)Alkyl substituiert ist, vorzugsweise (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist, weiter bevorzugt (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und (C₁-C₄)Alkoxycarbonyl substituiert ist,
oder
- R³ und R⁴: zusammmen mit dem direkt gebundenen N-Atom einen vier- bis achtgliedrigen heterocyclischen Ring, vorzugsweise 5- oder 6-gliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist,
oder
- R³ und R⁴: zusammen mit dem direkt gebundenen N-Atom die Gruppe -N=CR⁵-NR⁶R⁷, worin
R⁵ für Wasserstoff oder (C₁-C₄)Alkyl steht, wobei Wasserstoff bevorzugt ist, und
R⁶, R⁷ unabhängig voneinander für Wasserstoff oder (C₁-C₄)Alkyl, vorzugsweise für (C₁-C₂)Alkyl stehen oder R⁶ und R⁷ zusammen mit dem direkt gebundenen N-Atom einen fünf- oder sechsgliedrigen, vorzugsweise gesättigten heterocyclischen Ring, wie beispielsweise Piperidinyl, Pyrrolidinyl oder Morpholinyl bilden,
bedeuten:

Besonders bevorzugt ist auch die erfindungsgemäße Verwendung mit Verbindungen der Formel (I) oder deren Salzen, worin
- R¹: einen (C₁-C₆)Haloalkylrest, vorzugsweise aus der Gruppe CF₃, CF₂Cl, CF₂H, CF₂CF₃, CF₂CF₂H, CF₂CF₂Cl, CF₂CF₂CF₃ und C(CH₃)₂F, weiter bevorzugt CF₃, CF₂Cl, CF₂H, CF₂CF₂CF₃ oder CF₂CF₃, noch weiter bevorzugt CF₃, CF₂Cl, CF₂CF₂CF₃ oder CF₂CF₃, insbesondere CF₃, CF₂CF₃ oder CF₂Cl bedeutet und
- R²: Halogen bedeutet und
- R³: Wasserstoff bedeutet und
- R⁴: Wasserstoff bedeutet.

Ebenfalls besonders bevorzugt ist auch die erfindungsgemäße Verwendung mit Verbindungen der Formel (I) oder deren Salzen, worin
- R¹: CF₂Cl, CF₂H, CF₂CF₃, CF₂CF₂H oder CF₂CF₂Cl, weiter bevorzugt CF₂Cl oder CF₂H oder CF₂CF₃, weiter bevorzugt CF₂Cl oder CF₂CF₃, insbesondere CF₂Cl bedeutet
- R²: Wasserstoff bedeutet und
- R³: Wasserstoff oder (C₁-C₄)Alkyl bedeutet und
- R⁴: Wasserstoff bedeutet.

Besonders bevorzugt ist auch die erfindungsgemäße Verwendung mit Verbindungen der Formel (I) oder deren Salzen, worin die allgemeinen Reste den in den Tabellenbeispielen genannten Resten für R¹, R², R³ bzw. R⁴ entsprechen bzw. diese umfassen.

Besonders bevorzugt ist auch die erfindungsgemäße Verwendung mit neuen Verbindungen der Formel (I) oder deren Salzen, worin R¹, R², R³ bzw. R⁴ im übrigen vorzugsweise die zu den bevorzugten Verwendungen genannten Bedeutungen haben.

Die Verbindungen der allgemeinen Formel (I) können hergestellt werden, indem man beispielsweise
(a) eine Carbonsäure der allgemeinen Formel (II) 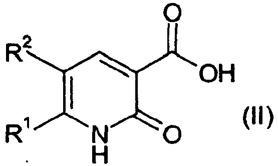 worin R¹ und R² wie in der herzustellenden Verbindung der Formel (I) definiert sind, mit einem Amin der Formel (III) oder dessen Salz, 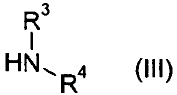 worin R³ und R⁴ wie in der herzustellenden Verbindung der Formel (I) definiert sind, gegebenenfalls in Gegenwart eines carbonsäureaktivierenden Reagenzes, beispielsweise N,N-Carbonyldiimidazol (CDI), oder eines wasserentziehenden Mittels, beispielsweise Dicyclohexylcarbodiimid (DCC), zur Verbindung der Formel (I) umsetzt oder
(b) einen Carbonsäureester der allgemeinen Formel (IV) 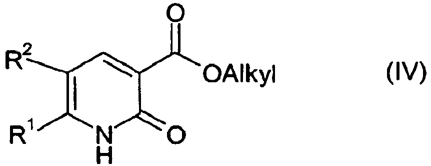 worin R¹ und R² wie in der herzustellenden Verbindung der Formel (I) definiert sind und "Alkyl" einen Alkylrest, beispielsweise Methyl oder Ethyl, bedeutet, mit einem Amin der Formel (III) oder dessen Salz, 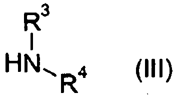 worin R³ und R⁴ wie in der herzustellenden Verbindung der Formel (I) definiert sind, zur Verbindung der Formel (I) umsetzt oder
(c) ein Carbonsäurehalogenid oder -anhydrid der allgemeinen Formel (V), 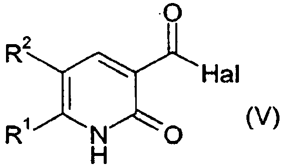 worin R¹ und R² wie in der herzustellenden Verbindung der Formel (I) definiert sind und Hal ein Halogenatom, beispielsweise Chlor, oder einen Acyloxyrest bedeutet, mit einem Amin der Formel (III) oder dessen Salz, 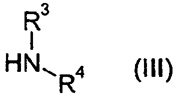 worin R³ und R⁴ wie in der herzustellenden Verbindung der Formel (I) definiert sind, zur Verbindung der Formel (I) umsetzt,
(d), im Falle, dass R³ und R⁴ in der herzustellenden Verbindung der Formel (I) jeweils Wasserstoff bedeutet,
   eine Verbindung der Formel (VI), 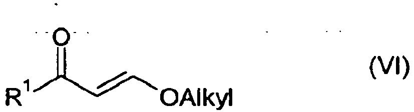 worin R¹ wie in der herzustellenden Verbindung der Formel (I) definiert ist und "Alkyl" einen Alkylrest, beispielsweise Methyl oder Ethyl, bedeutet, mit Malonsäurediamid zur Verbindung der Formel (I) umsetzt.

Die Amidbildungen nach Variante (a) können beispielsweise in einem inerten organischen Lösungsmittel in einem Temperaturbereich zwischen 0 °C und 150 °C, vorzugsweise 0 °C und 50 °C durchgeführt werden. Als organische Lösungsmittel eignen sich beispielsweise polare protische oder aprotische Lösungsmittel wie Ether, z. B. Diethylether, Tetrahydrofuran und Dioxan, oder Nitrile wie Acetonitril, oder Amide wie Dimethylformamid.

Die Amidbildungen nach Variante (b) können beispielsweise in einem inerten organischen Lösungsmittel in einem Temperaturbereich zwischen 0 °C und 150 °C, vorzugsweise 50 °C und 100 °C durchgeführt werden. Als organische Lösungsmittel eignen sich beispielsweise polare protische oder aprotische Lösungsmittel wie Ether, z. B. Tetrahydrofuran und Dioxan, oder Nitrile wie Acetonitril, oder Amide wie Dimethylformamid. Bevorzugt ist jedoch die Amidbildung nach Variante (b) bei erhöhten Temperaturen durch Umsetzung der Reaktionspartner in Substanz.

Die Amidbildungen nach Variante (c) können beispielsweise in Gegenwart eines säurebindenden Mittels in einem inerten organischen Lösungsmittel in einem Temperaturbereich zwischen 0 °C und 150 °C, vorzugsweise 0 °C und 50 °C durchgeführt werden. Als organische Lösungsmittel eignen sich beispielsweise polare protische oder aprotische Lösungsmittel wie Ether, z. B. Diethylether, Tetrahydrofuran und Dioxan, oder Nitrile wie Acetonitril, oder Amide wie Dimethylformamid. Säurebindende Mittel sind beispielsweise Alkali- oder Erdalkalimetall-carbonate wie z. B. Natrium-, Kalium- oder Calcium-carbonat, Alkalioder Erdalkalimetall-hydroxide, wie Natrium-, Kalium oder Calcium-hydroxid, oder Alkalimetall-hydride oder-amide, wie Natrium- oder Kalium-hydrid oder-amid, oder auch organische Basen, wie Triethylamin, Pyridin, Dimethylaminopyridin, DBU (1,8-Diazabicyclo[5.4.0]-undec-7-en), DBN (1,5-Diazabicyclo[4.3.0]non-5-en) und 1,4-Diaza-bicyclo[2.2.2]octan.

Die Amidbildungen nach Variante (d) können analog den Verfahren durchgeführt werden, wie sie in EP 522392 und Helv. Chim. Acta 71 (1988) 596-601 und GB 2305174 beschrieben sind. Das Malonsäurediamid kann in der Regel in einem organischen wasserfreien polaren protischen oder aprotischen Lösungsmittel, beispielsweise in einem Alkohol, mit einer starken Base wie einem Alkalimetall, Alkalimetallhydrid oder Alkalimetallalkoholat in ein reaktives Salz überführt und dann mit der Verbindung der Formel (VI) umgesetzt werden. Die Umsetzung mit der Verbindung (VI) kann in der Regel in einem Temperaturbereich zwischen 0 °C und dem Siedepunkt des Lösungsmittels (je nach Lösungsmittel etwa bis 150 °C) durchgeführt werden.

Die Verbindungen der allgemeinen Formeln (II), (III), (IV) und (V) sind entweder kommerziell erhältlich oder können nach oder analog dem Fachmann bekannten Methoden hergestellt werden (z. B. Helv. Chim. Acta 71 (1988) 596; EP 502740; EP 522392).

So sind beispielsweise die Verbindungen der Formel (IVa) 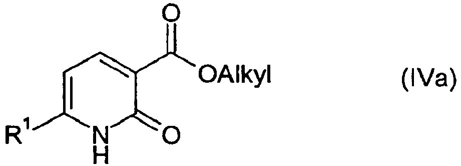 durch Umsetzung von Alkoxyvinylethem der Formel (VI) mit Malonsäurealkylesteramiden der Formel (VII) zugänglich.

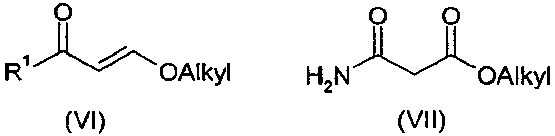

Die Edukte der Formel (VI) sind entweder kommerziell erhältlich oder können nach bekannten Methoden hergestellt werden (z. B. Synthesis 2000, 738-742; J. Fluor. Chem., 107, 2001, 285-300; Organometallics 15, 1996, 5374-5379).

Die Verbindungen der Formel (IV), worin R² ein Halogenatom bedeutet, lassen sich durch übliche Halogenierungen aus den Verbindungen der Formel (IVa) herstellen. Als Halogenierungsmittel für Pyridine finden beispielsweise Chlor (J. Org. Chem. 23, 1958, 1614), Brom (Synth. Commun. 19, 1989, 553-560; US P 2532055), lod (Tetrahedron Lett. 45, 2004, 6633-6636), Natriumhypochlorit (J. Org. Chem. 49, 1984, 4784-4786; J. Med. Chem. 36, 1993, 2676-2688, US P 4960896), Natriumhypobromit (J. Med. Chem. 32, 1989, 2178-2199), Thionylchlorid (Organic Letters, 6, 2004, 3-5), N-Chlorsuccinimid (J. Med. Chem. 46, 2003, 702-715), N-Bromsuccinimid (Chem. Pharm. Bull. 48, 2000, 1847-1853), N-Iodsuccinimid (J. Med. Chem. 36, 1993, 2676-2788) Verwendung.

Ferner können die Verbindungen der Formel (IV) aus den Verbindungen der Formel (IVa) sukzessive durch Nitrierung (z. B. J. Med. Chem. 36, 1993, 2676-2688; J. Heterocycl. Chem. 33, 1996, 287-294), Reduktion (z. B. J. Med. Chem. 33, 1990, 1859-1865), Diazotierung und anschließender Umsetzung der Diazoniumsalze mittels Sandmeyer- bzw. Schiemannn-Reaktion hergestellt werden.

Diejenigen Verbindungen der Formel (I), in denen R³ und R⁴ zusammen mit dem direkt gebundenen N-Atom die Gruppe -N=CR⁵-NR⁶R⁷ bedeuten, sind herstellbar, indem man eine Verbindung der Formel (I), worin R³ und R⁴ Wasserstoff bedeuten, mit Verbindungen der Formel (VIII), in denen R⁵, R⁶ und R⁷ die oben genannte Bedeutung haben, 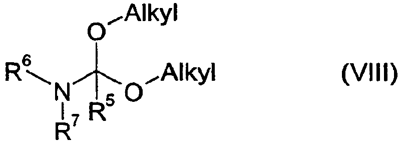 nach bekannten Methoden umsetzt (siehe z. B. Synthesis 1980, 119-121; J. Med. Chem., 33, 1990, 2052-2059).

Gegenstand der Erfindung ist auch das Verfahren zum Schützen von Kultur- oder Nutzpflanzen vor phytotoxischen Wirkungen von Agrochemikalien, wie Pestiziden, oder insbesondere Herbiziden, welche Schäden an den Kultur- oder Nutzpflanzen verursachen, dadurch gekennzeichnet, dass man Verbindungen der Formel (I) oder deren Salze als Safener anwendet, vorzugsweise eine effektive Menge der Verbindungen der Formel (I) oder deren Salzen auf die Pflanzen, Teile der Pflanzen oder deren Samen (oder Saatgut) appliziert.

Die Verbindungen (I) (= Safener) sind geeignet, zusammen mit Wirkstoffen (Pestiziden) zur selektiven Bekämpfung von Schadorganismen in einer Reihe von Pflanzenkulturen eingesetzt zu werden, beispielsweise in wirtschaftlich bedeutenden Kulturen wie Getreide (Weizen, Gerste, Triticale, Roggen, Reis, Mais, Hirse), Zuckerrübe, Zuckerrohr, Raps, Baumwolle, Sonnenblume, Erbsen, Bohnen und Soja. und Soja. Von besonderem Interesse ist dabei die Anwendung in monokotylen Kulturen wie Getreide (Weizen, Gerste, Roggen, Triticale, Sorghum), inklusive Mais und Reis, und monokotylen Gemüsekulturen, aber auch in dikotylen Kulturen wie beispielsweise Soja, Raps, Baumwolle, Wein, Gemüsepflanzen, Obstpflanzen und Zierpflanzen. Die Herbizid-Safener-Kombinationen mit Safenem (I) sind auch geeignet zur Bekämpfung von Schadpflanzen auf Beeten und Flächen von Nutz- und Zierpflanzen, wie beispielsweise Rasenflächen mit Nutz- oder Zierrasen, speziell Weidelgras, Rispengras oder Bermudagras.

Bei den Nutz- und Kulturpflanzen, in denen die Herbizid-Safener-Kombinationen mit Safenern (I) verwendet werden können, sind auch gegenüber einigen Pestiziden ganz oder partiell tolerante Mutantenkulturen oder ganz oder partiell tolerante transgene Kulturen von Interesse, z. B. Maiskulturen, die gegenüber Glufosinate oder Glyphosate resistent sind, oder Sojakulturen, die gegen herbizide Imidazolinone resistent sind. Der besondere Vorteil der neuartig eingesetzten Safener ist jedoch ihre effektive Wirkung in Kulturen, welche normalerweise nicht ausreichend tolerant gegenüber den zur Anwendung zu bringenden Pestiziden sind.

Die erfindungsgemäßen Verbindungen der Formel (I) können zur gemeinsamen Anwendung mit Pestiziden gleichzeitig oder in beliebiger Reihenfolge mit den Wirkstoffen ausgebracht werden und sind dann in der Lage, schädliche Nebenwirkungen dieser Wirkstoffe bei Kulturpflanzen zu reduzieren oder völlig aufzuheben, ohne die Wirksamkeit dieser Wirkstoffe gegen unerwünschte Schadorganismen zu beeinträchtigen oder wesentlich zu reduzieren. Dabei können auch Schädigungen, welche durch die Anwendung mehrerer Pestizide entstehen, z.B. durch mehrere Herbizide oder durch Herbizide in Kombination mit Insektiziden oder Fungiziden, wesentlich reduziert oder völlig aufgehoben werden. Hierdurch kann das Einsatzgebiet herkömmlicher Pestizide ganz erheblich erweitert werden.

Für den Fall, daß die erfindungsgemäßen Mittel Pestizide enthalten, werden diese Mittel nach entsprechender Verdünnung entweder direkt auf die Anbaufläche, auf die bereits gekeimten Schad- und/oder Nutzpflanzen oder auf die bereits aufgelaufenen Schad- und/oder Nutzpflanzen appliziert. Für den Fall, daß die erfindungsgemäßen Mittel kein Pestizid enthalten, können diese Mittel im sogenannten Tankmix-Verfahren - d.h. unmittelbar vor dem Aufbringen auf die zu behandelnde Fläche erfolgt beim Anwender die Vermischung und Verdünnung der separat formulierten Produkte (= nutzpflanzenschützendes Mittel und Pestizid) - oder zeitlich vor der Anwendung eines Pestizids, oder zeitlich nach der Anwendung eines Pestizids, oder zur Saatgut-Vorbehandlung, d.h. beispielsweise zur Beizung des Nutzpflanzensaatguts verwendet werden. Bevorzugt ist die zeitnahe Anwendung des Safeners mit dem Pestizid, insbesondere, wenn der Safener nach dem Herbizid auf die Pflanzen appliziert wird.

Die vorteilhaften Wirkungen der erfindungsgemäßen Verbindungen (I) werden beobachtet, wenn man sie zusammen mit den Pestiziden im Vorauflauf oder im Nachauflauf einsetzt, beispielsweise bei gleichzeitiger Applikation als Tank-mix oder als Co-formulierung oder bei einer separaten Applikation parallel oder nacheinander (Split-Applikation). Auch ist es möglich die Applikation mehrfach zu wiederholen. Manchmal kann es sinnvoll sein, eine Vorauflaufapplikation mit einer Nachauflaufapplikation zu kombinieren. Meist bietet sich die Anwendung als Nachauflaufapplikation auf die Nutz- oder Kulturpflanze mit gleichzeitiger oder späterer Applikation des Pestizids an. In Frage kommt auch die Anwendung der erfindungsgemäßen Verbindungen (I) bei der Saatgutbeizung, der (Tauch-)Behandlung von Keimpflanzen (z. B. Reis) oder Behandlung von anderem Vermehrungsgut (z. B. Kartoffelknollen).

Oftmals werden bei der Anwendung der erfindungsgemäßen Verbindungen (I) in Kombination mit Herbiziden neben der Safenerwirkung auch Wirkungsverstärkungen in der Herbizidwirkung gegenüber Schadpflanzen beobachtet. Weiterhin ist das Wachstum der Nutz- und Kulturpflanzen in vielen Fällen verbessert, und es können die Ernteerträge erhöht werden.

Die erfindungsgemäßen Mittel können ein oder mehrere Pestizide enthalten. Als Pestizide kommen beispielsweise Herbizide, Insektizide, Fungizide, Akarizide und Nematizide, welche jeweils bei alleiniger Anwendung phytotoxische Schäden an den Kulturpflanzen ergeben würden oder bei denen eine Schädigung wahrscheinlich wäre, in Frage. Von besonderem Interesse sind entsprechende pestizide Wirkstoffe aus den Gruppen der Herbizide, Insektizide, Akarizide, Nematizide und Fungizide, insbesondere Herbizide.

Das Gewichtsverhältnis Safener zu Pestizid kann innerhalb weiter Grenzen variiert werden und liegt in der Regel im Bereich von 1:100 bis 100:1, vorzugsweise 1:20 bis 20:1, insbesondere 1:10 bis 10:1. Das optimale Gewichtsverhältnis Safener zu Pestizid hängt in der Regel sowohl von dem jeweils eingesetzten Safener und dem jeweiligen Pestizid als auch von der Art der zu schützenden Nutz- oder Kulturpflanze ab. Die erforderliche Aufwandmenge an Safener kann je nach verwendetem Pestizid und Art der zu schützenden Nutzpflanze innerhalb weiter Grenzen variiert werden und liegt in der Regel im Bereich von 0,001 bis 10 kg, vorzugsweise 0,01 bis 1 kg, insbesondere 0,05 bis 0,5 kg Safener je Hektar. Die für eine erfolgreiche Behandlung notwendigen Mengen und Gewichtsverhältnisse können durch einfache Vorversuche ermittelt werden.

Im Falle einer Saatbeizung werden beispielsweise 0,005 bis 20 g Safener pro Kilogramm Saatgut, vorzugsweise 0,01 bis 10 g Safener pro Kilogramm Saatgut, insbesondere 0,05 bis 5 g Safener pro Kilogramm Saatgut eingesetzt.

Wenn Lösungen von Safener in der Saatbehandlung benutzt werden und das Saatgut oder Keimlinge mit den Lösungen benetzt werden, so liegt die geeignete Konzentration in der Regel im Bereich von 1 bis 10000 ppm, vorzugsweise 100 bis 1000 ppm bezogen auf das Gewicht. Die für eine erfolgreiche Behandlung notwendigen Mengen und Gewichtsverhältnisse können durch einfache Vorversuche ermittelt werden.

Die Safener können in üblicher Weise separat oder zusammen mit den Pestiziden formuliert werden. Gegenstand sind daher auch die nutzpflanzen- oder kulturpflanzenschützenden Mittel.

Bevorzugt ist die gemeinsame Anwendung von Safener und Pestizid, insbesondere die von Safener und Herbizid als Fertigformulierung oder die Anwendung im Tankmix-Verfahren.

Ebenso bevorzugt ist die Anwendung des Safeners (I) in der Saatgutbehandlung mit späterer Applikation von Pestiziden, vorzugsweise Herbiziden, nach der Aussaat im Vor- oder Nachauflaufverfahren.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenem, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Wirkstoffe in Mischungsformulierungen oder im Tank-Mix sind beispielsweise bekannte, vorzugsweise herbizide Wirkstoffe, die auf einer Inhibition von beispielsweise Acetolactat-Synthase, Acetyl-Coenzym-A-Carboxylase, PS I, PS II, HPPDO, Phytoene-Desaturase, Protoporphyrinogen-Oxidase, Glutamine-Synthetase, Cellulosebiosynthese, 5-Enolpyruvylshikimat-3-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder in dem Handbuch "The Pesticide Manual", 12. Auflage 2000, oder 13. Auflage 2003 oder 14. Auflage 2006/2007, oder in dem entsprechenden "e-Pesticide Manual", Version 4 (2006), jeweils herausgegeben vom British Crop Protection Council, (im Folgenden auch kurz "PM"), und dort zitierter Literatur beschrieben. Listen von "Common names" sind auch in "The Compendium of Pesticide Common Names" im Internet verfügbar. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acibenzolar-S-methyl; acifluorfen(-sodium); aclonifen; AD-67; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim(-sodium); ametryn; amicarbazone, amidochlor, amidosulfuron; aminopyralid; amitrol; AMS, d.h. Ammoniumsulfamat; ancimidol; anilofos; asulam; atrazin; aviglycine; azafenidin, azimsulfuron (DPX-A8947); aziprotryn; barban; BAS 516 H, d.h. 5-Fluor-2-phenyl-4H-3,1-benzoxazin-4-on; beflubutamid (UBH-509), benazolin(-ethyl); bencarbazone; benfluralin; benfuresate; benoxacor; bensulfuron(-methyl); bensulide; bentazone; benzfendizone; benzobicyclon, benzofenap; benzofluor; benzoylprop(-ethyl); benzthiazuron; bialaphos; bifenox; bispyribac(-sodium) (KIH-2023); borax; bromacil; bromobutide; bromofenoxim; bromoxynil; bromuron; buminafos; busoxinone; butachlor; butafenacil, butamifos; butenachlor (KH-218); buthidazole; butralin; butroxydim, butylate; cafenstrole (CH-900); carbetamide; carfentrazone(-ethyl); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlorbromuron; chlorbufam; chlorfenac; chlorfenprop; chlorflurecol(-methyl); chlorflurenol(-methyl); chloridazon; chlorimuron(-ethyl); chlormequat(-chloride); chlomitrofen; chlorophthalim (MK-616); chlorotoluron; chloroxuron; chlorpropham; chlorsulfuron; chlorthal-dimethyl; chlorthiamid; chlortoluron, cinidon(-methyl und -ethyl), cinmethylin; cinosulfuron; clefoxydim, clethodim; clodinafop und dessen Esterderivate (z.B. clodinafoppropargyl); clofencet; clomazone; clomeprop; cloprop; cloproxydim; clopyralid; clopyrasulfuron(-methyl), cloquintocet(-mexyl); cloransulam(-methyl), cumyluron (JC 940); cyanamide; cyanazine; cycloate; cyclosulfamuron (AC 104); cycloxydim; cycluron; cyhalofop und dessen Esterderivate (z.B. Butylester, DEH-112); cyperquat; cyprazine; cyprazole; cyprosulfamide; daimuron; 2,4-D, 2,4-DB; dalapon; daminozide; dazomet; n-decanol; desmedipham; desmetryn; di-allate; dicamba; dichlobenil; dichlormid; dichlorprop(-P)-salze; diclofop und dessen Ester wie diclofop-methyl; diclofop-P(-methyl); diclosulam, diethatyl(-ethyl); difenoxuron; difenzoquat(-metilsulfate); diflufenican; diflufenzopyr(-sodium); dimefuron; dimepiperate, dimethachlor; dimethametryn; dimethazone; dimethenamid (SAN-582H); dimethenamide-P; dimethylarsinic acid; dimethipin; dimetrasulfuron, dinitramine; dinoseb; dinoterb; diphenamid; dipropetryn; diquat-salze; dithiopyr; diuron; DNOC; eglinazine-ethyl; EL 77, d.h. 5-Cyano-1-(1,1-dimethylethyl)-N-methyl-1H-pyrazole-4-carboxamid; endothal; epoprodan, EPTC; esprocarb; ethalfluralin; ethametsulfuron-methyl; ethephon; ethidimuron; ethiozin; ethofumesate; ethoxyfen und dessen Ester (z.B. Ethylester, HN-252); ethoxysulfuron, etobenzanid (HW 52); F5231, d.h. N-[2-Chlor-4-fluor-5-[4-(3-fluorpropyl)-4,5-dihydro-5-oxo-1H-tetrazol-1-yl]-phenyl]-ethansulfonamid; fenchlorazole(-ethyl); fenclorim; fenoprop; fenoxan, fenoxaprop und fenoxaprop-P sowie deren Ester, z.B. fenoxaprop-P-ethyl und fenoxaprop-ethyl; fenoxydim; fentrazamide, fenuron; ferrous sulfate; flamprop(-methyl oder -isopropyl oder -isopropyl-L); flamprop-M(-methyl oder -isopropyl); flazasulfuron; florasulam, fluazifop und fluazifop-P und deren Ester, z.B. fluazifop-butyl und fluazifop-P-butyl; fluazolate, flucarbazone(-sodium), flucetosulfuron; fluchloralin; flufenacet; flufenpyr(-ethyl); flumetralin; flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupropanoate; flupyrsulfuron(-methyl)(-sodium); flurazole; flurenol(-butyl); fluridone; flurochloridone; fluroxypyr(-meptyl); flurprimidol, flurtamone; fluthiacet(-methyl) (KIH-9201); fluthiamide, fluxofenim; fomesafen; foramsulfuron, forchlorfenuron; fosamine; furilazole; furyloxyfen; gibberillic acid; glufosinate(-ammonium); glyphosate(-isopropylammonium); halosafen; halosulfuron(-methyl); haloxyfop und dessen Ester; haloxyfop-P (= R-haloxyfop) und dessen Ester; HC-252; hexazinone; imazamethabenz(-methyl); imazamethapyr, imazamox, imazapic, imazapyr; imazaquin und Salze wie das Ammoniumsalz; imazethamethapyr; imazethapyr; imazosulfuron; inabenfide; indanofan; indol-3-acetic acid; 4-indol-3-ylbutyric acid; iodosulfuron-methyl(-sodium); ioxynil; isocarbamid; isopropalin; isoproturon; isouron; isoxaben; isoxachlortole, isoxadifen(-ethyl); isoxaflutole, isoxapyrifop; karbutilate; lactofen; lenacil; linuron; Maleinsäurehydrazid (MH), MCPA; MCPB; mecoprop(-P); mefenacet; mefenpyr(-diethyl); mefluidid; mepiquat(-chloride); mesosulfuron(-methyl); mesotrione, metam; metamifop; metamitron; metazachlor; methabenzthiazuron; metham; methazole; methoxyphenone; methylarsonic acid; methyl-cyclopropene; methyldymron; methylisothiocyanate; methabenzthiazuron; metobenzuron; metobromuron; (alpha-)metolachlor; metosulam (XRD 511); metoxuron; metribuzin; metsulfuron-methyl; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclofen; nitralin; nitrofen; nitrophenolate mixture; nitrofluorfen; nonanoic acid; norflurazon; orbencarb; orthasulfamuron; oxabetrinil; oryzalin; oxadiargyl (RP-020630); oxadiazon; oxasulfuron, oxaziclomefone, oxyfluorfen; paclobutrazol; paraquat(-dichloride); pebulate; pelargonic acid, pendimethalin; penoxsulam; pentachlorophenol; pentanochlor; pentoxazone, perfluidone; pethoxamid; phenisopham; phenmedipham; picloram; picolinafen, pinoxaden, piperophos; piributicarb; pirifenop-butyl; pretilachlor; primisulfuron(-methyl); probenazole; procarbazone-(sodium), procyazine; prodiamine; profluralin; profoxydim; prohexadione(-calcium); prohydrojasmon; proglinazine(-ethyl); prometon; prometryn; propachlor; propanil; propaquizafop; propazine; propham; propisochlor; propoxycarbazone(-sodium) (MKH-6561); n-propyl-dihydrojasmonate; propyzamide; prosulfalin; prosulfocarb; prosulfuron (CGA-152005); prynachlor; pyraclonil; pyraflufen(-ethyl) (ET-751); pyrasulfotole; pyrazolynate; pyrazon; pyrazosulfuron(-ethyl); pyrazoxyfen; pyribenzoxim, pyributicarb, pyridafol, pyridate; pyriftalid; pyriminobac(-methyl) (KIH-6127); pyrimisulfan (KIH-5996); pyrithiobac(-sodium) (KIH-2031); pyroxasulfone (KIH-485); pyroxofop und dessen Ester (z.B. Propargylester); pyroxsulam; quinclorac; quinmerac; quinoclamine, quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; saflufenacil (N'-[2-Chlor-4-fluor-5-(3-methyl-2,6-dioxo-4-(triffuormethyl)-3,6-dihydro-1(2H)-pyrimidinyl)benzoyl]-N-isopropyl-N-methylsulfamid), secbumeton; sethoxydim; siduron; simazine; simetryn; sintofen; SN 106279, d.h. 2-[[7-[2-Chlor-4-(trifluor-methyl)-phenoxy]-2-naphthalenyl]-oxy]-propansäure und -methylester; sulcotrione, sulfentrazone (FMC-97285, F-6285); sulfazuron; sulfometuron(-methyl); sulfosate (ICI-A0224); sulfosulfuron, TCA; tebutam (GCP-5544); tebuthiuron; tecnacene; tembotrione; tefuryltrione; tepraloxydim, terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiafluamide, thiazafluron; thiazopyr (Mon-13200); thidiazimin (SN-24085); thidiazuron; thiencarbazone; thifensulfuron(-methyl); thiobencarb; Ti 35; tiocarbazil; topramezone; tralkoxydim; tri-allate; triasulfuron; triaziflam, triazofenamide; tribenuron(-methyl); triclopyr; tridiphane; trietazine; trifloxysulfuron; trifluralin; triflusulfuron und Ester (z.B. Methylester, DPX-66037); trimeturon; trinexapac; tritosulfuron, tsitodef; uniconazole; vemolate; WL 110547, d.h. 5-Phenoxy-1-[3-(trifluormethyl)-phenyl]-1 H-tetrazol; D-489; LS 82-556; KPP-300; NC-324; NC-330; DPX-N8189; SC-0774; DOWCO-535; DK-8910; V-53482; PP-600 und MBH-001.

Insektizide, die allein oder gemeinsam mit Herbiziden Pflanzenschädigungen verursachen können, sind beispielsweise folgende:

Organophosphate z.B. Terbufos (Counter^{®}), Fonofos (Dyfonate^{®}), Phorate (Thimet^{®}), Chlorpyriphos (Reldan^{®}), Carbamate, wie Carbofuran (Furadan^{®}), Pyrethroid-Insektizide, wie Tefluthrin (Force^{®}), Deltamethrin (Decis^{®}) und Tralomethrin (Scout^{®}) sowie andere insektizide Mittel mit andersartigem Wirkmechanismus.

Herbizide, deren phytotoxische Nebenwirkungen auf Kulturpflanzen mittels Verbindungen der Formel (I) herabgesetzt werden können, sind z.B. Herbizide aus der Gruppe der Carbamate, Thiocarbamate, Halogenacetanilide, substituierte Phenoxy-, Naphthoxy- und Phenoxyphenoxycarbonsäure-Derivate sowie Heteroaryloxy-phenoxyalkancarbonsäure-Derivate, wie Chinolyloxy-, Chinoxalyloxy-, Pyridyloxy-, Benzoxazolyloxy- und Benzthiazolyloxyphenoxyalkancarbonsäureester, Cyclohexandionoxime, Benzoylcyclohexandione, Benzoylisoxazole, Benzoylpyrazole, Imidazolinone, Pyrimidinyloxy-pyridincarbonsäure-Derivate, Pyrimidyloxy-benzoesäure-Derivate, Sulfonylharnstoffe, Sulfonylaminocarbonyltriazolinone, Triazoto-pyrimidin-sulfonamid-Derivate, Phosphinsäurederivate und deren Salze, Glyzinderivate, Triazolinone, Triazinone sowie S-(N-Aryl-N-alkylcarbamoylmethyl)-dithiophosphorsäureester, Pyridincarbonsäuren, Pyridine, Pyridincarboxamide, 1,3,5-Triazine, und weitere.

Bevorzugt sind dabei Phenoxyphenoxy- und Heteroaryloxyphenoxycarbonsäureester und -salze, Cyclohexandionoxime, Benzoylcyclohexandione, Benzoylisoxazole, Benzoylpyrazole, Sulfonylharnstoffe, Sulfonylaminocarbonyltriazolinone, Imidazolinone sowie Mischungen der genannten Wirkstoffe untereinander und/oder mit Wirkstoffen, die zur Erweiterung des Wirkungsspektrums der Herbizide eingesetzt werden, z.B. Bentazone, Cyanazine, Atrazine, Bromoxynil, Dicamba und andere Blattherbizide.

Geeignete Herbizide, die mit den erfindungsgemäßen Safenern kombiniert werden können, sind beispielsweise:
A) Herbizide vom Typ der Phenoxyphenoxy- und Heteroaryloxyphenoxycarbonsäure-Derivate, wie
   A1) Phenoxyphenoxy- und Benzyloxyphenoxy-carbonsäure-Derivate, z.B. 2-(4-(2,4-Dichlorphenoxy)-phenoxy)-propionsäuremethylester (Diclofop-methyl), 2-(4-(4-Brom-2-chlorphenoxy)phenoxy)propionsäuremethylester (DE-A 26 01 548), 2-(4-(4-Brom-2-fluorphenoxy)phenoxy)propionsäuremethylester (US-A 4,808,750), 2-(4-(2-Chlor-4-trifluormethylphenoxy)phenoxy)propionsäuremethylester (DE-A 24 33 067), 2-(4-(2-Fluor-4-trifluormethylphenoxy)phenoxy)propionsäuremethylester (US-A 4,808,750), 2-(4-(2,4-Dichlorbenzyl)phenoxy)propionsäuremethylester (DE-A 24 17 487), 4-(4-(4-Trifluormethylphenoxy)phenoxy)pent-2-en-säureethylester, 2-(4-(4-Trifluormethylphenoxy)phenoxy)propionsäuremethylester (DE-A 24 33 067); (*R*)-2-[4-(4-Cyano-2-fluorphenoxy)phenoxy]propionsäurebutylester (Cyhalofop-butyl)
   A2) "Einkemige" Heteroaryloxyphenoxy-alkancarbonsäure-Derivate, z.B. 2-(4-(3,5-Dichlorpyridyl-2-oxy)phenoxy)propionsäureethylester (EP-A 0 002 925), 2-(4-(3,5-Dichlorpyridyl-2-oxy)phenoxy)propionsäurepropargylester (EP-A 0 003 114),
      (*R*S)- oder (*R*)-2-(4-(3-Chlor-5-trifluormethyl-2-pyridyloxy)phenoxy)propionsäure-methylester (Haloxyfop-methyl bzw. Haloxyfop-P-methyl),
      2-(4-(3-Chlor-5-trifluormethyl-2-pyridyloxy)phenoxy)propionsäureethylester (EP-A 0 003 890),
      2-(4-(5-Chlor-3-fluor-2-pyridyloxy)phenoxy)propionsäurepropargylester (Clodinafop-propargyl),
      (*RS*)- oder (*R*)-2-(4-(5-Trifluormethyl-2-pyridyloxy)phenoxy)propionsäurebutylester (Fluazifop-butyl bzw. Fluazifop-P-butyl);
      (*R*)-2-[4-(3-chloro-5-trifluoromethyl-2-pyridyloxy)phenoxy]propionic acid
   A3) "Zweikernige" Heteroaryloxyphenoxy-alkancarbonsäure-Derivate, z.B. (*RS*)- oder (*R*)-2-(4-(6-Chlor-2-chinoxalyloxy)phenoxy)propionsäuremethylester und -ethylester (Quizalofop-methyl und -ethyl bzw. Quizalofop-P-methyl und -P-ethyl), 2-(4-(6-Fluor-2-chinoxalyloxy)phenoxy)propionsäuremethylester (s. J. Pest. Sci. Vol. 10, 61 (1985)),
      (*R*)-2-(4-(6-Chlor-2-chinoxalyloxy)phenoxy)propionsäure-2-isopropylidenaminooxy-ethylester (Propaquizafop),
      (*RS*)- oder (*R*)-2-(4-(6-Chlorbenzoxazol-2-yl-oxy)phenoxy)propionsäureethylester (Fenoxaprop-ethyl bzw. Fenoxaprop-P-ethyl),
      2-(4-(6-Chlorbenzthiazol-2-yloxy)phenoxy)propionsäureethylester (DE-A-26 40 730), (*RS)*- oder (*R*)-2-(4-(6-Chlorchinoxalyloxy)phenoxy)propionsäure-tetrahydro-2-furylmethylester (EP-A-0 323 727),
      (R)-2-[4-(6-Chlor-1,3-benzoxazol-2-yloxy)phenoxy]-2'-fluor-N-methylpropionanilid (Metamifop);
B) Herbizide aus der Reihe der Sulfonylharnstoffe, wie Pyrimidin- oder Triazinylaminocarbonyl-[benzol-, pyridin-, pyrazol-, thiophen- und (alkylsulfonyl)-alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind. Bevorzugte Substituenten im Benzol-, Pyridin-, Pyrazol-, Thiophen- oder (Alkylsulfonyl)-alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Halogenalkoxy, Halogenalkyl, Alkylcarbonyl, Alkoxyalkyl, (Alkansulfonyl)alkylamino. Solche geeignete Sulfonylharnstoffe sind beispielsweise
   B1) Phenyl- und Benzylsulfonylharnstoffe und verwandte Verbindungen, z.B. 1-(2-Chlorphenylsulfonyl)-3-(4-methoxy-6-methyl-1,3,5-triazin-2-yl)harnstoff (Chlorsulfuron),
      1-(2-Ethoxycarbonylphenylsulfonyl)-3-(4-chlor-6-methoxypyrimidin-2-yl)harnstoff (Chlorimuron-ethyl),
      1-(2-Methoxyphenylsulfonyl)-3-(4-methoxy-6-methyl-1,3,5-triazin-2-yl)harnstoff (Metsulfuron-methyl),
      1-(2-Chlorethoxyphenylsulfonyl)-3-(4-methoxy-6-methyl-1,3,5-triazin-2-yl)harnstoff (Triasulfuron),
      1-(2-Methoxycarbonylphenylsulfonyl)-3-(4,6-dimethylpyrimidin-2-yl)harnstoff (Sulfometuron-methyl),
      1-(2-Methoxycarbonylphenylsulfonyl)-3-(4-methoxy-6-methyl-1,3,5-triazin-2-yl)-3-methylharnstoff (Tribenuron-methyl),
      1-(2-Methoxycarbonylbenzylsulfonyl)-3-(4,6-dimethoxypyrimidin-2-yl)harnstoff (Bensulfuron-methyl),
      1-(2-Methoxycarbonylphenylsulfonyl)-3-(4,6-bis-(difluormethoxy)pyrimidin-2-yl)-harnstoff (Primisulfuron-methyl),
      3-(4-Ethyl-6-methoxy-1,3,5-triazin-2-yl)-1-(2,3-dihydro-1,1-dioxo-2-methylbenzo-[b]thiophen-7-sulfonyl)hamstoff (EP-A 0 796 83),
      3-(4-Ethoxy-6-ethyl-1,3,5-triazin-2-yl)-1-(2,3-dihydro-1,1-dioxo-2-methylbenzo[b]-thiophen-7-sulfonyl)hamstoff (EP-A 0 079 683),
      3-(4-Methoxy-6-methyl-1,3,5-triazin-2-yl)-1-(2-methoxycarbonyl-5-jod-phenyl-sulfonyl)harnstoff (WO 92/13845),
      2-[4-Dimethylamino-6-(2,2,2-trifluoroethoxy)-1,3,5-triazin-2-ylcarbamoylsulfamoyl]-3-methyl-benzoesäuremethylester (DPX-66037, Triflusulfuron-methyl),
      2-[(4,6-dimethylpyrimidin-2-yl)-carbamoylsulfamoyl]benzoesäureoxetan-3-ylester (CGA-277476, Oxasulfuron),
      4-Iod-2-[3-(4-methoxy-6-methyl-1,3,5-triazin-2-yl)ureidosulfonyl]-benzoesäuremethylester, Natriumsalz (lodosulfuron-methyl-Natrium),
      2-[3-(4,6-dimethoxypyrimidin-2-yl)ureidosulfonyl]-4-methansulfonylamino-methyl-benzoesäuremethylester (Mesosulfuron-methyl, WO 95/10507), N,N-Dimethyl-2-[3-(4,6-dimethoxypyrimidin-2-yl)ureidosulfonyl]-4-formylamino-benzamid (Foramsulfuron, WO 95/01344),
      1-(4,6-Dimethoxy-1,3,5-triazin-2-yl)-3-[2-(2-methoxyethoxy)phenylsulfonyl]harnstoff (Cinosulfuron),
      2-[(4-Ethoxy-6-methylamino-1,3,5-triazin-2-yl)carbamoylsulfamoyl]benzoesäure-methylester (Ethametsulfuron-methyl),
      1-(4-Methoxy-6-methyl-1,3,5-triazin-2-yl)3-[2-(3,3,3-trifluoropropyl)phenylsulfonyl]-harnstoff (Prosulfuron),
      1-(4-Methoxy-6-trifluoromethyl-1,3,5-triazin-2-yl)-3-(2-trifluoromethylbenzolsulfonyl)-harnstoff (Tritosulfuron),
      N-[(4-Methylpyrimidin-2-yl)carbamoyl]-2-nitrobenzolsulfonamid (Monosulfuron), Methyl-2-[({[4-methoxy-6-(methylthio)pyrimidin-2-yl]carbamoyl}amino)sulfonyl]-benzoat,
   B2) Thienylsulfonylharnstoffe, z.B.
      1-(2-Methoxycarbonylthiophen-3-yl)-3-(4-methoxy-6-methyl-1,3,5-triazin-2-yl)harnstoff (Thifensulfuron-methyl);
   B3) Pyrazolylsulfonylharnstoffe, z.B.
      1-(4-Ethoxycarbonyl-1-methylpyrazol-5-yl-sulfonyl)-3-(4,6-dimethoxypyrimidin-2-yl)harnstoff (Pyrazosulfuron-ethyl),
      3-Chlor-5-(4,6-dimethoxypyrimidin-2-ylcarbamoylsulfamoyl)-1-methyl-pyrazol-4-carbonsäuremethylester (Halosulfuron-methyl),
      5-(4,6-Dimethylpyrimidin-2-yl-carbamoylsulfamoyl)-1-(2-pyridyl)-pyrazol-4-carbonsäuremethylester (NC-330, s. Brighton Crop Prot. Conference'Weeds' 1991, Vol. 1, S. 45 ff.),
      1-(4,6-Dimethoxypyrimidin-2-yl)-3-[1-methyl-4-(2-methyl-2*H*-tetrazol-5-yl)pyrazol-5-ylsulfonyl]hamstoff (DPX-A8947, Azimsulfuron),
      N-[(4,6-Dimethoxypyrimidin-2-yl)carbamoyl]-4-(5,5-dimethyl-4,5-dihydroisoxazol-3-yl)-1,3-dimethyl-1H-pyrazol-5-sulfonamid;
   B4) Sulfondiamid-Derivate, z.B.
      3-(4,6-Dimethoxypyrimidin-2-yl)-1-(N-methyl-N-methylsulfonylaminosulfonyl)-harnstoff (Amidosulfuron) und dessen Strukturanaloge (EP-A 0 131 258 und Z. Pfl. Krankh. Pfl. Schutz, Sonderheft XII, 489-497 (1990));
   B5) Pyridylsulfonylharnstoffe, z.B.
      1-(3-N,N-Dimethylaminocarbonylpyridin-2-ylsulfonyl)-3-(4,6-dimethoxypyrimidin-2-yl)harnstoff (Nicosulfuron),
      1-(3-Ethylsulfonylpyridin-2-ylsulfonyl)-3-(4,6-dimethoxypyrimidin-2-yl)harnstoff (Rimsulfuron),
      2-[3-(4,6-Dimethoxypyrimidin-2-yl)ureidosulfonyl]-6-trifluormethyl-3-pyridin-carbonsäuremethylester, Natriumsalz (DPX-KE 459, Flupyrsulfuron-methyl-natrium),
      3-(4,6-Dimethoxypyrimidin-2-yl)-1-(3-N-methylsulfonyl-N-methyl-aminopyridin-2-yl)-sulfonylhamstoff oder dessen Salze (DE-A 40 00 503 und DE-A 40 30 577), 1-(4,6-Dimethoxypyrimidin-2-yl)-3-(3-trifluoromethyl-2-pyridylsulfonyl)harnstoff (Flazasulfuron),
      1-(4,6-Dimethoxypyrimidin-2-yl)-3-[3-(2,2,2-trifluoroethoxy)-2-pyridylsulfonyl]harnstoff-Natriumsalz (Trifloxysulfuron-natrium),
      (1*RS*,2*RS*;1*RS,*2*SR*)-1-{3-[(4,6-Dimethoxypyrimidin-2-ylcarbamoyl)sulfamoyl]-2-pyridyl}-2-fluorpropyl-methoxyacetate (Flucetosulfuron);
   B6) Alkoxyphenoxysulfonylharnstoffe, z. B.
      3-(4,6-Dimethoxypyrimidin-2-yl)-1-(2-ethoxyphenoxy)-sulfonylharnstoff oder dessen Salze (Ethoxysulfuron);
   B7) Imidazolylsulfonylharnstoffe, z.B.
      1-(4,6-Dimethoxypyrimidin-2-yl)-3-(2-ethylsulfonylimidazo[1,2-a]pyridin-3-yl)sulfonylharstoff (MON 37500, Sulfosulfuron),
      1-(2-Chloroimidazo[1,2-*a*]pyridin-3-ylsulfonyl)-3-(4,6-dimethoxypyrimidin-2-yl)harnstoff (Imazosulfuron),
      2-Chlor-N-[(4,6-dimethoxypyrimidin-2-yl)carbamoyl]-6-propylimidazo[1,2-b]pyridazin-3-sulfonamid;
   B8) Phenylaminosulfonylhamstoffe, z. B.
      1-[2-(Cyclopropylcarbonyl)phenylaminosulfonyl]-3-(4,6-dimethoxypyrimidin-2-yl)harnstoff (Cyclosulfamuron),
      1-(4,6-Dimethoxypyrimidin-2-yl)-3-[2-(dimethylcarbamoyl)phenylsulfamoyl]-harnstoff (Orthosulfamuron);
C) Chloracetanilide, z.B.
   Acetochlor, Alachlor, Butachlor, Dimethachlor, Dimethenamid, Dimethenamid-P; Metazachlor, Metolachlor, S-Metolachlor, Pethoxamid, Pretilachlor, Propachlor, Propisochlor und Thenylchlor; D) Thiocarbamate, z.B.
   S-Ethyl-N,N-dipropylthiocarbamat (EPTC),
   S-Ethyl-N,N-diisobutylthiocarbamat (Butylate),
   Cycloate, Dimepiperate, Esprocarb, Molinate, Orbencarb, Pebulate, Prosulfocarb, Thiobencarb, Tiocarbazil, Tri-allate, Vernolate;
E) Cyclohexandionoxime, z.B.
   Alloxydim, Butroxydim, Clethodim, Cloproxydim, Cycloxydim, Profoxydim, Sethoxydim, Tepraloxydim und Tralkoxydim;
F) Imidazolinone, z.B.
   Imazamethabenz-methyl, Imazapic, Imazamox, Imazapyr, Imazaquin und Imazethapyr;
G) Triazolopyrimidinsulfonamid-Derivate, z.B.
   Chloransulam-methyl, Diclosulam, Florasulam, Flumetsulam, Metosulam und Penoxsulam, d.h. 2-(2,2-Difluorethoxy)-N-(5,8-dimethoxy[1,2,4]triazolo[1,5-c]pyrimidin-2-yl)-6-(trifluormethyl)-benzolsulfonamid, und Pyroxsulam, d.h. N-(5,7-Dimethoxy[1,2,4]triazolo[1,5-a]pyrimidin-2-yl)-2-methoxy-4-(trifluormethyl)-3-pyridinsulfonamid;
H) Benzoylcyclohexandione, z.B.
   2-(2-Chlor-4-methylsulfonylbenzoyl)-cyclohexan-1,3-dion (SC-0051, Sulcotrione),
   2-(2-Nitrobenzoyl)-4,4-dimethyl-cyclohexan-1,3-dion (EP-A 0 274 634),
   2-(2-Nitro-3-methylsulfonylbenzoyl)-4,4-dimethylcyclohexan-1,3-dion (WO 91/13548),
   2-[4-(Methylsulfonyl)-2-nitrobenzoyl]-1,3-cyclohexandion (Mesotrione),
   2-[2-Chlor-3-(5-cyanomethyl-4,5-dihydroisoxazol-3-yl)-4-(ethylsulfonyl)-benzoyl]-1,3-cyclohexandion,
   2-[2-Chlor-3-(5-cyanomethyl-4,5-dihydroisoxazol-3-yl)-4-(methylsulfonyl)-benzoyl]-1,3-cyclohexandion,
   2-[2-Chlor-3-(5-ethoxymethyl-4,5-dihydroisoxazol-3-yl)-4-(ethylsulfonyl)-benzoyl]-1,3-cyclohexandion,
   2-[2-Chlor-3-(5-ethoxymethyl-4,5-dihydroisoxazol-3-yl)-4-(methylsulfonyl)-benzoyl]-1,3-cyclohexandion,
   2-[2-Chlor-3-[(2,2,2-trifluorethoxy)methyl]-4-(ethylsulfonyl)-benzoyl]-1,3-cyclohexandion,
   2-[2-Chlor-3-[(2,2,2-trifluorethoxy)methyl]-4-(methylsulfonyl)-benzoyl]-1,3-cyclohexandion (Tembotrione),
   2-[2-Chlor-3-[(2,2-difluorethoxy)methyl]-4-(ethylsulfonyl)-benzoyl]-1,3-cyclohexandion,
   2-[2-Chlor-3-[(2,2-difluorethoxy)methyl]-4-(methylsulfonyl)-benzoyl]-1,3-cyclohexandion,
   2-[2-Chlor-3-[(2,2,3,3-tetrafluor-propoxy)methyl]-4-(ethylsulfonyl)-benzoyl]-1,3-cyclohexandion,
   2-[2-Chlor-3-[(2,2,3,3-tetrafluor-propoxy)methyl]-4-(methylsulfonyl)benzoyl]-1,3-cyclohexandion,
   2-[2-Chlor-3-(cyclopropylmethoxy)-4-(ethylsulfonyl)-benzoyl]-1,3-cyclohexandion,
   2-[2-Chlor-3-(cyclopropylmethoxy)-4-(methylsulfonyl)-benzoyl]-1,3-cyclohexandion,
   2-[2-Chlor-3-(tetrahydrofuran-2-ylmethoxymethyl)-4-(ethylsulfonyl)-benzoyl]-1,3-cyclohexandion,
   2-[2-Chlor-3-(tetrahydrofuran-2-ylmethoxymethyl)-4-(methylsulfonyl)-benzoyl]-1,3-cyclohexandion (Tefuryltrione),
   2-[2-Chlor-3-[2-(2-methoxyethoxy)-ethoxymethyl]-4-(ethylsulfonyl)-benzoyl]-1,3-cyclohexandion,
   2-[2-Chlor-3-[2-(2-methoxyethoxy)-ethoxymethyl]-4-(methylsulfonyl)-benzoyl]-1,3-cyclohexandion,
   3-({2-[(2-methoxyethoxy)methyl]-6-(trifluoromethyl)pyridin-3-yl}carbonyl)-bicyclo[3.2.1]octane-2,4-dione (WO 2001094339);
I) Benzoylisoxazole, z. B.
   5-Cyclopropyl-[2-(methylsulfonyl)-4-(trifluoromethyl)benzoyl]isoxazol (Isoxaflutole), (4-Chlor-2-mesylphenyl)(5-cyclopropyl-1,2-oxazol-4-yl)methanon (Isoxachlortole);
J) Benzoylpyrazole, z. B.
   2-[4-(2,4-Dichlor-*m*-toluoyl)-1,3-dimethylpyrazol-5-yloxy]-4'-methylacetophenon (Benzofenap),
   4-(2,4-Dichlorbenzoyl)-1,3-dimethylpyrazol-5-yl toluene-4-sulfonate (Pyrazolynate),
   2-[4-(2,4-Dichlorbenzoyl)-1,3-dimethylpyrazol-5-yloxy]acetophenone (Pyrazoxyfen);
   5-Hydroxy-1-methyl-4-[2-(methylsulfonyl)-4-trifluormethyl-benzoyl]-pyrazol (WO 01/74785),
   1-Ethyl-5-hydroxy-4-[2-(methylsulfonyl)-4-trifluormethyl-benzoyl]-pyrazol (WO 01/74785),
   1,3-Dimethyl-5-hydroxy-4-[2-(methylsulfonyl)-4-trifluormethyl-benzoyl]-pyrazol (WO 01/74785),
   1-Ethyl-5-hydroxy-3-methyl-4-[2-(methylsulfonyl)-4-trifluormethyl-benzoyl]-pyrazol (Pyrasulfotole, WO 01/74785),
   5-Hydroxy-1-methyl-4-[-2-chlor-3-(4,5-dihydroisoxazol-3-yl)-4-methylsulfonyl-benzoyl]-pyrazol (WO 99/58509),
   5-Hydroxy-1-methyl-4-[3-(4,5-dihydroisoxazol-3-yl)-2-methyl-4-methylsulfonyl-benzoyl]-pyrazol (Topramezone, WO 99/58509),
   1-Ethyl-5-hydroxy-3-methyl-4-[2-methyl-4-methylsulfonyl-3-(2-methoxy-ethylamino)-benzoyl]-pyrazol (WO 96/26206),
   3-Cyclopropyl-5-hydroxy-1-methyl-4-[2-methyl-4-methylsulfonyl-3-(2-methoxy-ethylamino)-benzoyl]-pyrazol (WO 96/26206),
   5-Benzoxy-1-ethyl-4-[2-methyl-4-methylsulfonyl-3-(2-methoxy-ethylamino)-benzoyl]-pyrazol (WO 96/26206),
   1-Ethyl-5-hydroxy-4-(3-dimethylamino-2-methyl-4-methylsulfonyl-benzoyl)-pyrazol (WO 96/26206),
   5-Hydroxy-1-methyl-4-(2-chlor-3-dimethylamino-4-methylsulfonyl-benzoyl)-pyrazol (WO 96/26206),
   1-Ethyl-5-hydroxy-4-(3-allylamino-2-chlor-4-methylsulfonyl-benzoyl)-pyrazol (WO 96/26206),
   1-Ethyl-5-hydroxy-4-(2-methyl-4-methylsulfonyl-3-morpholino-benzoyl)-pyrazol (WO 96/26206),
   5-Hydroxy-1-isopropyl-4-(2-chlor-4-methylsulfonyl-3-morpholino-benzoyl)-pyrazol (WO 96/26206),
   3-Cyclopropyl-5-hydroxy-1-methyl-4-(2-chlor-4-methylsulfonyl-3-morpholino-benzoyl)-pyrazol (WO 96/26206),
   1,3-Dimethyl-5-hydroxy-4-(2-chlor-4-methylsulfonyl-3-pyrazol-1-yl-benzoyl)-pyrazol (WO 96/26206),
   1-Ethyl-5-hydroxy-3-methyl-4-(2-chlor-4-methylsulfonyl-3-pyrazol-1-yl-benzoyl)-pyrazol (WO 96/26206),
   1-Ethyl-5-hydroxy-4-(2-chlor-4-methylsulfonyl-3-pyrazol-1-yl-benzoyl)-pyrazol (WO 96/26206),
   (5-Hydroxy-1-methyl-1H-pyrazol-4-yl)(3,3,4-trimethyl-1,1-dioxido-2,3-dihydro-1-benzothien-5-yl)methanon (US2002/0016262),
   1-Methyl-4-[(3,3,4-trimethyl-1,1-dioxido-2,3-dihydro-1-benzothien-5-yl)carbonyl]-1H-pyrazol-5-yl-propan-1-sulfonat (US2002/0016262, WO 2002/015695);
   3-(2-Chlor-4-mesylbenzoyl)-2-phenylthiobicyclo[3.2.1]oct-2-en-4-on (Benzobicyclon);
K) Sulfonylaminocarbonyltriazolinone, z. B.
   4,5-Dihydro-3-methoxy-4-methyl-5-oxo-*N*-(2-trifluoromethoxyphenylsulfonyl)-1*H-*1,2,4-triazole-1-carboxamide Natriumsalz (Flucarbazone-Natrium),
   2-(4,5-Dihydro-4-methyl-5-oxo-3-propoxy-1*H*-1,2,4-triazol-1-yl)-carboxamidosulfonylbenzoesäure-methylester-Natriumsalz (Propoxycarbazone-Na),
   Methyl-4-[(4,5-dihydro-3-methoxy-4-methyl-5-oxo-1*H*-1,2,4-triazol-1-yl)carbonyl-sulfamoyl]-5-methylthiophen-3-carboxylat (Thiencarbazone-methyl);
L) Triazolinone, z. B.
   4-Amino-*N*-*tert*-butyl-4,5-dihydro-3-isopropyl-5-oxo-1,2,4-1*H*-triazole-1-carboxamid (Amicarbazone),
   2-(2,4-Dichlor-5-prop-2-ynyloxyphenyl)-5,6,7,8-tetrahydro-1,2,4-triazolo[4,3-*a*]pyridin-3(2*H*)-one (Azafenidin),
   (*RS*)-2-Chlor-3-[2-chloro-5-(4-difluoromethyl-4,5-dihydro-3-methyl-5-oxo-1*H*-1,2,4-triazol-1-yl)-4-fluorophenyl]propionsäureethylester (Carfentrazone-ethyl),
   2',4'-Dichlor-5'-(4-difluormethyl-4,5-dihydro-3-methyl-5-oxo-1*H-*1,2,4-triazol-1-yl)-methanesulfonanilid (Sulfentrazone),
   4-[4,5-Dihydro-4-methyl-5-oxo-3-(trifluormethyl)-1*H*-1,2,4-triazol-1-yl]-2-[(ethylsulfonyl)amino]-5-fluorbenzolcarbothioamid (Bencarbazone);
M) Phosphinsäuren und Derivate, z. B.
   4-[hydroxy(methyl)phosphinoyl]-L-homoalanyl-L-alanyl-L-alanin (Bilanafos), DL-Homoalanin-4-yl(methyl)phosphinsäure-ammoniumsalz (Glufosinateammonium);
N) Glyzinderivate, z. B.
   *N*-(Phosphonomethyl)glyzin und dessen Salze (Glyphosate und Salze, z. B. das Natriumsalz oder das Isopropylammoniumsalz),
   *N*-(Phosphonomethyl)glyzin-trimesiumsalz (Sulfosate);
O) Pyrimidinyloxy-pyridincarbonsäure-Derivate; Pyrimidinyloxybenzoesäure-Derivate bzw. Pyrimidinylthiobenzoesäurederivate, z.B.
   3-(4,6-Dimethoxypyrimidin-2-yl)-oxy-pyridin-2-carbonsäurebenzyl-ester (EP-A 0 249 707),
   3-(4,6-Dimethoxypyrimidin-2-yl)-oxy-pyridin-2-carbonsäuremethylester (EP-A 0 249 707),
   2,6-Bis[(4,6-dimethoxypyrimidin-2-yl)-oxy]-benzoesäure-1-(ethoxycarbonyl-oxyethyl)-ester (EP-A 0 472 113),
   2,6-Bis[(4,6-dimethoxypyrimidin-2-yl)-oxy]-benzoesäure (Bispyribac-natrium), Isopropyl-4-[[[2-[(4,6-dimethoxy-2-pyrimidinyl)oxy]phenyl]methyl]amino]benzoat (Pyribambenz-isopropyl, WO 2002034724),
   Propyl-4-[[[2-[(4,6-dimethoxy-2-pyrimidinyl)oxy]phenyl]methyl]amino]benzoat (Pyribambenz-propyl, WO 2002034724),
   Pyribenzoxim, Pyriftalid, Pyriminobac-methyl, Pyrithiobac-natrium, Pyrimisulfan;
P) S-(N-Aryl-N-alkyl-carbamoylmethyl)-dithiophosphonsäureester, wie S-[N-(4-Chlorphenyl)-N-isopropyl-carbamoylmethyl]-O,O-dimethyl-dithiophosphat (Anilophos);
Q) Triazinone, z. B.
   3-Cyclohexyl-6-dimethylamino-1-methyl-1,3,5-triazine-2,4-(1*H*,3*H*)-dion (Hexazinone),
   4-Amino-4,5-dihydro-3-methyl-6-phenyl-1,2,4-triazin-5-on (Metamitron), 4-Amino-6-*tert-*butyl-4,5-dihydro-3-methylthio-1,2,4-triazin-5-on (Metribuzin);
R) Pyridincarbonsäuren, z. B.
   Aminopyralid, Clopyralid, Fluroxypyr, Picloram und Triclopyr;
S) Pyridine, z. B.
   Dithiopyr und Thiazopyr;
T) Pyridincarboxamide, z. B.
   Diflufenican und Picolinafen;
U) 1,3,5-Triazine, z. B.
   Ametryn, Atrazine, Cyanazine, Dimethametrin, Prometon, Prometryn, Propazine, Simazine, Symetryn, Terbumeton, Terbuthylazine, Terbutryn und Trietazine;
V) Pflanzenwachstumsregulatoren, z. B.
   Forchlorfenuron und Thidiazuron;
W) Ketoenole, z. B.
   8-(2,6-diethyl-*p*-tolyl)-1,2,4,5-tetrahydro-7-oxo-7*H*-pyrazolo[1,2-*d*][1,4,5]oxadiazepin-9-yl 2,2-dimethylpropionate (Pinoxaden);
X) Pyrazole, z. B.
   3-[5-(difluoromethoxy)-1-methyl-3-(trifluoromethyl)pyrazol-4-ylmethylsulfonyl]-4,5-dihydro-5,5-dimethyl-1,2-oxazole (Pyroxasulfone).

Die Herbizide der Gruppen A bis W sind beispielsweise aus den oben jeweils genannten Schriften, aus "The Pesticide Manual", The British Crop Protection Council, 14th Edition, 2006, oder aus dem e-Pesticide Manual, Version 4.0, British Crop Protection Council 2006 oder auch aus dem "Compendium of Pesticide Common Names" bekannt.

Im Falle der Anwendung als Wirkstofformulierungen oder Coformulierungen-enthalten diese gegebenenfalls in der Regel die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die Verbindungen der allgemeinen Formel (I) und deren Kombinationen mit einem oder mehreren der genannten Pestizide können in Abhängigkeit von den vorgegebenen chemisch-physikalischen und biologischen Parametern auf verschiedene Arten formuliert werden. Als Formulierungsarten sind beispielsweise geeignet:
- Emulgierbare Konzentrate, die durch Auflösen der Wirkstoffe in einem organischen Lösungsmittel, z.B. Butanol, Cyclohexanon, Dimethylformamid, Xylol oder auch höher siedenden Kohlenwasserstoffen oder Mischungen der organischen Lösungsmittel unter Zusatz von einem oder mehreren Tensiden ionischer und/oder nichtionischer Art (Emulgatoren) hergestellt werden. Geeignete Emulgatoren sind beispielsweise alkylarylsulfonsaure Calcium-Salze, Fettsäurepolyglykolester, Alkylarylpolyglykolether, Fettalkoholpolyglykol-ether, Propylenoxid-Ethylenoxid-Kondensationsprodukte, Alkylpolyether, Sorbitanester und Polyoxyethylensorbitanfettsäureester;
- Stäubemittel, die durch Vermahlen der Wirkstoffe mit fein-verteilten festen anorganischen oder organischen Stoffen, z.B. Talkum, natürlichen Tonen, wie Kaolin, Bentonit und Pyrophyllit, Diatomeenerde oder Mehlen erhalten werden.
- auf Wasser oder Öl basierende Suspensionskonzentrate, die beispielsweise durch Naßvermahlung mittels Perlmühlen hergestellt werden können;
- wasserlösliche Pulver;
- wasserlösliche Konzentrate;
- Granulate, wie wasserlösliche Granulate, wasserdispergierbare Granulate sowie Granulate für die Streu- und Bodenapplikation;
- Spritzpulver, die neben Wirkstoff noch Verdünnungs- oder Inertstoffe und Tenside enthalten;
- Kapselsuspensionen und Mikrokapseln;
- Ultra-Low-Volume-Formulierungen.

Die oben genannten Formulierungsarten sind dem Fachmann bekannt und werden beispielsweise beschrieben in: K. Martens, "Spray Drying Handbook", 3rd Ed., G. Goodwin Ltd., London. 1979; W. van Valkenburg, "Pesticide Formulations", Marcel Dekker, N.Y. 1973; Winacker-Küchler, "Chemische Technologie", Band 7, C. Hanser Verlag München, 4. Auflage 1986; "Perry's Chemical Engineer's Handbook", 5th Ed., McGraw-Hill, N.Y. 1973, Seiten 8-57.

Die notwendigen Formulierungshilfsmittel wie Inertmaterialien, Tenside, Lösungsmittel und weitere Zusatzstoffe sind ebenfalls bekannt und werden beispielsweise beschrieben in: McCutcheon's "Detergents and Emulsifiers Annual", MC Publ. Corp., Ridgewood N.J.; C. Marsden, "Solvents Guide", 2nd Ed., Interscience, N.Y. 1963; H. von Olphen, "Introduction to Clay Colloid Chemistry", 2nd Ed., J. Wiley & Sons, N.Y.; Schönfeldt, "Grenzflächenaktive Äthylenoxidaddukte", Wiss. Verlagsgesellschaft, Stuttgart 1976; Sisley and Wood, "Encyclopedia of Surface Active Agents", Chem. Publ. Co. Inc., N.Y. 1964; Watkins, "Handbook of Insecticide Dust Diluents and Carriers", 2nd Ed., Darland Books, Caldwell N.J.; Winnacker-Küchler, "Chemische Technologie", Band 7, C. Hanser Verlag München, 4. Auflage 1986.

Außer den vorstehend genannten Formulierungshilfsmitteln können die nutzpflanzenschützenden Mittel gegebenenfalls übliche Haft-, Netz-, Dispergier-, Penetrations-, Emulgier-, Konservierungs-, Frostschutz-, Füll-, Träger- und Farbstoffe, Entschäumer, Verdunstungshemmer sowie den pH-Wert oder die Viskosität beeinflussende Mittel enthalten.

Je nach Art der Formulierung enthalten die nutzpflanzenschützenden Mittel in der Regel 0,1 bis 99 Gew.-%, insbesondere 0,2 bis 95 Gew.-%, eines oder mehrerer Safener der allgemeinen Formel (I) oder eine Kombination von Safener und Pestizid. Weiterhin enthalten sie 1 bis 99,9, insbesondere 4 bis 99,5 Gew.-%, eines oder mehrerer fester oder flüssiger Zusatzstoffe und 0 bis 25, insbesondere 0,1 bis 25 Gew.-% eines Tensids. In emulgierbaren Konzentraten beträgt die Wirkstoffkonzentration, d.h. die Konzentration von Safener und/oder Pestizid, in der Regel 1 bis 90, insbesondere 5 bis 80 Gew.-%. Stäubemittel enthalten üblicherweise 1 bis 30, vorzugsweise 5 bis 20 Gew.-% Wirkstoff. In Spritzpulvern beträgt die Wirkstoffkonzentration in der Regel 10 bis 90 Gew.-%. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u. a. variiert die erforderliche Aufwandmenge der Safener.

In den nachfolgenden Beispielen, die die Erfindung erläutern aber nicht limitieren, beziehen sich die Mengenangaben auf das Gewicht, wenn nicht Näheres definiert ist.

### BEISPIELE

### 1 FORMULIERUNGSBEISPIELE

### 1.1 STÄUBEMITTEL

Ein Stäubemittel wird erhalten, indem man 10 Gew.-Teile einer Verbindung der Formel (I) oder eines Wirkstoffgemischs aus einem Pestizid (z. B. Herbizid) und einem Safener der Formel (I) und 90 Gew.-Teile Talkum als Inertstoff mischt und in einer Schlagmühle zerkleinert.

### 1.2 WASSERDISPERGIERBARES PULVER

Ein in Wasser leicht dispergierbares, benetzbares Pulver wird erhalten, indem man 25 Gewichtsteile einer Verbindung der Formel (I) oder eines Wirkstoffgemischs aus einem Pestizid (z. B. Herbizid) und einem Safener der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 1.3 WASSERDISPERGIERBARES KONZENTRAT

Ein in Wasser leicht dispergierbares Dispersionskonzentrat wird erhalten, indem man 20 Gewichtsteile einer Verbindung der Formel (I) oder eines Wirkstoffgemischs aus einem Pestizid (z. B. Herbizid) und einem Safener der Formel (I) mit 6 Gew.-Teilen Alkylphenolpolyglykolether (^{®}Triton X 207), 3 Gew.-Teilen Isotridecanolpolyglykolether und 71 Gew.-Teilen paraffinischem Mineralöl mischt und in einer Reibkugelmühle auf eine Feinheit von unter 5 Mikron vermahlt.

### 1.4 EMULGIERBARES KONZENTRAT

Ein emulgierbares Konzentrat wird erhalten aus 15 Gew.-Teilen einer Verbindung der Formel (I) oder eines Wirkstoffgemischs aus einem Pestizid (z. B. Herbizid) und einem Safener der Formel (I), 75 Gew.-Teilen Cyclohexanon als Lösungsmittel und 10 Gew.-Teilen oxethyliertes Nonylphenol als Emulgator.

### 1.5 WASSERDISPERGIERBARES GRANULAT

Ein in Wasserdispergierbares Granulat wird erhalten, indem man

| | | |
|---|---|---|
| 75 | Gewichtsteile | eines Safeners der Formel (I) oder eines Gemischs eines Pestizids und eines Safeners der Formel (I), |
| 10 | " | ligninsulfonsaures Calcium, |
| 5 | " | Natriumlaurylsulfat, |
| 3 | " | Polyvinylalkohol und |
| 7 | " | Kaolin |

mischt, in einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | | |
|---|---|---|
| 25 | Gewichtsteile | eines Safeners der Formel (I) oder eines Gemischs eines Pestizids und eines Safeners der Formel (I), |
| 5 | " | 2,2'-dinaphthylmethan-6,6'-disulfonsaures Natrium, |
| 2 | " | oleoylmethyltaurinsaures Natrium, |
| 17 | " | Calciumcarbonat, |
| 50 | " | Wasser und |
| 1 | Gewichtsteil | Polyvinylalkohol |

auf einer Kolloidmühle homogenisiert, zerkleinert, dann in einer Perlmühle mahlt und die so erhaltene Suspension in einem Sprühturm mittels einer Einstoffdüse zerstäubt und trocknet.

### 2. HERSTELLUNGSBEISPIELE

### Beispiel A1

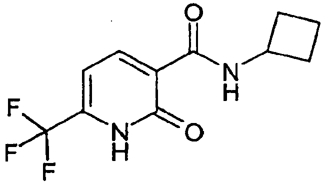

### N-Cyclobutyl-2-oxo-6-(trifluormethyl)-1,2-dihydropyridin-3-carboxamid

### A1.1) 4-Butoxy-1,1,1-trifluorbut-3-en-2-on

Ein Gemisch aus 29,9 g (0,38 mol) Pyridin und 50,0 g (0,38 mol) Butylvinylether in 200 ml Trichlormethan wurde unter Rühren bei 5 °C mit 79,9 g (0,38 mol) Trifluoressigsäureanhydrid - gelöst in 100 ml Trichlormethan - versetzt. Nach Zugabe wurde noch 15 h bei Raumtemperatur nachgerührt. Die Mischung wurde anschließend mit 300 ml Wasser versetzt, die organische Phase abgetrennt, getrocknet und eingeengt. Man erhielt 59 g (79 % d. Th.) eines gelblichen Öls. 1 H-NMR: [CDCl₃] 0,96 (t, 3H); 1,41 (m, 2H); 1,73 (m, 2H); 4,04 (t, 2H); 5,85 (d, 1 H); 7,90 (d, 1H).

### A1.2) 2-Oxo-6-(trifluormethyl)-1,2-dihydropyridin-3-carbonsäuremethylester

Nach Auflösung von 2,15 g (94 mmol) Natrium in 300 ml Methanol wurden 15,3 g (78 mmol) 4-Butoxy-1,1,1-trifluorbut-3-en-2-on und 9,13 g (78 mmol) Methylmalonat-monoamid zugesetzt und die Mischung 18 h unter Rückfluß erhitzt. Nach Einengung der Mischung wurde der Rückstand in Wasser aufgenommen und mit Dichlormethan gewaschen. Die wässrige Phase wurde anschließend durch Zugabe von 2n Salzsäure auf pH 2 eingestellt und mit Dichlormethan extrahiert. Nach Trocknung und Einengung des Extraktes verblieben 12 g (69 % d. Th.) eines farblosen Pulvers. ¹H-NMR: [CDCl₃] 4,03 (s, 3H); 7,31 (d, 1H); 8,39 (d, 1H).

### A1.3) 2-Oxo-6-(trifluormethyl)-1,2-dihydropyridin-3-carbonsäure

Bei Raumtemperatur wurden 4,73 g (21,4 mmol) 2-Oxo-6-(trifluormethyl)-1,2-dihydropyridin-3-carbonsäuremethylester in 45 ml Methanol und 15 ml Wasser gelöst, mit 1,80 g (42,8 mmol) Lithiumhydroxid-monohydrat versetzt und anschließend 2 h unter Rückfluß erhitzt. Nach Einengung der Mischung auf etwa 15 ml wurde mit Dichlormethan gewaschen und die wässrige Phase durch Zugabe von 2n Salzsäure auf pH 2 eingestellt. Erneut wurde mit Dichlormethan extrahiert, die organische Phase getrocknet und eingeengt. Man erhielt 4,2 g (94 % d. Th.) eines farblosen Pulvers.
¹H-NMR: [DMSO] 7,41 (d, 1 H); 8,35 (d, 1 H).

### A1.4) N-Cyclobutyl-2-oxo-6-(trifluormethyl)-1,2-dihydropyridin-3-carboxamid

Nach Auflösung von 600 mg (2,9 mmol) 2-Oxo-6-(trifluormethyl)-1,2-dihydropyridin-3-carbonsäure in 5 ml Tetrahydrofuran und Zugabe von 658 mg (4,1 mmol) N,N-Carbonyldiimidazol wurde zunächst 30 min bei Raumtemperatur sowie 30 min unter Rückfluß erhitzt. Anschließend tropfte man eine Lösung von 247 mg (3,5 mmol) Cyclobutylamin in 5 ml Tetrahydrofuran hinzu und refluxierte die Mischung für weitere 2 h. Nach Einengung der Lösung bis zur Trockne wurde in Essigsäureethylester aufgenommen, mit 1n Salzsäure und Wasser gewaschen, getrocknet und eingeengt. Nach säulenchromatographischer Reinigung erhielt man 160 mg (19 % d. Th.) eines hellbraunen Pulvers.
¹H-NMR: [CDCl₃] 1,80 (m, 2H); 2,00 (m, 2H); 2,42 (m, 2H); 4,55 (m, 1 H); 6,88 (d, 1H); 8,65 (d, 1H); 9,50 (br, 1H).

### Beispiel A2

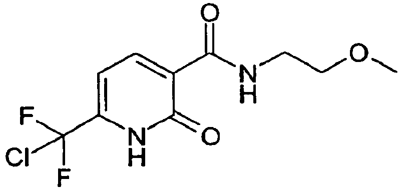

### 6-[Chlor(difluor)methyl]-N-(2-methoxyethyl)-2-oxo-1,2-dihydropyridin-3-carboxamid

### A2.1) 4-Butoxy-1-chlor-1,1-difluorbut-3-en-2-on

Ein Gemisch aus 1,60 g (20,2 mmol) Pyridin und 2,1 g (20,2 mmol) Butylvinylether in 30 ml Trichlormethan wurde unter Rühren bei 5 °C mit 5,0 g (20,2 mmol) Chlordifluoressigsäureanhydrid - gelöst in 10 ml Trichlormethan - versetzt. Nach Zugabe wurde noch 15 h bei Raumtemperatur nachgerührt. Die Mischung wurde anschließend mit 100 ml Wasser versetzt, die organische Phase abgetrennt, getrocknet und eingeengt. Man erhielt 3,4 g (80 % d. Th.) eines gelblichen Öls. ¹H-NMR: [DMSO] 0,90 (t, 3H); 1,35 (m, 2H); 1,65 (m, 2H); 4,20 (t, 2H); 6,04 (d, 1 H); 8,10 (d, 1H).

### A2.2) 6-[Chlor(difluor)methyl]-2-oxo-1,2-dihydropyridin-3-carbonsäuremethylester

Nach Auflösung von1,47 g (61 mmol) Natrium in 220 ml Methanol wurden 10 g (47 mmol) 4-Butoxy-1-chlor-1,1-difluorbut-3-en-2-on und 5,51 g (47 mmol) Methylmalonat-monoamid zugesetzt und die Mischung 21 h unter Rückfluß erhitzt. Durch Zugabe von 2n Salzsäure wurde auf ph 4-5 eingestellt und anschließend ca. 200 ml abdestilliert. Die zurückbleibende Lösung wurde mit Essigester extrahiert, der Extrakt getrocknet und eingeengt. Der Rückstand wurde mit Diisopropylether verrieben, abgesaugt und getrocknet. Man erhielt 7,4 g (66 % d. Th.) eines farblosen Pulvers.
¹H-NMR: [CDCl₃] 4,01 (s, 3H); 7,30 (d, 1H); 8,38 (d, 1H); 11,5 (br, 1H).

### A2.3a) 6-[Chlor(difluor)methyl]-2-oxo-1,2-dihydropyridin-3-carbonsäure

Nach Auflösung von 1,70 g (74 mmol) Natrium in 250 ml Methanol wurden 13,1 g (61,7 mmol) 4-Butoxy-1-chlor-1,1-difluorbut-3-en-2-on und 7,22 g (61,7 mmol) Methylmalonat-monoamid zugesetzt und die Mischung 21 h unter Rückfluß erhitzt. Anschließend gab man 250 ml Wasser und 2,9 g (67,9 mmol) Lithiumhydroxid-monohydrat hinzu und refluxierte weitere 2h. Nach Einengung der Mischung auf etwa 200 ml wusch man mit Dichlormethan und säuerte die wäßrige Phase mit 2n Salzsäure auf pH 2 an. Den ausgefallenen Feststoff saugte man ab und trocknete im Vakuum: 9,3 g (68 % d. Th.).

### A2.3b) 6-[Chlor(difluor)methyl]-2-oxo-1,2-dihydropyridin-3-carbonsäure

11,0 g (49,4 mmol) 6-[Chlor(difluor)methyl]-2-oxo-1,2-dihydropyridin-3-carboxamid wurden in 77 ml 50%iger Schwefelsäure 7 h erhitzt. Anschließend wurde die Mischung auf Eiswasser gegeben, der Niederschlag abgesaugt und getrocknet. Man erhielt 7,2 g (65 % d. Th.) eines gelblichen Pulvers.
Schmp.: 145-147 °C
¹H-NMR: [DMSO] 7,36 (d, 1H); 8,34 (d, 1H).

### A2.4) 6-[Chlor(difluor)methyl]-N-(2-methoxyethyl)-2-oxo-1,2-dihydropyridin-3-carboxamid

Nach Auflösung von 500 mg (2,2 mmol) 6-[Chlor(difluor)methyl]-2-oxo-1,2-dihydropyridin-3-carbonsäure in 10 ml Tetrahydrofuran und Zugabe von 508 mg (3,1 mmol) N,N-Carbonyldiimidazol wurde zunächst 30 min bei Raumtemperatur sowie 30 min unter Rückfluß erhitzt. Anschließend tropfte man eine Lösung von 202 mg (2,7 mmol) 2-Methoxyethylamin in 2 ml Tetrahydrofuran hinzu und refluxierte die Mischung für weitere 2 h. Nach Einengung der Lösung bis zur Trockne wurde in Essigsäureethylester aufgenommen, mit 1 n Salzsäure und Wasser gewaschen, getrocknet und eingeengt. Man erhielt 360 mg (54 % d. Th.) eines hellbraunen Pulvers.
¹H-NMR: [CDCl₃] 3,40 (s, 3H); 3,58 (m, 2H); 3,67 (m, 2H); 6,84 (d, 1H), 8,62 (d, 1 H); 9,40 (br, 1 H).

### Beispiel A3

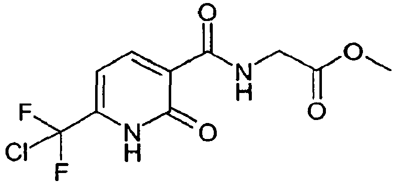

### Methyl N-({6-[chlor(difluor)methyl]-2-oxo-1,2-dihydropyridin-3-yl}carbonyl)glycinat

Nach Auflösung von 300 mg (1,34 mmol) 6-[Chlor(difluor)methyl]-2-oxo-1,2-dihydropyridin-3-carbonsäure in 5 ml Tetrahydrofuran und Zugabe von 435 mg (2,68 mmol) N,N-Carbonyldiimidazol wurde zunächst 30 min bei Raumtemperatur sowie 30 min unter Rückfluß erhitzt. Anschließend gab man eine Mischung von 168 mg (1,34 mmol) Glycinmethylester-hydrochlorid in 3 ml Tetrahydrofuran hinzu und refluxierte die Mischung für weitere 2 h. Nach Einengung der Lösung bis zur Trockne wurde in Essigsäureethylester aufgenommen, mit 1 n Salzsäure und Wasser gewaschen, getrocknet und eingeengt. Man erhielt 92 mg (23 % d. Th.) eines hellbraunen Pulvers.
¹H-NMR: [DMSO] 3,65 (s, 3H); 4, 12 (d, 2H); 7,28 (d, br, 1H); 8,42 (d, 1H); 9,25 (t, br, 1H), 13,6 (br, 1H)

### Beispiel A4

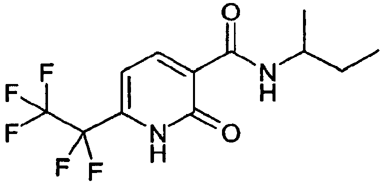

### N-(1-Methylpropyl)-2-oxo-6-(pentafluorethyl)-1,2-dihydropyridin-3-carboxamid

### A4.1) 1-Butoxy-4,4,5,5,5-pentafluorpent-1-en-3-on

Ein Gemisch aus 8,11 g (102 mmol) Pyridin und 9,33 g (93,2 mmol) Butylvinylether in 200 ml Dichlormethan wurde unter Rühren bei -10 °C mit 17,0 g (93,2 mmol) Pentafluorpropionylchlorid - gelöst in 20 ml Dichlormethan - versetzt. Nach Zugabe wurde noch 15 h bei Raumtemperatur nachgerührt. Die Mischung wurde anschließend mit 200 ml Wasser versetzt, die organische Phase abgetrennt, getrocknet und eingeengt. Man erhielt 18,9 g (82 % d. Th.) eines gelblichen Öls. ¹H-NMR: [DMSO] 0,85 (t, 3H); 1,32 (m, 2H); 1,63 (m, 2H); 4,20 (t, 2H); 6,06 (d, 1H); 8,11 (d, 1H).

### A4.2) 2-Oxo-6-(pentafluorethyl)-1,2-dihydropyridin-3-carbonsäureethylester

Nach Auflösung von 0,56 g (24,4 mmol) Natrium in 150 ml Ethanol wurden 5 g (20,3 mmol) 1-Butoxy-4,4,5,5,5-pentafluorpent-1-en-3-on und 2,66 g (20,3 mmol) Methylmalonat-monoamid zugesetzt und die Mischung 6 h unter Rückfluß erhitzt. Nach Einengung der Mischung auf etwa 50 ml wurde mit 500 ml 1 n Salzsäure versetzt und anschließend mit Essigsäurethylester extrahiert. Nach Trocknung und Einengung erhielt man 2,3 g (40 % d. Th.) eines gelblichen Harzes.
¹H-NMR: [CDCl₃] 1,45 (t, 3H); 4,50 (q, 2H); 7,35 (d, 1H); 8,40 (d, 1H); 11,4 (br, 1H).

### A4.3) N-(1-Methylpropyl)-2-oxo-6-(pentafluorethyl)-1,2-dihydropyridin-3-carboxamid

160 mg (0,56 mmol) 2-Oxo-6-(pentafluorethyl)-1,2-dihydropyridin-3-carbonsäureethylester wurden bei Raumtemperatur in 5 ml 2-Butylamin 14 h gerührt. Anschließend stellte man durch Zugabe von 1 n Salzsäure auf pH 2 ein, wobei ein farbloser Feststoff ausfiel. Nach Absaugung und Trocknung erhielt man 160 mg (91 % d. Th.) des Produktes.
¹H-NMR: [CDCl₃] 0,97 (t, 3H); 1,22 (d, 3H); 1,59 (m, 2H); 4,10 (m, 1 H); 6,88 (d, 1 H); 8,72 (d, 1H); 9,24 (d, br, 1H); 13,6 (br).

### Beispiel A5

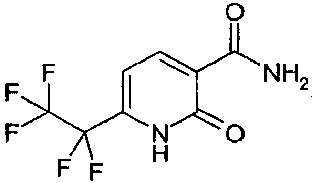

### 2-Oxo-6-(pentafluorethyl)-1,2-dihydropyridin-3-carboxamid

Nach Auflösung von 0,22 g (9,7 mmol) Natrium in 50 ml Ethanol wurden 2 g (8,1 mmol) 1-Butoxy-4,4,5,5,5-pentafluorpent-1-en-3-on und 0,86 g (8,1 mmol) Malonsäurediamid zugesetzt und die Mischung 7 h unter Rückfluß erhitzt. Nach Einengung der Mischung wurde mit 1 n Salzsäure versetzt. Der dabei ausfallende Niederschlag wurde abgesaugt und getrocknet. Man erhielt 1,9 g (94 % d. Th.) eines gelben Pulvers.
¹H-NMR: [DMSO] 7,45 (d, 1H); 8,15 (br, 1 H); 8,45 (br, 1H); 8,50 (d, 1H); 13,7 (br, 1H).

### Beispiel A6

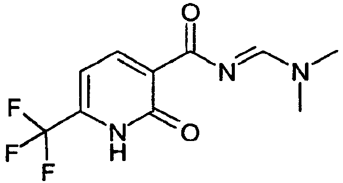

### N-[(Dimethylamino)methylen]-2-oxo-6-(trifluormethyl)-1,2-dihydropyridin-3-carboxamid

1,00 g (4,85 mmol) 2-Oxo-6-(trifluormethyl)-1,2-dihydropyridin-3-carboxamid in 5 ml Toluol wurden mit 0,87 g (7,28 mmol) N,N-Dimethylformamid-dimethylacetal versetzt und 4 h unter Rückfluß erhitzt. Nach Abkühlung wurde der ausgefallene Niederschlag abgesaugt und getrocknet: 0,5g (39 % d. Th.).
¹H-NMR: [CDCl₃] 3,25 (s, 3H); 3,35 (3, 3H); 7,25 (d, 1 H); 8,58 (d, 1 H); 8,76 (s, 1 H) ¹³C-NMR. [CDCl₃] 35 (NMe₂); 106 (C-5); 115 (C-3); 122 (q, CF₃); 143 (C-4); 151 (C-6); 164 (N=CN); 167 (C-2); 171 (CON).

### Beispiel A7

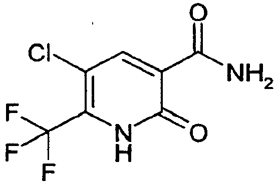

### 5-Chlor-2-oxo-6-(trifluormethyl)-1,2-dihydropyridin-3-carboxamid

300 mg (1,46 mmol) 2-Oxo-6-(trifluormethyl)-1,2-dihydropyridin-3-carboxamid in 5 ml 1,2-Dichlorethan und 2,3 g (0,25 ml) Pyridin wurden bei Raumtemperatur mit 0,39 g (2,9 mmol) Sulfurylchlorid versetzt und anschließend 2h refluxiert. Die Mischung wurde danach auf 0,5n Salzsäure gegeben. Nach Abtrennung der organischen Phase wurde diese getrocknet und eingeengt, wobei 144 mg (41 % d. Th.) eines bräunlichen Pulvers erhalten wurde.
¹H-NMR: [DMSO] 8,25 (br, 1H); 8,35 (br, 1H); 8,50 (s, 1H); 13,8 (br, 1H) ¹³C-NMR. [DMSO] 118 (C-5); 120 (C-6),: 120,5 (q, CF₃); 144 (C-4), 161 (C-2); 166 (COONH₂).

### Beispiel A8

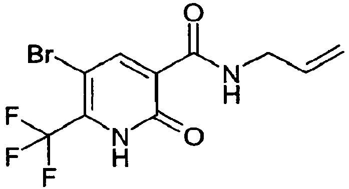

### N-Allyl-5-brom-2-oxo-6-(trifluormethyl)-1,2-dihydropyridin-3-carboxamid

### A8.1) 5-Brom-2-oxo-6-(trifluormethyl)-1,2-dihydropyridin-3-carbonsäuremethylester

Eine Suspension von 0,3 g (1,36 mmol) 2-Oxo-6-(trifluormethyl)-1,2-dihydropyridin-3-carbonsäuremethylester in 10 ml Eisessig wurde bei Raumtemperatur mit 0,36 g (2,04 mmol) N-Bromsuccinimid versetzt und anschließend 2 Tage refluxiert. Das Reaktionsgemisch wurde danach auf Wasser gegeben und mit Dichlormethan extrahiert. Nach Einengung des Extraktes erfolgte die weitere Reinigung durch präparative HPLC. Dabei wurden 227 mg (56 % d. Th.) eines farblosen Pulvers erhalten.
¹H-NMR: [DMSO] 3,85 (s, 3H); 8,45 (s, 1H); 12,80 (br, 1H);
¹³C-NMR. [DMSO] 53 (OCH₃); 105 (C-3); 148 (C-4), 161 (C-2); 164 (COOMe).

### A8.2) N-Allyl-5-brom-2-oxo-6-(trifluormethyl)-1,2-dihydropyridin3-carboxamid

250 mg (0,83 mmol) Methyl-5-brom-2-oxo-6-(trifluormethyl)-1,2-dihydropyridin-3-carboxylat wurden bei Raumtemperatur in 5 ml Allylamin 18 h gerührt. Anschließend stellte man durch Zugabe von 2n Salzsäure auf pH 2 ein, wobei ein farbloser Feststoff ausfiel. Nach Absaugung und Trocknung erhielt man 134 mg (50 % d. Th.) des Produktes.
¹H-NMR: [DMSO] 3,94 (t, 2H); 5,18 (dd, 2H); 5,89 (m, 1H); 8,52 (s, 1H); 8,85 (t, br, 1H).

### Beispiel A9

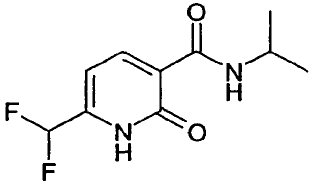

### 6-Difluormethyl-N-isopropyl-2-oxo-1,2-dihydropyridin-3-carboxamid

### A9.1) 4-Butoxy-1,1-difluorbut-3-en-2-on

Ein Gemisch aus 4,41 g (4,5 ml, 55,7 mmol) Pyridin und 5,7 g (55,7 mmol) Butylvinylether in 330 ml Trichlormethan wurde unter Rühren bei 5 °C mit 10,0 g (55,7 mmol) Difluoressigsäureanhydrid versetzt. Nach Zugabe wurde noch 15 h bei Raumtemperatur nachgerührt. Die Mischung wurde anschließend mit 300 ml Wasser versetzt, die organische Phase abgetrennt, getrocknet und eingeengt. Man erhielt 5,7 g (57 % d. Th.) eines gelblichen Öls.
¹H-NMR: [CDCl₃] 0,95 (t, 3H); 1,42 (m, 2H); 1,72 (m, 2H); 4,00 (t, 2H); 5,78 (t, 1H); 5,90 (d, 1 H); 7,85 (d, 1 H).

### A9.2) 6-(Difluormethyl)-2-oxo-1,2-dihydropyridin-3-carbonsäuremethylester

Nach Auflösung von 1,21 g (52,9 mmol) Natrium in 200 ml Methanol wurden 7,9 g (44 mmol) 4-Butoxy-1,1-difluorbut-3-en-2-on und 5,3 g (44 mmol) Methylmalonat-monoamid zugesetzt und die Mischung 15 h unter Rückfluß erhitzt. Nach Einengung der Mischung wurde der Rückstand in 100 ml 1n Salzsäure verrührt. Der erhaltene Feststoff wurde abgesaugt und getrocknet: 7,4 g (82 % d. Th.).
¹H-NMR: [DMSO] 3,82 (s, 3H); 6,85 (t, 1H); 7,02 (d, br, 1H); 8,20 (d, 1H); 12,4 (br, 1H).

### A9.3) 6-(Difluormethyl)-2-oxo-1,2-dihydropyridin-3-carbonsäure

7,4 g (36,4 mmol) 6-(Difluormethyl)-2-oxo-1,2-dihydropyridin-3-carbonsäuremethylester wurden in 100 ml Methanol gelöst und mit einer Lösung aus 1,3 g (54,6 mmol) Lithiumhydroxid in 50 ml Wasser versetzt. Nach zweistündigem Erhitzen unter Rückfluß wurde auf ein Volumen von etwa 50 ml eingeengt und die Lösung mit Dichlormethan gewaschen. Anschließend säuerte man die wässrige Phase mit 2n Salzsäure an und extrahierte mit Essigsäureethylester. Nach Trocknung und Einengung des Extraktes verblieben 5,6 g (82 % d. Th.) eines bräunlichen Pulvers.

### A9.4) 6-(Difluormethyl)-N-isopropyl-2-oxo-1,2-dihydropyridin-3-carboxamid

Nach Auflösung von 200 mg (1,1 mmol) 6-(Difluormethyl)-2-oxo-1,2-dihydropyridin-3-carbonsäure in 25 ml Tetrahydrofuran und Zugabe von 206 mg (1,3 mmol) N,N-Carbonyldiimidazol wurde zunächst 30 min bei Raumtemperatur sowie 30 min unter Rückfluß erhitzt. Anschließend tropfte man eine Lösung von 66 mg (1,1 mmol) Isopropylamin in 5 ml Tetrahydrofuran hinzu und refluxierte die Mischung für weitere 2 h. Nach Einengung der Lösung bis zur Trockne wurde in Essigsäureethylester aufgenommen, mit 2n Salzsäure und Wasser gewaschen, getrocknet und eingeengt. Nach säulenchromatographischer Reinigung erhielt man 150 mg (61 % d. Th.) eines farblosen Pulvers.
¹H-NMR: [CDCl₃] 1,25 (d, 6H); 4,22 (m, 1H); 6,55 (t, 1H); 6,69 (d, 1H); 8,64 (d, 1H); 9,42 (d, br, 1H); 13,0 (br, 1H).

### Beispiel A10

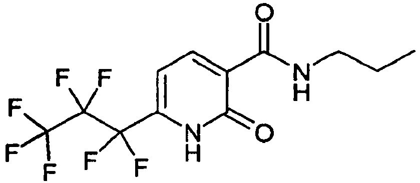

### 6-(Heptafluorpropyl)-2-oxo-N-propyl-1,2-dihydropyridin-3-carboxamid

### A10.1) 1-Butoxy-4,4,5,5,6,6,6-heptafluorhex-1-en-3-on

Ein Gemisch aus 8,11 g (102 mmol) Pyridin und 9,33 g (93,2 mmol) Butylvinylether in 200 ml Dichlormethan wurde unter Rühren bei -10 °C mit 21,7 g (93,2 mmol) Heptafluorbutyrylchlorid - gelöst in 50 ml Dichlormethan - versetzt. Nach Zugabe wurde noch 15 h bei Raumtemperatur nachgerührt. Die Mischung wurde anschließend mit 300 ml Wasser versetzt, die organische Phase abgetrennt, getrocknet und eingeengt.
Man erhielt 28,2 g (82 % d. Th.) eines gelblichen Öls, welches ohne weitere Reinigung weiter umgesetzt wurde.
¹H-NMR: [CDCl₃] 0,96 (t, 3H); 1,40 (m, 2H); 1,75 (m, 2H); 4,04 (t, 2H); 5,95 (d, 1 H); 7,93 (d, 1H).

### A10.2) 6-(Heptafluorpropyl)-2-oxo-1,2-dihydropyridin-3-carbonsäuremethylester

Nach Auflösung von 2,63 g (114 mmol) Natrium in 250 ml Methanol wurden 28,2 g (95,5 mmol) 1-Butoxy-4,4,5,5,6,6,6-heptafluorhex-1-en-3-on und 11,18 g (95,5 mmol) Methylmalonat-monoamid zugesetzt und die Mischung 18 h unter Rückfluß erhitzt. Nach Einengung der Mischung wurde der Rückstand in Wasser aufgenommen und mit Dichlormethan gewaschen. Die wässrige Phase wurde anschließend durch Zugabe von 2n Salzsäure auf pH 2 eingestellt und mit Dichlormethan extrahiert. Nach Trocknung, Einengung und säulenchromatographischer Reinigung des Extraktes verblieben 15,2 g (49 % d. Th.) eines gelben Pulvers.
¹H-NMR: [CDCl₃] 4,06 (s, 3H); 7,35 (d, 1 H); 8,40 (d, 1H); 11,4 (br, 1H).

### A10.3) 6-(Heptafluorpropyl)-2-oxo-N-propyl-1,2-dihydropyridin-3-carboxamid

In 5 ml Propylamin wurden 250 mg (0,78 mmol) Methyl-6-(heptafluorpropyl)-2-oxo-1,2-dihydropyridin-3-carboxylat 5 h refluxiert. Anschließend stellte man durch Zugabe von 1 n Salzsäure auf pH 2 ein und extrahierte die Mischung mit Dichlormethan. Nach Trocknung und Einengung wurden 240 mg (88 % d. Th.) eines beigen Pulvers erhalten.
¹H-NMR: [DMSO] 0,90 (t, 3H); 1,54 (m, 2H); 3,25 (q, 2H); 7,41 (d, 1H); 8,40 (d, 1H); 8,85 (br, 1 H); 13,45 (br, 1 H).

### Beispiel A11

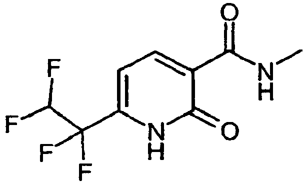

### N-Methyl-2-oxo-6-(1,1,2,2-tetrafluorethyl)-1,2-dihydropyridin-3-carboxamid

### A11.1) 1-Butoxy-4,4,5,5-tetrafluorpent-1-en-3-on

Ein Gemisch aus 14,4 g (182 mmol) Pyridin und 15,2 g (152 mmol) Butylvinylether in 250 ml Dichlormethan wurde unter Rühren bei 0 °C mit 25,0 g (152 mmol) 3H-Tetra-fluorpropionylchlorid - gelöst in 20 ml Dichlormethan - versetzt. Nach Zugabe wurde noch 15 h bei Raumtemperatur nachgerührt. Die Mischung wurde anschließend mit 250 ml Wasser versetzt, die organische Phase abgetrennt, getrocknet und eingeengt. Man erhielt 12,5 g (36 % d. Th.) eines gelblichen Öls.
¹H-NMR: [CDCl₃] 0,96 (t, 3H); 1,46 (m, 2H); 1,75 (m, 2H); 4,03 (t, 2H); 6,00 (d, 1H); 6,09 (tt, 1H); 7,90 (d, 1 H).

### A11.2) 2-Oxo-6-(1,1,2,2-tetrafluorethyl)-1,2-dihydropyridin-3-carbonsäure-methylester

Nach Auflösung von 1,39 g (60,3 mmol) Natrium in 500 ml Methanol wurden 11,5 g (50,3 mmol) 1-Butoxy-4,4,5,5-tetrafluorpent-1-en-3-on und 6,07 g (50,3 mmol) Methylmalonat-monoamid zugesetzt und die Mischung 6 h unter Rückfluß erhitzt. Nach Einengung der Mischung auf etwa 100 ml wurde mit 500 ml 1 n Salzsäure versetzt und anschließend mit Essigsäurethylester extrahiert. Nach Trocknung und Einengung erhielt man 10,3 g (67 % d. Th.) eines gelblichen Harzes.
¹H-NMR: [CDCl₃] 4,03 (s, 3H); 6,40 (tt, 1 H); 7,38 (d, 1 H); 8,39 (d, 1 H).

### A11.3) N-Methyl-2-oxo-6-(1,1,2,2-tetrafluorethyl)-1,2-dihydropyridin-3-carboxamid

300 mg (1,19 mmol) 2-Oxo-6-(1,1,2,2-tetrafluorethyl)-1,2-dihydropyridin-3-carbon-säuremethylester wurden bei Raumtemperatur in 3,3 ml 40%iger wässriger Methylamin-Lösung 14 h gerührt. Anschließend stellte man durch Zugabe von 1 n Salzsäure auf pH 2 ein und extrahierte mit Dichlormethan. Nach Trocknung und Einengung des Extraktes erhielt man 230 mg (77 % d. Th.) des Produktes.
¹H-NMR: [DMSO] 2,83 (d, 3H); 6,81 (tt, 1H); 7,22 (br, 1H); 8,41 (d, 1H); 9,00 (br, 1 H); 13,4 (br, 1H).

### Beispiel A12

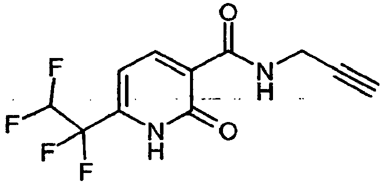

### 2-Oxo-N-(2-propinyl)-6-(1,1,2,2-tetrafluorethyl)-1,2-dihydropyridin-3-carboxamid

300 mg (1,19 mmol) 2-Oxo-6-(1,1,2,2-tetrafluorethyl)-1,2-dihydropyridin-3-carbon-säuremethylester wurden bei Raumtemperatur in 3,3 ml (41 mmol) Propargylamin 14 h gerührt. Anschließend stellte man durch Zugabe von 1 n Salzsäure auf pH 2 ein und extrahierte mit Dichlormethan. Nach Trocknung und Einengung des Extraktes erhielt man 280 mg (85 % d. Th.) des Produktes.
¹H-NMR: [DMSO] 3,15 (t, 1H); 4,13 (m, 2H); 6,82 (tt, 1H); 7,18 (br, 1H); 8,41 (d, 1H); 9,35 (br, 1H); 13,3 (br, 1H).

### Beispiel A13

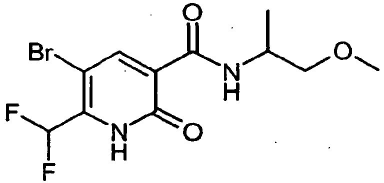

### 5-Brom-6-(difluormethyl)-N-(2-methoxy-1-methylethyl)-2-oxo-1,2-dihydropyridin-3-carboxamid

### A13.1) 5-Brom-6-(difluormethyl)-2-oxo-1,2-dihydropyridin-3-carbonsäure-methylester

4,1 g (20 mmol) 6-(Difluormethyl)-2-oxo-1,2-dihydropyridin-3-carbonsäuremethylester wurden in 20 ml N,N-Dimethylformamid gelöst und bei Raumtemperatur mit 5,35 g (30 mmol) N-Bromsuccinimid versetzt. Nach 2stdg. Rühren gab man das Reaktionsgemisch auf Wasser und extrahierte mit tert.Butyl-methylether. Der Extrakt wurde anschließend mehrmals mit Wasser gewaschen. Nach Trocknung und Einengung erhielt man schließlich 3,7 g (65 % d. Th.) des Produktes.
¹H-NMR: [DMSO] 3,83 (s, 3H); 7,07 (t, 1H); 8,34 (s, 1H); 12,5 (s, br, 1H).

### A13.2) 5-Brom-6-(difluormethyl)-N-(2-methoxy-1-methylethyl)-2-oxo-1,2-dihydropyridin-3-carboxamid

334 mg (1,19 mmol) 5-Brom-6-(difluormethyl)-2-oxo-1,2-dihydropyridin-3-carbonsäuremethylester wurden bei Raumtemperatur in 3,3 ml (41 mmol) 2-Amino-1-methoxypropan 14 h gerührt. Anschließend stellte man durch Zugabe von 1 n Salzsäure auf pH 2 ein und extrahierte mit Dichlormethan. Nach Trocknung und Einengung des Extraktes erhielt man 360 mg (89 % d. Th.) des Produktes.
¹H-NMR: [CDCl3] 1,27 (d, 3H); 3,40 (s, 3H); 3,43 (m, 2H); 4,37 (m, 1 H); 6,85 (t, 1 H); 8,68 (s, 1H); 9,42 (d, br, 1H); 12,7 (br, 1H).

### Beispiel A14

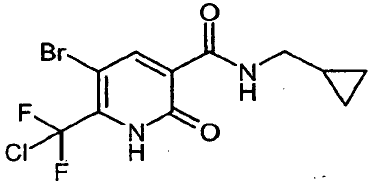

### 5-Brom-6-[chlor(difluor)methyl]-N-(cyclopropylmethyl)-2-oxo-1,2-dihydropyridin-3-carboxamid

### A14.1) 5-Brom-6-[chlor(difluor)methyl]-2-oxo-1,2-dihydropyridin-3-carbon-säuremethylester

1,30 g (5,47 mmol) 6-[Chlor(difluor)methyl]-2-oxo-1,2-dihydropyridin-carbonsäure-methylester wurden in 50 ml N,N-Dimethylformamid gelöst und bei Raumtemperatur mit 1,46 g (8,21 mmol) N-Bromsuccinimid versetzt. Nach 3stdg. Rühren gab man das Reaktionsgemisch auf Wasser, wobei ein farbloser Niederschlag ausfiel. Nach Absaugung und Trocknung verblieben 1,23 g (71 % d. Th.) des Produktes. ¹H-NMR: [DMSO] 3,85 (s, 3H); 8,45 (s, 1H); 12,7 (s, br, 1H).

### A14.2) 5-Brom-6-[chlor(difluor)methyl]-N-(cyclopropylmethyl)-2-oxo-1,2-dihydropyridin-3-carboxamid

350 mg (1,11 mmol) 5-Brom-6-[chlor(difluor)methyl]-2-oxo-1,2-dihydropyridin-3-carbonsäuremethylester wurden bei Raumtemperatur in 5 ml Acetonitril gelöst und mit 0,5 ml Aminomethylcyclopropan 12 h gerührt. Anschließend gab man das Reaktionsgemisch auf 1 n Salzsäure und saugte den dabei ausfallenden Niederschlag ab. Nach dessen Trocknung wurde nochmals mit 1 n Salzsäure verrührt, abgesaugt und mit Wasser nachgewaschen. Nach Trocknung erhielt man 240 mg (62 % d. Th.) des Produktes.
¹H-NMR: [DMSO] 0,25 (m, 2H); 0,46 (m, 2H); 1,04 (m, 1H); 3,20 (t, 2H); 8,55 (s, 1 H); 8,82 (t, br, 1 H); 13,6 (br, 1 H).

### Beispiel A15

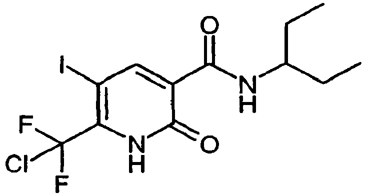

### 6-[Chlor(difluor)methyl]-N-(1-ethylpropyl)-5-iod-2-oxo-1,2-dihydropyridin-3-carboxamid

### A15.1) 6-[Chlor(difluor)methyl]-5-iod-2-oxo-1,2-dihydropyridin-3-carbonsäuremethylester

10,0 g (42,1 mmol) 6-[Chlor(difluor)methyl]-2-oxo-1,2-dihydropyridincarbonsäuremethylester wurden in 50 ml N,N-Dimethylformamid gelöst und bei Raumtemperatur mit 6,32 g (63,1 mmol) Calciumcarbonat und 20,5 g (126 mmol) lodchlorid versetzt. Nach 6stdg. Rühfen bei 50 °C gab man das Reaktionsgemisch auf Wasser, wobei ein farbloser Niederschlag ausfiel, der mit verdünnter Natriumbisulfit-Lösung und Wasser gewaschen wurde. Nach Absaugung und Trocknung verblieben 13,6 g (89 % d. Th.) des Produktes.
¹H-NMR: [DMSO] 3,84 (s, 3H); 8,61 (s, 1H); 12,6 (s, br, 1H).

### A15.2) 6-[Chlor(difluor)methyl]-N-(1-ethylpropyl)-5-iod-2-oxo-1,2-dihydro-pyridin-3-carboxamid

400 mg (1,1 mmol) 6-[Chlor(difluor)methyl]-5-iod-2-oxo-1,2-dihydropyridin-3-carbon-säuremethylester wurden bei Raumtemperatur in 5 ml Acetonitril gelöst und mit 0,5 ml 3-Pentylamin 12 h gerührt. Anschließend gab man das Reaktionsgemisch auf 1 n Salzsäure, saugte den dabei ausfallenden Niederschlag ab und wusch ihn mit Wasser. Nach Trocknung erhielt man 370 mg (72 % d. Th.) des Produktes. ¹H-NMR: [DMSO] 0,87 (t, 6H); 1,43 (m, 2H); 1,55 (m, 2H); 3,78 (m, 1H); 8,55 (br, 1 H); 8,67 (s, 1 H); 13,5 (br, 1 H).

### Beispiel A16

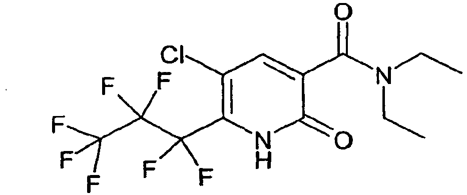

### 5-Chlor-N,N-diethyl-6-(heptafluorpropyl)-2-oxo-1,2-dihydropyridin-3-carboxamid

### A16.1) 5-Chlor-6-(heptafluorpropy)-2-oxo-1,2-dihydropyridin-3-carbonsäure-methylester

16,7 g (52 mmol) 6-(Heptafluorpropyl)-2-oxo-1,2-dihydropyridin-3-carbonsäure-methylester wurden in 160 ml N,N-Dimethylformamid gelöst und bei Raumtemperatur mit 7,08 g (52 mmol) N-Chlorsuccinimid versetzt. Nach 3stdg. Rühren bei 50 °C gab man das Reaktionsgemisch auf Wasser und extrahierte mehrmals mit Essigsäureethylester. Die organische Phase wurde anschließend abgetrennt, mit Wasser gewaschen und getrocknet. Das erhaltene Rohprodukt wurde danach mittels präparativer RP-HPLC gereinigt. Man erhielt 10,1 g (54 % d. Th.) des Produktes als farbloses Pulver.
¹H-NMR: [DMSO] 3,85 (s, 3H); 8,35 (s, 1H); 12,8 (s, br, 1H).

### A16.2) 5-Chlor-6-(heptafluorpropyl)-2-oxo-1,2-dihydropyridin-3-carbonsäure

1,54 g (4,33 mmol) 5-Chlor-6-(heptafluorpropy)-2-oxo-1,2-dihydropyridin-3-carbonsäuremethylester wurden in 23 ml Methanol gelöst und mit einer Lösung aus 0,16 g (6,5 mmol) Lithiumhydroxid in 13 ml Wasser versetzt. Nach zweistündigem Erhitzen unter Rückfluß wurde das Methanol abdestilliert und die verbliebene wässrige Lösung mit Dichlormethan gewaschen. Anschließend säuerte man die wässrige Phase mit 2n Salzsäure an und extrahierte mit Dichlormethan. Nach Trocknung und Einengung des Extraktes verblieben 1,45 g (98 % d. Th.) eines bräunlichen Pulvers. ¹H-NMR: [DMSO] 8,30 (s, 1H).

### A16.3) 5-Chlor-N,N-diethyl-6-(heptafluorpropyl)-2-oxo-1,2-dihydropyridin-3-carboxamid

Nach Auflösung von 355 mg (1,04 mmol) 5-Chlor-6-(heptafluorpropyl)-2-oxo-1,2-dihydropyridin-3-carbonsäure in 30 ml Tetrahydrofuran und Zugabe von 337 mg (2,08 mmol) N,N-Carbonyldiimidazol wurde zunächst 30 min bei Raumtemperatur sowie 30 min unter Rückfluß erhitzt. Anschließend gab man 152 mg (0,22 ml, 2,08 mmol) Diethylamin hinzu und refluxierte die Mischung für weitere 2 h. Nach Einengung der Lösung bis zur Trockne wurde in Dichlormethan aufgenommen, mit 1 n Salzsäure und Wasser gewaschen, getrocknet und eingeengt. Man erhielt 300 mg (72 % d. Th.) des Produktes.
¹H-NMR: [DMSO] 1,03 (t, 3H); 1,15 (t, 3H); 3,12 (q, 2H); 3,45 (q, 2H); 8,05 (s, 1H); 12,5 (br, 1H).

### Beispiel A17

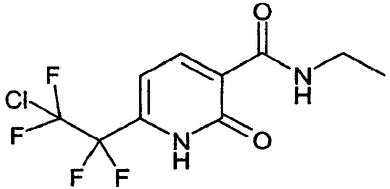

### 6-(2-Chlor-1,1,2,2-tetrafluorethyl)-N-ethyl-2-oxo-1,2-dihydropyridin-3-carboxamid

### A17.1) 1-Butoxy-5-chlor-4,4,5,5-tetrafluorpent-1-en-3-on

Ein Gemisch aus 3,97 g (50,2 mmol) Pyridin und 5,03 g (50,2 mmol) Butylvinylether in 80 ml Chloroform wurde unter Rühren bei 0 °C mit 10,0 g (50,2 mmol) 3-Chlortetrafluorpropionylchlorid - gelöst in 20 ml Chloroform - versetzt. Nach Zugabe wurde noch 15 h bei Raumtemperatur nachgerührt. Die Mischung wurde anschließend mit 100 ml Wasser versetzt, die organische Phase abgetrennt, getrocknet und eingeengt. Man erhielt 10,4 g (78 % d. Th.) eines gelblichen Öls. ¹H-NMR: [CDCl₃] 0,96 (t, 3H); 1,43 (m, 2H); 1,75 (m, 2H); 4,04 (t, 2H); 5,97 (d, 1H); 7,91 (d, 1H).

### A17.2) 6-(2-Chlor-1,1,2,2-tetrafluorethyl)-2-oxo-1,2-dihydropyridin-3-carbonsäuremethylester

Nach Auflösung von 0,32 g (13,7 mmol) Natrium in 50 ml Methanol wurden 3,0 g (11,4 mmol) 1-Butoxy-5-chfor-4,4,5,5-tetrafluorpent-1-en-3-on und 1,34 g (11,4 mmol) Methylmalonat-monoamid zugesetzt und die Mischung 6 h unter Rückfluß erhitzt. Nach Einengung der Mischung auf etwa 30 ml wurde mit 50 ml 1n Salzsäure versetzt und anschließend mehrmals mit Essigsäurethylester extrahiert. Nach Trocknung und Einengung wurde der erhaltene Rückstand in Diethylether aufgenommen und filtriert. Nach Einengung des Filtrates erhielt man 1,74 g (52 % d. Th.) eines gelblichen Pulvers.
¹H-NMR [DMSO] 3,84 (s, 3H); 7,44 (d, 1H); 8,32 (d, 1H); 12,4 (br, 1H).

### A17.3) 6-(2-Chlor-1,1,2,2-tetrafluorethyl)-N-ethyl-2-oxo-1,2-dihydropyridin-3-carboxamid

300 mg (1,04 mmol) 6-(2-Chlor-1,1,2,2-tetrafluorethyl)-2-oxo-1,2-dihydropyridin-3-carbonsäuremethylester wurden bei Raumtemperatur in 5 ml wässriger Ethylamin-Lösung (70 %) 14 h gerührt. Anschließend stellte man durch Zugabe von 1 n Salzsäure auf pH 2 ein und extrahierte mit Dichlormethan. Nach Trocknung und Einengung des Extraktes erhielt man 310 mg (98 % d. Th.) des Produktes.
¹H-NMR: [CDCl3] 1,27 (t, 3H); 3,50 (m, 2H); 6,88 (d, 1H); 8,68 (d, 1 H); 9,32 (br, 1H); 12,6 (br, 1 H).

### Beispiel A18

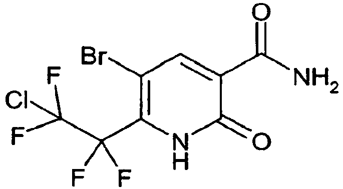

### 5-Brom-6-(2-chlor-1,1,2,2-tetrafluorethyl)-2-oxo-1,2-dihydropyridin-3-carboxamid

### A18.1) 6-(2-Chlor-1,1,2,2-tetrafluorethyl)-2-oxo-1,2-dihydropyridin-3-carboxamid

Nach Auflösung von 0,53 g (22,8 mmol) Natrium in 50 ml Methanol wurden 5,0 g (19,0 mmol) 1-Butoxy-5-chlor-4,4,5,5-tetrafluorpent-1-en-3-on und 2,0 g (19,0 mmol) Malonsäurediamid zugesetzt und die Mischung 6 h refluxiert. Nach Einengung der Mischung wurde mit 1 n Salzsäure versetzt. Der dabei ausfallende Niederschlag wurde abgesaugt und getrocknet. Man erhielt 3,8 g (73 % d. Th.) eines bräunlichen Pulvers.
¹H-NMR: [DMSO] 7,42 (br, 1H); 8,10 (br, 1H); 8,40 (br, 1H); 8,46 (d, 1H), 13,6 (br, 1 H).

### A18.2) 5-Brom-6-(2-chlor-1,1,2,2-tetrafluorethyl)-2-oxo-1,2-dihydropyridin-3-carboxamid

0,49 g (1,8 mmol) 6-(2-Chlor-1,1,2,2-tetrafluorethyl)-2-oxo-1,2-dihydropyridin-3-carboxamid wurden in 5 ml N,N-Dimethylformamid gelöst und bei Raumtemperatur mit 0,48 g (2,7 mmol) N-Bromsuccinimid versetzt. Nach 2stdg. Rühren bei Raumtemperatur gab man das Reaktionsgemisch auf Wasser, wobei ein Niederschlag ausfiel. Nach Absaugen, waschen mit Wasser und Trocknung erhielt man 610 mg (96 % d. Th.) des Produktes als gelbliches Pulver.
¹H-NMR: [DMSO] 8,25 (br, 1 H); 8,35 (br, 1H); 8,61 (s, 1H); 13,7 (br, 1H).

### Beispiel A19

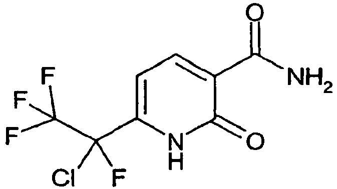

### 6-(1-Chlor-1,2,2,2-tetrafluorethyl)-2-oxo-1,2-dihydropyridin-3-carboxamid

### A19.1) 1-Butoxy-4-chlor-4,5,5,5-tetrafluorpent-1-en-3-on

Ein Gemisch aus 4,19 g (50,0 mmol) Pyridin und 4,83 g (48,2 mmol) Butylvinylether in 75 ml Dichlormethan wurde unter Rühren bei 0 °C tropfenweise mit 9,59 g (48,2 mmol) 2-Chlortetrafluorpropionylchlorid versetzt. Nach Zugabe wurde noch 15 h bei Raumtemperatur nachgerührt. Die Mischung wurde anschließend mit 1 n Salzsäure und 2 x 100 ml Wasser gewaschen, die organische Phase abgetrennt, getrocknet und eingeengt. Man erhielt 6,33 g (50 % d. Th.) eines braunen Öls.
¹H-NMR: [CDCl₃] 0,97 (t, 3H); 1,48 (m, 2H); 1,75 (m, 2H); 4,03 (t, 2H); 5,98 (d, 1H); 7,89 (d, 1H).

### A19.2) 6-(1-Chlor-1,2,2,2-tetrafluorethyl)-2-oxo-1,2-dihydropyridin-3-carboxamid

Nach Auflösung von 0,33 g (14,4 mmol) Natrium in 50 ml Methanol wurden 3,16 g (12,03 mmol) 1-Butoxy-4-chlor-4,5,5,5-tetrafluorpent-1-en-3-on und 1,27 g (12,03 mmol) Malonsäurediamid zugesetzt und die Mischung 7 h unter Rückfluß erhitzt. Nach Einengung der Mischung wurde mit 1 n Salzsäure versetzt. Der dabei ausfallende Niederschlag wurde abgesaugt und getrocknet. Man erhielt 380 mg (12 % d. Th.) eines gelblichen Pulvers.
¹H-NMR: [DMSO] 7,45 (d, 1 H); 8,14 (br, 1 H); 8,42 (br, 1 H); 8,48 (d, 1 H); 13,7 (br, 1H).

In der nachfolgenden Tabelle 1 ist beispielhaft eine Reihe von Verbindungen der allgemeinen Formel (I) aufgeführt, die in analoger Weise zu den obigen Beispielen A1 bis A10 und den weiter oben erwähnten Methoden erhalten werden können.

In der Tabelle bedeuten:

| | | | |
|---|---|---|---|
| Bu | = Butyl | Et | = Ethyl |
| Me | = Methyl | Ph | = Phenyl |
| Pr | = Propyl | | |
| i | = iso | s | = sekundär |
| t | = tertiär | c | = cyclo |

Entsprechendes gilt für die zusammengesetzten Ausdrücke wie
- iPr: = Isopropyl
- iBu: = Isobutyl
- sBu: = sec.-Butyl
- tBu: = tert.-Butyl
- cPr: = Cyclopropyl
- cPentyl: = Cyclopentyl
- cHexyl: = Cyclohexyl

Ist in den Tabellen ein Alkylrest ohne weitere Kennzeichnung aufgeführt, so handelt es sich um den geradkettigen Alkylrest, d. h. beispielsweise Bu = n-Bu = n-Butyl.

Die Zahlenindizes in den Formelausdrücken sind in der Tabelle nicht tiefgestellt, sondern in derselben Zeilenhöhe und Schriftgröße wie die Atomsymbole angeordnet.

Beispielsweise entspricht die Formel CF3 in der Tabelle der Formel CF₃ gemäß üblicher Schreibweise mit tiefgestelltem Index oder die Formel CH2CH(CH2CH3)2 der Formel CH₂CH(CH₂CH₃)₂ mit tiefgestellten Indices.

Zu einigen Verbindungen (I) in Tabelle 1 sind in der Tabelle 2 physikalischchemische Daten (in der Regel ¹H-NMR-Daten) aufgeführt. Die Zuordnung der Daten zu den Verbindungen erfolgt dabei über die Beispielnummer gemäß Tabelle 1.

**Tabelle 1: Verbindungen der Formel (I)**

| | | | | |
|---|---|---|---|---|
| 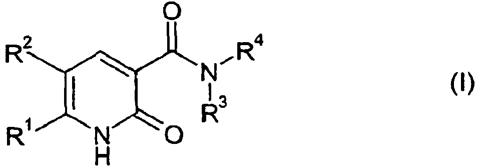 | | | | |

| Bsp. | R¹ | R² | R³ | R⁴ |
|---|---|---|---|---|
| 1 | CF3 | H | H | Me |
| 2 | CF3 | H | H | Et |
| 3 | CF3 | H | H | Pr |
| 4 | CF3 | H | H | iPr |
| 5 | CF3 | H | H | cPr |
| 6 | CF3 | H | H | Bu |
| 7 | CF3 | H | H | cBu |
| 8 | CF3 | H | H | tBu |
| 9 | CF3 | H | Me | Me |
| 10 | CF3 | H | Me | Et |
| 11 | CF3 | H | Me | Bu |
| 12 | CF3 | H | Me | Pr |
| 13 | CF3 | H | Me | iPr |
| 14 | CF3 | H | Et | Et |
| 15 | CF3 | H | Et | Pr |
| 16 | CF3 | H | Et | iPr |
| 17 | CF3 | H | Pr | Pr |
| 18 | CF3 | H | H | cPentyl |
| 19 | CF3 | H | H | cHexyl |
| 20 | CF3 | H | H | CH2(CH2)3CH3 |
| 21 | CF3 | H | H | CH2(CH2)4CH3 |
| 22 | CF3 | H | H | CH2-cPr |
| 23 | CF3 | H | H | CH2-CN |
| 24 | CF3 | H | H | CH2-C(CH3)3 |
| 25 | CF3 | H | H | CH2CF2CF3 |
| 26 | CF3 | H | H | CH2CF3 |
| 27 | CF3 | H | H | CH2(CF2)2CF3 |
| 28 | CF3 | H | H | CH2CH(CH3)CH2CH3 |
| 29 | CF3 | H | H | CH2C(CH3)2CH2F |
| 30 | CF3 | H | H | CH2CH(CH3)2 |
| 31 | CF3 | H | H | CH2CH(CH2CH3)2 |
| 32 | CF3 | H | H | CH2CH2CH(CH3)2 |
| 33 | CF3 | H | H | CH2CH2C(CH3)3 |
| 34 | CF3 | H | H | CH2CH=CH2 |
| 35 | CF3 | H | Me | CH2CH=CH2 |
| 36 | CF3 | H | CH2CH=CH2 | CH2CH=CH2 |
| 37 | CF3 | H | H | CH2CH=CHCH3 |
| 38 | CF3 | H | H | CH2-C(CH3)=CH2 |
| 39 | CF3 | H | H | CH2-C≡CH |
| 40 | CF3 | H | Me | CH2-C≡CH |
| 41 | CF3 | H | H | CH(CH3)CH2CH3 |
| 42 | CF3 | H | H | CH(CH3)cPr |
| 43 | CF3 | H | H | CH(CH3)(CH2)2CH3 |
| 44 | CF3 | H | H | CH(CH3)(CH2)4CH3 |
| 45 | CF3 | H | H | CH(CH3)(CH2)5CH3 |
| 46 | CF3 | H | H | CH(CH2CH3)(CH2)3CH3 |
| 47 | CF3 | H | H | CH(CH3)CH2CH(CH3)2 |
| 48 | CF3 | H | H | CH(CH3)C(CH3)3 |
| 49 | CF3 | H | H | CH(CH3)CH(CH3)2 |
| 50 | CF3 | H | H | CH(CH3)CH2CH2CH(CH3)2 |
| 51 | CF3 | H | H | CH(CH2CH3)2 |
| 52 | CF3 | H | H | C(CH3)2CH2CH3 |
| 53 | CF3 | H | H | C(CH3)2CH2C(CH3)3 |
| 54 | CF3 | H | H | CH2-CH(OMe)2 |
| 55 | CF3 | H | H | CH2-CH(OEt)2 |
| 56 | CF3 | H | H | CH2CH2-OH |
| 57 | CF3 | H | H | CH2CH2-OMe |
| 58 | CF3 | H | Me | CH2CH2-OMe |
| 59 | CF3 | H | H | CH2CH2-OEt |
| 60 | CF3 | H | H | CH2CH2-SMe |
| 61 | CF3 | H | H | CH2CH2-CN |
| 62 | CF3 | H | H | CH2CH2-NMe2 |
| 63 | CF3 | H | H | CH2CH2-Morpholin-4-yl |
| 64 | CF3 | H | H | CH(CH3)CH2-OMe |
| 65 | CF3 | H | H | CH(CH3)CH2-NMe2 |
| 66 | CF3 | H | H | CH2CH2CH2-OMe |
| 67 | CF3 | H | H | CH2CH2CH2-SMe |
| 68 | CF3 | H | H | CH2CH2CH2-OEt |
| 69 | CF3 | H | H | CH2CH2CH2-OiPr |
| 70 | CF3 | H | H | CH2CH2CH2-OBu |
| 71 | CF3 | H | H | CH2-COOCH3 |
| 72 | CF3 | H | Me | CH2-COOCH3 |
| 73 | CF3 | H | H | CH(CH3)COOMe |
| 74 | CF3 | H | H | CH(CH3)COOEt |
| 75 | CF3 | H | H | CH2CH2-COOCH3 |
| 76 | CF3 | H | H | CH(COOCH3)2 |
| 77 | CF3 | H | H | CH(COOEt)CH2-CH(CH3)2 |
| 78 | CF3 | H | H | CH(COOMe)CH(CH3)2 |
| 79 | CF3 | H | H | O-CH2CH3 |
| 80 | CF3 | H | Me | O-CH3 |
| 81 | CF3 | H | H | O-CH2CH=CH2 |
| 82 | CF3 | H | H | O-tBu |
| 83 | CF3 | H | H | O-Pr |
| 84 | CF3 | H | H | O-CH2cPr |
| 85 | CF3 | H | H | O-CH2CH(CH3)2 |
| 86 | CF3 | H | H | O-CH2CF3 |
| 87 | CF3 | H | H | O-CH(CH3)cPr |
| 88 | CF3 | H | H | O-CH2CH2Cl |
| 89 | CF3 | H | H | O-CH2C≡CH |
| 90 | CF3 | H | H | O-CH2C≡CCH3 |
| 91 | CF3 | H | H | O-CH(CH3)C≡CH |
| 92 | CF3 | H | H | CH2-Ph |
| 93 | CF3 | H | Me | CH2-Ph |
| 94 | CF3 | H | H | CH2-Pyridin-3-yl |
| 95 | CF3 | H | H | CH2-6-Cl-Pyridin-3-yl |
| 96 | CF3 | H | H | CH(CH3)Ph |
| 97 | CF3 | H | H | CH2CH2-Ph |
| 98 | CF3 | H | H | CH2-2-CF3-Ph |
| 99 | CF3 | H | H | CH2CH2CHPh2 |
| 100 | CF3 | H | Morpholin-4-yl | |
| 101 | CF3 | H | Piperidin-1-yl | |
| 102 | CF3 | H | Thiazolidin-3-yl | |
| 103 | CF3 | H | Pyrrolidin-1-yl | |
| 104 | CF3 | H | 2-Methyl-pyrrolidin-1-yl | |
| 105 | CF3 | H | =CH-N(CH3)2 | |
| 106 | CF3 | H | =C(CH3)N(CH3)2 | |
| 107 | CF3 | H | =CH-N(C2H5)2 | |
| 108 | CF3 | H | =C(CH3)N(C2H5)2 | |
| 109 | CF₃ | H | =CH-Piperidin | |
| 110 | CF3 | H | =CH-Morpholin | |
| 111 | CF3 | H | =CH-Pyrrolidin | |
| 112 | CF3 | H | H | Indan-1-yl |
| 113 | CF3 | H | H | Tetrahydrofuran-2-ylmethyl |
| 114 | CF2Cl | H | H | H |
| 115 | CF2Cl | H | H | Me |
| 116 | CF2Cl | H | H | Et |
| 117 | CF2Cl | H | H | Pr |
| 118 | CF2Cl | H | H | iPr |
| 119 | CF2Cl | H | H | cPr |
| 120 | CF2Cl | H | H | Bu |
| 121 | CF2Cl | H | H | cBu |
| 122 | CF2Cl | H | H | tBu |
| 123 | CF2Cl | H | Me | Me |
| 124 | CF2Cl | H | Me | Et |
| 125 | CF2Cl | H | Me | Bu |
| 126 | CF2Cl | H | Me | Pr |
| 127 | CF2Cl | H | Me | iPr |
| 128 | CF2Cl | H | Et | Et |
| 129 | CF2Cl | H | Et | Pr |
| 130 | CF2Cl | H | Et | iPr |
| 131 | CF2Cl | H | Pr | Pr |
| 132 | CF2Cl | H | H | cPentyl |
| 133 | CF2Cl | H | H | cHexyl |
| 134 | CF2Cl | H | H | CH2(CH2)3CH3 |
| 135 | CF2Cl | H | H | CH2(CH2)4CH3 |
| 136 | CF2Cl | H | H | CH2-cPr |
| 137 | CF2Cl | H | H | CH2-CN |
| 138 | CF2Cl | H | H | CH2-C(CH3)3 |
| 139 | CF2Cl | H | H | CH2CF2CF3 |
| 140 | CF2Cl | H | H | CH2CF3 |
| 141 | CF2Cl | H | H | CH2(CF2)2CF3 |
| 142 | CF2Cl | H | H | CH2CH(CH3)CH2CH3 |
| 143 | CF2Cl | H | H | CH2C(CH3)2CH2F |
| 144 | CF2Cl | H | H | CH2CH(CH3)2 |
| 145 | CF2Cl | H | H | CH2CH(CH2CH3)2 |
| 146 | CF2Cl | H | H | CH2CH2CH(CH3)2 |
| 147 | CF2Cl | H | H | CH2CH2C(CH3)3 |
| 148 | CF2Cl | H | H | CH2CH=CH2 |
| 149 | CF2Cl | H | Me | CH2CH=CH2 |
| 150 | CF2Cl | H | CH2CH=CH2 | CH2CH=CH2 |
| 151 | CF2Cl | H | H | CH2CH=CHCH3 |
| 152 | CF2Cl | H | H | CH2-C(CH3)=CH2 |
| 153 | CF2Cl | H | H | CH2-C≡CH |
| 154 | CF2Cl | H | Me | CH2-C=CH |
| 155 | CF2Cl | H | H | CH(CH3)CH2CH3 |
| 156 | CF2Cl | H | H | CH(CH3)cPr |
| 157 | CF2Cl | H | H | CH(CH3)(CH2)2CH3 |
| 158 | CF2Cl | H | H | CH(CH3)(CH2)4CH3 |
| 159 | CF2Cl | H | H | CH(CH3)(CH2)5CH3 |
| 160 | CF2Cl | H | H | CH(CH2CH3)(CH2)3CH3 |
| 161 | CF2Cl | H | H | CH(CH3)CH2CH(CH3)2 |
| 162 | CF2Cl | H | H | CH(CH3)C(CH3)3 |
| 163 | CF2Cl | H | H | CH(CH3)CH(CH3)2 |
| 164 | CF2Cl | H | H | CH(CH3)CH2CH2CH(CH3)2 |
| 165 | CF2Cl | H | H | CH(CH2CH3)2 |
| 166 | CF2Cl | H | H | C(CH3)2CH2CH3 |
| 167 | CF2Cl | H | H | C(CH3)2CH2C(CH3)3 |
| 168 | CF2Cl | H | H | CH2-CH(OMe)2 |
| 169 | CF2Cl | H | H | CH2-CH(OEt)2 |
| 170 | CF2Cl | H | H | CH2CH2-OH |
| 171 | CF2Cl | H | H | CH2CH2-OMe |
| 172 | CF2Cl | H | Me | CH2CH2-OMe |
| 173 | CF2Cl | H | H | CH2CH2-OEt |
| 174 | CF2Cl | H | H | CH2CH2-SMe |
| 175 | CF2Cl | H | H | CH2CH2-CN |
| 176 | CF2Cl | H | H | CH2CH2-NMe2 |
| 177 | CF2Cl | H | H | CH2CH2-Morpholin-4-yl |
| 178 | CF2Cl | H | H | CH(CH3)CH2-OMe |
| 179 | CF2Cl | H | H | CH(CH3)CH2-NMe2 |
| 180 | CF2Cl | H | H | CH2CH2CH2-OMe |
| 181 | CF2Cl | H | H | CH2CH2CH2-SMe |
| 182 | CF2Cl | H | H | CH2CH2CH2-OEt |
| 183 | CF2Cl | H | H | CH2CH2CH2-OiPr |
| 184 | CF2Cl | H | H | CH2CH2CH2-OBu |
| 185 | CF2Cl | H | H | CH2-COOCH3 |
| 186 | CF2Cl | H | Me | CH2-COOCH3 |
| 187 | CF2Cl | H | H | CH(CH3)COOMe |
| 188 | CF2Cl | H | H | CH(CH3)COOEt |
| 189 | CF2Cl | H | H | CH2CH2-COOCH3 |
| 190 | CF2Cl | H | H | CH(COOCH3)2 |
| 191 | CF2Cl | H | H | CH(COOEt)CH2-CH(CH3)2 |
| 192 | CF2Cl | H | H | CH(COOMe)CH(CH3)2 |
| 193 | CF2Cl | H | H | O-CH2CH3 |
| 194 | CF2Cl | H | H | O-CH3 |
| 195 | CF2Cl | H | H | O-CH2CH=CH2 |
| 196 | CF2Cl | H | H | O-tBu |
| 197 | CF2Cl | H | H | O-Pr |
| 198 | CF2Cl | H | H | O-CH2cPr |
| 199 | CF2Cl | H | H | O-CH2CH(CH3)2 |
| 200 | CF2Cl | H | H | O-CH2CF3 |
| 201 | CF2Cl | H | H | O-CH(CH3)cPr |
| 202 | CF2Cl | H | H | O-CH2CH2Cl |
| 203 | CF2Cl | H | H | O-CH2C≡CH |
| 204 | CF2Cl | H | H | O-CH2C≡CCH3 |
| 205 | CF2Cl | H | H | O-CH(CH3)C≡CH |
| 206 | CF2Cl | H | H | CH2-Ph |
| 207 | CF2Cl | H | Me | CH2-Ph |
| 208 | CF2Cl | H | H | CH2-Pyridin-3-yl |
| 209 | CF2Cl | H | H | CH2-6-Cl-Pyridin-3-yl |
| 210 | CF2Cl | H | H | CH(CH3)Ph |
| 211 | CF2Cl | H | H | CH2CH2-Ph |
| 212 | CF2Cl | H | H | CH2-2-CF3-Ph |
| 213 | CF2Cl | H | H | CH2CH2CHPh |
| 214 | CF2Cl | H | Morpholin-4-yl | |
| 215 | CF2Cl | H | Piperidin-1-yl | |
| 216 | CF2Cl | H | Thiazolidin-3-yl | |
| 217 | CF2Cl | H | Pyrrolidin-1-yl | |
| 218 | CF2Cl | H | 2-Methyl-pyrrolidin-1-yl | |
| 219 | CF2Cl | H | =CH-N(CH3)2 | |
| 220 | CF2Cl | H | =C(CH3)N(CH3)2 | |
| 221 | CF2Cl | H | =CH-N(C2H5)2 | |
| 222 | CF2Cl | H | =C(CH3)N(C2H5)2 | |
| 223 | CF2Cl | H | =CH-Piperidin | |
| 224 | CF2Cl | H | =CH-Morpholin | |
| 225 | CF2Cl | H | =CH-Pyrrolidin | |
| 226 | CF2Cl | H | H | Indan-1-yl |
| 227 | CF2Cl | H | H | Tetrahydrofuran-2-ylmethyl |
| 228 | CHF2 | H | H | H |
| 229 | CHF2 | H | H | Me |
| 230 | CHF2 | H | H | Et |
| 231 | CHF2 | H | H | Pr |
| 232 | CHF2 | H | H | iPr |
| 233 | CHF2 | H | H | cPr |
| 234 | CHF2 | H | H | Bu |
| 235 | CHF2 | H | H | cBu |
| 236 | CHF2 | H | H | tBu |
| 237 | CHF2 | H | Me | Me |
| 238 | CHF2 | H | Me | Et |
| 239 | CHF2 | H | Me | Bu |
| 240 | CHF2 | H | Me | Pr |
| 241 | CHF2 | H | Me | iPr |
| 242 | CHF2 | H | Et | Et |
| 243 | CHF2 | H | Et | Pr |
| 244 | CHF2 | H | Et | iPr |
| 245 | CHF2 | H | Pr | Pr |
| 246 | CHF2 | H | H | cPentyl |
| 247 | CHF2 | H | H | cHexyl |
| 248 | CHF2 | H | H | CH2(CH2)3CH3 |
| 249 | CHF2 | H | H | CH2(CH2)4CH3 |
| 250 | CHF2 | H | H | CH2-cPr |
| 251 | CHF2 | H | H | CH2-CN |
| 252 | CHF2 | H | H | CH2-C(CH3)3 |
| 253 | CHF2 | H | H | CH2CF2CF3 |
| 254 | CHF2 | H | H | CH2CF3 |
| 255 | CHF2 | H | H | CH2(CF2)2CF3 |
| 256 | CHF2 | H | H | CH2CH(CH3)CH2CH3 |
| 257 | CHF2 | H | H | CH2C(CH3)2CH2F |
| 258 | CHF2 | H | H | CH2CH(CH3)2 |
| 259 | CHF2 | H | H | CH2CH(CH2CH3)2 |
| 260 | CHF2 | H | H | CH2CH2CH(CH3)2 |
| 261 | CHF2 | H | H | CH2CH2C(CH3)3 |
| 262 | CHF2 | H | H | CH2CH=CH2 |
| 263 | CHF2 | H | Me | CH2CH=CH2 |
| 264 | CHF2 | H | CH2CH=CH2 | CH2CH=CH2 |
| 265 | CHF2 | H | H | CH2CH=CHCH3 |
| 266 | CHF2 | H | H | CH2-C(CH3)=CH2 |
| 267 | CHF2 | H | H | CH2-C=CH |
| 268 | CHF2 | H | Me | CH2-C=CH |
| 269 | CHF2 | H | H | CH(CH3)CH2CH3 |
| 270 | CHF2 | H | H | CH(CH3)cPr |
| 271 | CHF2 | H | H | CH(CH3)(CH2)2CH3 |
| 272 | CHF2 | H | H | CH(CH3)(CH2)4CH3 |
| 273 | CHF2 | H | H | CH(CH3)(CH2)5CH3 |
| 274 | CHF2 | H | H | CH(CH2CH3)(CH2)3CH3 |
| 275 | CHF2 | H | H | CH(CH3)CH2CH(CH3)2 |
| 276 | CHF2 | H | H | CH(CH3)C(CH3)3 |
| 277 | CHF2 | H | H | CH(CH3)CH(CH3)2 |
| 278 | CHF2 | H | H | CH(CH3)CH2CH2CH(CH3)2 |
| 279 | CHF2 | H | H | CH(CH2CH3)2 |
| 280 | CHF2 | H | H | C(CH3)2CH2CH3 |
| 281 | CHF2 | H | H | C(CH3)2CH2C(CH3)3 |
| 282 | CHF2 | H | H | CH2-CH(OMe)2 |
| 283 | CHF2 | H | H | CH2-CH(OEt)2 |
| 284 | CHF2 | H | H | CH2CH2-OH |
| 285 | CHF2 | H | H | CH2CH2-OMe |
| 286 | CHF2 | H | H | CH2CH2-OEt |
| 287 | CHF2 | H | H | CH2CH2-SMe |
| 288 | CHF2 | H | H | CH2CH2-CN |
| 289 | CHF2 | H | H | CH2CH2-NMe2 |
| 290 | CHF2 | H | H | CH2CH2-Morpholin-4-yl |
| 291 | CHF2 | H | H | CH(CH3)CH2-OMe |
| 292 | CHF2 | H | H | CH(CH3)CH2-NMe2 |
| 293 | CHF2 | H | H | CH2CH2CH2-OMe |
| 294 | CHF2 | H | H | CH2CH2CH2-SMe |
| 295 | CHF2 | H | H | CH2CH2CH2-OEt |
| 296 | CHF2 | H | H | CH2CH2CH2-OiPr |
| 297 | CHF2 | H | H | CH2CH2CH2-OBu |
| 298 | CHF2 | H | H | CH2-COOCH3 |
| 299 | CHF2 | H | Me | CH2-COOCH3 |
| 300 | CHF2 | H | H | CH(CH3)COOMe |
| 301 | CHF2 | H | H | CH(CH3)COOEt |
| 302 | CHF2 | H | H | CH2CH2-COOCH3 |
| 303 | CHF2 | H | H | CH(COOCH3)2 |
| 304 | CHF2 | H | H | CH(COOEt)CH2-CH(CH3)2 |
| 305 | CHF2 | H | H | CH(COOMe)CH(CH3)2 |
| 306 | CHF2 | H | H | O-CH2CH3 |
| 307 | CHF2 | H | H | O-CH3 |
| 308 | CHF2 | H | H | O-CH2CH=CH2 |
| 309 | CHF2 | H | H | O-tBu |
| 310 | CHF2 | H | H | O-Pr |
| 311 | CHF2 | H | H | O-CH2cPr |
| 312 | CHF2 | H | H | O-CH2CH(CH3)2 |
| 313 | CHF2 | H | H | O-CH2CF3 |
| 314 | CHF2 | H | H | O-CH(CH3)cPr |
| 315 | CHF2 | H | H | O-CH2CH2Cl |
| 316 | CHF2 | H | H | O-CH2C≡CH |
| 317 | CHF2 | H | H | O-CH2C≡CCH3 |
| 318 | CHF2 | H | H | O-CH(CH3)C≡CH |
| 319 | CHF2 | H | H | CH2-Ph |
| 320 | CHF2 | H | Me | CH2-Ph |
| 321 | CHF2 | H | H | CH2-Pyridin-3-yl |
| 322 | CHF2 | H | H | CH2-6-Cl-Pyridin-3-yl |
| 323 | CHF2 | H | H | CH(CH3)Ph |
| 324 | CHF2 | H | H | CH2CH2-Ph |
| 325 | CHF2 | H | H | CH2-2-CF3-Ph |
| 326 | CHF2 | H | H | CH2CH2CHPh |
| 327 | CHF2 | H | Morpholin-4-yl | |
| 328 | CHF2 | H | Piperidin-1-yl | |
| 329 | CHF2 | H | Thiazolidin-3-yl | |
| 330 | CHF2 | H | Pyrrolidin-1-yl | |
| 331 | CHF2 | H | 2-Methyl-pyrrolidin-1-yl | |
| 332 | CHF2 | H | =CH-N(CH3)2 | |
| 333 | CHF2 | H | =C(CH3)N(CH3)2 | |
| 334 | CHF2 | H | =CH-N(C2H5)2 | |
| 335 | CHF2 | H | =C(CH3)N(C2H5)2 | |
| 336 | CHF2 | H | =CH-Piperidin | |
| 337 | CHF2 | H | =CH-Morpholin | |
| 338 | CHF2 | H | =CH-Pyrrolidin | |
| 339 | CHF2 | H | H | Indan-1-yl |
| 340 | CHF2 | H | H | Tetrahydrofuran-2-ylmethyl |
| 341 | CF2CF3 | H | H | H |
| 342 | CF2CF3 | H | H | Me |
| 343 | CF2CF3 | H | H | Et |
| 344 | CF2CF3 | H | H | Pr |
| 345 | CF2CF3 | H | H | iPr |
| 346 | CF2CF3 | H | H | cPr |
| 347 | CF2CF3 | H | H | Bu |
| 348 | CF2CF3 | H | H | cBu |
| 349 | CF2CF3 | H | H | tBu |
| 350 | CF2CF3 | H | Me | Me |
| 351 | CF2CF3 | H | Me | Et |
| 352 | CF2CF3 | H | Me | Bu |
| 353 | CF2CF3 | H | Me | Pr |
| 354 | CF2CF3 | H | Me | iPr |
| 355 | CF2CF3 | H | Et | Et |
| 356 | CF2CF3 | H | Et | Pr |
| 357 | CF2CF3 | H | Et | iPr |
| 358 | CF2CF3 | H | Pr | Pr |
| 359 | CF2CF3 | H | H | cPentyl |
| 360 | CF2CF3 | H | H | cHexyl |
| 361 | CF2CF3 | H | H | CH2(CH2)3CH3 |
| 362 | CF2CF3 | H | H | CH2(CH2)4CH3 |
| 363 | CF2CF3 | H | H | CH2-cPr |
| 364 | CF2CF3 | H | H | CH2-CN |
| 365 | CF2CF3 | H | H | CH2-C(CH3)3 |
| 366 | CF2CF3 | H | H | CH2CF2CF3 |
| 367 | CF2CF3 | H | H | CH2CF3 |
| 368 | CF2CF3 | H | H | CH2(CF2)2CF3 |
| 369 | CF2CF3 | H | H | CH2CH(CH3)CH2CH3 |
| 370 | CF2CF3 | H | H | CH2C(CH3)2CH2F |
| 371 | CF2CF3 | H | H | CH2CH(CH3)2 |
| 372 | CF2CF3 | H | H | CH2CH(CH2CH3)2 |
| 373 | CF2CF3 | H | H | CH2CH2CH(CH3)2 |
| 374 | CF2CF3 | H | H | CH2CH2C(CH3)3 |
| 375 | CF2CF3 | H | H | CH2CH=CH2 |
| 376 | CF2CF3 | H | Me | CH2CH=CH2 |
| 377 | CF2CF3 | H | CH2CH=CH2 | CH2CH=CH2 |
| 378 | CF2CF3 | H | H | CH2CH=CHCH3 |
| 379 | CF2CF3 | H | H | CH2-C(CH3)=CH2 |
| 380 | CF2CF3 | H | H | CH2-C=CH |
| 381 | CF2CF3 | H | Me | CH2-C=CH |
| 382 | CF2CF3 | H | H | CH(CH3)CH2CH3 |
| 383 | CF2CF3 | H | H | CH(CH3)cPr |
| 384 | CF2CF3 | H | H | CH(CH3)(CH2)2CH3 |
| 385 | CF2CF3 | H | H | CH(CH3)(CH2)4CH3 |
| 386 | CF2CF3 | H | H | CH(CH3)(CH2)5CH3 |
| 387 | CF2CF3 | H | H | CH(CH2CH3)(CH2)3CH3 |
| 388 | CF2CF3 | H | H | CH(CH3)CH2CH(CH3)2 |
| 389 | CF2CF3 | H | H | CH(CH3)C(CH3)3 |
| 390 | CF2CF3 | H | H | CH(CH3)CH(CH3)2 |
| 391 | CF2CF3 | H | H | CH(CH3)CH2CH2CH(CH3)2 |
| 392 | CF2CF3 | H | H | CH(CH2CH3)2 |
| 393 | CF2CF3 | H | H | C(CH3)2CH2CH3 |
| 394 | CF2CF3 | H | H | C(CH3)2CH2C(CH3)3 |
| 395 | CF2CF3 | H | H | CH2-CH(OMe)2 |
| 396 | CF2CF3 | H | H | CH2-CH(OEt)2 |
| 397 | CF2CF3 | H | H | CH2CH2-OH |
| 398 | CF2CF3 | H | H | CH2CH2-OMe |
| 399 | CF2CF3 | H | Me | CH2CH2-OMe |
| 400 | CF2CF3 | H | H | CH2CH2-OEt |
| 401 | CF2CF3 | H | H | CH2CH2-SMe |
| 402 | CF2CF3 | H | H | CH2CH2-CN |
| 403 | CF2CF3 | H | H | CH2CH2-NMe2 |
| 404 | CF2CF3 | H | H | CH2CH2-Morpholin-4-yl |
| 405 | CF2CF3 | H | H | CH(CH3)CH2-OMe |
| 406 | CF2CF3 | H | H | CH(CH3)CH2-NMe2 |
| 407 | CF2CF3 | H | H | CH2CH2CH2-OMe |
| 408 | CF2CF3 | H | H | CH2CH2CH2-SMe |
| 409 | CF2CF3 | H | H | CH2CH2CH2-OEt |
| 410 | CF2CF3 | H | H | CH2CH2CH2-OiPr |
| 411 | CF2CF3 | H | H | CH2CH2CH2-OBu |
| 412 | CF2CF3 | H | H | CH2-COOCH3 |
| 413 | CF2CF3 | H | Me | CH2-COOCH3 |
| 414 | CF2CF3 | H | H | CH(CH3)COOMe |
| 415 | CF2CF3 | H | H | CH(CH3)COOEt |
| 416 | CF2CF3 | H | H | CH2CH2-COOCH3 |
| 417 | CF2CF3 | H | H | CH(COOCH3)2 |
| 418 | CF2CF3 | H | H | CH(COOEt)CH2-CH(CH3)2 |
| 419 | CF2CF3 | H | H | CH(COOMe)CH(CH3)2 |
| 420 | CF2CF3 | H | H | O-CH2CH3 |
| 421 | CF2CF3 | H | H | O-CH3 |
| 422 | CF2CF3 | H | H | O-CH2CH=CH2 |
| 423 | CF2CF3 | H | H | O-tBu |
| 424 | CF2CF3 | H | H | O-Pr |
| 425 | CF2CF3 | H | H | O-CH2cPr |
| 426 | CF2CF3 | H | H | O-CH2CH(CH3)2 |
| 427 | CF2CF3 | H | H | O-CH2CF3 |
| 428 | CF2CF3 | H | H | O-CH(CH3)cPr |
| 429 | CF2CF3 | H | H | O-CH2CH2Cl |
| 430 | CF2CF3 | H | H | O-CH2C≡CH |
| 431 | CF2CF3 | H | H | O-CH2C≡CCH3 |
| 432 | CF2CF3 | H | H | O-CH(CH3)C≡CH |
| 433 | CF2CF3 | H | H | CH2-Ph |
| 434 | CF2CF3 | H | Me | CH2-Ph |
| 435 | CF2CF3 | H | H | CH2-Pyridin-3-yl |
| 436 | CF2CF3 | H | H | CH2-6-Cl-Pyridin-3-yl |
| 437 | CF2CF3 | H | H | CH(CH3)Ph |
| 438 | CF2CF3 | H | H | CH2CH2-Ph |
| 439 | CF2CF3 | H | H | CH2-2-CF3-Ph |
| 440 | CF2CF3 | H | H | CH2CH2CHPh |
| 441 | CF2CF3 | H | Morpholin-4-yl | |
| 442 | CF2CF3 | H | Piperidin-1-yl | |
| 443 | CF2CF3 | H | Thiazolidin-3-yl | |
| 444 | CF2CF3 | H | Pyrrolidin-1-yl | |
| 445 | CF2CF3 | H | 2-Methyl-pyrrolidin-1-yl | |
| 446 | CF2CF3 | H | =CH-N(CH3)2 | |
| 447 | CF2CF3 | H | =C(CH3)N(CH3)2 | |
| 448 | CF2CF3 | H | =CH-N(C2H5)2 | |
| 449 | CF2CF3 | H | =C(CH3)N(C2H5)2 | |
| 450 | CF2CF3 | H | =CH-Piperidin | |
| 451 | CF2CF3 | H | =CH-Morpholin | |
| 452 | CF2CF3 | H | =CH-Pyrrolidin | |
| 453 | CF2CF3 | H | H | Indan-1-yl |
| 454 | CF2CF3 | H | H | Tetrahydrofuran-2-ylmethyl |
| 455 | CF3 | Cl | H | H |
| 456 | CF3 | Cl | H | Me |
| 457 | CF3 | Cl | H | Et |
| 458 | CF3 | Cl | H | Pr |
| 459 | CF3 | Cl | H | iPr |
| 460 | CF3 | Cl | H | cPr |
| 461 | CF3 | Cl | H | Bu |
| 462 | CF3 | Cl | H | cBu |
| 463 | CF3 | Cl | H | tBu |
| 464 | CF3 | Cl | Me | Me |
| 465 | CF3 | Cl | Me | Et |
| 466 | CF3 | Cl | Me | Bu |
| 467 | CF3 | Cl | Me | Pr |
| 468 | CF3 | Cl | Me | iPr |
| 469 | CF3 | Cl | Et | Et |
| 470 | CF3 | Cl | Et | Pr |
| 471 | CF3 | Cl | Et | iPr |
| 472 | CF3 | Cl | Pr | Pr |
| 473 | CF3 | Cl | H | cPentyl |
| 474 | CF3 | Cl | H | cHexyl |
| 475 | CF3 | Cl | H | CH2(CH2)3CH3 |
| 476 | CF3 | Cl | H | CH2(CH2)4CH3 |
| 477 | CF3 | Cl | H | CH2-cPr |
| 478 | CF3 | Cl | H | CH2-CN |
| 479 | CF3 | Cl | H | CH2-C(CH3)3 |
| 480 | CF3 | Cl | H | CH2CF2CF3 |
| 481 | CF3 | Cl | H | CH2CF3 |
| 482 | CF3 | Cl | H | CH2(CF2)2CF3 |
| 483 | CF3 | Cl | H | CH2CH(CH3)CH2CH3 |
| 484 | CF3 | Cl | H | CH2C(CH3)2CH2F |
| 485 | CF3 | Cl | H | CH2CH(CH3)2 |
| 486 | CF3 | Cl | H | CH2CH(CH2CH3)2 |
| 487 | CF3 | Cl | H | CH2CH2CH(CH3)2 |
| 488 | CF3 | Cl | H | CH2CH2C(CH3)3 |
| 489 | CF3 | Cl | H | CH2CH=CH2 |
| 490 | CF3 | Cl | Me | CH2CH=CH2 |
| 491 | CF3 | Cl | CH2CH=CH2 | CH2CH=CH2 |
| 492 | CF3 | Cl | H | CH2CH=CHCH3 |
| 493 | CF3 | Cl | H | CH2-C(CH3)=CH2 |
| 494 | CF3 | Cl | H | CH2-C=CH |
| 495 | CF3 | Cl | Me | CH2-C=CH |
| 496 | CF3 | Cl | H | CH(CH3)CH2CH3 |
| 497 | CF3 | Cl | H | CH(CH3)cPr |
| 498 | CF3 | Cl | H | CH(CH3)(CH2)2CH3 |
| 499 | CF₃ | Cl | H | CH(CH3)(CH2)4CH3 |
| 500 | CF3 | Cl | H | CH(CH3)(CH2)5CH3 |
| 501 | CF3 | Cl | H | CH(CH2CH3)(CH2)3CH3 |
| 502 | CF3 | Cl | H | CH(CH3)CH2CH(CH3)2 |
| 503 | CF3 | Cl | H | CH(CH3)C(CH3)3 |
| 504 | CF3 | Cl | H | CH(CH3)CH(CH3)2 |
| 505 | CF3 | Cl | H | CH(CH3)CH2CH2CH(CH3)2 |
| 506 | CF3 | Cl | H | CH(CH2CH3)2 |
| 507 | CF3 | Cl | H | C(CH3)2CH2CH3 |
| 508 | CF3 | Cl | H | C(CH3)2CH2C(CH3)3 |
| 509 | CF3 | Cl | H | CH2-CH(OMe)2 |
| 510 | CF3 | Cl | H | CH2-CH(OEt)2 |
| 511 | CF3 | Cl | H | CH2CH2-OH |
| 512 | CF3 | Cl | H | CH2CH2-OMe |
| 513 | CF3 | Cl | Me | CH2CH2-OMe |
| 514 | CF3 | Cl | H | CH2CH2-OEt |
| 515 | CF3 | Cl | H | CH2CH2-SMe |
| 516 | CF3 | Cl | H | CH2CH2-CN |
| 517 | CF3 | Cl | H | CH2CH2-NMe2 |
| 518 | CF3 | Cl | H | CH2CH2-Morpholin-4-yl |
| 519 | CF3 | Cl | H | CH(CH3)CH2-OMe |
| 520 | CF3 | Cl | H | CH(CH3)CH2-NMe2 |
| 521 | CF3 | Cl | H | CH2CH2CH2-OMe |
| 522 | CF3 | Cl | H | CH2CH2CH2-SMe |
| 523 | CF3 | Cl | H | CH2CH2CH2-OEt |
| 524 | CF3 | Cl | H | CH2CH2CH2-OiPr |
| 525 | CF3 | Cl | H | CH2CH2CH2-OBu |
| 526 | CF3 | Cl | H | CH2-COOCH3 |
| 527 | CF3 | Cl | Me | CH2-COOCH3 |
| 528 | CF3 | Cl | H | CH(CH3)COOMe |
| 529 | CF3 | Cl | H | CH(CH3)COOEt |
| 530 | CF3 | Cl | H | CH2CH2-COOCH3 |
| 531 | CF3 | Cl | H | CH(COOCH3)2 |
| 532 | CF3 | Cl | H | CH(COOEt)CH2-CH(CH3)2 |
| 533 | CF3 | Cl | H | CH(COOMe)CH(CH3)2 |
| 534 | CF3 | Cl | H | O-CH2CH3 |
| 535 | CF3 | Cl | Me | O-CH3 |
| 536 | CF3 | Cl | H | O-CH2CH=CH2 |
| 537 | CF3 | Cl | H | O-tBu |
| 538 | CF3 | Cl | H | O-Pr |
| 539 | CF3 | Cl | H | O-CH2cPr |
| 540 | CF3 | Cl | H | O-CH2CH(CH3)2 |
| 541 | CF3 | Cl | H | O-CH2CF3 |
| 542 | CF3 | Cl | H | O-CH(CH3)cPr |
| 543 | CF3 | Cl | H | O-CH2CH2Cl |
| 544 | CF3 | Cl | H | O-CH2C≡CH |
| 545 | CF3 | Cl | H | O-CH2C≡CCH3 |
| 546 | CF3 | Cl | H | O-CH(CH3)C≡CH |
| 547 | CF3 | Cl | H | CH2-Ph |
| 548 | CF3 | Cl | Me | CH2-Ph |
| 549 | CF3 | Cl | H | CH2-Pyridin-3-yl |
| 550 | CF3 | Cl | H | CH2-6-Cl-Pyridin-3-yl |
| 551 | CF3 | Cl | H | CH(CH3)Ph |
| 552 | CF3 | Cl | H | CH2CH2-Ph |
| 553 | CF3 | Cl | H | CH2-2-CF3-Ph |
| 554 | CF3 | Cl | H | CH2CH2CHPh2 |
| 555 | CF3 | Cl | Morpholin-4-yl | |
| 556 | CF3 | Cl | Piperidin-1-yl | |
| 557 | CF3 | Cl | Thiazolidin-3-yl | |
| 558 | CF3 | Cl | Pyrrolidin-1-yl | |
| 559 | CF3 | Cl | 2-Methyl-pyrrolidin-1-yl | |
| 560 | CF3 | Cl | =CH-N(CH3)2 | |
| 561 | CF3 | Cl | =C(CH3)N(CH3)2 | |
| 562 | CF3 | Cl | =CH-N(C2H5)2 | |
| 563 | CF3 | Cl | =C(CH3)N(C2H5)2 | |
| 564 | CF3 | Cl | =CH-Piperidin | |
| 565 | CF3 | Cl | =CH-Morpholin | |
| 566 | CF3 | Cl | =CH-Pyrrolidin | |
| 567 | CF3 | Cl | H | Indan-1-yl |
| 568 | CF3 | Cl | H | Tetrahydrofuran-2-ylmethyl |
| 569 | CF2Cl | Cl | H | H |
| 570 | CF2Cl | Cl | H | Me |
| 571 | CF2Cl | Cl | H | Et |
| 572 | CF2Cl | Cl | H | Pr |
| 573 | CF2Cl | Cl | H | iPr |
| 574 | CF2Cl | Cl | H | cPr |
| 575 | CF2Cl | Cl | H | Bu |
| 576 | CF2Cl | Cl | H | cBu |
| 577 | CF2Cl | Cl | H | tBu |
| 578 | CF2Cl | Cl | Me | Me |
| 579 | CF2Cl | Cl | Me | Et |
| 580 | CF2Cl | Cl | Me | Bu |
| 581 | CF2Cl | Cl | Me | Pr |
| 582 | CF2Cl | Cl | Me | iPr |
| 583 | CF2Cl | Cl | Et | Et |
| 584 | CF2Cl | Cl | Et | Pr |
| 585 | CF2Cl | Cl | Et | iPr |
| 586 | CF2Cl | Cl | Pr | Pr |
| 587 | CF2Cl | Cl | H | cPentyl |
| 588 | CF2Cl | Cl | H | cHexyl |
| 589 | CF2Cl | Cl | H | CH2(CH2)3CH3 |
| 590 | CF2Cl | Cl | H | CH2(CH2)4CH3 |
| 591 | CF2Cl | Cl | H | CH2-cPr |
| 592 | CF2Cl | Cl | H | CH2-CN |
| 593 | CF2Cl | Cl | H | CH2-C(CH3)3 |
| 594 | CF2Cl | Cl | H | CH2CF2CF3 |
| 595 | CF2Cl | Cl | H | CH2CF3 |
| 596 | CF2Cl | Cl | H | CH2(CF2)2CF3 |
| 597 | CF2Cl | Cl | H | CH2CH(CH3)CH2CH3 |
| 598 | CF2Cl | Cl | H | CH2C(CH3)2CH2F |
| 599 | CF2Cl | Cl | H | CH2CH(CH3)2 |
| 600 | CF2Cl | Cl | H | CH2CH(CH2CH3)2 |
| 601 | CF2Cl | Cl | H | CH2CH2CH(CH3)2 |
| 602 | CF2Cl | Cl | H | CH2CH2C(CH3)3 |
| 603 | CF2Cl | Cl | H | CH2CH=CH2 |
| 604 | CF2Cl | Cl | Me | CH2CH=CH2 |
| 605 | CF2Cl | Cl | CH2CH=CH2 | CH2CH=CH2 |
| 606 | CF2Cl | Cl | H | CH2CH=CHCH3 |
| 607 | CF2Cl | Cl | H | CH2-C(CH3)=CH2 |
| 608 | CF2Cl | Cl | H | CH2-C=CH |
| 609 | CF2Cl | Cl | Me | CH2-C=CH |
| 610 | CF2Cl | Cl | H | CH(CH3)CH2CH3 |
| 611 | CF2Cl | Cl | H | CH(CH3)cPr |
| 612 | CF2Cl | Cl | H | CH(CH3)(CH2)2CH3 |
| 613 | CF2Cl | Cl | H | CH(CH3)(CH2)4CH3 |
| 614 | CF2Cl | Cl | H | CH(CH3)(CH2)5CH3 |
| 615 | CF2Cl | Cl | H | CH(CH2CH3)(CH2)3CH3 |
| 616 | CF2Cl | Cl | H | CH(CH3)CH2CH(CH3)2 |
| 617 | CF2Cl | Cl | H | CH(CH3)C(CH3)3 |
| 618 | CF2Cl | Cl | H | CH(CH3)CH(CH3)2 |
| 619 | CF2Cl | Cl | H | CH(CH3)CH2CH2CH(CH3)2 |
| 620 | CF2Cl | Cl | H | CH(CH2CH3)2 |
| 621 | CF2Cl | Cl | H | C(CH3)2CH2CH3 |
| 622 | CF2Cl | Cl | H | C(CH3)2CH2C(CH3)3 |
| 623 | CF2Cl | Cl | H | CH2-CH(OMe)2 |
| 624 | CF2Cl | Cl | H | CH2-CH(OEt)2 |
| 625 | CF2Cl | Cl | H | CH2CH2-OH |
| 626 | CF2Cl | Cl | H | CH2CH2-OMe |
| 627 | CF2Cl | Cl | Me | CH2CH2-OMe |
| 628 | CF2Cl | Cl | H | CH2CH2-OEt |
| 629 | CF2Cl | Cl | H | CH2CH2-SMe |
| 630 | CF2Cl | Cl | H | CH2CH2-CN |
| 631 | CF2Cl | Cl | H | CH2CH2-NMe2 |
| 632 | CF2Cl | Cl | H | CH2CH2-Morpholin-4-yl |
| 633 | CF2Cl | Cl | H | CH(CH3)CH2-OMe |
| 634 | CF2Cl | Cl | H | CH(CH3)CH2-NMe2 |
| 635 | CF2Cl | Cl | H | CH2CH2CH2-OMe |
| 636 | CF2Cl | Cl | H | CH2CH2CH2-SMe |
| 637 | CF2Cl | Cl | H | CH2CH2CH2-OEt |
| 638 | CF2Cl | Cl | H | CH2CH2CH2-OiPr |
| 639 | CF2Cl | Cl | H | CH2CH2CH2-OBu |
| 640 | CF2Cl | Cl | H | CH2-COOCH3 |
| 641 | CF2Cl | Cl | Me | CH2-COOCH3 |
| 642 | CF2Cl | Cl | H | CH(CH3)COOMe |
| 643 | CF2Cl | Cl | H | CH(CH3)COOEt |
| 644 | CF2Cl | Cl | H | CH2CH2-COOCH3 |
| 645 | CF2Cl | Cl | H | CH(COOCH3)2 |
| 646 | CF2Cl | Cl | H | CH(COOEt)CH2-CH(CH3)2 |
| 647 | CF2Cl | Cl | H | CH(COOMe)CH(CH3)2 |
| 648 | CF2Cl | Cl | H | O-CH2CH3 |
| 649 | CF2Cl | Cl | H | O-CH3 |
| 650 | CF2Cl | Cl | H | O-CH2CH=CH2 |
| 651 | CF2Cl | Cl | H | O-tBu |
| 652 | CF2Cl | Cl | H | O-Pr |
| 653 | CF2Cl | Cl | H | O-CH2cPr |
| 654 | CF2Cl | Cl | H | O-CH2CH(CH3)2 |
| 655 | CF2Cl | Cl | H | O-CH2CF3 |
| 656 | CF2Cl | Cl | H | O-CH(CH3)cPr |
| 657 | CF2Cl | Cl | H | O-CH2CH2Cl |
| 658 | CF2Cl | Cl | H | O-CH2C≡CH |
| 659 | CF2Cl | Cl | H | O-CH2C≡CCH3 |
| 660 | CF2Cl | Cl | H | O-CH(CH3)C≡CH |
| 661 | CF2Cl | Cl | H | CH2-Ph |
| 662 | CF2Cl | Cl | Me | CH2-Ph |
| 663 | CF2Cl | Cl | H | CH2-Pyridin-3-yl |
| 664 | CF2Cl | Cl | H | CH2-6-Cl-Pyridin-3-yl |
| 665 | CF2Cl | Cl | H | CH(CH3)Ph |
| 666 | CF2Cl | Cl | H | CH2CH2-Ph |
| 667 | CF2Cl | Cl | H | CH2-2-CF3-Ph |
| 668 | CF2Cl | Cl | H | CH2CH2CHPh |
| 669 | CF2Cl | Cl | Morpholin-4-yl | |
| 670 | CF2Cl | Cl | Piperidin-1-yl | |
| 671 | CF2Cl | Cl | Thiazolidin-3-yl | |
| 672 | CF2Cl | Cl | Pyrrolidin-1-yl | |
| 673 | CF2Cl | Cl | 2-Methyl-pyrrolidin-1-yl | |
| 674 | CF2Cl | Cl | =CH-N(CH3)2 | |
| 675 | CF2Cl | Cl | =C(CH3)N(CH3)2 | |
| 676 | CF2Cl | Cl | =CH-N(C2H5)2 | |
| 677 | CF2Cl | Cl | =C(CH3)N(C2H5)2 | |
| 678 | CF2Cl | Cl | =CH-Piperidin | |
| 679 | CF2Cl | Cl | =CH-Morpholin | |
| 680 | CF2Cl | Cl | =CH-Pyrrolidin | |
| 681 | CF2Cl | Cl | H | Indan-1-yl |
| 682 | CF2Cl | Cl | H | Tetrahydrofuran-2-ylmethyl |
| 683 | CHF2 | Cl | H | H |
| 684 | CHF2 | Cl | H | Me |
| 685 | CHF2 | Cl | H | Et |
| 686 | CHF2 | Cl | H | Pr |
| 687 | CHF2 | Cl | H | iPr |
| 688 | CHF2 | Cl | H | cPr |
| 689 | CHF2 | Cl | H | Bu |
| 690 | CHF2 | Cl | H | cBu |
| 691 | CHF2 | Cl | H | tBu |
| 692 | CHF2 | Cl | Me | Me |
| 693 | CHF2 | Cl | Me | Et |
| 694 | CHF2 | Cl | Me | Bu |
| 695 | CHF2 | Cl | Me | Pr |
| 696 | CHF2 | Cl | Me | iPr |
| 697 | CHF2 | Cl | Et | Et |
| 698 | CHF2 | Cl | Et | Pr |
| 699 | CHF2 | Cl | Et | iPr |
| 700 | CHF2 | Cl | Pr | Pr |
| 701 | CHF2 | Cl | H | cPentyl |
| 702 | CHF2 | Cl | H | cHexyl |
| 703 | CHF2 | Cl | H | CH2(CH2)3CH3 |
| 704 | CHF2 | Cl | H | CH2(CH2)4CH3 |
| 705 | CHF2 | Cl | H | CH2-cPr |
| 706 | CHF2 | Cl | H | CH2-CN |
| 707 | CHF2 | Cl | H | CH2-C(CH3)3 |
| 708 | CHF2 | Cl | H | CH2CF2CF3 |
| 709 | CHF2 | Cl | H | CH2CF3 |
| 710 | CHF2 | Cl | H | CH2(CF2)2CF3 |
| 711 | CHF2 | Cl | H | CH2CH(CH3)CH2CH3 |
| 712 | CHF2 | Cl | H | CH2C(CH3)2CH2F |
| 713 | CHF2 | Cl | H | CH2CH(CH3)2 |
| 714 | CHF2 | Cl | H | CH2CH(CH2CH3)2 |
| 715 | CHF2 | Cl | H | CH2CH2CH(CH3)2 |
| 716 | CHF2 | Cl | H | CH2CH2C(CH3)3 |
| 717 | CHF2 | Cl | H | CH2CH=CH2 |
| 718 | CHF2 | Cl | Me | CH2CH=CH2 |
| 719 | CHF2 | Cl | CH2CH=CH2 | CH2CH=CH2 |
| 720 | CHF2 | Cl | H | CH2CH=CHCH3 |
| 721 | CHF2 | Cl | H | CH2-C(CH3)=CH2 |
| 722 | CHF2 | Cl | H | CH2-C≡CH |
| 723 | CHF2 | Cl | Me | CH2-C≡CH |
| 724 | CHF2 | Cl | H | CH(CH3)CH2CH3 |
| 725 | CHF2 | Cl | H | CH(CH3)cPr |
| 726 | CHF2 | Cl | H | CH(CH3)(CH2)2CH3 |
| 727 | CHF2 | Cl | H | CH(CH3)(CH2)4CH3 |
| 728 | CHF2 | Cl | H | CH(CH3)(CH2)5CH3 |
| 729 | CHF2 | Cl | H | CH(CH2CH3)(CH2)3CH3 |
| 730 | CHF2 | Cl | H | CH(CH3)CH2CH(CH3)2 |
| 731 | CHF2 | Cl | H | CH(CH3)C(CH3)3 |
| 732 | CHF2 | Cl | H | CH(CH3)CH(CH3)2 |
| 733 | CHF2 | Cl | H | CH(CH3)CH2CH2CH(CH3)2 |
| 734 | CHF2 | Cl | H | CH(CH2CH3)2 |
| 735 | CHF2 | Cl | H | C(CH3)2CH2CH3 |
| 736 | CHF2 | Cl | H | C(CH3)2CH2C(CH3)3 |
| 737 | CHF2 | Cl | H | CH2-CH(OMe)2 |
| 738 | CHF2 | Cl | H | CH2-CH(OEt)2 |
| 739 | CHF2 | Cl | H | CH2CH2-OH |
| 740 | CHF2 | Cl | H | CH2CH2-OMe |
| 741 | CHF2 | Cl | Me | CH2CH2-OMe |
| 742 | CHF2 | Cl | H | CH2CH2-OEt |
| 743 | CHF2 | Cl | H | CH2CH2-SMe |
| 744 | CHF2 | Cl | H | CH2CH2-CN |
| 745 | CHF2 | Cl | H | CH2CH2-NMe2 |
| 746 | CHF2 | Cl | H | CH2CH2-Morpholin-4-yl |
| 747 | CHF2 | Cl | H | CH(CH3)CH2-OMe |
| 748 | CHF2 | Cl | H | CH(CH3)CH2-NMe2 |
| 749 | CHF2 | Cl | H | CH2CH2CH2-OMe |
| 750 | CHF2 | Cl | H | CH2CH2CH2-SMe |
| 751 | CHF2 | Cl | H | CH2CH2CH2-OEt |
| 752 | CHF2 | Cl | H | CH2CH2CH2-OiPr |
| 753 | CHF2 | Cl | H | CH2CH2CH2-OBu |
| 754 | CHF2 | Cl | H | CH2-COOCH3 |
| 755 | CHF2 | Cl | Me | CH2-COOCH3 |
| 756 | CHF2 | Cl | H | CH(CH3)COOMe |
| 757 | CHF2 | Cl | H | CH(CH3)COOEt |
| 758 | CHF2 | Cl | H | CH2CH2-COOCH3 |
| 759 | CHF2 | Cl | H | CH(COOCH3)2 |
| 760 | CHF2 | Cl | H | CH(COOEt)CH2-CH(CH3)2 |
| 761 | CHF2 | Cl | H | CH(COOMe)CH(CH3)2 |
| 762 | CHF2 | Cl | H | O-CH2CH3 |
| 763 | CHF2 | Cl | H | O-CH3 |
| 764 | CHF2 | Cl | H | O-CH2CH=CH2 |
| 765 | CHF2 | Cl | H | O-tBu |
| 766 | CHF2 | Cl | H | O-Pr |
| 767 | CHF2 | Cl | H | O-CH2cPr |
| 768 | CHF2 | Cl | H | O-CH2CH(CH3)2 |
| 769 | CHF2 | Cl | H | O-CH2CF3 |
| 770 | CHF2 | Cl | H | O-CH(CH3)cPr |
| 771 | CHF2 | Cl | H | O-CH2CH2Cl |
| 772 | CHF2 | Cl | H | O-CH2C≡CH |
| 773 | CHF2 | Cl | H | O-CH2C≡CCH3 |
| 774 | CHF2 | Cl | H | O-CH(CH3)C≡CH |
| 775 | CHF2 | Cl | H | CH2-Ph |
| 776 | CHF2 | Cl | Me | CH2-Ph |
| 777 | CHF2 | Cl | H | CH2-Pyridin-3-yl |
| 778 | CHF2 | Cl | H | CH2-6-Cl-Pyridin-3-yl |
| 779 | CHF2 | Cl | H | CH(CH3)Ph |
| 780 | CHF2 | Cl | H | CH2CH2-Ph |
| 781 | CHF2 | Cl | H | CH2-2-CF3-Ph |
| 782 | CHF2 | Cl | H | CH2CH2CHPh |
| 783 | CHF2 | Cl | Morpholin-4-yl | |
| 784 | CHF2 | Cl | Piperidin-1-yl | |
| 785 | CHF2 | Cl | Thiazolidin-3-yl | |
| 786 | CHF2 | Cl | Pyrrolidin-1-yl | |
| 787 | CHF2 | Cl | 2-Methyl-pyrrolidin-1-yl | |
| 788 | CHF2 | Cl | =CH-N(CH3)2 | |
| 789 | CHF2 | Cl | =C(CH3)N(CH3)2 | |
| 790 | CHF2 | Cl | =CH-N(C2H5)2 | |
| 791 | CHF2 | Cl | =C(CH3)N(C2H5)2 | |
| 792 | CHF2 | Cl | =CH-Piperidin | |
| 793 | CHF2 | Cl | =CH-Morpholin | |
| 794 | CHF2 | Cl | =CH-Pyrrolidin | |
| 795 | CHF2 | Cl | H | Indan-1-yl |
| 796 | CHF2 | Cl | H | Tetrahydrofuran-2-ylmethyl |
| 797 | CF2CF3 | Cl | H | H |
| 798 | CF2CF3 | Cl | H | Me |
| 799 | CF2CF3 | Cl | H | Et |
| 800 | CF2CF3 | Cl | H | Pr |
| 801 | CF2CF3 | Cl | H | iPr |
| 802 | CF2CF3 | Cl | H | cPr |
| 803 | CF2CF3 | Cl | H | Bu |
| 804 | CF2CF3 | Cl | H | cBu |
| 805 | CF2CF3 | Cl | H | tBu |
| 806 | CF2CF3 | Cl | Me | Me |
| 807 | CF2CF3 | Cl | Me | Et |
| 808 | CF2CF3 | Cl | Me | Bu |
| 809 | CF2CF3 | Cl | Me | Pr |
| 810 | CF2CF3 | Cl | Me | iPr |
| 811 | CF2CF3 | Cl | Et | Et |
| 812 | CF2CF3 | Cl | Et | Pr |
| 813 | CF2CF3 | Cl | Et | iPr |
| 814 | CF2CF3 | Cl | Pr | Pr |
| 815 | CF2CF3 | Cl | H | cPentyl |
| 816 | CF2CF3 | Cl | H | cHexyl |
| 817 | CF2CF3 | Cl | H | CH2(CH2)3CH3 |
| 818 | CF2CF3 | Cl | H | CH2(CH2)4CH3 |
| 819 | CF2CF3 | Cl | H | CH2-cPr |
| 820 | CF2CF3 | Cl | H | CH2-CN |
| 821 | CF2CF3 | Cl | H | CH2-C(CH3)3 |
| 822 | CF2CF3 | Cl | H | CH2CF2CF3 |
| 823 | CF2CF3 | Cl | H | CH2CF3 |
| 824 | CF2CF3 | Cl | H | CH2(CF2)2CF3 |
| 825 | CF2CF3 | Cl | H | CH2CH(CH3)CH2CH3 |
| 826 | CF2CF3 | Cl | H | CH2C(CH3)2CH2F |
| 827 | CF2CF3 | Cl | H | CH2CH(CH3)2 |
| 828 | CF2CF3 | Cl | H | CH2CH(CH2CH3)2 |
| 829 | CF2CF3 | Cl | H | CH2CH2CH(CH3)2 |
| 830 | CF2CF3 | Cl | H | CH2CH2C(CH3)3 |
| 831 | CF2CF3 | Cl | H | CH2CH=CH2 |
| 832 | CF2CF3 | Cl | Me | CH2CH=CH2 |
| 833 | CF2CF3 | Cl | CH2CH=CH2 | CH2CH=CH2 |
| 834 | CF2CF3 | Cl | H | CH2CH=CHCH3 |
| 835 | CF2CF3 | Cl | H | CH2-C(CH3)=CH2 |
| 836 | CF2CF3 | Cl | H | CH2-C=CH |
| 837 | CF2CF3 | Cl | Me | CH2-C=CH |
| 838 | CF2CF3 | Cl | H | CH(CH3)CH2CH3 |
| 839 | CF2CF3 | Cl | H | CH(CH3)cPr |
| 840 | CF2CF3 | Cl | H | CH(CH3)(CH2)2CH3 |
| 841 | CF2CF3 | Cl | H | CH(CH3)(CH2)4CH3 |
| 842 | CF2CF3 | Cl | H | CH(CH3)(CH2)5CH3 |
| 843 | CF2CF3 | Cl | H | CH(CH2CH3)(CH2)3CH3 |
| 844 | CF2CF3 | Cl | H | CH(CH3)CH2CH(CH3)2 |
| 845 | CF2CF3 | Cl | H | CH(CH3)C(CH3)3 |
| 846 | CF2CF3 | Cl | H | CH(CH3)CH(CH3)2 |
| 847 | CF2CF3 | Cl | H | CH(CH3)CH2CH2CH(CH3)2 |
| 848 | CF2CF3 | Cl | H | CH(CH2CH3)2 |
| 849 | CF2CF3 | Cl | H | C(CH3)2CH2CH3 |
| 850 | CF2CF3 | Cl | H | C(CH3)2CH2C(CH3)3 |
| 851 | CF2CF3 | Cl | H | CH2-CH(OMe)2 |
| 852 | CF2CF3 | Cl | H | CH2-CH(OEt)2 |
| 853 | CF2CF3 | Cl | H | CH2CH2-OH |
| 854 | CF2CF3 | Cl | H | CH2CH2-OMe |
| 855 | CF2CF3 | Cl | Me | CH2CH2-OMe |
| 856 | CF2CF3 | Cl | H | CH2CH2-OEt |
| 857 | CF2CF3 | Cl | H | CH2CH2-SMe |
| 858 | CF2CF3 | Cl | H | CH2CH2-CN |
| 859 | CF2CF3 | Cl | H | CH2CH2-NMe2 |
| 860 | CF2CF3 | Cl | H | CH2CH2-Morpholin-4-yl |
| 861 | CF2CF3 | Cl | H | CH(CH3)CH2-OMe |
| 862 | CF2CF3 | Cl | H | CH(CH3)CH2-NMe2 |
| 863 | CF2CF3 | Cl | H | CH2CH2CH2-OMe |
| 864 | CF2CF3 | Cl | H | CH2CH2CH2-SMe |
| 865 | CF2CF3 | Cl | H | CH2CH2CH2-OEt |
| 866 | CF2CF3 | Cl | H | CH2CH2CH2-OiPr |
| 867 | CF2CF3 | Cl | H | CH2CH2CH2-OBu |
| 868 | CF2CF3 | Cl | H | CH2-COOCH3 |
| 869 | CF2CF3 | Cl | Me | CH2-COOCH3 |
| 870 | CF2CF3 | Cl | H | CH2CH2-COOCH3 |
| 871 | CF2CF3 | Cl | H | CH(COOCH3)2 |
| 872 | CF2CF3 | Cl | H | CH(COOEt)CH2-CH(CH3)2 |
| 873 | CF2CF3 | Cl | H | CH(COOMe)CH(CH3)2 |
| 874 | CF2CF3 | Cl | H | O-CH2CH3 |
| 875 | CF2CF3 | Cl | H | O-CH3 |
| 876 | CF2CF3 | Cl | H | O-CH2CH=CH2 |
| 877 | CF2CF3 | Cl | H | O-tBu |
| 878 | CF2CF3 | Cl | H | O-Pr |
| 879 | CF2CF3 | Cl | H | O-CH2cPr |
| 880 | CF2CF3 | Cl | H | O-CH2CH(CH3)2 |
| 881 | CF2CF3 | Cl | H | O-CH2CF3 |
| 882 | CF2CF3 | Cl | H | O-CH(CH3)cPr |
| 883 | CF2CF3 | Cl | H | O-CH2CH2Cl |
| 884 | CF2CF3 | Cl | H | O-CH2C≡CH |
| 885 | CF2CF3 | Cl | H | O-CH2C≡CCH3 |
| 886 | CF2CF3 | Cl | H | O-CH(CH3)C≡CH |
| 887 | CF2CF3 | Cl | H | CH2-Ph |
| 888 | CF2CF3 | Cl | Me | CH2-Ph |
| 889 | CF2CF3 | Cl | H | CH2-Pyridin-3-yl |
| 890 | CF2CF3 | Cl | H | CH2-6-Cl-Pyridin-3-yl |
| 891 | CF2CF3 | Cl | H | CH(CH3)Ph |
| 892 | CF2CF3 | Cl | H | CH2CH2-Ph |
| 893 | CF2CF3 | Cl | H | CH2-2-CF3-Ph |
| 894 | CF2CF3 | Cl | H | CH2CH2CHPh |
| 895 | CF2CF3 | Cl | Morpholin-4-yl | |
| 896 | CF2CF3 | Cl | Piperidin-1-yl | |
| 897 | CF2CF3 | Cl | Thiazolidin-3-yl | |
| 898 | CF2CF3 | Cl | Pyrrolidin-1-yl | |
| 899 | CF2CF3 | Cl | 2-Methyl-pyrrolidin-1-yl | |
| 900 | CF2CF3 | Cl | =CH-N(CH3)2 | |
| 901 | CF2CF3 | Cl | =C(CH3)N(CH3)2 | |
| 902 | CF2CF3 | Cl | =CH-N(C2H5)2 | |
| 903 | CF2CF3 | Cl | =C(CH3)N(C2H5)2 | |
| 904 | CF2CF3 | Cl | =CH-Piperidin | |
| 905 | CF2CF3 | Cl | =CH-Morpholin | |
| 906 | CF2CF3 | Cl | =CH-Pyrrolidin | |
| 907 | CF2CF3 | Cl | H | Indan-1-yl |
| 908 | CF2CF3 | Cl | H | Tetrahydrofuran-2-ylmethyl |
| 909 | CF3 | Br | H | H |
| 910 | CF3 | Br | H | Me |
| 911 | CF3 | Br | H | Et |
| 912 | CF3 | Br | H | Pr |
| 913 | CF3 | Br | H | iPr |
| 914 | CF3 | Br | H | cPr |
| 915 | CF3 | Br | H | Bu |
| 916 | CF3 | Br | H | cBu |
| 917 | CF3 | Br | H | tBu |
| 918 | CF3 | Br | Me | Me |
| 919 | CF3 | Br | Me | Et |
| 920 | CF3 | Br | Me | Bu |
| 921 | CF3 | Br | Me | Pr |
| 922 | CF3 | Br | Me | iPr |
| 923 | CF3 | Br | Et | Et |
| 924 | CF3 | Br | Et | Pr |
| 925 | CF3 | Br | Et | iPr |
| 926 | CF3 | Br | Pr | Pr |
| 927 | CF3 | Br | H | cPentyl |
| 928 | CF3 | Br | H | cHexyl |
| 929 | CF3 | Br | H | CH2(CH2)3CH3 |
| 930 | CF3 | Br | H | CH2(CH2)4CH3 |
| 931 | CF3 | Br | H | CH2-cPr |
| 932 | CF3 | Br | H | CH2-CN |
| 933 | CF3 | Br | H | CH2-C(CH3)3 |
| 934 | CF3 | Br | H | CH2CF2CF3 |
| 935 | CF3 | Br | H | CH2CF3 |
| 936 | CF3 | Br | H | CH2(CF2)2CF3 |
| 937 | CF3 | Br | H | CH2CH(CH3)CH2CH3 |
| 938 | CF3 | Br | H | CH2C(CH3)2CH2F |
| 939 | CF3 | Br | H | CH2CH(CH3)2 |
| 940 | CF3 | Br | H | CH2CH(CH2CH3)2 |
| 941 | CF3 | Br | H | CH2CH2CH(CH3)2 |
| 942 | CF3 | Br | H | CH2CH2C(CH3)3 |
| 943 | CF3 | Br | H | CH2CH=CH2 |
| 944 | CF3 | Br | Me | CH2CH=CH2 |
| 945 | CF3 | Br | CH2CH=CH2 | CH2CH=CH2 |
| 946 | CF3 | Br | H | CH2CH=CHCH3 |
| 947 | CF3 | Br | H | CH2-C(CH3)=CH2 |
| 948 | CF3 | Br | H | CH2-C≡CH |
| 949 | CF3 | Br | Me | CH2-C≡CH |
| 950 | CF3 | Br | H | CH(CH3)CH2CH3 |
| 951 | CF3 | Br | H | CH(CH3)cPr |
| 952 | CF3 | Br | H | CH(CH3)(CH2)2CH3 |
| 953 | CF3 | Br | H | CH(CH3)(CH2)4CH3 |
| 954 | CF3 | Br | H | CH(CH3)(CH2)5CH3 |
| 955 | CF3 | Br | H | CH(CH2CH3)(CH2)3CH3 |
| 956 | CF3 | Br | H | CH(CH3)CH2CH(CH3)2 |
| 957 | CF3 | Br | H | CH(CH3)C(CH3)3 |
| 958 | CF3 | Br | H | CH(CH3)CH(CH3)2 |
| 959 | CF3 | Br | H | CH(CH3)CH2CH2CH(CH3)2 |
| 960 | CF3 | Br | H | CH(CH2CH3)2 |
| 961 | CF3 | Br | H | C(CH3)2CH2CH3 |
| 962 | CF3 | Br | H | C(CH3)2CH2C(CH3)3 |
| 963 | CF3 | Br | H | CH2-CH(OMe)2 |
| 964 | CF3 | Br | H | CH2-CH(OEt)2 |
| 965 | CF3 | Br | H | CH2CH2-OH |
| 966 | CF3 | Br | H | CH2CH2-OMe |
| 967 | CF3 | Br | Me | CH2CH2-OMe |
| 968 | CF3 | Br | H | CH2CH2-OEt |
| 969 | CF3 | Br | H | CH2CH2-SMe |
| 970 | CF3 | Br | H | CH2CH2-CN |
| 971 | CF3 | Br | H | CH2CH2-NMe2 |
| 972 | CF3 | Br | H | CH2CH2-Morpholin-4-yl |
| 973 | CF3 | Br | H | CH(CH3)CH2-OMe |
| 974 | CF3 | Br | H | CH(CH3)CH2-NMe2 |
| 975 | CF3 | Br | H | CH2CH2CH2-OMe |
| 976 | CF3 | Br | H | CH2CH2CH2-SMe |
| 977 | CF3 | Br | H | CH2CH2CH2-OEt |
| 978 | CF3 | Br | H | CH2CH2CH2-OiPr |
| 979 | CF3 | Br | H | CH2CH2CH2-OBu |
| 980 | CF3 | Br | H | CH2-COOCH3 |
| 981 | CF3 | Br | Me | CH2-COOCH3 |
| 982 | CF3 | Br | H | CH(CH3)COOMe |
| 983 | CF3 | Br | H | CH(CH3)COOEt |
| 984 | CF3 | Br | H | CH2CH2-COOCH3 |
| 985 | CF3 | Br | H | CH(COOCH3)2 |
| 986 | CF3 | Br | H | CH(COOEt)CH2-CH(CH3)2 |
| 987 | CF3 | Br | H | CH(COOMe)CH(CH3)2 |
| 988 | CF3 | Br | H | O-CH2CH3 |
| 989 | CF3 | Br | Me | O-CH3 |
| 990 | CF3 | Br | H | O-CH2CH=CH2 |
| 991 | CF3 | Br | H | O-tBu |
| 992 | CF3 | Br | H | O-Pr |
| 993 | CF3 | Br | H | O-CH2cPr |
| 994 | CF3 | Br | H | O-CH2CH(CH3)2 |
| 995 | CF3 | Br | H | O-CH2CF3 |
| 996 | CF3 | Br | H | O-CH(CH3)cPr |
| 997 | CF3 | Br | H | O-CH2CH2Cl |
| 998 | CF3 | Br | H | O-CH2C≡CH |
| 999 | CF3 | Br | H | O-CH2C≡CCH3 |
| 1000 | CF3 | Br | H | O-CH(CH3)C≡CH |
| 1001 | CF3 | Br | H | CH2-Ph |
| 1002 | CF3 | Br | Me | CH2-Ph |
| 1003 | CF3 | Br | H | CH2-Pyridin-3-yl |
| 1004 | CF3 | Br | H | CH2-6-Cl-Pyridin-3-yl |
| 1005 | CF3 | Br | H | CH(CH3)Ph |
| 1006 | CF3 | Br | H | CH2CH2-Ph |
| 1007 | CF3 | Br | H | CH2-2-CF3-Ph |
| 1008 | CF3 | Br | H | CH2CH2CHPh2 |
| 1009 | CF3 | Br | Morpholin-4-yl | |
| 1010 | CF3 | Br | Piperidin-1-yl | |
| 1011 | CF3 | Br | Thiazolidin-3-yl | |
| 1012 | CF3 | Br | Pyrrolid in-1-yl | |
| 1013 | CF3 | Br | 2-Methyl-pyrrolidin-1-yl | |
| 1014 | CF3 | Br | =CH-N(CH3)2 | |
| 1015 | CF3 | Br | =C(CH3)N(CH3)2 | |
| 1016 | CF3 | Br | =CH-N(C2H5)2 | |
| 1017 | CF3 | Br | =C(CH3)N(C2H5)2 | |
| 1018 | CF3 | Br | =CH-Piperidin | |
| 1019 | CF3 | Br | =CH-Morpholin | |
| 1020 | CF3 | Br | =CH-Pyrrolidin | |
| 1021 | CF3 | Br | H | Indan-1-yl |
| 1022 | CF3 | Br | H | Tetrahydrofuran-2-ylmethyl |
| 1023 | CF2Cl | Br | H | H |
| 1024 | CF2Cl | Br | H | Me |
| 1025 | CF2Cl | Br | H | Et |
| 1026 | CF2Cl | Br | H | Pr |
| 1027 | CF2Cl | Br | H | iPr |
| 1028 | CF2Cl | Br | H | cPr |
| 1029 | CF2Cl | Br | H | Bu |
| 1030 | CF2Cl | Br | H | cBu |
| 1031 | CF2Cl | Br | H | tBu |
| 1032 | CF2Cl | Br | Me | Me |
| 1033 | CF2Cl | Br | Me | Et |
| 1034 | CF2Cl | Br | Me | Bu |
| 1035 | CF2Cl | Br | Me | Pr |
| 1036 | CF2Cl | Br | Me | iPr |
| 1037 | CF2Cl | Br | Et | Et |
| 1038 | CF2Cl | Br | Et | Pr |
| 1039 | CF2Cl | Br | Et | iPr |
| 1040 | CF2Cl | Br | Pr | Pr |
| 1041 | CF2Cl | Br | H | cPentyl |
| 1042 | CF2Cl | Br | H | cHexyl |
| 1043 | CF2Cl | Br | H | CH2(CH2)3CH3 |
| 1044 | CF2Cl | Br | H | CH2(CH2)4CH3 |
| 1045 | CF2Cl | Br | H | CH2-cPr |
| 1046 | CF2Cl | Br | H | CH2-CN |
| 1047 | CF2Cl | Br | H | CH2-C(CH3)3 |
| 1048 | CF2Cl | Br | H | CH2CF2CF3 |
| 1049 | CF2Cl | Br | H | CH2CF3 |
| 1050 | CF2Cl | Br | H | CH2(CF2)2CF3 |
| 1051 | CF2Cl | Br | H | CH2CH(CH3)CH2CH3 |
| 1052 | CF2Cl | Br | H | CH2C(CH3)2CH2F |
| 1053 | CF2Cl | Br | H | CH2CH(CH3)2 |
| 1054 | CF2Cl | Br | H | CH2CH(CH2CH3)2 |
| 1055 | CF2Cl | Br | H | CH2CH2CH(CH3)2 |
| 1056 | CF2Cl | Br | H | CH2CH2C(CH3)3 |
| 1057 | CF2Cl | Br | H | CH2CH=CH2 |
| 1058 | CF2Cl | Br | Me | CH2CH=CH2 |
| 1059 | CF2Cl | Br | CH2CH=CH2 | CH2CH=CH2 |
| 1060 | CF2Cl | Br | H | CH2CH=CHCH3 |
| 1061 | CF2Cl | Br | H | CH2-C(CH3)=CH2 |
| 1062 | CF2Cl | Br | H | CH2-C≡CH |
| 1063 | CF2Cl | Br | Me | CH2-C=CH |
| 1064 | CF2Cl | Br | H | CH(CH3)CH2CH3 |
| 1065 | CF2Cl | Br | H | CH(CH3)cPr |
| 1066 | CF2Cl | Br | H | CH(CH3)(CH2)2CH3 |
| 1067 | CF2Cl | Br | H | CH(CH3)(CH2)4CH3 |
| 1068 | CF2Cl | Br | H | CH(CH3)(CH2)5CH3 |
| 1069 | CF2Cl | Br | H | CH(CH2CH3)(CH2)3CH3 |
| 1070 | CF2Cl | Br | H | CH(CH3)CH2CH(CH3)2 |
| 1071 | CF2Cl | Br | H | CH(CH3)C(CH3)3 |
| 1072 | CF2Cl | Br | H | CH(CH3)CH(CH3)2 |
| 1073 | CF2Cl | Br | H | CH(CH3)CH2CH2CH(CH3)2 |
| 1074 | CF2Cl | Br | H | CH(CH2CH3)2 |
| 1075 | CF2Cl | Br | H | C(CH3)2CH2CH3 |
| 1076 | CF2Cl | Br | H | C(CH3)2CH2C(CH3)3 |
| 1077 | CF2Cl | Br | H | CH2-CH(OMe)2 |
| 1078 | CF2Cl | Br | H | CH2-CH(OEt)2 |
| 1079 | CF2Cl | Br | H | CH2CH2-OH |
| 1080 | CF2Cl | Br | H | CH2CH2-OMe |
| 1081 | CF2Cl | Br | Me | CH2CH2-OMe |
| 1082 | CF2Cl | Br | H | CH2CH2-OEt |
| 1083 | CF2Cl | Br | H | CH2CH2-SMe |
| 1084 | CF2Cl | Br | H | CH2CH2-CN |
| 1085 | CF2Cl | Br | H | CH2CH2-NMe2 |
| 1086 | CF2Cl | Br | H | CH2CH2-Morpholin-4-yl |
| 1087 | CF2Cl | Br | H | CH(CH3)CH2-OMe |
| 1088 | CF2Cl | Br | H | CH(CH3)CH2-NMe2 |
| 1089 | CF2Cl | Br | H | CH2CH2CH2-OMe |
| 1090 | CF2Cl | Br | H | CH2CH2CH2-SMe |
| 1091 | CF2Cl | Br | H | CH2CH2CH2-OEt |
| 1092 | CF2Cl | Br | H | CH2CH2CH2-OiPr |
| 1093 | CF2Cl | Br | H | CH2CH2CH2-OBu |
| 1094 | CF2Cl | Br | H | CH2-COOCH3 |
| 1095 | CF2Cl | Br | Me | CH2-COOCH3 |
| 1096 | CF2Cl | Br | H | CH(CH3)COOMe |
| 1097 | CF2Cl | Br | H | CH(CH3)COOEt |
| 1098 | CF2Cl | Br | H | CH2CH2-COOCH3 |
| 1099 | CF2Cl | Br | H | CH(COOCH3)2 |
| 1100 | CF2Cl | Br | H | CH(COOEt)CH2-CH(CH3)2 |
| 1101 | CF2Cl | Br | H | CH(COOMe)CH(CH3)2 |
| 1102 | CF2Cl | Br | H | O-CH2CH3 |
| 1103 | CF2Cl | Br | Me | O-CH3 |
| 1104 | CF2Cl | Br | H | O-CH2CH=CH2 |
| 1105 | CF2Cl | Br | H | O-tBu |
| 1106 | CF2Cl | Br | H | O-Pr |
| 1107 | CF2Cl | Br | H | O-CH2cPr |
| 1108 | CF2Cl | Br | H | O-CH2CH(CH3)2 |
| 1109 | CF2Cl | Br | H | O-CH2CF3 |
| 1110 | CF2Cl | Br | H | O-CH(CH3)cPr |
| 1111 | CF2Cl | Br | H | O-CH2CH2Cl |
| 1112 | CF2Cl | Br | H | O-CH2C≡CH |
| 1113 | CF2Cl | Br | H | O-CH2C≡CCH3 |
| 1114 | CF2Cl | Br | H | O-CH(CH3)C≡CH |
| 1115 | CF2Cl | Br | H | CH2-Ph |
| 1116 | CF2Cl | Br | Me | CH2-Ph |
| 1117 | CF2Cl | Br | H | CH2-Pyridin-3-yl |
| 1118 | CF2Cl | Br | H | CH2-6-Cl-Pyridin-3-yl |
| 1119 | CF2Cl | Br | H | CH(CH3)Ph |
| 1120 | CF2Cl | Br | H | CH2CH2-Ph |
| 1121 | CF2Cl | Br | H | CH2-2-CF3-Ph |
| 1122 | CF2Cl | Br | H | CH2CH2CHPh2 |
| 1123 | CF2Cl | Br | Morpholin-4-yl | |
| 1124 | CF2Cl | Br | Piperidin-1-yl | |
| 1125 | CF2Cl | Br | Thiazolidin-3-yl | |
| 1126 | CF2Cl | Br | Pyrrolidin-1-yl | |
| 1127 | CF2Cl | Br | 2-Methyl-pyrrolidin-1-yl | |
| 1128 | CF2Cl | Br | =CH-N(CH3)2 | |
| 1129 | CF2Cl | Br | =C(CH3)N(CH3)2 | |
| 1130 | CF2Cl | Br | =CH-N(C2H5)2 | |
| 1131 | CF2Cl | Br | =C(CH3)N(C2H5)2 | |
| 1132 | CF2Cl | Br | =CH-Piperidin | |
| 1133 | CF2Cl | Br | =CH-Morpholin | |
| 1134 | CF2Cl | Br | =CH-Pyrrolidin | |
| 1135 | CF2Cl | Br | H | Indan-1-yl |
| 1136 | CF2Cl | Br | H | Tetrahydrofuran-2-ylmethyl |
| 1137 | CHF2 | Br | H | H |
| 1138 | CHF2 | Br | H | Me |
| 1139 | CHF2 | Br | H | Et |
| 1140 | CHF2 | Br | H | Pr |
| 1141 | CHF2 | Br | H | iPr |
| 1142 | CHF2 | Br | H | cPr |
| 1143 | CHF2 | Br | H | Bu |
| 1144 | CHF2 | Br | H | cBu |
| 1145 | CHF2 | Br | H | tBu |
| 1146 | CHF2 | Br | Me | Me |
| 1147 | CHF2 | Br | Me | Et |
| 1148 | CHF2 | Br | Me | Bu |
| 1149 | CHF2 | Br | Me | Pr |
| 1150 | CHF2 | Br | Me | iPr |
| 1151 | CHF2 | Br | Et | Et |
| 1152 | CHF2 | Br | Et | Pr |
| 1153 | CHF2 | Br | Et | iPr |
| 1154 | CHF2 | Br | Pr | Pr |
| 1155 | CHF2 | Br | H | cPentyl |
| 1156 | CHF2 | Br | H | cHexyl |
| 1157 | CHF2 | Br | H | CH2(CH2)3CH3 |
| 1158 | CHF2 | Br | H | CH2(CH2)4CH3 |
| 1159 | CHF2 | Br | H | CH2-cPr |
| 1160 | CHF2 | Br | H | CH2-CN |
| 1161 | CHF2 | Br | H | CH2-C(CH3)3 |
| 1162 | CHF2 | Br | H | CH2CF2CF3 |
| 1163 | CHF2 | Br | H | CH2CF3 |
| 1164 | CHF2 | Br | H | CH2(CF2)2CF3 |
| 1165 | CHF2 | Br | H | CH2CH(CH3)CH2CH3 |
| 1166 | CHF2 | Br | H | CH2C(CH3)2CH2F |
| 1167 | CHF2 | Br | H | CH2CH(CH3)2 |
| 1168 | CHF2 | Br | H | CH2CH(CH2CH3)2 |
| 1169 | CHF2 | Br | H | CH2CH2CH(CH3)2 |
| 1170 | CHF2 | Br | H | CH2CH2C(CH3)3 |
| 1171 | CHF2 | Br | H | CH2CH=CH2 |
| 1172 | CHF2 | Br | Me | CH2CH=CH2 |
| 1173 | CHF2 | Br | CH2CH=CH2 | CH2CH=CH2 |
| 1174 | CHF2 | Br | H | CH2CH=CHCH3 |
| 1175 | CHF2 | Br | H | CH2-C(CH3)=CH2 |
| 1176 | CHF2 | Br | H | CH2-C≡CH |
| 1177 | CHF2 | Br | Me | CH2-C≡CH |
| 1178 | CHF2 | Br | H | CH(CH3)CH2CH3 |
| 1179 | CHF2 | Br | H | CH(CH3)cPr |
| 1180 | CHF2 | Br | H | CH(CH3)(CH2)2CH3 |
| 1181 | CHF2 | Br | H | CH(CH3)(CH2)4CH3 |
| 1182 | CHF2 | Br | H | CH(CH3)(CH2)5CH3 |
| 1183 | CHF2 | Br | H | CH(CH2CH3)(CH2)3CH3 |
| 1184 | CHF2 | Br | H | CH(CH3)CH2CH(CH3)2 |
| 1185 | CHF2 | Br | H | CH(CH3)C(CH3)3 |
| 1186 | CHF2 | Br | H | CH(CH3)CH(CH3)2 |
| 1187 | CHF2 | Br | H | CH(CH3)CH2CH2CH(CH3)2 |
| 1188 | CHF2 | Br | H | CH(CH2CH3)2 |
| 1189 | CHF2 | Br | H | C(CH3)2CH2CH3 |
| 1190 | CHF2 | Br | H | C(CH3)2CH2C(CH3)3 |
| 1191 | CHF2 | Br | H | CH2-CH(OMe)2 |
| 1192 | CHF2 | Br | H | CH2-CH(OEt)2 |
| 1193 | CHF2 | Br | H | CH2CH2-OH |
| 1194 | CHF2 | Br | H | CH2CH2-OMe |
| 1195 | CHF2 | Br | Me | CH2CH2-OMe |
| 1196 | CHF2 | Br | H | CH2CH2-OEt |
| 1197 | CHF2 | Br | H | CH2CH2-SMe |
| 1198 | CHF2 | Br | H | CH2CH2-CN |
| 1199 | CHF2 | Br | H | CH2CH2-NMe2 |
| 1200 | CHF2 | Br | H | CH2CH2-Morpholin-4-yl |
| 1201 | CHF2 | Br | H | CH(CH3)CH2-OMe |
| 1202 | CHF2 | Br | H | CH(CH3)CH2-NMe2 |
| 1203 | CHF2 | Br | H | CH2CH2CH2-OMe |
| 1204 | CHF2 | Br | H | CH2CH2CH2-SMe |
| 1205 | CHF2 | Br | H | CH2CH2CH2-OEt |
| 1206 | CHF2 | Br | H | CH2CH2CH2-OiPr |
| 1207 | CHF2 | Br | H | CH2CH2CH2-OBu |
| 1208 | CHF2 | Br | H | CH2-COOCH3 |
| 1209 | CHF2 | Br | Me | CH2-COOCH3 |
| 1210 | CHF2 | Br | H | CH(CH3)COOMe |
| 1211 | CHF2 | Br | H | CH(CH3)COOEt |
| 1212 | CHF2 | Br | H | CH2CH2-COOCH3 |
| 1213 | CHF2 | Br | H | CH(COOCH3)2 |
| 1214 | CHF2 | Br | H | CH(COOEt)CH2-CH(CH3)2 |
| 1215 | CHF2 | Br | H | CH(COOMe)CH(CH3)2 |
| 1216 | CHF2 | Br | H | O-CH2CH3 |
| 1217 | CHF2 | Br | Me | O-CH3 |
| 1218 | CHF2 | Br | H | O-CH2CH=CH2 |
| 1219 | CHF2 | Br | H | O-tBu |
| 1220 | CHF2 | Br | H | O-Pr |
| 1221 | CHF2 | Br | H | O-CH2cPr |
| 1222 | CHF2 | Br | H | O-CH2CH(CH3)2 |
| 1223 | CHF2 | Br | H | O-CH2CF3 |
| 1224 | CHF2 | Br | H | O-CH(CH3)cPr |
| 1225 | CHF2 | Br | H | O-CH2CH2Cl |
| 1226 | CHF2 | Br | H | O-CH2C≡CH |
| 1227 | CHF2 | Br | H | O-CH2C≡CCH3 |
| 1228 | CHF2 | Br | H | O-CH(CH3)C≡CH |
| 1229 | CHF2 | Br | H | CH2-Ph |
| 1230 | CHF2 | Br | Me | CH2-Ph |
| 1231 | CHF2 | Br | H | CH2-Pyridin-3-yl |
| 1232 | CHF2 | Br | H | CH2-6-Cl-Pyridin-3-yl |
| 1233 | CHF2 | Br | H | CH(CH3)Ph |
| 1234 | CHF2 | Br | H | CH2CH2-Ph |
| 1235 | CHF2 | Br | H | CH2-2-CF3-Ph |
| 1236 | CHF2 | Br | H | CH2CH2CHPh2 |
| 1237 | CHF2 | Br | Morpholin-4-yl | |
| 1238 | CHF2 | Br | Piperidin-1-yl | |
| 1239 | CHF2 | Br | Thiazolidin-3-yl | |
| 1240 | CHF2 | Br | Pyrrolidin-1-yl | |
| 1241 | CHF2 | Br | 2-Meihyl-pyrrolidin-1-yl | |
| 1242 | CHF2 | Br | =CH-N(CH3)2 | |
| 1243 | CHF2 | Br | =C(CH3)N(CH3)2 | |
| 1244 | CHF2 | Br | =CH-N(C2H5)2 | |
| 1245 | CHF2 | Br | =C(CH3)N(C2H5)2 | |
| 1246 | CHF2 | Br | =CH-Piperidin | |
| 1247 | CHF2 | Br | =CH-Morpholin | |
| 1248 | CHF2 | Br | =CH-Pyrrolidin | |
| 1249 | CHF2 | Br | H | Indan-1-yl |
| 1250 | CHF2 | Br | H | Tetrahydrofuran-2-ylmethyl |
| 1251 | CF2CF3 | Br | H | H |
| 1252 | CF2CF3 | Br | H | Me |
| 1253 | CF2CF3 | Br | H | Et |
| 1254 | CF2CF3 | Br | H | Pr |
| 1255 | CF2CF3 | Br | H | iPr |
| 1256 | CF2CF3 | Br | H | cPr |
| 1257 | CF2CF3 | Br | H | Bu |
| 1258 | CF2CF3 | Br | H | cBu |
| 1259 | CF2CF3 | Br | H | tBu |
| 1260 | CF2CF3 | Br | Me | Me |
| 1261 | CF2CF3 | Br | Me | Et |
| 1262 | CF2CF3 | Br | Me | Bu |
| 1263 | CF2CF3 | Br | Me | Pr |
| 1264 | CF2CF3 | Br | Me | iPr |
| 1265 | CF2CF3 | Br | Et | Et |
| 1266 | CF2CF3 | Br | Et | Pr |
| 1267 | CF2CF3 | Br | Et | iPr |
| 1268 | CF2CF3 | Br | Pr | Pr |
| 1269 | CF2CF3 | Br | H | cPentyl |
| 1270 | CF2CF3 | Br | H | cHexyl |
| 1271 | CF2CF3 | Br | H | CH2(CH2)3CH3 |
| 1272 | CF2CF3 | Br | H | CH2(CH2)4CH3 |
| 1273 | CF2CF3 | Br | H | CH2-cPr |
| 1274 | CF2CF3 | Br | H | CH2-CN |
| 1275 | CF2CF3 | Br | H | CH2-C(CH3)3 |
| 1276 | CF2CF3 | Br | H | CH2CF2CF3 |
| 1277 | CF2CF3 | Br | H | CH2CF3 |
| 1278 | CF2CF3 | Br | H | CH2(CF2)2CF3 |
| 1279 | CF2CF3 | Br | H | CH2CH(CH3)CH2CH3 |
| 1280 | CF2CF3 | Br | H | CH2C(CH3)2CH2F |
| 1281 | CF2CF3 | Br | H | CH2CH(CH3)2 |
| 1282 | CF2CF3 | Br | H | CH2CH(CH2CH3)2 |
| 1283 | CF2CF3 | Br | H | CH2CH2CH(CH3)2 |
| 1284 | CF2CF3 | Br | H | CH2CH2C(CH3)3 |
| 1285 | CF2CF3 | Br | H | CH2CH=CH2 |
| 1286 | CF2CF3 | Br | Me | CH2CH=CH2 |
| 1287 | CF2CF3 | Br | CH2CH=CH2 | CH2CH=CH2 |
| 1288 | CF2CF3 | Br | H | CH2CH=CHCH3 |
| 1289 | CF2CF3 | Br | H | CH2-C(CH3)=CH2 |
| 1290 | CF2CF3 | Br | H | CH2-C≡CH |
| 1291 | CF2CF3 | Br | Me | CH2-C≡CH |
| 1292 | CF2CF3 | Br | H | CH(CH3)CH2CH3 |
| 1293 | CF2CF3 | Br | H | CH(CH3)cPr |
| 1294 | CF2CF3 | Br | H | CH(CH3)(CH2)2CH3 |
| 1295 | CF2CF3 | Br | H | CH(CH3)(CH2)4CH3 |
| 1296 | CF2CF3 | Br | H | CH(CH3)(CH2)5CH3 |
| 1297 | CF2CF3 | Br | H | CH(CH2CH3)(CH2)3CH3 |
| 1298 | CF2CF3 | Br | H | CH(CH3)CH2CH(CH3)2 |
| 1299 | CF2CF3 | Br | H | CH(CH3)C(CH3)3 |
| 1300 | CF2CF3 | Br | H | CH(CH3)CH(CH3)2 |
| 1301 | CF2CF3 | Br | H | CH(CH3)CH2CH2CH(CH3)2 |
| 1302 | CF2CF3 | Br | H | CH(CH2CH3)2 |
| 1303 | CF2CF3 | Br | H | C(CH3)2CH2CH3 |
| 1304 | CF2CF3 | Br | H | C(CH3)2CH2C(CH3)3 |
| 1305 | CF2CF3 | Br | H | CH2-CH(OMe)2 |
| 1306 | CF2CF3 | Br | H | CH2-CH(OEt)2 |
| 1307 | CF2CF3 | Br | H | CH2CH2-OH |
| 1308 | CF2CF3 | Br | H | CH2CH2-OMe |
| 1309 | CF2CF3 | Br | Me | CH2CH2-OMe |
| 1310 | CF2CF3 | Br | H | CH2CH2-OEt |
| 1311 | CF2CF3 | Br | H | CH2CH2-SMe |
| 1312 | CF2CF3 | Br | H | CH2CH2-CN |
| 1313 | CF2CF3 | Br | H | CH2CH2-NMe2 |
| 1314 | CF2CF3 | Br | H | CH2CH2-Morpholin-4-yl |
| 1315 | CF2CF3 | Br | H | CH(CH3)CH2-OMe |
| 1316 | CF2CF3 | Br | H | CH(CH3)CH2-NMe2 |
| 1317 | CF2CF3 | Br | H | CH2CH2CH2-OMe |
| 1318 | CF2CF3 | Br | H | CH2CH2CH2-SMe |
| 1319 | CF2CF3 | Br | H | CH2CH2CH2-OEt |
| 1320 | CF2CF3 | Br | H | CH2CH2CH2-OiPr |
| 1321 | CF2CF3 | Br | H | CH2CH2CH2-OBu |
| 1322 | CF2CF3 | Br | H | CH2-COOCH3 |
| 1323 | CF2CF3 | Br | Me | CH2-COOCH3 |
| 1324 | CF2CF3 | Br | H | CH(CH3)COOMe |
| 1325 | CF2CF3 | Br | H | CH(CH3)COOEt |
| 1326 | CF2CF3 | Br | H | CH2CH2-COOCH3 |
| 1327 | CF2CF3 | Br | H | CH(COOCH3)2 |
| 1328 | CF2CF3 | Br | H | CH(COOEt)CH2-CH(CH3)2 |
| 1329 | CF2CF3 | Br | H | CH(COOMe)CH(CH3)2 |
| 1330 | CF2CF3 | Br | H | O-CH2CH3 |
| 1331 | CF2CF3 | Br | Me | O-CH3 |
| 1332 | CF2CF3 | Br | H | O-CH2CH=CH2 |
| 1333 | CF2CF3 | Br | H | O-tBu |
| 1334 | CF2CF3 | Br | H | O-Pr |
| 1335 | CF2CF3 | Br | H | O-CH2cPr |
| 1336 | CF2CF3 | Br | H | O-CH2CH(CH3)2 |
| 1337 | CF2CF3 | Br | H | O-CH2CF3 |
| 1338 | CF2CF3 | Br | H | O-CH(CH3)cPr |
| 1339 | CF2CF3 | Br | H | O-CH2CH2Cl |
| 1340 | CF2CF3 | Br | H | O-CH2C≡CH |
| 1341 | CF2CF3 | Br | H | O-CH2C≡CCH3 |
| 1342 | CF2CF3 | Br | H | O-CH(CH3)C≡CH |
| 1343 | CF2CF3 | Br | H | CH2-Ph |
| 1344 | CF2CF3 | Br | Me | CH2-Ph |
| 1345 | CF2CF3 | Br | H | CH2-Pyridin-3-yl |
| 1346 | CF2CF3 | Br | H | CH2-6-Cl-Pyridin-3-yl |
| 1347 | CF2CF3 | Br | H | CH(CH3)Ph |
| 1348 | CF2CF3 | Br | H | CH2CH2-Ph |
| 1349 | CF2CF3 | Br | H | CH2-2-CF3-Ph |
| 1350 | CF2CF3 | Br | H | CH2CH2CHPh2 |
| 1351 | CF2CF3 | Br | Morpholin-4-yl | |
| 1352 | CF2CF3 | Br | Piperidin-1-yl | |
| 1353 | CF2CF3 | Br | Thiazolidin-3-yl | |
| 1354 | CF2CF3 | Br | Pyrrolidin-1-yl | |
| 1355 | CF2CF3 | Br | 2-Methyl-pyrrolidin-1-yl | |
| 1356 | CF2CF3 | Br | =CH-N(CH3)2 | |
| 1357 | CF2CF3 | Br | =C(CH3)N(CH3)2 | |
| 1358 | CF2CF3 | Br | =CH-N(C2H5)2 | |
| 1359 | CF2CF3 | Br | =C(CH3)N(C2H5)2 | |
| 1360 | CF2CF3 | Br | =CH-Piperidin | |
| 1361 | CF2CF3 | Br | =CH-Morpholin | |
| 1362 | CF2CF3 | Br | =CH-Pyrrolidin | |
| 1363 | CF2CF3 | Br | H | Indan-1-yl |
| 1364 | CF2CF3 | Br | H | Tetrahydrofuran-2-ylmethyl |
| 1365 | CF2CF3 | I | H | H |
| 1366 | CF3 | I | H | H |
| 1367 | CF2CHF2 | H | H | H |
| 1368 | CF2CHF2 | H | H | Me |
| 1369 | CF2CHF2 | H | H | Et |
| 1370 | CF2CHF2 | H | H | Pr |
| 1371 | CF2CHF2 | H | H | iPr |
| 1372 | CF2CHF2 | H | H | cPr |
| 1373 | CF2CHF2 | H | H | Bu |
| 1374 | CF2CHF2 | H | H | cBu |
| 1375 | CF2CHF2 | H | H | tBu |
| 1376 | CF2CHF2 | H | Me | Me |
| 1377 | CF2CHF2 | H | Me | Et |
| 1378 | CF2CHF2 | H | Me | Bu |
| 1379 | CF2CHF2 | H | Me | Pr |
| 1380 | CF2CHF2 | H | Me | iPr |
| 1381 | CF2CHF2 | H | Et | Et |
| 1382 | CF2CHF2 | H | Et | Pr |
| 1383 | CF2CHF2 | H | Et | iPr |
| 1384 | CF2CHF2 | H | Pr | Pr |
| 1385 | CF2CHF2 | H | H | cPentyl |
| 1386 | CF2CHF2 | H | H | cHexyl |
| 1387 | CF2CHF2 | H | H | CH2(CH2)3CH3 |
| 1388 | CF2CHF2 | H | H | CH2(CH2)4CH3 |
| 1389 | CF2CHF2 | H | H | CH2-cPr |
| 1390 | CF2CHF2 | H | H | CH2-CN |
| 1391 | CF2CHF2 | H | H | CH2-C(CH3)3 |
| 1392 | CF2CHF2 | H | H | CH2CF2CF3 |
| 1393 | CF2CHF2 | H | H | CH2CF3 |
| 1394 | CF2CHF2 | H | H | CH2(CF2)2CF3 |
| 1395 | CF2CHF2 | H | H | CH2CH(CH3)CH2CH3 |
| 1396 | CF2CHF2 | H | H | CH2C(CH3)2CH2F |
| 1397 | CF2CHF2 | H | H | CH2CH(CH3)2 |
| 1398 | CF2CHF2 | H | H | CH2CH(CH2CH3)2 |
| 1399 | CF2CHF2 | H | H | CH2CH2CH(CH3)2 |
| 1400 | CF2CHF2 | H | H | CH2CH2C(CH3)3 |
| 1401 | CF2CHF2 | H | H | CH2CH=CH2 |
| 1402 | CF2CHF2 | H | Me | CH2CH=CH2 |
| 1403 | CF2CHF2 | H | CH2CH=CH2 | CH2CH=CH2 |
| 1404 | CF2CHF2 | H | H | CH2CH=CHCH3 |
| 1405 | CF2CHF2 | H | H | CH2-C(CH3)=CH2 |
| 1406 | CF2CHF2 | H | H | CH2-C≡CH |
| 1407 | CF2CHF2 | H | Me | CH2-C≡CH |
| 1408 | CF2CHF2 | H | H | CH(CH3)CH2CH3 |
| 1409 | CF2CHF2 | H | H | CH(CH3)cPr |
| 1410 | CF2CHF2 | H | H | CH(CH3)(CH2)2CH3 |
| 1411 | CF2CHF2 | H | H | CH(CH3)(CH2)4CH3 |
| 1412 | CF2CHF2 | H | H | CH(CH3)(CH2)5CH3 |
| 1413 | CF2CHF2 | H | H | CH(CH2CH3)(CH2)3CH3 |
| 1414 | CF2CHF2 | H | H | CH(CH3)CH2CH(CH3)2 |
| 1415 | CF2CHF2 | H | H | CH(CH3)C(CH3)3 |
| 1416 | CF2CHF2 | H | H | CH(CH3)CH(CH3)2 |
| 1417 | CF2CHF2 | H | H | CH(CH3)CH2CH2CH(CH3)2 |
| 1418 | CF2CHF2 | H | H | CH(CH2CH3)2 |
| 1419 | CF2CHF2 | H | H | C(CH3)2CH2CH3 |
| 1420 | CF2CHF2 | H | H | C(CH3)2CH2C(CH3)3 |
| 1421 | CF2CHF2 | H | H | CH2-CH(OMe)2 |
| 1422 | CF2CHF2 | H | H | CH2-CH(OEt)2 |
| 1423 | CF2CHF2 | H | H | CH2CH2-OH |
| 1424 | CF2CHF2 | H | H | CH2CH2-OMe |
| 1425 | CF2CHF2 | H | Me | CH2CH2-OMe |
| 1426 | CF2CHF2 | H | H | CH2CH2-OEt |
| 1427 | CF2CHF2 | H | H | CH2CH2-SMe |
| 1428 | CF2CHF2 | H | H | CH2CH2-CN |
| 1429 | CF2CHF2 | H | H | CH2CH2-NMe2 |
| 1430 | CF2CHF2 | H | H | CH2CH2-Morpholin-4-yl |
| 1431 | CF2CHF2 | H | H | CH(CH3)CH2-OMe |
| 1432 | CF2CHF2 | H | H | CH(CH3)CH2-NMe2 |
| 1433 | CF2CHF2 | H | H | CH2CH2CH2-OMe |
| 1434 | CF2CHF2 | H | H | CH2CH2CH2-SMe |
| 1435 | CF2CHF2 | H | H | CH2CH2CH2-OEt |
| 1436 | CF2CHF2 | H | H | CH2CH2CH2-OiPr |
| 1437 | CF2CHF2 | H | H | CH2CH2CH2-OBu |
| 1438 | CF2CHF2 | H | H | CH2-COOCH3 |
| 1439 | CF2CHF2 | H | Me | CH2-COOCH3 |
| 1440 | CF2CHF2 | H | H | CH(CH3)COOMe |
| 1441 | CF2CHF2 | H | H | CH(CH3)COOEt |
| 1442 | CF2CHF2 | H | H | CH2CH2-COOCH3 |
| 1443 | CF2CHF2 | H | H | CH(COOCH3)2 |
| 1444 | CF2CHF2 | H | H | CH(COOEt)CH2-CH(CH3)2 |
| 1445 | CF2CHF2 | H | H | CH(COOMe)CH(CH3)2 |
| 1446 | CF2CHF2 | H | H | O-CH2CH3 |
| 1447 | CF2CHF2 | H | H | O-CH3 |
| 1448 | CF2CHF2 | H | H | O-CH2CH=CH2 |
| 1449 | CF2CHF2 | H | H | O-tBu |
| 1450 | CF2CHF2 | H | H | O-Pr |
| 1451 | CF2CHF2 | H | H | O-CH2cPr |
| 1452 | CF2CHF2 | H | H | O-CH2CH(CH3)2 |
| 1453 | CF2CHF2 | H | H | O-CH2CF3 |
| 1454 | CF2CHF2 | H | H | O-CH(CH3)cPr |
| 1455 | CF2CHF2 | H | H | O-CH2CH2Cl |
| 1456 | CF2CHF2 | H | H | O-CH2C≡CH |
| 1457 | CF2CHF2 | H | H | O-CH2C≡CCH3 |
| 1458 | CF2CHF2 | H | H | O-CH(CH3)C≡CH |
| 1459 | CF2CHF2 | H | H | CH2-Ph |
| 1460 | CF2CHF2 | H | Me | CH2-Ph |
| 1461 | CF2CHF2 | H | H | CH2-Pyridin-3-yl |
| 1462 | CF2CHF2 | H | H | CH2-6-Cl-Pyridin-3-yl |
| 1463 | CF2CHF2 | H | H | CH(CH3)Ph |
| 1464 | CF2CHF2 | H | H | CH2CH2-Ph |
| 1465 | CF2CHF2 | H | H | CH2-2-CF3-Ph |
| 1466 | CF2CHF2 | H | H | CH2CH2CHPh |
| 1467 | CF2CHF2 | H | Morpholin-4-yl | |
| 1468 | CF2CHF2 | H | Piperidin-1-yl | |
| 1469 | CF2CHF2 | H | Thiazolidin-3-yl | |
| 1470 | CF2CHF2 | H | Pyrrolidin-1-yl | |
| 1471 | CF2CHF2 | H | 2-Methyl-pyrrolidin-1-yl | |
| 1472 | CF2CHF2 | H | =CH-N(CH3)2 | |
| 1473 | CF2CHF2 | H | =C(CH3)N(CH3)2 | |
| 1474 | CF2CHF2 | H | =CH-N(C2H5)2 | |
| 1475 | CF2CHF2 | H | =C(CH3)N(C2H5)2 | |
| 1476 | CF2CHF2 | H | =CH-Piperidin | |
| 1477 | CF2CHF2 | H | =CH-Morpholin | |
| 1478 | CF2CHF2 | H | =CH-Pyrrolidin | |
| 1479 | CF2CHF2 | H | H | Indan-1-yl |
| 1480 | CF2CHF2 | H | H | Tetrahydrofuran-2-ylmethyl |
| 1481 | CF2CF2Cl | H | H | H |
| 1482 | CF2CF2Cl | H | H | Me |
| 1483 | CF2CF2Cl | H | H | Et |
| 1484 | CF2CF2Cl | H | H | Pr |
| 1485 | CF2CF2Cl | H | H | iPr |
| 1486 | CF2CF2Cl | H | H | cPr |
| 1487 | CF2CF2Cl | H | H | Bu |
| 1488 | CF2CF2Cl | H | H | cBu |
| 1489 | CF2CF2Cl | H | H | tBu |
| 1490 | CF2CF2Cl | H | Me | Me |
| 1491 | CF2CF2Cl | H | Me | Et |
| 1492 | CF2CF2Cl | H | Me | Bu |
| 1493 | CF2CF2Cl | H | Me | Pr |
| 1494 | CF2CF2Cl | H | Me | iPr |
| 1495 | CF2CF2Cl | H | Et | Et |
| 1496 | CF2CF2Cl | H | Et | Pr |
| 1497 | CF2CF2Cl | H | Et | iPr |
| 1498 | CF2CF2Cl | H | Pr | Pr |
| 1499 | CF2CF2Cl | H | H | cPentyl |
| 1500 | CF2CF2Cl | H | H | cHexyl |
| 1501 | CF2CF2Cl | H | H | CH2(CH2)3CH3 |
| 1502 | CF2CF2Cl | H | H | CH2(CH2)4CH3 |
| 1503 | CF2CF2Cl | H | H | CH2-cPr |
| 1504 | CF2CF2Cl | H | H | CH2-CN |
| 1505 | CF2CF2Cl | H | H | CH2-C(CH3)3 |
| 1506 | CF2CF2Cl | H | H | CH2CF2CF3 |
| 1507 | CF2CF2Cl | H | H | CH2CF3 |
| 1508 | CF2CF2Cl | H | H | CH2(CF2)2CF3 |
| 1509 | CF2CF2Cl | H | H | CH2CH(CH3)CH2CH3 |
| 1510 | CF2CF2Cl | H | H | CH2C(CH3)2CH2F |
| 1511 | CF2CF2Cl | H | H | CH2CH(CH3)2 |
| 1512 | CF2CF2Cl | H | H | CH2CH(CH2CH3)2 |
| 1513 | CF2CF2Cl | H | H | CH2CH2CH(CH3)2 |
| 1514 | CF2CF2Cl | H | H | CH2CH2C(CH3)3 |
| 1515 | CF2CF2Cl | H | H | CH2CH=CH2 |
| 1516 | CF2CF2Cl | H | Me | CH2CH=CH2 |
| 1517 | CF2CF2Cl | H | CH2CH=CH2 | CH2CH=CH2 |
| 1518 | CF2CF2Cl | H | H | CH2CH=CHCH3 |
| 1519 | CF2CF2Cl | H | H | CH2-C(CH3)=CH2 |
| 1520 | CF2CF2Cl | H | H | CH2-C≡CH |
| 1521 | CF2CF2Cl | H | Me | CH2-C=CH |
| 1522 | CF2CF2Cl | H | H | CH(CH3)CH2CH3 |
| 1523 | CF2CF2Cl | H | H | CH(CH3)cPr |
| 1524 | CF2CF2Cl | H | H | CH(CH3)(CH2)2CH3 |
| 1525 | CF2CF2Cl | H | H | CH(CH3)(CH2)4CH3 |
| 1526 | CF2CF2Cl | H | H | CH(CH3)(CH2)5CH3 |
| 1527 | CF2CF2Cl | H | H | CH(CH2CH3)(CH2)3CH3 |
| 1528 | CF2CF2Cl | H | H | CH(CH3)CH2CH(CH3)2 |
| 1529 | CF2CF2Cl | H | H | CH(CH3)C(CH3)3 |
| 1530 | CF2CF2Cl | H | H | CH(CH3)CH(CH3)2 |
| 1531 | CF2CF2Cl | H | H | CH(CH3)CH2CH2CH(CH3)2 |
| 1532 | CF2CF2Cl | H | H | CH(CH2CH3)2 |
| 1533 | CF2CF2Cl | H | H | C(CH3)2CH2CH3 |
| 1534 | CF2CF2Cl | H | H | C(CH3)2CH2C(CH3)3 |
| 1535 | CF2CF2Cl | H | H | CH2-CH(OMe)2 |
| 1536 | CF2CF2Cl | H | H | CH2-CH(OEt)2 |
| 1537 | CF2CF2Cl | H | H | CH2CH2-OH |
| 1538 | CF2CF2Cl | H | H | CH2CH2-OMe |
| 1539 | CF2CF2Cl | H | Me | CH2CH2-OMe |
| 1540 | CF2CF2Cl | H | H | CH2CH2-OEt |
| 1541 | CF2CF2Cl | H | H | CH2CH2-SMe |
| 1542 | CF2CF2Cl | H | H | CH2CH2-CN |
| 1543 | CF2CF2Cl | H | H | CH2CH2-NMe2 |
| 1544 | CF2CF2Cl | H | H | CH2CH2-Morpholin-4-yl |
| 1545 | CF2CF2Cl | H | H | CH(CH3)CH2-OMe |
| 1546 | CF2CF2Cl | H | H | CH(CH3)CH2-NMe2 |
| 1547 | CF2CF2Cl | H | H | CH2CH2CH2-OMe |
| 1548 | CF2CF2Cl | H | H | CH2CH2CH2-SMe |
| 1549 | CF2CF2Cl | H | H | CH2CH2CH2-OEt |
| 1550 | CF2CF2Cl | H | H | CH2CH2CH2-OiPr |
| 1551 | CF2CF2Cl | H | H | CH2CH2CH2-OBu |
| 1552 | CF2CF2Cl | H | H | CH2-COOCH3 |
| 1553 | CF2CF2Cl | H | Me | CH2-COOCH3 |
| 1554 | CF2CF2Cl | H | H | CH(CH3)COOMe |
| 1555 | CF2CF2Cl | H | H | CH(CH3)COOEt |
| 1556 | CF2CF2Cl | H | H | CH2CH2-COOCH3 |
| 1557 | CF2CF2Cl | H | H | CH(COOCH3)2 |
| 1558 | CF2CF2Cl | H | H | CH(COOEt)CH2-CH(CH3)2 |
| 1559 | CF2CF2Cl | H | H | CH(COOMe)CH(CH3)2 |
| 1560 | CF2CF2Cl | H | H | O-CH2CH3 |
| 1561 | CF2CF2Cl | H | H | O-CH3 |
| 1562 | CF2CF2Cl | H | H | O-CH2CH=CH2 |
| 1563 | CF2CF2Cl | H | H | O-tBu |
| 1564 | CF2CF2Cl | H | H | O-Pr |
| 1565 | CF2CF2Cl | H | H | O-CH2cPr |
| 1566 | CF2CF2Cl | H | H | O-CH2CH(CH3)2 |
| 1567 | CF2CF2Cl | H | H | O-CH2CF3 |
| 1568 | CF2CF2Cl | H | H | O-CH(CH3)cPr |
| 1569 | CF2CF2Cl | H | H | O-CH2CH2Cl |
| 1570 | CF2CF2Cl | H | H | O-CH2C≡CH |
| 1571 | CF2CF2Cl | H | H | O-CH2C≡CCH3 |
| 1572 | CF2CF2Cl | H | H | O-CH(CH3)C≡CH |
| 1573 | CF2CF2Cl | H | H | CH2-Ph |
| 1574 | CF2CF2Cl | H | Me | CH2-Ph |
| 1575 | CF2CF2Cl | H | H | CH2-Pyridin-3-yl |
| 1576 | CF2CF2Cl | H | H | CH2-6-Cl-Pyridin-3-yl |
| 1577 | CF2CF2Cl | H | H | CH(CH3)Ph |
| 1578 | CF2CF2Cl | H | H | CH2CH2-Ph |
| 1579 | CF2CF2Cl | H | H | CH2-2-CF3-Ph |
| 1580 | CF2CF2Cl | H | H | CH2CH2CHPh |
| 1581 | CF2CF2Cl | H | Morpholin-4-yl | |
| 1582 | CF2CF2Cl | H | Piperidin-1-yl | |
| 1583 | CF2CF2Cl | H | Thiazolidin-3-yl | |
| 1584 | CF2CF2Cl | H | Pyrrolidin-1-yl | |
| 1585 | CF2CF2Cl | H | 2-Methyl-pyrrolidin-1-yl | |
| 1586 | CF2CF2Cl | H | =CH-N(CH3)2 | |
| 1587 | CF2CF2Cl | H | =C(CH3)N(CH3)2 | |
| 1588 | CF2CF2Cl | H | =CH-N(C2H5)2 | |
| 1589 | CF2CF2Cl | H | =C(CH3)N(C2H5)2 | |
| 1590 | CF2CF2Cl | H | =CH-Piperidin | |
| 1591 | CF2CF2Cl | H | =CH-Morpholin | |
| 1592 | CF2CF2Cl | H | =CH-Pyrrolidin | |
| 1593 | CF2CF2Cl | H | H | Indan-1-yl |
| 1594 | CF2CF2Cl | H | H | Tetrahydrofuran-2-ylmethyl |
| 1595 | C3F7 | H | H | H |
| 1596 | C3F7 | H | H | Me |
| 1597 | C3F7 | H | H | Et |
| 1598 | C3F7 | H | H | Pr |
| 1599 | C3F7 | H | H | iPr |
| 1600 | C3F7 | H | H | cPr |
| 1601 | C3F7 | H | H | Bu |
| 1602 | C3F7 | H | H | cBu |
| 1603 | C3F7 | H | H | tBu |
| 1604 | C3F7 | H | Me | Me |
| 1605 | C3F7 | H | Me | Et |
| 1606 | C3F7 | H | Me | Bu |
| 1607 | C3F7 | H | Me | Pr |
| 1608 | C3F7 | H | Me | iPr |
| 1609 | C3F7 | H | Et | Et |
| 1610 | C3F7 | H | Et | Pr |
| 1611 | C3F7 | H | Et | iPr |
| 1612 | C3F7 | H | Pr | Pr |
| 1613 | C3F7 | H | H | cPentyl |
| 1614 | C3F7 | H | H | cHexyl |
| 1615 | C3F7 | H | H | CH2(CH2)3CH3 |
| 1616 | C3F7 | H | H | CH2(CH2)4CH3 |
| 1617 | C3F7 | H | H | CH2-cPr |
| 1618 | C3F7 | H | H | CH2-CN |
| 1619 | C3F7 | H | H | CH2-C(CH3)3 |
| 1620 | C3F7 | H | H | CH2CF2CF3 |
| 1621 | C3F7 | H | H | CH2CF3 |
| 1622 | C3F7 | H | H | CH2(CF2)2CF3 |
| 1623 | C3F7 | H | H | CH2CH(CH3)CH2CH3 |
| 1624 | C3F7 | H | H | CH2C(CH3)2CH2F |
| 1625 | C3F7 | H | H | CH2CH(CH3)2 |
| 1626 | C3F7 | H | H | CH2CH(CH2CH3)2 |
| 1627 | C3F7 | H | H | CH2CH2CH(CH3)2 |
| 1628 | C3F7 | H | H | CH2CH2C(CH3)3 |
| 1629 | C3F7 | H | H | CH2CH=CH2 |
| 1630 | C3F7 | H | Me | CH2CH=CH2 |
| 1631 | C3F7 | H | CH2CH=CH2 | CH2CH=CH2 |
| 1632 | C3F7 | H | H | CH2CH=CHCH3 |
| 1633 | C3F7 | H | H | CH2-C(CH3)=CH2 |
| 1634 | C3F7 | H | H | CH2-C=CH |
| 1635 | C3F7 | H | Me | CH2-C=CH |
| 1636 | C3F7 | H | H | CH(CH3)CH2CH3 |
| 1637 | C3F7 | H | H | CH(CH3)cPr |
| 1638 | C3F7 | H | H | CH(CH3)(CH2)2CH3 |
| 1639 | C3F7 | H | H | CH(CH3)(CH2)4CH3 |
| 1640 | C3F7 | H | H | CH(CH3)(CH2)5CH3 |
| 1641 | C3F7 | H | H | CH(CH2CH3)(CH2)3CH3 |
| 1642 | C3F7 | H | H | CH(CH3)CH2CH(CH3)2 |
| 1643 | C3F7 | H | H | CH(CH3)C(CH3)3 |
| 1644 | C3F7 | H | H | CH(CH3)CH(CH3)2 |
| 1645 | C3F7 | H | H | CH(CH3)CH2CH2CH(CH3)2 |
| 1646 | C3F7 | H | H | CH(CH2CH3)2 |
| 1647 | C3F7 | H | H | C(CH3)2CH2CH3 |
| 1648 | C3F7 | H | H | C(CH3)2CH2C(CH3)3 |
| 1649 | C3F7 | H | H | CH2-CH(OMe)2 |
| 1650 | C3F7 | H | H | CH2-CH(OEt)2 |
| 1651 | C3F7 | H | H | CH2CH2-OH |
| 1652 | C3F7 | H | H | CH2CH2-OMe |
| 1653 | C3F7 | H | Me | CH2CH2-OMe |
| 1654 | C3F7 | H | H | CH2CH2-OEt |
| 1655 | C3F7 | H | H | CH2CH2-SMe |
| 1656 | C3F7 | H | H | CH2CH2-CN |
| 1657 | C3F7 | H | H | CH2CH2-NMe2 |
| 1658 | C3F7 | H | H | CH2CH2-Morpholin-4-yl |
| 1659 | C3F7 | H | H | CH(CH3)CH2-OMe |
| 1660 | C3F7 | H | H | CH(CH3)CH2-NMe2 |
| 1661 | C3F7 | H | H | CH2CH2CH2-OMe |
| 1662 | C3F7 | H | H | CH2CH2CH2-SMe |
| 1663 | C3F7 | H | H | CH2CH2CH2-OEt |
| 1664 | C3F7 | H | H | CH2CH2CH2-OiPr |
| 1665 | C3F7 | H | H | CH2CH2CH2-OBu |
| 1666 | C3F7 | H | H | CH2-COOCH3 |
| 1667 | C3F7 | H | Me | CH2-COOCH3 |
| 1668 | C3F7 | H | H | CH(CH3)COOMe |
| 1669 | C3F7 | H | H | CH(CH3)COOEt |
| 1670 | C3F7 | H | H | CH2CH2-COOCH3 |
| 1671 | C3F7 | H | H | CH(COOCH3)2 |
| 1672 | C3F7 | H | H | CH(COOEt)CH2-CH(CH3)2 |
| 1673 | C3F7 | H | H | CH(COOMe)CH(CH3)2 |
| 1674 | C3F7 | H | H | O-CH2CH3 |
| 1675 | C3F7 | H | H | O-CH3 |
| 1676 | C3F7 | H | H | O-CH2CH=CH2 |
| 1677 | C3F7 | H | H | O-tBu |
| 1678 | C3F7 | H | H | O-Pr |
| 1679 | C3F7 | H | H | O-CH2cPr |
| 1680 | C3F7 | H | H | O-CH2CH(CH3)2 |
| 1681 | C3F7 | H | H | O-CH2CF3 |
| 1682 | C3F7 | H | H | O-CH(CH3)cPr |
| 1683 | C3F7 | H | H | O-CH2CH2Cl |
| 1684 | C3F7 | H | H | O-CH2C≡CH |
| 1685 | C3F7 | H | H | O-CH2C≡CCH3 |
| 1686 | C3F7 | H | H | O-CH(CH3)C≡CH |
| 1687 | C3F7 | H | H | CH2-Ph |
| 1688 | C3F7 | H | Me | CH2-Ph |
| 1689 | C3F7 | H | H | CH2-Pyridin-3-yl |
| 1690 | C3F7 | H | H | CH2-6-Cl-Pyridin-3-yl |
| 1691 | C3F7 | H | H | CH(CH3)Ph |
| 1692 | C3F7 | H | H | CH2CH2-Ph |
| 1693 | C3F7 | H | H | CH2-2-CF3-Ph |
| 1694 | C3F7 | H | H | CH2CH2CHPh |
| 1695 | C3F7 | H | Morpholin-4-yl | |
| 1696 | C3F7 | H | Piperidin-1-yl | |
| 1697 | C3F7 | H | Thiazolidin-3-yl | |
| 1698 | C3F7 | H | Pyrrolidin-1-yl | |
| 1699 | C3F7 | H | 2-Methyl-pyrrolidin-1-yl | |
| 1700 | C3F7 | H | =CH-N(CH3)2 | |
| 1701 | C3F7 | H | =C(CH3)N(CH3)2 | |
| 1702 | C3F7 | H | =CH-N(C2H5)2 | |
| 1703 | C3F7 | H | =C(CH3)N(C2H5)2 | |
| 1704 | C3F7 | H | =CH-Piperidin | |
| 1705 | C3F7 | H | =CH-Morpholin | |
| 1706 | C3F7 | H | =CH-Pyrrolidin | |
| 1707 | C3F7 | H | H | Indan-1-yl |
| 1708 | C3F7 | H | H | Tetrahydrofuran-2-ylmethyl |
| 1709 | CF(CF3)2 | H | H | H |
| 1710 | C3F7 | Cl | H | H |
| 1711 | C3F7 | Cl | H | Me |
| 1712 | C3F7 | Cl | H | Et |
| 1713 | C3F7 | Cl | H | Pr |
| 1714 | C3F7 | Cl | H | iPr |
| 1715 | C3F7 | Cl | H | cPr |
| 1716 | C3F7 | Cl | H | Bu |
| 1717 | C3F7 | Cl | H | cBu |
| 1718 | C3F7 | Cl | H | tBu |
| 1719 | C3F7 | Cl | Me | Me |
| 1720 | C3F7 | Cl | Et | Et |
| 1721 | C3F7 | Cl | H | CH2-cPr |
| 1722 | C3F7 | Cl | H | CH2-C(CH3)3 |
| 1723 | C3F7 | Cl | H | CH2CH(CH3)2 |
| 1724 | C3F7 | Cl | H | CH2CH(CH2CH3)2 |
| 1725 | C3F7 | Cl | H | CH2CH2CH(CH3)2 |
| 1726 | C3F7 | Cl | H | CH2CH2C(CH3)3 |
| 1727 | C3F7 | Cl | H | CH2CH=CH2 |
| 1728 | C3F7 | Cl | H | CH2-C(CH3)=CH2 |
| 1729 | C3F7 | Cl | H | CH2-CCH |
| 1730 | C3F7 | Cl | Me | CH2-CCH |
| 1731 | C3F7 | Cl | H | CH(CH3)CH2CH3 |
| 1732 | C3F7 | Cl | H | CH(CH3)cPr |
| 1733 | C3F7 | Cl | H | CH(CH3)(CH2)2CH3 |
| 1734 | C3F7 | Cl | H | CH(CH3)(CH2)4CH3 |
| 1735 | C3F7 | Cl | H | CH(CH3)CH2CH(CH3)2 |
| 1736 | C3F7 | Cl | H | CH(CH3)C(CH3)3 |
| 1737 | C3F7 | Cl | H | CH(CH3)CH(CH3)2 |
| 1738 | C3F7 | Cl | H | CH(CH2CH3)2 |
| 1739 | C3F7 | Cl | H | CH2-CH(OMe)2 |
| 1740 | C3F7 | Cl | H | CH2-CH(OEt)2 |
| 1741 | C3F7 | Cl | H | CH2CH2-OH |
| 1742 | C3F7 | Cl | H | CH2CH2-OMe |
| 1743 | C3F7 | Cl | Me | CH2CH2-OMe |
| 1744 | C3F7 | Cl | H | CH(CH3)CH2-OMe |
| 1745 | C3F7 | Cl | H | CH2CH2CH2-OMe |
| 1746 | C3F7 | Cl | H | CH2-COOCH3 |
| 1747 | C3F7 | Cl | Me | CH2-COOCH3 |
| 1748 | C3F7 | Cl | H | CH(CH3)COOMe |
| 1749 | C3F7 | Cl | H | CH(CH3)COOEt |
| 1750 | C3F7 | Cl | H | CH2CH2-COOCH3 |
| 1751 | C3F7 | Br | H | H |
| 1752 | C3F7 | Br | H | Me |
| 1753 | C3F7 | Br | H | Et |
| 1754 | C3F7 | Br | H | Pr |
| 1755 | C3F7 | Br | H | iPr |
| 1756 | C3F7 | Br | H | cPr |
| 1757 | C3F7 | Br | H | Bu |
| 1758 | C3F7 | Br | H | cBu |
| 1759 | C3F7 | Br | H | tBu |
| 1760 | C3F7 | Br | Me | Me |
| 1761 | C3F7 | Br | Et | Et |
| 1762 | C3F7 | Br | H | CH2-cPr |
| 1763 | C3F7 | Br | H | CH2-C(CH3)3 |
| 1764 | C3F7 | Br | H | CH2CH(CH3)2 |
| 1765 | C3F7 | Br | H | CH2CH(CH2CH3)2 |
| 1766 | C3F7 | Br | H | CH2CH2CH(CH3)2 |
| 1767 | C3F7 | Br | H | CH2CH2C(CH3)3 |
| 1768 | C3F7 | Br | H | CH2CH=CH2 |
| 1769 | C3F7 | Br | H | CH2-C(CH3)=CH2 |
| 1770 | C3F7 | Br | H | CH2-CCH |
| 1771 | C3F7 | Br | Me | CH2-CCH |
| 1772 | C3F7 | Br | H | CH(CH3)CH2CH3 |
| 1773 | C3F7 | Br | H | CH(CH3)cPr |
| 1774 | C3F7 | Br | H | CH(CH3)(CH2)2CH3 |
| 1775 | C3F7 | Br | H | CH(CH3)(CH2)4CH3 |
| 1776 | C3F7 | Br | H | CH(CH3)CH2CH(CH3)2 |
| 1777 | C3F7 | Br | H | CH(CH3)C(CH3)3 |
| 1778 | C3F7 | Br | H | CH(CH3)CH(CH3)2 |
| 1779 | C3F7 | Br | H | CH(CH3)CH2CH2CH(CH3)2 |
| 1780 | C3F7 | Br | H | CH(CH2CH3)2 |
| 1781 | C3F7 | Br | H | CH2-CH(OMe)2 |
| 1782 | C3F7 | Br | H | CH2-CH(OEt)2 |
| 1783 | C3F7 | Br | H | CH2CH2-OH |
| 1784 | C3F7 | Br | H | CH2CH2-OMe |
| 1785 | C3F7 | Br | H | CH(CH3)CH2-OMe |
| 1786 | C3F7 | Br | H | CH2CH2CH2-OMe |
| 1787 | C3F7 | Br | H | CH2-COOCH3 |
| 1788 | C3F7 | Br | Me | CH2-COOCH3 |
| 1789 | C3F7 | Br | H | CH(CH3)COOMe |
| 1790 | C3F7 | Br | H | CH(CH3)COOEt |
| 1791 | C3F7 | Br | H | CH2CH2-COOCH3 |
| 1792 | C3F7 | I | H | H |
| 1793 | C3F7 | I | H | Me |
| 1794 | C3F7 | I | H | Et |
| 1795 | C3F7 | I | H | Pr |
| 1796 | C3F7 | I | H | iPr |
| 1797 | C3F7 | I | H | cPr |
| 1798 | C3F7 | I | H | Bu |
| 1799 | C3F7 | I | H | cBu |
| 1800 | C3F7 | I | H | tBu |
| 1801 | C3F7 | I | Me | Me |
| 1802 | C3F7 | I | Et | Et |
| 1803 | C3F7 | I | H | CH2-cPr |
| 1804 | C3F7 | I | H | CH2-C(CH3)3 |
| 1805 | C3F7 | I | H | CH2CH(CH3)2 |
| 1806 | C3F7 | I | H | CH2CH(CH2CH3)2 |
| 1807 | C3F7 | I | H | CH2CH2CH(CH3)2 |
| 1808 | C3F7 | I | H | CH2CH2C(CH3)3 |
| 1809 | C3F7 | I | H | CH2CH=CH2 |
| 1810 | C3F7 | I | H | CH2-C(CH3)=CH2 |
| 1811 | C3F7 | I | H | CH2-CCH |
| 1812 | C3F7 | I | Me | CH2-CCH |
| 1813 | C3F7 | I | H | CH(CH3)CH2CH3 |
| 1814 | C3F7 | I | H | CH(CH3)cPr |
| 1815 | C3F7 | I | H | CH(CH3)(CH2)2CH3 |
| 1816 | C3F7 | I | H | CH(CH3)(CH2)4CH3 |
| 1817 | C3F7 | I | H | CH(CH3)CH2CH(CH3)2 |
| 1818 | C3F7 | I | H | CH(CH3)C(CH3)3 |
| 1819 | C3F7 | I | H | CH(CH3)CH(CH3)2 |
| 1820 | C3F7 | I | H | CH(CH2CH3)2 |
| 1821 | C3F7 | I | H | CH2-CH(OMe)2 |
| 1822 | C3F7 | I | H | CH2-CH(OEt)2 |
| 1823 | C3F7 | I | H | CH2CH2-OH |
| 1824 | C3F7 | I | H | CH2CH2-OMe |
| 1825 | C3F7 | I | Me | CH2CH2-OMe |
| 1826 | C3F7 | I | H | CH(CH3)CH2-OMe |
| 1827 | C3F7 | I | H | CH2CH2CH2-OMe |
| 1828 | C3F7 | I | H | CH2-COOCH3 |
| 1829 | C3F7 | I | Me | CH2-COOCH3 |
| 1830 | C3F7 | I | H | CH(CH3)COOMe |
| 1831 | C3F7 | I | H | CH(CH3)COOEt |
| 1832 | C3F7 | I | H | CH2CH2-COOCH3 |
| 1833 | CF3 | I | H | Me |
| 1834 | CF3 | I | H | Et |
| 1835 | CF3 | I | H | Pr |
| 1836 | CF3 | I | H | iPr |
| 1837 | CF3 | I | H | cPr |
| 1838 | CF3 | I | H | Bu |
| 1839 | CF3 | I | H | cBu |
| 1840 | CF3 | I | H | tBu |
| 1841 | CF3 | I | Me | Me |
| 1842 | CF3 | I | Et | Et |
| 1843 | CF3 | I | H | CH2(CH2)3CH3 |
| 1844 | CF3 | I | H | CH2(CH2)4CH3 |
| 1845 | CF3 | I | H | CH2-cPr |
| 1846 | CF3 | I | H | CH2-C(CH3)3 |
| 1847 | CF3 | I | H | CH2CH(CH3)2 |
| 1848 | CF3 | I | H | CH2CH(CH2CH3)2 |
| 1849 | CF3 | I | H | CH2CH2CH(CH3)2 |
| 1850 | CF3 | I | H | CH2CH2C(CH3)3 |
| 1851 | CF3 | I | H | CH2CH=CH2 |
| 1852 | CF3 | I | H | CH2-C(CH3)=CH2 |
| 1853 | CF3 | I | H | CH2-CCH |
| 1854 | CF3 | I | H | CH(CH3)CH2CH3 |
| 1855 | CF3 | I | H | CH(CH3)cPr |
| 1856 | CF3 | I | H | CH(CH3)(CH2)2CH3 |
| 1857 | CF3 | I | H | CH(CH3)(CH2)4CH3 |
| 1858 | CF3 | I | H | CH(CH3)CH2CH(CH3)2 |
| 1859 | CF3 | I | H | CH(CH3)C(CH3)3 |
| 1860 | CF3 | I | H | CH(CH3)CH(CH3)2 |
| 1861 | CF3 | I | H | CH(CH2CH3)2 |
| 1862 | CF3 | I | H | C(CH3)2CH2CH3 |
| 1863 | CF3 | I | H | C(CH3)2CH2C(CH3)3 |
| 1864 | CF3 | I | H | CH2-CH(OMe)2 |
| 1865 | CF3 | I | H | CH2-CH(OEt)2 |
| 1866 | CF3 | I | H | CH2CH2-OH |
| 1867 | CF3 | I | H | CH2CH2-OMe |
| 1868 | CF3 | I | Me | CH2CH2-OMe |
| 1869 | CF3 | I | H | CH(CH3)CH2-OMe |
| 1870 | CF3 | I | H | CH2CH2CH2-OMe |
| 1871 | CF3 | I | H | CH2-COOCH3 |
| 1872 | CF3 | I | Me | CH2-COOCH3 |
| 1873 | CF3 | I | H | CH(CH3)COOMe |
| 1874 | CF3 | I | H | CH(CH3)COOEt |
| 1875 | CF3 | I | H | CH2CH2-COOCH3 |
| 1876 | CF2Cl | I | H | H |
| 1877 | CF2Cl | I | H | Me |
| 1878 | CF2Cl | I | H | Et |
| 1879 | CF2Cl | I | H | Pr |
| 1880 | CF2Cl | I | H | iPr |
| 1881 | CF2Cl | I | H | cPr |
| 1882 | CF2Cl | I | H | Bu |
| 1883 | CF2Cl | I | H | cBu |
| 1884 | CF2Cl | I | H | tBu |
| 1885 | CF2Cl | I | Me | Me |
| 1886 | CF2Cl | I | H | CH2-cPr |
| 1887 | CF2Cl | I | H | CH2-C(CH3)3 |
| 1888 | CF2Cl | I | H | CH2CH(CH3)2 |
| 1889 | CF2Cl | I | H | CH2CH(CH2CH3)2 |
| 1890 | CF2Cl | I | H | CH2CH2CH(CH3)2 |
| 1891 | CF2Cl | I | H | CH2CH2C(CH3)3 |
| 1892 | CF2Cl | I | H | CH2CH=CH2 |
| 1893 | CF2Cl | I | Me | CH2CH=CH2 |
| 1894 | CF2Cl | I | H | CH2-C(CH3)=CH2 |
| 1895 | CF2Cl | I | H | CH2-CCH |
| 1896 | CF2Cl | I | Me | CH2-CCH |
| 1897 | CF2Cl | I | H | CH(CH3)CH2CH3 |
| 1898 | CF2Cl | I | H | CH(CH3)cPr |
| 1899 | CF2Cl | I | H | CH(CH3)(CH2)2CH3 |
| 1900 | CF2Cl | I | H | CH(CH3)(CH2)4CH3 |
| 1901 | CF2Cl | I | H | CH(CH3)(CH2)5CH3 |
| 1902 | CF2Cl | I | H | CH(CH2CH3)(CH2)3CH3 |
| 1903 | CF2Cl | I | H | CH(CH3)CH2CH(CH3)2 |
| 1904 | CF2Cl | I | H | CH(CH3)C(CH3)3 |
| 1905 | CF2Cl | I | H | CH(CH3)CH(CH3)2 |
| 1906 | CF2Cl | I | H | CH(CH3)CH2CH2CH(CH3)2 |
| 1907 | CF2Cl | I | H | CH(CH2CH3)2 |
| 1908 | CF2Cl | I | H | C(CH3)2CH2CH3 |
| 1909 | CF2Cl | I | H | C(CH3)2CH2C(CH3)3 |
| 1910 | CF2Cl | I | H | CH2-CH(OMe)2 |
| 1911 | CF2Cl | I | H | CH2-CH(OEt)2 |
| 1912 | CF2Cl | I | H | CH2CH2-OH |
| 1913 | CF2Cl | I | H | CH2CH2-OMe |
| 1914 | CF2Cl | I | Me | CH2CH2-OMe |
| 1915 | CF2Cl | I | H | CH2CH2-OEt |
| 1916 | CF2Cl | I | H | CH(CH3)CH2-OMe |
| 1917 | CF2Cl | I | H | CH2CH2CH2-OMe |
| 1918 | CF2Cl | I | H | CH2-COOCH3 |
| 1919 | CF2Cl | I | Me | CH2-COOCH3 |
| 1920 | CF2Cl | I | H | CH(CH3)COOMe |
| 1921 | CF2Cl | I | H | CH(CH3)COOEt |
| 1922 | CF2Cl | I | H | CH2CH2-COOCH3 |
| 1923 | CF2CF3 | I | H | Me |
| 1924 | CF2CF3 | I | H | Et |
| 1925 | CF2CF3 | I | H | Pr |
| 1926 | CF2CF3 | I | H | iPr |
| 1927 | CF2CF3 | I | H | cPr |
| 1928 | CF2CF3 | I | H | Bu |
| 1929 | CF2CF3 | I | H | cBu |
| 1930 | CF2CF3 | I | H | tBu |
| 1931 | CF2CF3 | I | Me | Me |
| 1932 | CF2CF3 | I | Et | Et |
| 1933 | CF2CF3 | I | H | CH2-cPr |
| 1934 | CF2CF3 | I | H | CH2-C(CH3)3 |
| 1935 | CF2CF3 | I | H | CH2CH(CH3)2 |
| 1936 | CF2CF3 | I | H | CH2CH(CH2CH3)2 |
| 1937 | CF2CF3 | I | H | CH2CH2CH(CH3)2 |
| 1938 | CF2CF3 | I | H | CH2CH2C(CH3)3 |
| 1939 | CF2CF3 | I | H | CH2CH=CH2 |
| 1940 | CF2CF3 | I | Me | CH2CH=CH2 |
| 1941 | CF2CF3 | I | H | CH2-C(CH3)=CH2 |
| 1942 | CF2CF3 | I | H | CH2-CCH |
| 1943 | CF2CF3 | I | Me | CH2-CCH |
| 1944 | CF2CF3 | I | H | CH(CH3)CH2CH3 |
| 1945 | CF2CF3 | I | H | CH(CH3)cPr |
| 1946 | CF2CF3 | I | H | CH(CH3)(CH2)2CH3 |
| 1947 | CF2CF3 | I | H | CH(CH3)(CH2)4CH3 |
| 1948 | CF2CF3 | I | H | CH(CH3)CH2CH(CH3)2 |
| 1949 | CF2CF3 | I | H | CH(CH3)C(CH3)3 |
| 1950 | CF2CF3 | I | H | CH(CH3)CH(CH3)2 |
| 1951 | CF2CF3 | I | H | CH(CH2CH3)2 |
| 1952 | CF2CF3 | I | H | CH2-CH(OMe)2 |
| 1953 | CF2CF3 | I | H | CH2-CH(OEt)2 |
| 1954 | CF2CF3 | I | H | CH2CH2-OH |
| 1955 | CF2CF3 | I | H | CH2CH2-OMe |
| 1956 | CF2CF3 | I | Me | CH2CH2-OMe |
| 1957 | CF2CF3 | I | H | CH(CH3)CH2-OMe |
| 1958 | CF2CF3 | I | H | CH2CH2CH2-OMe |
| 1959 | CF2CF3 | I | H | CH2-COOCH3 |
| 1960 | CF2CF3 | I | Me | CH2-COOCH3 |
| 1961 | CF2CF3 | I | H | CH(CH3)COOMe |
| 1962 | CF2CF3 | I | H | CH(CH3)COOEt |
| 1963 | CF2CF3 | I | H | CH2CH2-COOCH3 |
| 1964 | CF2CF2Cl | Cl | H | H |
| 1965 | CF2CF2Cl | Cl | H | Me |
| 1966 | CF2CF2Cl | Cl | H | Et |
| 1967 | CF2CF2Cl | Cl | H | Pr |
| 1968 | CF2CF2Cl | Cl | H | iPr |
| 1969 | CF2CF2Cl | Cl | H | cPr |
| 1970 | CF2CF2Cl | Cl | H | Bu |
| 1971 | CF2CF2Cl | Cl | H | cBu |
| 1972 | CF2CF2Cl | Cl | H | tBu |
| 1973 | CF2CF2Cl | Cl | Me | Me |
| 1974 | CF2CF2Cl | Cl | Et | Et |
| 1975 | CF2CF2Cl | Cl | H | CH2-cPr |
| 1976 | CF2CF2Cl | Cl | H | CH2-C(CH3)3 |
| 1977 | CF2CF2Cl | Cl | H | CH2CH(CH3)2 |
| 1978 | CF2CF2Cl | Cl | H | CH2CH(CH2CH3)2 |
| 1979 | CF2CF2Cl | Cl | H | CH2CH2CH(CH3)2 |
| 1980 | CF2CF2Cl | Cl | H | CH2CH2C(CH3)3 |
| 1981 | CF2CF2Cl | Cl | H | CH2CH=CH2 |
| 1982 | CF2CF2Cl | Cl | Me | CH2CH=CH2 |
| 1983 | CF2CF2Cl | Cl | H | CH2-C(CH3)=CH2 |
| 1984 | CF2CF2Cl | Cl | H | CH2-CCH |
| 1985 | CF2CF2Cl | Cl | Me | CH2-CCH |
| 1986 | CF2CF2Cl | Cl | H | CH(CH3)CH2CH3 |
| 1987 | CF2CF2Cl | Cl | H | CH(CH3)cPr |
| 1988 | CF2CF2Cl | Cl | H | CH(CH3)(CH2)2CH3 |
| 1989 | CF2CF2Cl | Cl | H | CH(CH3)(CH2)4CH3 |
| 1990 | CF2CF2Cl | Cl | H | CH(CH3)CH2CH(CH3)2 |
| 1991 | CF2CF2Cl | Cl | H | CH(CH3)C(CH3)3 |
| 1992 | CF2CF2Cl | Cl | H | CH(CH3)CH(CH3)2 |
| 1993 | CF2CF2Cl | Cl | H | CH(CH2CH3)2 |
| 1994 | CF2CF2Cl | Cl | H | CH2-CH(OMe)2 |
| 1995 | CF2CF2Cl | Cl | H | CH2-CH(OEt)2 |
| 1996 | CF2CF2Cl | Cl | H | CH2CH2-OH |
| 1997 | CF2CF2Cl | Cl | H | CH2CH2-OMe |
| 1998 | CF2CF2Cl | Cl | Me | CH2CH2-OMe |
| 1999 | CF2CF2Cl | Cl | H | CH(CH3)CH2-OMe |
| 2000 | CF2CF2Cl | Cl | H | CH2CH2CH2-OMe |
| 2001 | CF2CF2Cl | Cl | H | CH2-COOCH3 |
| 2002 | CF2CF2Cl | Cl | Me | CH2-COOCH3 |
| 2003 | CF2CF2Cl | Cl | H | CH(CH3)COOMe |
| 2004 | CF2CF2Cl | Cl | H | CH(CH3)COOEt |
| 2005 | CF2CF2Cl | Cl | H | CH2CH2-COOCH3 |
| 2006 | CF2CF2Cl | Br | H | H |
| 2007 | CF2CF2Cl | Br | H | Me |
| 2008 | CF2CF2Cl | Br | H | Et |
| 2009 | CF2CF2Cl | Br | H | Pr |
| 2010 | CF2CF2Cl | Br | H | iPr |
| 2011 | CF2CF2Cl | Br | H | cPr |
| 2012 | CF2CF2Cl | Br | H | Bu |
| 2013 | CF2CF2Cl | Br | H | cBu |
| 2014 | CF2CF2Cl | Br | H | tBu |
| 2015 | CF2CF2Cl | Br | Me | Me |
| 2016 | CF2CF2Cl | Br | Et | Et |
| 2017 | CF2CF2Cl | Br | H | CH2-cPr |
| 2018 | CF2CF2Cl | Br | H | CH2-C(CH3)3 |
| 2019 | CF2CF2Cl | Br | H | CH2CH(CH3)2 |
| 2020 | CF2CF2Cl | Br | H | CH2CH(CH2CH3)2 |
| 2021 | CF2CF2Cl | Br | H | CH2CH2CH(CH3)2 |
| 2022 | CF2CF2Cl | Br | H | CH2CH2C(CH3)3 |
| 2023 | CF2CF2Cl | Br | H | CH2CH=CH2 |
| 2024 | CF2CF2Cl | Br | Me | CH2CH=CH₂ |
| 2025 | CF2CF2Cl | Br | H | CH2-C(CH3)=CH2 |
| 2026 | CF2CF2Cl | Br | H | CH2-CCH |
| 2027 | CF2CF2Cl | Br | Me | CH2-CCH |
| 2028 | CF2CF2Cl | Br | H | CH(CH3)CH2CH3 |
| 2029 | CF2CF2Cl | Br | H | CH(CH3)cPr |
| 2030 | CF2CF2Cl | Br | H | CH(CH3)(CH2)2CH3 |
| 2031 | CF2CF2Cl | Br | H | CH(CH3)(CH2)4CH3 |
| 2032 | CF2CF2Cl | Br | H | CH(CH3)CH2CH(CH3)2 |
| 2033 | CF2CF2Cl | Br | H | CH(CH3)C(CH3)3 |
| 2034 | CF2CF2Cl | Br | H | CH(CH3)CH(CH3)2 |
| 2035 | CF2CF2Cl | Br | H | CH(CH2CH3)2 |
| 2036 | CF2CF2Cl | Br | H | CH2-CH(OMe)2 |
| 2037 | CF2CF2Cl | Br | H | CH2-CH(OEt)2 |
| 2038 | CF2CF2Cl | Br | H | CH2CH2-OH |
| 2039 | CF2CF2Cl | Br | H | CH2CH2-OMe |
| 2040 | CF2CF2Cl | Br | Me | CH2CH2-OMe |
| 2041 | CF2CF2Cl | Br | H | CH(CH3)CH2-OMe |
| 2042 | CF2CF2Cl | Br | H | CH2CH2CH2-OMe |
| 2043 | CF2CF2Cl | Br | H | CH2-COOCH3 |
| 2044 | CF2CF2Cl | Br | Me | CH2-COOCH3 |
| 2045 | CF2CF2Cl | Br | H | CH(CH3)COOMe |
| 2046 | CF2CF2Cl | Br | H | CH(CH3)COOEt |
| 2047 | CF2CF2Cl | Br | H | CH2CH2-COOCH3 |
| 2048 | CF2CF2Cl | I | H | H |
| 2049 | CF2CF2Cl | I | H | Me |
| 2050 | CF2CF2Cl | I | H | Et |
| 2051 | CF2CF2Cl | I | H | Pr |
| 2052 | CF2CF2Cl | I | H | iPr |
| 2053 | CF2CF2Cl | I | H | cPr |
| 2054 | CF2CF2Cl | I | H | Bu |
| 2055 | CF2CF2Cl | I | H | cBu |
| 2056 | CF2CF2Cl | I | H | tBu |
| 2057 | CF2CF2Cl | I | Me | Me |
| 2058 | CF2CF2Cl | I | Et | Et |
| 2059 | CF2CF2Cl | I | H | CH2-cPr |
| 2060 | CF2CF2Cl | I | H | CH2-C(CH3)3 |
| 2061 | CF2CF2Cl | I | H | CH2CH(CH3)2 |
| 2062 | CF2CF2Cl | I | H | CH2CH(CH2CH3)2 |
| 2063 | CF2CF2Cl | I | H | CH2CH2CH(CH3)2 |
| 2064 | CF2CF2Cl | I | H | CH2CH2C(CH3)3 |
| 2065 | CF2CF2Cl | I | H | CH2CH=CH2 |
| 2066 | CF2CF2Cl | I | Me | CH2CH=CH2 |
| 2067 | CF2CF2Cl | I | H | CH2-C(CH3)=CH2 |
| 2068 | CF2CF2Cl | I | H | CH2-CCH |
| 2069 | CF2CF2Cl | I | Me | CH2-CCH |
| 2070 | CF2CF2Cl | I | H | CH(CH3)CH2CH3 |
| 2071 | CF2CF2Cl | I | H | CH(CH3)cPr |
| 2072 | CF2CF2Cl | I | H | CH(CH3)(CH2)2CH3 |
| 2073 | CF2CF2Cl | I | H | CH(CH3)(CH2)4CH3 |
| 2074 | CF2CF2Cl | I | H | CH(CH3)CH2CH(CH3)2 |
| 2075 | CF2CF2Cl | I | H | CH(CH3)C(CH3)3 |
| 2076 | CF2CF2Cl | I | H | CH(CH3)CH(CH3)2 |
| 2077 | CF2CF2Cl | I | H | CH(CH2CH3)2 |
| 2078 | CF2CF2Cl | I | H | CH2-CH(OMe)2 |
| 2079 | CF2CF2Cl | I | H | CH2-CH(OEt)2 |
| 2080 | CF2CF2Cl | I | H | CH2CH2-OH |
| 2081 | CF2CF2Cl | I | H | CH2CH2-OMe |
| 2082 | CF2CF2Cl | I | Me | CH2CH2-OMe |
| 2083 | CF2CF2Cl | I | H | CH(CH3)CH2-OMe |
| 2084 | CF2CF2Cl | I | H | CH2CH2CH2-OMe |
| 2085 | CF2CF2Cl | I | H | CH2-COOCH3 |
| 2086 | CF2CF2Cl | I | Me | CH2-COOCH3 |
| 2087 | CF2CF2Cl | I | H | CH(CH3)COOMe |
| 2088 | CF2CF2Cl | I | H | CH(CH3)COOEt |
| 2089 | CF2CF2Cl | I | H | CH2CH2-COOCH3 |
| 2090 | CFClCF3 | H | H | H |
| 2091 | CFCICF3 | H | H | Me |
| 2092 | CFCICF3 | H | H | Et |
| 2093 | CFCICF3 | H | H | Pr |
| 2094 | CFClCF3 | H | H | iPr |
| 2095 | CFClCF3 | H | H | cPr |
| 2096 | CFClCF3 | H | H | Bu |
| 2097 | CFClCF3 | H | H | cBu |
| 2098 | CFClCF3 | H | H | tBu |
| 2099 | CFClCF3 | H | Me | Me |
| 2100 | CFClCF3 | H | Et | Et |
| 2101 | CFClCF3 | H | H | CH2-cPr |
| 2102 | CFClCF3 | H | H | CH2-C(CH3)3 |
| 2103 | CFClCF3 | H | H | CH2CH(CH3)2 |
| 2104 | CFClCF3 | H | H | CH2CH(CH2CH3)2 |
| 2105 | CFClCF3 | H | H | CH2CH2CH(CH3)2 |
| 2106 | CFClCF3 | H | H | CH2CH2C(CH3)3 |
| 2107 | CFClCF3 | H | H | CH2CH=CH2 |
| 2108 | CFClCF3 | H | Me | CH2CH=CH2 |
| 2109 | CFClCF3 | H | H | CH2-C(CH3)=CH2 |
| 2110 | CFClCF3 | H | H | CH2-CCH |
| 2111 | CFClCF3 | H | Me | CH2-CCH |
| 2112 | CFClCF3 | H | H | CH(CH3)CH2CH3 |
| 2113 | CFClCF3 | H | H | CH(CH3)cPr |
| 2114 | CFClCF3 | H | H | CH(CH3)(CH2)2CH3 |
| 2115 | CFClCF3 | H | H | CH(CH3)(CH2)4CH3 |
| 2116 | CFClCF3 | H | H | CH(CH3)CH2CH(CH3)2 |
| 2117 | CFClCF3 | H | H | CH(CH3)C(CH3)3 |
| 2118 | CFClCF3 | H | H | CH(CH3)CH(CH3)2 |
| 2119 | CFClCF3 | H | H | CH(CH2CH3)2 |
| 2120 | CFClCF3 | H | H | CH2-CH(OMe)2 |
| 2121 | CFClCF3 | H | H | CH2-CH(OEt)2 |
| 2122 | CFClCF3 | H | H | CH2CH2-OH |
| 2123 | CFClCF3 | H | H | CH2CH2-OMe |
| 2124 | CFClCF3 | H | Me | CH2CH2-OMe |
| 2125 | CFClCF3 | H | H | CH(CH3)CH2-OMe |
| 2126 | CFClCF3 | H | H | CH2CH2CH2-OMe |
| 2127 | CFClCF3 | H | H | CH2-COOCH3 |
| 2128 | CFClCF3 | H | Me | CH2-COOCH3 |
| 2129 | CFClCF3 | H | H | CH(CH3)COOMe |
| 2130 | CFClCF3 | H | H | CH(CH3)COOEt |
| 2131 | CFClCF3 | H | H | CH2CH2-COOCH3 |
| 2132 | CFClCF3 | Br | H | H |
| 2133 | CFClCF3 | Br | H | Me |
| 2134 | CFClCF3 | Br | H | Et |
| 2135 | CFClCF3 | Br | H | Pr |
| 2136 | CFClCF3 | Br | H | iPr |
| 2137 | CFClCF3 | Br | H | cPr |
| 2138 | CFClCF3 | Br | H | Bu |
| 2139 | CFClCF3 | Br | H | cBu |
| 2140 | CFClCF3 | Br | H | tBu |
| 2141 | CFClCF3 | Br | Me | Me |
| 2142 | CFClCF3 | Br | Et | Et |
| 2143 | CFClCF3 | Br | H | CH2-cPr |
| 2144 | CFClCF3 | Br | H | CH2-C(CH3)3 |
| 2145 | CFClCF3 | Br | H | CH2CH(CH3)2 |
| 2146 | CFClCF3 | Br | H | CH2CH(CH2CH3)2 |
| 2147 | CFClCF3 | Br | H | CH2CH2CH(CH3)2 |
| 2148 | CFClCF3 | Br | H | CH2CH2C(CH3)3 |
| 2149 | CFClCF3 | Br | H | CH2CH=CH2 |
| 2150 | CFClCF3 | Br | Me | CH2CH=CH2 |
| 2151 | CFClCF3 | Br | H | CH2-C(CH3)=CH2 |
| 2152 | CFClCF3 | Br | H | CH2-C=CH |
| 2153 | CFClCF3 | Br | Me | CH2-C=CH |
| 2154 | CFClCF3 | Br | H | CH(CH3)CH2CH3 |
| 2155 | CFClCF3 | Br | H | CH(CH3)cPr |
| 2156 | CFClCF3 | Br | H | CH(CH3)(CH2)2CH3 |
| 2157 | CFClCF3 | Br | H | CH(CH3)(CH2)4CH3 |
| 2158 | CFClCF3 | Br | H | CH(CH3)CH2CH(CH3)2 |
| 2159 | CFClCF3 | Br | H | CH(CH3)C(CH3)3 |
| 2160 | CFClCF3 | Br | H | CH(CH3)CH(CH3)2 |
| 2161 | CFClCF3 | Br | H | CH(CH2CH3)2 |
| 2162 | CFClCF3 | Br | H | CH2-CH(OMe)2 |
| 2163 | CFClCF3 | Br | H | CH2-CH(OEt)2 |
| 2164 | CFClCF3 | Br | H | CH2CH2-OH |
| 2165 | CFClCF3 | Br | H | CH2CH2-OMe |
| 2166 | CFClCF3 | Br | Me | CH2CH2-OMe |
| 2167 | CFClCF3 | Br | H | CH(CH3)CH2-OMe |
| 2168 | CFClCF3 | Br | H | CH2CH2CH2-OMe |
| 2169 | CFClCF3 | Br | H | CH2-COOCH3 |
| 2170 | CFClCF3 | Br | Me | CH2-COOCH3 |
| 2171 | CFClCF3 | Br | H | CH(CH3)COOMe |
| 2172 | CFClCF3 | Br | H | CH(CH3)COOEt |
| 2173 | CFClCF3 | Br | H | CH2CH2-COOCH3 |

**Tabelle 2: NMR-Daten zu Verbindungen aus Tabelle 1**

| Bsp.Nr. | 1H-NMR-Daten |
|---|---|
| 1 | [CDCl3] 3,03 (d, 3H); 6,88 (d, 1H); 8,65 (d, 1H); 9,22 (br, 1H) |
| 2 | [CDCl3] 1,25 (t, 3H); 3,5 (m, 2H); 6,92 (d, 1H); 8,72 (d, 1H), 9,33 (br, 1H), 13,4 (br, 1H) |
| 3 | [CDCl3] 1,02 (t, 3H); 1,62 (m, 2H); 3,42 (m, 2H); 6,90 (d, 1H); 8,70 (d, 1H); 9,35 (br, 1H); 13,3 (br, 1H) |
| 4 | [CDCl3] 1,25 (d, 6H); 4,22 (m, 1H); 6,90 (d, 1H); 8,68 (d, 1H); 9,22 (d, br, 1H) |
| 5 | [CDCl3] 0,61 (m, 2H); 0,89 (m, 2H); 3,00 (m, 1H); 6,90 (d, 1 H); 8,68 (d, 1H), 9,31 (br, 1H) |
| 6 | [CDCl3] 0,96 (t, 3H); 1,41 (m, 2H); 1,61 (m, 2H); 3,48 (q, 2H); 6,90 (d, 1H); 8,70 (d, 1H); 9,31 (br, 1H); 13,3 (br, 1H) |
| 7 | [CDCl3] 1,80 (m, 2H); 2,00 (m, 2H); 2,42 (m, 2H); 4,55 (m, 1H); 6,88 (d, 1H); 8,65 (d, 1H); 9,50 (br, 1H) |
| 8 | [CDCl3] 1,44 (s, 9H); 6,90 (d, 1H); 8,62 (d, 1H); 9,32 (br, 1H); 13, 4 (br, 1H) |
| 9 | [CDCl3] 3,05 (s, 6H); 7,10 (d, 1H); 7,77 (d, 1H) |
| 10 | [CDCl3] 1,22 (t, 3H); 3,02 (s, 3H); 3,46 (m, 2H); 7,08 (d,1H); 7,77 (d, 1H) |
| 11 | [CDCl3] 0,90 (t, 3H); 1,30 (br, 2H); 1,60 (m, 2H); 3,03 (s, 3H); 3,42 (br, 2H); 7,10 (d, 1H); 7,74 (d, 1H) |
| 13 | [CDCl3] 1,20 (d, 6H); 2,90 (s, 3H); 4,05 (m, br, 1H); 7,03 (d, 1H); 8,68 (d, 1H); 11,2 (br) |
| 14 | [CDCl3] 1,20 (t, 6H); 3,40 (m, 4H); 7,05 (d, 1H); 8,70 (d, 1H); 11,1 (br) |
| 16 | [CDCl3] 1,21 (m, 9H); 3,39 (q, 2H); 4,0 (br, 1H); 7,04 (d, 1H); 7,68 (d, 1H); 10,6 (br, 1H) |
| 18 | [CDCl3] 1,5-1,8 (m, 8H); 4,40 (m, 1H); 6,90 (d, 1H); 8,68 (d, 1H); 9,40 (d, br, 1H); 13,5 (br) |
| 19 | [CDCl3] 1,2-1,5 (m, 6H); 1,76 (m, 2H); 2,00 (m, 2H); 4,00 (m, 1H); 6,90 (d, 1H); 8,68 (d, 1H); 9,30 (d,br, 1H); 13,4 (br, 1H) |
| 20 | [CDCl3] 0,90 (t, 3H); 1,38 (m, 4H); 1,61 (m, 2H); 3,41 (m, 2H); 6,86 (d,1H); 8,63 (d, 1H); 9,20 (t, 1H) |
| 22 | [CDCl3] 0,30 (m, 2H); 0,60 (m, 2H); 1,05 (m, 1H); 3,30 (m, 2H); 6,90 (d, 1H), 8,70 (d, 1H); 9,38 (br, 1H) |
| 24 | [CDCl3] 1,00 (s, 9H); 3,28 (d, 2H); 6,90 (d, 1H); 8,73 (d, 1H); 9,4 (br, 1H); 13,2 (br, 1H) |
| 25 | [CDCl3] 4,18 (dt, 2H); 6,92 (d, 1H); 8,72 (d, 1H); 9,8 (t, br, 1H) |
| 26 | [CDCl3] 4,11 (m, 2H); 6,90 (d, 1H); 8,70 (d, 1H); 9,80 (br, 1H); 12,16 (br, 1H) |
| 27 | [CDCl3] 4,22 (dt; 2H); 6,93 (d, 1H); 8,72 (d, 1H); 9,80 (t, br, 1H) |
| 30 | [CDCl3] 1,00 (d, 6H); 1,87 (m, 1H); 3,29 (t, 2H); 6,88 (d, 1H); 8,64 (d, 1H); 9,32 (br, 1H); 12,5 (br, 1H) |
| 32 | [CDCl3] 0,95 (d, 6H); 1,48 (m, 2H); 1,70 (m, 1H); 3,48 (m, 2H); 6,88 (d, 1H); 8,65 (d, 1H); 9,22 (br, 1H), 12,6 (br) |
| 34 | [CDCl3] 4,10 (t, 2H); 5,22 (dd, 2H); 5,95 (m, 1H); 6,90 (d, 1H); 8,70 (d, 1H); 9,40 (br, 1H); 12,7 (br, 1H) |
| 36 | [CDCl3] 4,0 (m, 4H); 5,21 (d, 4H); 5,80 (m, 2H); 7,04 (d, 1H); 7,78 (d, 1H); 11,0 (br) |
| 38 | [CDCl3] 1,80 (s, 3H); 4,02 (d, 2H); 4,93 (d, 2H); 6,89 (d 1H); 8,70 (d, 1H); 9,43 (br, 1H); 13,2 (br, 1H) |
| 39 | [CDCl3] 2,24 (t, 1H); 4,22 (m, 2H); 6,88 (d, 1H); 8,68 (d, 1H); 9,50 (br, 1H) |
| 40 | [CDCl3] 2,30 (s, 1H); 3,12 (s, 3H); 4,22 (br, 2H); 7,04 (d, 1H); 7,84 (d, 1H); 10,9 (br) |
| 41 | [CDCl3] 0,95 (t, 3H); 1,22 (d, 3H); 1,58 (m, 2H); 4,10 (m, 1H); 6,88 (d, 1H); 8,66 (d, 1H); 9,20 (br, 1H); 13,0 (br, 1H) |
| 43 | [DMSO] 0,93 (t, 3H); 1,16 (d, 3H); 1,30 (m, 2H); 1,48 (m, 2H); 4,00 (m, 1H); 7,35 (br, 1H); 7,39 (d, 1H); 8,40 (d, 1H); 13,40 (br) |
| 44 | [DMSO] 0,87 (t, 3H); 1,15 (d, 3H); 1,27 (m, 6H); 1,48 (m, 2H); 4,00 (m, 1H); 7,33 (d, 1H); 8,40 (d, 1H); 8,80 (br, 1H), 13,4 (br, 1H) |
| 47 | [CDCl3] 0,93 (d, 6H); 1,22 (d, 3H); 1,35 (m, 1H), 1,50 (m, 1H); 1,65 (m, 1H); 4,23 (m, 1H); 6,90 (d, 1H); 8,64 (d, 1H); 9,20 (d, br, 1H); 13,3 (br, 1H) |
| 48 | [CDCl3] 0,96 (s, 9H); 1,18 (d, 3H); 4,10 (m, 1H); 6,90 (d, 1H); 8,74 (d, 1H); 9,37 (d, br, 1 H), 13,6 (br, 1H) |
| 49 | [CDCl3] 0,95 (dt, 6H); 1,20 (d, 3H); 1,80 (m, 1H); 4,07 (m, 1H); 6,90 (d, 1H); 8,70 (d, 1H); 9,30 (d, br, 1H); 13,6 (br) |
| 51 | [DMSO] 0,85 (t, 6H); 1,45 (m, 2H); 1,55 (m, 2H); 3,80 (m, 1H); 7,33 (d, br, 1H); 8,38 (d, 1H); 8,65 (br, 1H); 13,5 (br, 1H) |
| 54 | [CDCl3] 3,40 (s, 6H); 3,62 (t, 2H); 4,50 (t, 1H); 6,89 (d, 1H); 8,65 (d, 1H); 9,30 (br, 1H); 12,4 (br, 1H) |
| 55 | [CDCl3] 1,21 (t, 6H); 3,60 (m, 4H); 3,72 (m, 2H); 4,61 (t, 1H); 6,88 (d, 1H); 8,61 (d, 1H); 9,28 (br, 1H), 12,0 (br) |
| 56 | [DMSO] 3,40 (m, 2H); 3,53 (m, 2H); 7,32 (d, br, 1H); 8,45 (d, 1H); 9,05 (br, 1H); 13,6 (br, 1H) |
| 57 | [CDCl3] 3,40 (s, 3H); 3,56 (t, 2H); 3,64 (m, 2H); 6,90 (d, 1H), 8,65 (d, 1H); 9,35 (br, 1H) |
| 64 | [CDCl3] 1,25 (d, 3H); 3,40 (s, 3H); 3,43 (d, 2H); 4,37 (m, 1H); 6,89 (d, 1H); 8,64 (d, 1H); 9,30 (d, br, 1H); 13,0 (br, 1H) |
| 66 | (CDCl3] 1,90 (m, 2H); 3,34 (s, 3H); 3,46 (t, 2H); 3,55 (q, 2H); 6,90 (d, 1H); 8,60 (d, 1H); 9,23 (br, 1H); 12,5 (br) |
| 71 | [CDCl3] 3,80 (s, 3H); 4,22 (d, 2H); 6,88 (d, 1H); 8,64 (d, 1H); 9,62 (t, 1H), |
| 72 | [CDCl3] 3,10 (s, 3H); 3,75 (s, 3H); 4,25 (s, 2H); 7,18 (d, 1H); 7,80 (d, 1H) |
| 75 | [CDCl3] 2,64 (t, 2H); 3,69 (s, 3H); 3,73 (m, 2H); 6,88 (d, 1H); 8,61 (d, 1H); 9,41 (t, br, 1H); 12,2 (br, 1H) |
| 76 | [CDCl3] 3,83 (s, 6H); 5,42 (d, 1H); 6,90 (d, 1H); 8,63 (d, 1H), 10,2 (br, 1H), |
| 77 | [CDCl3] 0,99 (t, 6H); 1,25 (t, 3H)1,75 (m, 3H); 4,20 (m, 2H); 4,72 (m, 1H); 6,90 (d, 1H); 8,64 (d, 1H); 9,60 (d, br, 1H) |
| 78 | [CDCl3] 1,05 (t, 6H); 2,30 (m, 1H); 3,78 (s, 3H); 4,72 (m, 1H); 6,90 (d, 1H); 8,70 (d, 1H); 9,76 (d, br, 1H), 13,6 (br, 1H) |
| 79 | [CDCl3] 1,35 (t, 3H); 4,10 (q, 2H); 6,91 (d, 1H); 8,71 (d, 1H); 11,46 (s, 1H) |
| 80 | (CDCl3] 3,41 (s, 3H); 3,68 (s, 3H); 7,18 (d, 1H); 8,32 (d, 1H) |
| 81 | [CDCl3] 4,50 (d, 2H); 5,3 (m, 2H); 6,0 (m, 1H); 6,90 (d, 1H); 8,70 (d, 1H); 11,43 (s, 1H) |
| 82 | [CDCl3] 1,36 (s, 9H); 6,92 (d, 1H); 8,72 (d, 1H); 11,22 (s, 1H); 13,0 (br, 1H) |
| 92 | [CDCl3] 4,62 (d, 2H), 6,84 (d, 1H); 7,3 (m, 5H); 8,70 (d, 1H); 9,65 (br, 1H), 13,0 (br, 1H) |
| 93 | [CDCl3] 2,95 (s, 3H); 4,65 (br, 2H); 7,05 (d,1H); 7,3 (m, 5H), 7,82 (d, 1H); 11,7 (br) |
| 94 | [DMSO] 4,55 (d, 2H); 7,33 (d, 1H); 7,37 (dd, 1H); 7,75 (dt, 1H); 8,40 (d, 1H); 8,48 (dd, 1H); 8,58 (s, 1H); 9,45 (br, 1H); 13,4 (br, 1H) |
| 95 | [DMSO] 4,55 (d, 2H); 7,30 (d, br, 1H); 7,50 (d, 1H); 7,80 (dd, 1H); 8,37 (m, 2H); 9,55 (br, 1H); 13,4 (br, 1H) |
| 96 | [CDCl3] 1,59 (d, 3H); 5,25 (m, 1H); 6,88 (d, 1H); 7,2-7,4 (m, 5H); 8,68 (d, 1H); 9,75 (d, br, 1H); 13,5 (br, 1H) |
| 97 | [CDCl3] 2,94 (t, 2H); 3,70 (m, 2H); 6,85 (d, 1H); 7,2-7,4 (m, 5H); 8,62 (d, 1H); 9,24 (br, 1H) |
| 98 | [CDCl3] 4,81 (d, 2H); 6,85 (d, 1H); 7,40 (t, 1H); 7,52 (t, 1H); 7,61 (d, 1H); 7,67 (d, 1H); 8,68 (d, 1H); 9,68 (t, br, 1H) |
| 99 | [DMSO] 2,28 (q, 2H); 3,20 (q, 2H); 4,03 (t, 1H); 7,1-7,4 (m, 11H); 8,35 (d, 1H); 9,0 (br), 13,4 (br,1H) |
| 100 | [CDCl3] 3,45-3,55 (br, 4H); 3,75 (m, 4H); 7,03 (d,1H); 7,80 (d, 1H); 11,3 (br, 1H) |
| 101 | [CDCl3] 1,65 (m, 6H); 3,52 (m, 4H); 7,10 (d, 1H); 7,72 (d, 1H) |
| 102 | [CDCl3] 3,06 (t, 2H); 3,90 (m, 2H); 4,65 (m, 2H); 7,08 (d, 1H); 7,90 (d, 1H); 11,0 (br) |
| 103 | [CDCl3] 2,00 (m, 4H); 3,67 (m, 4H); 7,20 (d, 1H); 7,97 (d, 1H), 11,7 (br, 1H) |
| 104 | [CDCl3] 1,30 (m, 3H); 1,65 (m, 1H); 1,85 (m, 1H); 2,00 (m, 1H); 2,19 (m, 1H); 3,62 (m, 2H); 4,30 (m, 1H); 7,10 (d, 1H); 7,89 (d, 1H) |
| 105 | [CDCl3] 3,25 (s, 3H); 3,35 (3, 3H);7,25 (d, 1H); 8,58 (d, 1H); 8,76 (s, 1H) |
| 112 | [CDCl3] 1,95 (m, 1H); 2,65 (m, 1H); 2,90 (m, 1H); 3,05 (m, 1H); 5,60 (m, 1H); 6,85 (d, 1H); 7,2-7,4 (m, 4H); 8,68 (d, 1H); 9,52 (d, br, 1H) |
| 113 | [CDCl3] 1,60 (m, 1H), 1,90 (m, 2H), 2,01 (m, 1H); 3,42 (m,1H), 3,65-3,82 (m, 2H), 3,90 (m, 1H), 4,09 (m, 1H); 6,88 (d, 1H); 8,63 (d, 1H); 9,37 (br, 1H) |
| 114 | [DMSO] 7,35 (d, 1H); 8,10 (br, 1H); 8,40 (d, 1H); 8,41 (br, 1H); 13,6 (br, 1H) |
| 115 | [CDCl3] 3,01 (d, 3H); 6,81 (d, 1H); 8,63 (d, 1H); 9,22 (br, 1H); 11,7 (br, 1H) |
| 116 | [CDCl3] 1,25 (t, 3H); 3,5 (m, 2H); 6,85 (d, 1H); 8,66 (d, 1H), 9,33 (br, 1H), 13,0 (br, 1H) |
| 117 | [CDCl3] 1,01 (t, 3H); 1,62 (m, 2H); 3,42 (m, 2H); 6,86 (d, 1H); 8,66 (d, 1H); 9,35 (br, 1H); 12,6 (br, 1H) |
| 118 | [CDCl3] 1,27 (d, 6H); 4,25 (m, 1H); 6,85 (d, 1H); 8,64 (d,1H); 9,27 (br, 1H); 13,1 (br, 1H) |
| 119 | [CDCl3] 0,62 (m, 2H); 0,85 (m, 2H); 3,02 (m, 1H); 6,84 (d, 1H); 8,78 (d, 1H), 9,33 (br, 1H) |
| 120 | [CDCl3] 0,98 (t, 3H); 1,41 (m, 2H); 1,61 (m, 2H); 3,45 (q, 2H); 6,83 (d, 1H); 8,65 (d, 1H); 9,33 (br, 1H); 12,9 (br, 1H) |
| 121 | [CDCl3] 1,80 (m, 2H); 2,05 (m, 2H); 2,44 (m, 2H); 4,58 (m, 1H); 6,84 (d, 1H); 8,63 (d, 1H); 9,60 (br, 1H); 13,1 (br) |
| 122 | [CDCl3] 1,46 (s, 9H); 6,86 (d, 1H); 8,62 (d, 1H); 9,34 (br, 1H); 13, 4 (br, 1H) |
| 123 | [CDCl3] 3,08 (s, 6H); 7,06 (d, 1H); 7,79 (d, 1H) |
| 124 | [CDCl3] 1,23 (t, 3H); 3,04 (s, 3H); 3,46 (m, 2H); 7,02 (d,1H); 7,72 (d, 1H) |
| 125 | [CDCl3] 0,92 (t, 3H); 1,30 (br, 2H); 1,60 (m, 2H); 3,04 (s, 3H); 3,41 (br, 2H); 7,04 (d, 1H); 7,71 (d, 1H) |
| 127 | [CDCl3] 1,20 (d, 6H); 2,90 (s, 3H); 4,05 (m, br, 1H); 7,00 (d, 1H); 7,68 (d, 1H) |
| 128 | [CDCl3] 1,20 (t, 6H); 3,41 (m, 4H); 6,98 (d, 1H); 7,66 d, 1H); 11,0 (br) |
| 130 | [CDCl3] 1,20 (m, 9H); 3,40 (q, 2H); 4,06 (m, br, 1H); 6,98 (d, 1H); 8,64 (d, 1H); 10,2 (br) |
| 132 | [CDCl3] 1,5-1,8 (m, 8H); 4,40 (m, 1H); 6,85 (d, 1H); 8,65 (d, 1H); 9,40 (d, br, 1H); 13,1 (br, 1H) |
| 133 | [CDCl3] 1,2-1,7 (m, 6H); 1,80 (m, 2H); 2,00 (m, 2H); 3,98 (m, 1H); 6,85 (d, 1H); 8,64 (d, 1H); 9,30 (d,br, 1 H); 13,0 (br) |
| 134 | [CDCl3] 0,90 (t, 3H); 1,40 (m, 4H); 1,62 (m, 2H); 3,44 (m, 2H); 6,84 (d,1H); 8,64 (d, 1H); 9,30 (br, 1H) |
| 136 | [CDCl3] 0,30 (m, 2H); 0,59 (m, 2H); 1,05 (m, 1H); 3,32 (m, 2H); 6,86 (d, 1H), 8,65 (d, 1H); 9,40 (br) |
| 138 | [CDCl3] 1,00 (s, 9H); 3,26 (d, 2H); 6,86 (d, 1H); 8,70 (d, 1H); 9,42 (br, 1H); 12,5 (br, 1H) |
| 139 | [DMSO] 4,25 (dt, 2H); 7,25 (d, 1H); 8,40 (d, 1H); 9,35 (br, 1H); 13,5 (br, 1H) |
| 140 | [CDCl3] 4,15 (m, 2H); 6,88 (d, 1H); 8,70 (d, 1H); 9,80 (br, 1H) |
| 141 | [CDCl3] 4,20 (dt; 2H); 6,90 (d, 1H); 8,70 (d, 1H); 9,83 (t, br, 1H); 12,80 (br) |
| 144 | [CDCl3] 1,00 (d, 6H); 1,90 (m, 1H); 3,30 (t, 2H); 6,83 (d, 1H); 8,65 (d, 1H); 9,35 (br, 1H); 12,2 (br) |
| 146 | [CDCl3] 0,96 (d, 6H); 1,50 (m, 2H); 1,70 (m, 1H); 3,48 (m, 2H); 6,83 (d, 1H); 8,67 (d, 1H); 9,30 (br, 1H), 12,9 (br, 1H) |
| 148 | [CDCl3] 4,62 (d, 2H); 5,36 (dd, 2H); 5,95 (m, 1H); 7,80 (d, 1H); 8,65 (d, 1H) |
| 150 | [CDCl3] 4,0 (m, 4H); 5,22 (d, 4H); 5,80 (m, 2H); 6,93 (d, 1H); 7,72 (d, 1H) |
| 152 | [CDCl3] 1,80 (s, 3H); 4,02 (d, 2H); 4,90 (d, 2H); 6,85 (d 1H); 8,68 (d, 1H); 9,48 (br, 1H); 12,8 (br, 1H) |
| 153 | [CDCl3] 2,26 (t, 1H); 4,23 (m, 2H); 6,84 (d, 1H); 8,64 (d, 1H); 9,58 (br, 1H); 12,4 (br) |
| 154 | [CDCl3] 2,30 (s, 1H); 3,12 (s, 3H); 4,24 (br, 2H); 7,01 (d, 1H); 7,82 (d, 1H); 10,9 (br) |
| 155 | [CDCl3] 0,98 (t, 3H); 1,22 (d, 3H); 1,59 (m, 2H); 4,10 (m, 1H); 6,85 (d, 1H); 8,65 (d, 1H); 9,26 (d, br, 1H); 13,4 (br, 1H) |
| 157 | [CDCl3] 0,92 (t, 3H); 1,22 (d, 3H); 1,40 (m, 2H); 1,55 (m, 2H); 4,20 (m, 1H); 6,85 (d, 1H); 8,68 (d, 1H); 9,30 (d, br, 1H) |
| 158 | [CDCl3] 0,87 (t, 3H); 1,12 (d, 3H); 1,30 (m, 4H); 1,58 (m, 4H); 4,15 (m, 1H); 6,84 (d, 1H); 8,65 (d, 1H); 9,24 (br, 1H), 13,1 (br) |
| 161 | [CDCl3] 0,90 (d, 6H); 1,22 (d, 3H), 1,30 (m, 1H); 1,55 (m, 2H); 4,23 (m, 1H); 6,86 (d, 1H); 8,64 (d, 1H); 9,20 (d, br, 1H); 13,0 (br, 1H) |
| 162 | [CDCl3] 0,98 (s, 9H); 1,20 (d, 3H); 4,10 (m, 1H); 6,87 (d, 1H); 8,70 (d, 1H); 9,38 (d, br, 1H), 13,2 (br, 1H) |
| 163 | [CDCl3] 0,95 (dt, 6H); 1,20 (d, 3H); 1,82 (m, 1H); 4,05 (m, 1H); 6,84 (d, 1H); 8,68 (d, 1H); 9,30 (d, br, 1H); 13,1 (br) |
| 165 | [CDCl3] 0,96 (t, 6H); 1,50 (m, 2H); 1,65 (m, 2H); 4,00 (m, 1H); 6,85 (d, 1H); 8,68 (d, 1H); 9,22 (d, br, 1H); 13,5 (br, 1H) |
| 166 | (CDCl3] 0,90 (t, 3H); 1,41 (s; 6H); 1,85 (q, 2H); 6,82 (d, 1H); 8,61 (d, 1H); 9,20 (br, 1H); 13,1 (br) |
| 168 | [CDCl3] 3,40 (s, 6H); 3,63 (t, 2H); 4,50 (t, 1H); 6,85 (d, 1H); 8,65 (d, 1H); 9,40 (br, 1H); 12,4 (br, 1H) |
| 169 | [CDCl3] 1,21 (t, 6H); 3,60 (m, 4H); 3,74 (m, 2H); 4,62 (t, 1H); 6,84 (d, 1H); 8,60 (d, 1H); 9,35 (br, 1H), 11,5 (br) |
| 171 | [CDCl3] 3,40 (s, 3H); 3,58 (m, 2H); 3,67 (m, 2H); 6,84 (d, 1H), 8,62 (d, 1H); 9,40 (br, 1H) |
| 178 | [CDCl3] 1,24 (d, 3H); 3,40 (s, 3H); 3,42 (d, 2H); 4,40 (m, 1H); 6,86 (d, 1H); 8,64 (d, 1H); 9,35 (br, 1H); 13,0 (br) |
| 180 | [DMSO] 1,75 (m, 2H); 3,22 (s, 3H); 3,36 (m, 4H); 7,3 (br, 1H); 8,4 (d, 1H); 9,0 (br); 13,4 (br, 1H) |
| 185 | [DMSO] 3,65 (s, 3H); 4,12 (d, 2H); 7,28 (d, br, 1H); 8,42 (d, 1H); 9,25 (t, br,1H), 13,6 (br, 1H) |
| 189 | [CDCl3] 2,66 (t, 2H); 3,70 (s, 3H); 3,76 (m, 2H); 6,83 (d, 1H); 8,61 (d, 1H); 9,51 (t, br, 1H); 12,4 (br, 1H) |
| 193 | [CDCl3] 1,37 (t, 3H); 4,08 (q, 2H); 6,88 (d, 1H); 8,69 (d, 1H); 11,48 (s, 1H) |
| 194 | [CDCl3] 3,88 (s, 3H); 6,88 (d, 1H); 8,65 (d, 1H); 11,5 (s, 1H) |
| 195 | [CDCl3] 4,50 (d, 2H); 5,34 (m, 2H); 6,05 (m, 1H); 6,88 (d, 1H); 8,70 (d, 1H); 11,44 (s, 1H) |
| 196 | [CDCl3] 1,39 (s, 9H); 6,88 (d, 1H); 8,70 (d, 1H); 11,22 (s, 1H) |
| 206 | [CDCl3] 4,62 (d, 2H), 6,82 (d, 1H); 7,2-7,3 (m, 5H); 8,68 (d, 1H); 9,65 (t, br, 1H), 12,8 (br, 1H) |
| 207 | [CDCl3] 2,99 (s, 3H); 4,67 (br, 2H); 7,00 (d,1H); 7,3-7,4 (m, 5H), 7,78 (d, 1H); 10,2 (br) |
| 210 | [CDCl3] 1,60 (d, 3H); 5,30 (m, 1H); 6,85 (d, 1H); 7,2-7,4 (m, 5H); 8,65 (d, 1H); 9,78 (d, br, 1H); 13,2 (br, 1H) |
| 211 | [CDCl3] 2,95 (t, 2H); 3,70 (m, 2H); 6,82 (d, 1H); 7,2-7,35 (m, 5H); 8,63 (d, 1H); 9,40 (t, br, 1H); 12,2 (br, 1H) |
| 214 | [CDCl3] 3,45-3,55 (br, 4H); 3,75 (m, 4H); 6,96 (d,1H); 7,78 (d, 1H) |
| 215 | [CDCl3] 1,65 (m, 6H); 3,53 (m, 4H); 7,00 (d, 1H); 7,70 (d, 1H) |
| 216 | [CDCl3] 3,05 (t, 2H); 3,92 (m, 2H); 4,66 (m, 2H); 7,04 (d, 1H); 7,86 (d, 1H); 10,4 (br) |
| 217 | [CDCl3] 2,00 (m, 4H); 3,70 (m, 4H); 7,16 (d,1H); 7,97 (d, 1H), 11,7 (br, 1H) |
| 218 | [CDCl3] 1,30 (m, 3H); 1,65 (m, 1H); 1,85 (m, 1H); 2,04 (m, 1H); 2,20 (m, 1H); 3,63 (m, 2H); 4,33 (m, 1H); 7,12 (d, 1H); 7,89 (d, 1H), 11,5 (br, 1H) |
| 227 | [CDCl3] 1,60 (m, 1H), 1,90 (m, 2H), 2,00 (m, 1H); 3,42 (m,1H), 3,65-3,82 (m, 2H), 3,90 (m, 1H), 4,10 (m, 1H); 6,88 (d, 1H); 8,65 (d, 1H); 9,5 (br, 1H) |
| 228 | [DMSO] 6,80 (br, 1H); 6,90 (t, 1H); 7,72 (br, 1H); 8,40 (d, 1H); 8,90 (br, 1H); 13,20 (br, 1H) |
| 229 | [DMSO] 2,82 (d, 3H); 6,75 (d, br, 1H); 6,90 (t, 1H); 8,89 (d, 1H); 9,50 (br, 1H); 13,2 (br, 1H) |
| 230 | [DMSO] 1,11 (t, 3H); 3,32 (m, 2H); 6,75 (d, br, 1H); 6,90 (t, 1H); 8,39 (d, 1H), 9,60 (br, 1H), 13,2 (br, 1H) |
| 232 | [CDCl3] 1,25 (d, 6H); 4,22 (m, 1H); 6,55 (t, 1H); 6,69 (d, 1H); 8,64 (d, 1H); 9,42 (d, br, 1H); 13,0 (br, 1H) |
| 233 | [DMSO] 0,51 (m, 2H); 0,74 (m, 2H); 2,84 (m, 1H); 6,79 (d, br, 1H); 6,90 (t, 1H); 8,37 (d, 1H); 9,65 (br, 1H); 13,1 (br, 1H) |
| 250 | [DMSO] 0,23 (m, 2H); 0,46 (m, 2H); 1,02 (m, 1H); 3,20 (m, 2H); 6,75 (br, 1H), 6,92 (t, 1H); 8,40 (d, 1H); 9,70 (br, 1H); 13,1 (br, 1H) |
| 252 | [DMSO] 0,91 (s, 9H); 3,15 (d, 2H); 6,80 (br, 1H); 6,91 (t, 1H); 8,40 (d, 1H); 9,85 (br, 1H); 13,2 (br, 1H) |
| 258 | [DMSO] 0,90 (d, 6H); 1,80 (m, 1H); 3,15 (t, 2H); 6,80 (br, 1H); 6,91 (t, 1H); 8,40 (d, 1H); 9,73 (br, 1H); 13,1 (br, 1H) |
| 262 | [DMSO] 3,96 (m, 2H); 5,15 (dd, 2H); 5,94 (m, 1H); 6,80 (br, 1H); 6,92 (t, 1H); 8,40 (d, 1H); 9,75 (br, 1H); 13,2 (br, 1H) |
| 271 | [CDCl3] 0,92 (t, 3H); 1,25 (d, 3H); 1,42 (m, 2H); 1,55 (m, 2H); 4,19 (m, 1H); 6,60 (t, 1H); 6,73 (d, 1H); 8,66 (d, 1H); 9,50 (d, br, 1H) |
| 272 | [CDCl3] 0,90 (t, 3H); 1,2-1,4 (m, 9H); 1,58 (m, 2H); 4,17 (m, 1H); 6,60 (t, 1H); 6,75 (d, 1H); 8,68 (d, 1H); 9,48 (d, br, 1H) |
| 279 | [CDCl3] 0,85 (t, 6H); 1,45 (m, 2H); 1,55 (m, 2H); 3,80 (m, 1H); 6,80 (br, 1H); 6,91 (t, 1H); 8,40 (d, 1H); 9,51 (br, 1H); 13,1 (br, 1H) |
| 280 | [DMSO] 0,86 (t, 3H); 1,12 (d, 3H); 1,50 (m, 2H); 3,90 (m, 1H); 6,75 (br, 1H); 6,91 (t, 1H); 8,39 (d, 1H); 9,57 (br, 1H); 13,1 (br, 1H) |
| 285 | [DMSO] 3,29 (s, 3H); 3,46 (m, 4H); 6,76 (br, 1H), 6,91 (t, 1H); 8,50 (d, 1H); 9,73 (br, 1H), 13,1 (br, 1H) |
| 291 | [DMSO] 1,14 (d, 3H); 3,27 (s, 3H); 3,35 (m, 2H); 4,15 (m, 1H); 6,80 (br, 1H); 6,91 (t, 1H); 8,40 (d, 1H); 9,70 (br, 1H); 13,1 (br, 1H)) |
| 293 | [DMSO] 1,72 (m, 2H); 3,22 (s, 3H); 3,37 (m, 4H); 6,75 (br, 1H); 6,90 (t, 1H); 8,40 (d, 1H); 9,70 (br, 1H)); 13,2 (br, 1H) |
| 341 | [DMSO] 7,45 (d, 1H); 8,15 (br, 1H); 8,45 (br, 1H); 8,50 (d, 1H); 13,7 (br, 1H) |
| 342 | [CDCl3] 3,01 (d, 3H); 6,90 (d, 1H); 8,72 (d, 1H); 9,34 (br, 1H) |
| 343 | [DMSO] 1,11 (t, 3H); 3,31 (m, 2H); 7,41(d, 1H); 8,42 (d, 1H); 8,85 (br, 1H), 13,4 (br,1H) |
| 344 | [CDCl3] 1,02 (t, 3H); 1,61 (m, 2H); 3,42 (q, 2H); 6,88 (d, 1H); 8,72 (d, 1H); 9,38 (br,1H) |
| 345 | [CDCl3] 1,25 (d, 6H); 4,25 (m, 1H); 6,90 (d, 1H); 8,72 (d,1H); 9,30 (d, br, 1H); 13,8 (br, 1H) |
| 346 | [CDCl3] 0,60 (m, 2H); 0,88 (m, 2H); 3,02 (m, 1H); 6,90 (d, 1H); 8,72 (d, 1H); 9,42 (br, 1H); 13,4 (br) |
| 347 | [DMSO] 0,88 (t, 3H); 1,34 (m, 2H); 1,50 (m, 2H); 3,31 (q, 2H); 7,27 (d, 1H); 8,35 (d, 1H); 9,21 (br, 1H); 12,4 (br, 1H) |
| 348 | [DMSO] 1,70 (m, 2H); 2,02 (m, 2H); 2,27 (m, 2H); 4,40 (m, 1H); 7,41 (d, 1H); 8,48 (d, 1H); 9,00 (br, 1H); 13,4 (br, 1H) |
| 349 | [DMSO] 1,36 (s, 9H); 7,35 (d, br, 1H); 8,40 (d, 1H); 8,60 (br, 1H); 13,3 (br, 1H) |
| 350 | [CDCl3] 3,08 (s, 6H); 7,08 (d, 1H); 7,81 (d, 1H) |
| 355 | [CDCl3] 1,21 (t, 6H); 3,42 (m, 4H); 7,04 (d, 1H); 7,71 (d, 1H); 10,8 (br) |
| 363 | [DMSO] 0,27 (m, 2H); 0,46 (m, 2H); 1,05 (m, 1H); 3,15 (m, 2H); 7,45 (d, br, 1H); 8,45 (d, 1H); 8,95 (br, 1H); 13,4 (br, 1H) |
| 365 | [DMSO] 0,91 (s, 9H); 3,15 (d, 2H); 7,38 (d, br, 1H); 8,40 (d, 1H); 8,83 (br, 1H); 13,4 (br, 1H) |
| 373 | [DMSO] 0,90 (d, 6H); 1,42 (m, 2H); 1,63 (m, 1H); 3,33 (m, 2H); 7,40 (d, br, 1H); 8,40 (d, 1H); 8,83 (br, 1H), 13,4 (br, 1H) |
| 375 | [DMSO] 3,95 (m, 2H); 5,15 (dd, 2H); 5,90 (m, 1H); 7,40 (d, 1H); 8,41 (d, 1H); 8,98 (br, 1H); 13,4 (br, 1H) |
| 379 | [DMSO] 1,73 (s, 3H); 3,88 (m, 2H); 4,75 (d, 2H); 7,40 (d, br, 1H); 8,41 (d, 1H); 8,97 (br, 1H); 13,4 (br, 1H) |
| 380 | [DMSO] 3,15 (t, 1H); 4,21 (m, 2H); 7,40 (d, 1H); 8,40 (d, 1H); 9,12 (br, 1H); 13,3 (br, 1H) |
| 382 | [CDCl3] 0,97 (t, 3H); 1,22 (d, 3H); 1,59 (m, 2H); 4,10 (m, 1H); 6,88 (d, 1H); 8,72 (d, 1H); 9,24 (d, br, 1H); 13,6 (br) |
| 384 | [CDCl3] 0,92 (t, 3H); 1,21 (d, 3H); 1,40 (m, 2H); 1,50 (m, 2H); 4,20 (m, 1H); 6,88 (d, 1H); 8,72 (d, 1H); 9,28 (d, br, 1H) |
| 385 | [DMSO] 0,85 (t, 3H); 1,1-1,35 (m, 9H); 1,50 (m, 2H); 4,00 (m, 1H); 7,41 (d, 1H); 8,40 (d, 1H); 8,65 (br, 1H); 13,4 (br, 1H) |
| 388 | [DMSO] 0,88 (d, 6H); 1,15 (d, 3H), 1,28 (m, 1H); 1,48 (m, 1H); 1,62 (m, 1H); 4,11 (m, 1H); 7,40 (d, br, 1H); 8,40 (d, 1H); 8,52 (d, br, 1H); 13,4 (br, 1H) |
| 389 | [DMSO] 0,90 (s, 9H); 1,10 (d, 3H); 3,90 (m, 1H); 7,38 (d, br, 1H); 8,40 (d, 1H); 8,70 (br, 1H), 13,4 (br, 1H) |
| 390 | [DMSO] 0,79 (dt, 6H); 1,10 (d, 3H); 1,75 (m, 1H); 3,87 (m, 1H); 7,40 (d, br, 1H); 8,41 (d, 1H); 8,65 (d, br, 1H); 13,4 (br, 1H) |
| 392 | [CDCl3] 0,95 (t, 6H); 1,50 (m, 2H); 1,65 (m, 2H); 4,00 (m, 1H); 6,90 (d, 1H); 8,75 (d, 1H); 9,20 (d, br, 1H); 13,7 (br, 1H) |
| 395 | [DMSO] 3,30 (s, 6H); 3,42 (t, 2H); 4,50 (t, 1H); 7,40 (d, br, 1H); 8,45 (d, 1H); 8,90 (br, 1H); 13,4 (br, 1H) |
| 397 | [DMSO] 3,38 (m, 2H); 3,52 (m, 2H); 4,80 (t, 1H); 7,40 (d, br, 1H); 8,43 (d, 1H); 8,95 (br, 1H); 13,4 (br, 1H) |
| 398 | [CDCl3] 3,38 (s, 3H); 3,58 (m, 2H); 3,65 (m, 2H); 6,88 (d, 1H), 8,70 (d, 1H); 9,43 (br, 1H) |
| 405 | [CDCl3] 1,25 (d, 3H); 3,38 (s, 3H); 3,42 (d, 2H); 4,40 (m, 1H); 6,88 (d, 1H); 8,70 (d, 1H); 9,38 (d, br, 1H) |
| 407 | [CDCl3] 1,89 (m, 2H); 3,32 (s, 3H); 3,48 (t, 2H); 3,58 (m, 2H); 6,92 (d, 1H); 8,64 (d, 1H); 9,30 (br, 1H) |
| 455 | [DMSO] 8,25 (br, 1H); 8,35 (br, 1H); 8,50 (s, 1H); 13,8 (br, 1H) |
| 456 | [DMSO] 2,84 (d, 3H); 8,40 (s, 1H); 8,72 (d, br, 1H) |
| 457 | [DMSO] 1,12 (t, 3H); 3,35 (q, 2H); 8,40 (s, 1H); 8,75 (t, br, 1H); 13,5 (br, 1H) |
| 458 | [DMSO] 0,89 (t, 3H); 1,52 (m, 2H); 3,25 (m, 2H); 8,41 (s, 1H); 8,73 (t, br, 1H); 13,4 (br, 1H) |
| 459 | [DMSO] 1,19 (d, 6H); 4,08 (m, 1H); 8,43 (s, 1H); 8,59 (d, br, 1H); 13,4 (br, 1H) |
| 460 | [DMSO] 0,59 (m, 2H); 0,75 (m, 2H); 2,87 (m, 1H); 8,39 (s, 1H); 8,73 (d, br, 1 H) |
| 461 | [DMSO] 0,90 (t, 3H); 1,32 (m, 2H); 1,50 (m, 2H); 3,30 (m, 2H); 8,41 (s, 1H); 8,74 (t, br, 1H); 13,5 (br, 1H) |
| 462 | [DMSO] 1,70 (m, 2H); 2,03 (m, 2H); 2,25 (m, 2H); 4,40 (m, 1H); 8,41 (s, 1H); 8,90 (d, br, 1H); 13,5 (br, 1H) |
| 477 | [DMSO] 0,25 (m, 2H); 0,47 (m, 2H); 1,02 (m, 1H); 3,19 (t, 2H); 8,45 (s, 1H); 8,80 (t, br, 1H) |
| 479 | [DMSO] 0,90 (s, 9H); 3,14 (d, 2H); 8,38 (s, 1H); 8,60 (t, br, 1H); 13,7 (br, 1H) |
| 485 | [DMSO] 0,89 (d, 6H); 1,82 (m, 1H); 3,13 (t, 2H); 8,40 (s, 1H); 8,72 (t, br, 1H) |
| 487 | [DMSO] 0,90 (d, 6H); 1,41 (q, 2H); 1,62 (m, 1H); 3,32 (m, 2H); 8,40 (s, 1H); 8,72 (t, br, 1H); 13,5 (br, 1H) |
| 489 | [CDCl3] 4,10 (d, 2H); 5,25 (dd, 2H); 5,93 (m, 1H); 8,60 (s, 1H); 9,10 (br, 1H); 12,4 (br, 1H) |
| 493 | [DMSO] 1,71 (s, 3H); 3,86 (d, 2H); 4,84 (d, 2H); 8,40 (s, 1H); 8,85 (t, br, 1H); 13,5 (br) |
| 494 | [DMSO] 3,17 (m, 1H); 4,11 (m, 2H); 8,39 (s, 1H); 8,98 (t, br, 1H), 13,4 (br) |
| 496 | [CDCl3] 0,95 (t, 3H); 1,25 (d, 3H); 1,60 (m, 2H); 4,12 (m, 1H); 8,48 (s, 1H); 8,52 (br, 1 H) |
| 498 | [CDCl3] 0,95 (t, 3H); 1,25 (d, 3H); 1,40 (m, 2H); 1,55 (m, 2H); 4,17 (m, 1H); 8,53 (s, 1H); 8,70 (br, 1H) |
| 499 | [CDCl3] 0,87 (t, 3H); 1,21 (d, 3H); 1,32 (m, 6H); 1,53 (m, 2H); 4,15 (m, 1H); 8,62 (s, 1H); 8,99 (d, br,1H); 13,0 (br) |
| 502 | [CDCl3] 0,91 (d, 6H); 1,23 (d, 3H); 1,35 (m, 1H); 1,48 (m, 1H); 1,66 (m, 1H); 4,25 (m, 1H); 8,62 (s, 1H); 8,96 (d, br, 1H); 12,9 (br, 1H) |
| 504 | [CDCl3] 0,95 (m, 6H); 1,18 (d, 3H); 1,81 (m, 1H); 4,07 (m, 1H); 8,61 (s, 1H); 8,97 (d, br, 1H); 12,9 (br, 1H) |
| 506 | [CDCl3] 0,94 (t, 6H); 1,50 (m, 2H); 1,65 (m, 2H), 4,00 (m, 1H); 8,56 (s, 1H); 8,73 (d, br, 1H) |
| 512 | [DMSO] 3,28 (s, 3H); 3,48 (m, 4H); 8,42 (s, 1H); 8,78 (br, 1H); 13,7 (br, 1H) |
| 519 | [DMSO] 1,16 (d, 3H); 3,30 (s, 3H); 3,42 (m, 2H); 4,18 (m, 1H); 8,42 (s, 1H); 8,60 (d, br, 1H); 13,7 (br, 1H) |
| 521 | [DMSO] 1,74 (m, 2H); 3,26 (s, 3H); 3,39 (m, 4H); 8,40 (s, 1H); 8,80 (t, br, 1H); 13,6 (br, 1H) |
| 569 | [DMSO] 8,25 (br, 1H); 8,35 (br, 1H); 8,50 (s, 1H); 13,7 (br, 1H) |
| 570 | [DMSO] 2,82 (d, 3H); 8,40 (s, 1H); 8,71 (br, 1H); 13,5 (br, 1H) |
| 571 | [DMSO] 1,12 (t, 3H); 3,35 (q, 2H); 8,40 (s, 1H); 8,75 (t, br, 1H); 13,7 (br, 1H) |
| 572 | [DMSO] 0,89 (t, 3H); 1,52 (m, 2H); 3,26 (m, 2H); 8,39 (s, 1H); 8,82 (br, 1H) |
| 573 | [DMSO] 1,18 (d, 6H); 4,09 (m, 1H); 8,42 (s, 1H); 8,53 (d, br, 1H); 13,5 (br, 1H) |
| 574 | [DMSO] 0,59 (m, 2H); 0,74 (m, 2H); 2,85 (m, 1H); 8,37 (s, 1H); 8,72 (d, br, 1H) |
| 575 | [DMSO] 0,89 (t, 3H); 1,35 (m, 2H); 1,50 (m, 2H); 3,35 (m, 2H); 8,39 (s, 1H); 8,75 (br, 1H) |
| 576 | [DMSO] 1,70 (m, 2H); 2,05 (m, 2H); 2,25 (m, 2H); 4,40 (m, 1H); 8,40 (s, 1H); 8,94 (br, 1 H) |
| 591 | [DMSO] 0,25 (m, 2H); 0,45 (m, 2H); 1,03 (m, 1H); 3,21 (t, 2H); 8,40 (s, 1H); 8,84 (br,1H) |
| 599 | [DMSO] 0,89 (d, 6H); 1,82 (m, 1H); 3,14 (m, 2H); 8,38 (s, 1H); 8,74 (br, 1H) |
| 601 | [CDCl3] 0,96 (d, 6H); 1,51 (m, 2H); 1,70 (m, 1H); 3,49 (m, 2H); 8,59 (s, 1H); 9,02 (br,1H);12,2(br) |
| 603 | [DMSO] 3,95 (t, 2H); 5,18 (dd, 2H); 5,89 (m, 1H); 8,40 (s, 1H); 8,85 (t, br, 1H); 13,6 (br, 1H) |
| 612 | [CDCl3] 0,91 (t, 3H); 1,24 (d, 3H); 1,40 (m, 2H); 1,54 (m, 2H); 4,18 (m, 1H); 8,57 (s, 1H); 8,90 (d, br, 1H); 12,2 (br, 1H) |
| 620 | [CDCl3] 0,94 (t, 6H); 1,51 (m, 2H); 1,67 (m, 2H), 3,99 (m, 1H); 8,57 (s, 1H); 8,82 (d, br, 1H) |
| 626 | [DMSO] 3,29 (s, 3H); 3,48 (m, 4H); 8,41 (s, 1H); 8,78 (br, 1H); 13,7 (br, 1H) |
| 633 | [CDCl3] 1,28 (d, 3H); 3,40 (s, 3H); 3,45 (m, 2H); 4,37 (m, 1H); 8,48 (s, 1H); 8,78 (br, 1H) |
| 635 | [DMSO] 1,75 (m, 2H); 3,25 (s, 3H); 3,39 (m, 4H); 8,40 (s, 1H); 8,81 (br, 1H); 13,6 (br, 1H) |
| 797 | [DMSO] 8,27 (br, 1H); 8,38 (br, 1H); 8,52 (s, 1H); 13,7 (br, 1H) |
| 798 | [DMSO] 2,82 (d, 3H); 8,40 (s, 1H); 8,75 (d, br, 1H); 13,6 (br, 1H) |
| 799 | [DMSO] 1,12 (t, 3H); 3,33 (m, 2H); 8,41 (s, 1H); 8,78 (t, br, 1H); 13,6 (br, 1H) |
| 800 | [DMSO] 0,89 (t, 3H); 1,52 (m, 2H); 3,27 (q, 2H); 8,41 (s, 1H); 8,76 (t, br, 1H); 13,5 (br, 1H) |
| 801 | [DMSO] 1,18 (d, 6H); 4,09 (m, 1H); 8,42 (s, 1H); 8,60 (d, br, 1H) |
| 802 | [DMSO] 0,55 (m, 2H); 0,75, (m, 2H); 2,85 (m, 1H); 8,31 (s, 1H); 9,24 (br, 1H); 13,4 (br, 1H) |
| 803 | [DMSO] 0,89 (t, 3H); 1,33 (m, 2H); 1,51 (m, 2H); 3,30 (m, 2H); 8,40 (s, 1H); 8,75 (t, br, 1H), 13,4 (br) |
| 804 | [DMSO] 1,70 (m, 2H); 2,04 (m, 2H); 2,25 (m, 2H); 4,39 (m, 1H); 8,43 (s, 1H); 8,95 (d, br, 1H); 13,4 (br, 1H) |
| 806 | [DMSO] 2,82 (s, 3H); 2,98 (s, 3H); 8,06 (s, 1H); 12,6 (s, br, 1H) |
| 811 | [CDCl3] 1,24 (t, 6H); 3,44 (m, 4H); 7,72 (s, 1H) |
| 819 | [DMSO] 0,25 (m, 2H); 0,46 (m, 2H); 1,03 (m, 1H); 3,20 (t, 2H); 8,45 (s, 1H); 8,87 (t, br, 1H); 13,4 (br, 1H) |
| 821 | [DMSO] 0,90 (s, 9H); 3,13 (d, 2H); 8,37 (s, 1H); 8,61 (t, br, 1H); 13,4 (br, 1H) |
| 827 | [DMSO] 0,88 (d, 6H); 1,82 (m, 1H); 3,13 (t, 2H); 8,40 (s, 1H); 8,76 (t, br, 1H); 13,5 (br, 1H) |
| 829 | [DMSO] 0,90 (d, 6H); 1,42 (m, 2H); 1,61 (m, 1H); 3,30 (m, 2H); 8,41 (s, 1H); 8,76 (t, br, 1 H); 13,5 (br, 1 H) |
| 831 | [DMSO] 3,95 (t, 2H); 5,19 (dd, 2H); 5,89 (m, 1H); 8,41 (s, 1H); 8,88 (t, br, 1H), 13,5 (br, 1H) |
| 835 | [DMSO] 1,72 (s, 3H); 3,87 (d, 2H); 4,86 (d, 2H); 8,39 (s, 1H); 8,90 (t, br, 1H); 13,4 (br, 1H) |
| 836 | [DMSO] 3,18 (m, 1H); 4,10 (m, 2H); 8,36 (s, 1H); 9,01 (t, br, 1H); 13,4 (br, 1H) |
| 838 | [DMSO] 0,88 (t, 3H); 1,14 (d, 3H); 1,51 (m, 2H); 3,91 (m, 1H); 8,41 (s, 1H); 8,54 (d, br, 1H); 13,5 (br, 1H) |
| 840 | [DMSO] 0,88 (t, 3H); 1,14 (d, 3H); 1,33 (m, 2H), 1,47 (m, 2H); 4,01 (m, 1H); 8,42 (s, 1H); 853 (d, br, 1H); 13,5 (br, 1H) |
| 841 | [DMSO] 0,85 (t, 3H); 1,14 (d, 3H); 1,28 (m, 6H); 1,49 (m, 2H); 3,98 (m, 1H); 8,41 (s, 1H); 8,60 (d, br, 1H); 13,5 (br, 1H) |
| 844 | [DMSO] 0,87 (d, 6H); 1,14 (d, 3H); 1,27 (m, 1H); 1,48 (m, 1H); 1,62 (m, 1H); 4,09 (m, 1H); 8,41 (s, 1H); 8,52 (d, br, 1H); 13,5 (br, 1H) |
| 845 | [DMSO] 0,90 (s, 9H); 1,08 (d, 3H); 3,90 (m, 1H); 8,35 (s, 1H); 8,38 (d, br, 1H); 13,4 (br) |
| 846 | [DMSO] 0,89 (d, 6H); 1,12 (d, 3H); 1,75 (m, 1H); 3,87 (m, 1H); 8,40 (s, 1H); 8,50 (d, br, 1H); 13,5 (br) |
| 848 | [DMSO] 0,87 (t, 6H); 1,44 (m, 2H); 1,55 (m, 2H), 3,79 (m, 1H); 8,40 (s, 1H); 8,42 (d, br, 1H); 13,4 (br, 1H) |
| 851 | [CDCl3] 3,42 (s, 6H); 3,63 (t, 2H); 4,50 (t, 1H); 8,55 (s, 1H); 8,95 (t, br, 1H); 12,1 (br, 1H) |
| 853 | [CDCl3] 3,65 (m, 2H); 3,86 (m, 2H); 8,47 (s, 1 H); 8,98 (br, 1H) |
| 854 | [CDCl3] 3,39 (s, 3H); 3,58 (m, 2H); 3,69 (m, 2H); 8,49 (s, 1 H); 8,80 (br, 1 H); 11,9 (br, 1H) |
| 861 | [CDCl3] 1,28 (d, 3H); 3,38 (s, 3H); 3,45 (m, 2H); 4,39 (m, 1H); 8,51 (s, 1H); 8,85 (br, 1H); 12,4 (br, 1H) |
| 863 | [CDCl3] 1,90 (m, 2H); 3,40 (s, 3H); 3,57 (m, 4H); 8,30 (s, 1H); 8,70 (br, 1H) |
| 868 | [CDCl3] 3,80 (s, 3H); 4,25 (d, 2H); 8,52 (s, 1H); 9,16 (br, 1H) |
| 909 | [DMSO] 8,22 (br, 1H); 8,32 (br, 1H); 8,61 (s, 1H); 13,8 (br, 1H) |
| 910 | [DMSO] 2,83 (d, 3H); 8,51 (s, 1H); 8,74 (br, 1H); 13,7 (br, 1H) |
| 911 | [DMSO] 1,12 (t, 3H); 3,32 (q, 2H); 8,54 (s, 1H); 8,75 (t, br, 1H); 13,7 (br, 1H) |
| 912 | [DMSO] 0,89 (t, 3H); 1,52 (m, 2H); 3,25 (q, 2H); 8,54 (s, 1H); 8,75 (t, br, 1H); 13,6 (br, 1H) |
| 913 | [CDCl3] 1,25 (d, 6H); 4,25 (m, 1H); 8,54 (br, 1H); 8,70 (s, 1H) |
| 914 | [DMSO] 0,58 (m, 2H); 0,75, (m, 2H); 2,86 (m, 1H); 8,50 (s, 1H); 8,75 (d, 1H); 13,6 (br, 1H) |
| 915 | [DMSO] 0,89 (t, 3H); 1,32 (m, 2H); 1,50 (m, 2H); 3,32 (m, 2H); 8,53 (s, 1H); 8,74 (t, br, 1H), 13,5 (br) |
| 916 | [DMSO] 1,70 (m, 2H); 2,05 (m, 2H); 2,25 (m, 2H); 4,40 (m, 1H); 8,52 (s, 1H); 8,98 (d, br, 1H); 13,5 (br, 1H) |
| 917 | [CDCl3] 1,50 (s, 9H); 8,65 (s, 1H); 8,80 (s, br, 1H); 12,3 (br, 1H) |
| 918 | [DMSO] 2,82 (s, 3H); 2,99 (s, 3H); 8,14 (s, 1H); 12,6 (br, 1H) |
| 923 | [DMSO] 1,02 (t, 3H); 1,12 (t, 3H); 3,10 (q, 2H); 3,40 (q, 2H); 8,15 (s, 1H), 12,5 (br, 1H) |
| 931 | [DMSO] 0,24 (m, 2H); 0,46 (m, 2H); 1,04 (m, 1H); 3,18 (t, 2H); 8,55 (s, 1H); 8,82 (t, br, 1H) |
| 933 | [DMSO] 0,90 (s, 9H); 3,13 (d, 2H); 8,50 (s, 1H); 8,61 (t, br, 1H) |
| 939 | [DMSO] 0,88 (d, 6H); 1,83 (m, 1H); 3,14 (t, 2H); 8,52 (s, 1H); 8,72 (t, br, 1H); 13,6 (br, 1H) |
| 941 | [DMSO] 0,90 (d, 6H); 1,43 (m, 2H); 1,62 (m, 1H); 3,35 (m, 2H); 8,52 (s, 1H); 8,72 (t, br, 1H); 13,5 (br, 1H) |
| 943 | [DMSO] 3,94 (t, 2H); 5,18 (dd, 2H); 5,89 (m, 1H); 8,52 (s, 1H); 8,85 (t, br, 1H) |
| 947 | [DMSO] 1,72 (s, 3H); 3,87 (d, 2H); 4,85 (d, 2H); 8,51 (s, 1H); 8,87 (t, br, 1H); 13,5 (br) |
| 948 | [DMSO] 3,18 (m, 1H); 4,12 (m, 2H); 8,50 (s, 1H); 9,00 (t, br, 1H) |
| 950 | [DMSO] 0,92 (t, 3H); 1,21 (d, 3H); 1,57 (m, 2H); 3,97 (m, 1H); 8,55 (d, br, 1H); 8,60 (s, 1H); 13,7 (br, 1H) |
| 952 | [CDCl3] 0,90 (t, 3H); 1,24 (d, 3H); 1,40 (m, 2H), 1,55 (m, 2H); 4,18 (m, 1H); 8,72 (s, 1H); 8,90 (d, br, 1H) |
| 953 | [DMSO] 0,77 (t, 3H); 1,15 (d, 3H); 1,27 (m, 6H); 1,49 (m, 2H); 3,99 (m, 1H); 8,50 (d, br, 1H); 8,55 (s, 1H); 13,6 (br) |
| 956 | [DMSO] 0,88 (d, 6H); 1,15 (d, 3H); 1,27 (m, 1H); 1,48 (m, 1H); 1,62 (m, 1H); 4,10 (m, 1H); 8,50 (d, br, 1H); 8,55 (s, 1H); 13,6 (br, 1H) |
| 957 | [DMSO] 0,91 (s, 9H); 1,09 (d, 3H); 3,80 (m, 1H); 8,39 (d, br, 1H); 8,49 (s, 1H); 13,6 (br, 1H) |
| 958 | [DMSO] 0,89 (d, 6H); 1,11 (d, 3H); 1,75 (m, 1H); 3,86 (m, 1H); 8,48 (d, br, 1H); 8,52 (s, 1H); 13,6 (br) |
| 960 | [CDCl3] 0,95 (t, 6H); 1,50 (m, 2H); 1,62 (m, 2H), 4,00 (m, 1H); 8,73 (s, 1H); 8,90 (d, br, 1H) |
| 963 | [CDCl3] 3,43 (s, 6H); 3,65 (t, 2H); 4,50 (t, 1H); 8,62 (s, 1H); 8,78 (br, 1H) |
| 965 | [DMSO] 3,40 (m, 2H); 3,52 (m, 2H); 8,57 (s, 1H); 8,78 (t, br, 1H); 13,6 (br, 1H) |
| 966 | [DMSO] 3,29 (s, 3H); 3,48 (m, 4H); 8,54 (s, 1H); 8,78 (br, 1H); 13,6 (br, 1H) |
| 973 | [CDCl3] 1,29 (d, 3H); 3,40 (s, 3H); 3,45 (m, 2H); 4,36 (m, 1H); 8,60 (s, 1H); 8,72 (br, 1H) |
| 975 | [DMSO] 1,75 (m, 2H); 3,20 (s, 3H); 3,38 (m, 4H); 8,51 (s, 1H); 8,81 (t, br, 1H); 13,6 (br) |
| 1023 | [DMSO] 8,22 (br, 1H); 8,35 (br, 1H); 8,61 (s, 1H); 13,8 (br, 1H) |
| 1024 | [DMSO] 2,82 (d, 3H); 8,50 (s, 1H); 8,75 (br, 1H); 13,7 (br, 1H) |
| 1025 | [DMSO] 1,13 (t, 3H); 3,35 (m, 2H); 8,52 (s, 1H); 8,75 (br, 1H); 13,5 (br, 1H) |
| 1026 | [DMSO] 0,90 (t, 3H); 1,53 (m, 2H); 3,25 (m, 2H); 8,52 (s, 1H); 8,75 (t, br, 1H); 13,5 (br, 1H) |
| 1027 | [DMSO] 1,18 (d, 6H); 4,08 (m, 1H); 8,54 (s, 1H); 8,56 (br, 1H) |
| 1028 | [DMSO] 0,58 (m, 2H); 0,75 (m, 2H); 3,86 (m, 1H); 8,48 (s, 1H); 8,75 (br, 1H) |
| 1029 | [DMSO] 0,89 (t, 3H); 1,34 (m, 2H); 1,50 (m, 2H); 3,26 (m, 2H); 8,51 (s, 1H); 8,81 (br, 1H); 13,6 (br, 1H) |
| 1030 | [DMSO] 1,70 (m, 2H); 2,04 (m, 2H); 2,25 (m, 2H); 4,39 (m, 1H); 8,53 (s, 1H); 8,91 (d, br, 1H); 13,4 (br, 1H) |
| 1031 | [CDCl3] 1,46 (s, 9H); 8,63 (s, 1H); 8,83 (br, 1H); 111,9 (br, 1H) |
| 1032 | [DMSO] 2,82 (s, br, 3H); 2,96 (s, br, 3H); 8,11 (s, 1H); 12,5 (br, 1H) |
| 1037 | [CDCl3] 1,23 (t, 6H); 3,42 (br, 4H); 7,88 (s, 1H) |
| 1045 | [DMSO] 0,25 (m, 2H); 0,46 (m, 2H); 1,04 (m, 1H); 3,20 (t, 2H); 8,55 (s, 1H); 8,82 (t, br, 1H); 13,6 (br, 1H) |
| 1047 | [DMSO] 0,90 (s, 9H); 3,13 (d, 2H); 8,48 (s, 1H); 8,61 (t, br, 1H) |
| 1053 | [DMSO] 0,90 (d, 6H); 1,82 (m, 1H); 3,13 (m, 2H); 8,50 (s, 1H); 8,75 (br, 1H); 13,5 (br, 1H) |
| 1055 | [DMSO] 0,89 (d, 6H); 1,41 (m, 2H); 1,61 (m, 1H); 3,31 (m, 2H); 8,49 (s, 1H); 8,90 (br, 1H); 13,5 (br, 1H) |
| 1057 | [DMSO] 3,95 (t, 2H); 5,18 (dd, 2H); 5,90 (m, 1H); 8,50 (s, 1H); 8,85 (t, br, 1H) |
| 1061 | [CDCl3] 1,80 (s, 3H); 4,03 (d, 2H); 4,93 (d, 2H); 8,76 (s, 1H); 9,22 (t, br, 1H); |
| 1062 | [DMSO] 3,18 (m, 1H); 4,10 (m, 2H); 8,48 (s, 1H); 9,00 (br, 1H) |
| 1064 | [DMSO] 0,88 (t, 3H); 1,16 (d, 3H); 1,51 (m, 2H); 3,93 (m, 1H); 8,50 (d, br, 1H); 8,55 (s, 1H); 13,6 (br, 1H) |
| 1066 | [DMSO] 0,86 (t, 3H); 1,15 (d, 3H); 1,30 (m, 2H), 1,48 (m, 2H); 4,00 (m, 1H); 8,50 (d, br, 1H); 8,52 (s, 1H); 13,6 (br, 1H) |
| 1067 | [CDCl3] 0,86 (t, 3H); 1,22 (d, 3H); 1,25 (m, 6H); 1,48 (m, 2H); 4,16 (m, 1H); 8,62 (s, 1H); 8,68 (br, 1H) |
| 1070 | [CDCl3] 0,93 (d, 6H); 1,23 (d, 3H); 1,27 (m, 1H); 1,50 (m, 1H); 1,70 (m, 1H); 4,25 (m, 1H); 8,63 (br, 2H) |
| 1071 | [CDCl3] 0,95 (s, 9H); 1,17 (d, 3H); 4,08 (m, 1H); 8,70 (s, 1H); 9,00 (d, br, 1H) |
| 1072 | [CDCl3] 0,95 (m, 6H); 1,20 (d, 3H); 1,82 (m, 1H); 4,07 (m, 1H); 8,76 (s, 1H); 9,13 (br, 1H) |
| 1074 | [CDCl3] 0,92 (t, 6H); 1,50 (m, 2H); 1,65 (m, 2H), 3,96 (m, 1H); 8,72 (s, 1H); 9,00 (d, br, 1H) |
| 1077 | [CDCl3] 3,42 (s, 6H); 3,62 (t, 2H); 4,50 (t, 1H); 8,62 (s, 1H); 8,90 (br, 1H) |
| 1079 | [CDCl3] 3,65 (m, 2H); 3,85 (m, 2H); 8,64 (s, 1H); 9,22 (br, 1H) |
| 1080 | [DMSO] 3,30 (s, 3H); 3,46 (m, 4H); 8,52 (s, 1H); 8,75 (br, 1H) |
| 1087 | [CDCl3] 1,29 (d, 3H); 3,39 (s, 3H); 3,45 (m, 2H); 4,37 (m, 1H); 8,66 (s, 1H);8,98 (br, 1H); 12,50 (br,1H) |
| 1089 | [DMSO] 1,75 (m, 2H); 3,23 (s, 3H); 3,38 (m, 4H); 8,52 (s, 1H); 8,80 (t, br, 1H) |
| 1094 | [CDCl3] 3,80 (s, 3H); 4,24 (d, 2H); 8,61 (s, 1H); 9,19 (br, 1H) |
| 1138 | [DMSO] 2,83 (d, 3H); 7,07 (t, 1H); 8,40 (s, 1H); 8,94 (br, 1H); 13,4 (br, 1H) |
| 1139 | [DMSO] 1,12 (t, 3H); 3,33 (m, 2H); 7,08 (t, 1H); 8,40 (s, 1H); 9,00 (br, 1H); 13,4 (br, 1H) |
| 1171 | [DMSO] 3,90 (t, 2H); 5,15 (dd, 2H); 5,90 (m, 1H); 7,10 (t, 1H); 8,40 (s, 1H); 9,16 (br, 1H); 13,4 (br, 1H) |
| 1178 | [CDCl3] 0,95 (t, 3H); 1,21 (d, 3H); 1,58 (m, 2H); 4,08 (m, 1H); 6,86 (t, 1H); 8,70 (s, 1H); 9,34 (d, br, 1H); 12,9 (br, 1H) |
| 1180 | [CDCl3] 0,93 (t, 3H); 1,21 (d, 3H); 1,40 (m, 2H); 1,52 (m, 2H); 4,16 (m, 1H); 6,87 (t, 1H); 8,70 (s, 1H); 9,31 (d, br, 1H); 12,9 (br, 1H) |
| 1188 | [CDCl3] 0,92 (t, 6H); 1,50 (m, 2H); 1,65 (m, 2H); 3,98 (m, 1H); 6,86 (t, 1H); 8,71 (s, 1H); 9,28 (d, br, 1H); 12,9 (br, 1H) |
| 1201 | [CDCl3] 1,27 (d, 3H); 3,40 (s, 3H); 3,43 (m, 2H); 4,37 (m, 1H); 6,85 (t, 1H); 8,68 (s, 1H); 9,42 (d, br, 1H); 12,7 (br, 1H) |
| 1251 | [DMSO] 8,26 (br, 1H), 8,42 (br, 1H); 8,62 (s, 1H) |
| 1252 | [CDCl3] 3,00 (d, 3H); 8,66 (s, 1H); 8,82 (br, 1H) |
| 1253 | [CDCl3] 1,24 (t, 3H); 3,50 (m, 2H); 8,68 (s, 1H); 8,86 (br, 1H) |
| 1254 | [CDCl3] 1,00 (t, 3H); 1,62 (m, 2H); 3,42 (m, 2H); 8,67 (s, 1H); 8,90 (br, 1H) |
| 1255 | [CDCl3] 1,25 (d, 6H); 4,25 (m, 1H); 8,70 (s, 1H); 8,84 (br, 1H) |
| 1256 | [CDCl3] 0,60 (m, 2H); 0,89, (m, 2H); 3,00 (m, 1H); 8,70 (s, 1H); 9,00 (br, 1H) |
| 1257 | [CDCl3] 0,96 (t, 3H); 1,42, (m, 2H); 1,60 (m, 2H); 3,45 (m, 2H); 8,70 (s, 1H); 8,88 (br, 1H); 12,1 (br, 1H) |
| 1258 | [CDCl3] 1,77 (m, 2H); 1,98, (m, 2H); 2,43 (m, 2H); 4,55 (m, 1H); 8,68 (s, 1H); 9,13 (br, 1H); 12,5 (br, 1H) |
| 1259 | [CDCl3] 1,45 (s, 9H); 8,67 (s, 1H); 8,78 (br, 1H); 12,4 (br, 1H) |
| 1260 | [CDCl3] 3,11 (s, 6H); 7,95 (s, 1H) |
| 1265 | [CDCl3] 1,25 (t, 6H); 3,45 (br, 4H); 7,89 (s, 1H) |
| 1273 | [CDCl3] 0,27 (m, 2H); 0,56, (m, 2H); 1,04 (m, 1H); 3,32 (m, 2H); 8,67 (s, 1H); 8,92 (br, 1H); 12,3 (br, 1H) |
| 1275 | [CDCl3] 0,97 (s, 9H); 3,29 (d, 2H); 8,73 (s, 1H); 9,00 (br, 1H); 11,9 (br, 1H) |
| 1281 | [CDCl3] 0,98 (d, 6H); 1,89 (m, 1H); 3,29 (t, 2H); 8,70 (s, 1H); 8,98 (br, 1H); 12,1 (br, 1H) |
| 1283 | [CDCl3] 0,95 (d, 6H); 1,50 (m, 2H); 1,68 (m 1H); 3,46 (m, 2H); 8,69 (s, 1H); 8,83 (br, 1H); 12,1 (br, 1H) |
| 1285 | [CDCl3] 4,09 (t, 2H); 5,22 (dd, 2H); 5,91 (m, 1H); 8,70 (s, 1H); 9,01 (br, 1H); 12,2 (br, 1H) |
| 1289 | [DMSO] 0,26 (m, 2H); 0,47 (m, 2H); 1,05 (m, 1H); 3,20 (m, 2H); 6,85 (tt, 1H); 7,25 (br, 1H); 8,45 (d, 1H); 9,15 (br, 1H), 13,4 (br, 1H) |
| 1290 | [CDCl3] 2,25 (m, 1H); 4,23 (m, 2H); 8,72 (s, 1H); 9,20 (br, 1H), 12,0 (br, 1H) |
| 1292 | [CDCl3] 0,95 (t, 3H); 1,21 (d, 3H); 1,55 (m 2H); 4,10 (m, 1H); 8,70 (s, 1H); 8,82 (br, 1H) |
| 1294 | [CDCl3] 0,93 (t, 3H); 1,22 (d, 3H); 1,38 (m, 2H); 1,52 (m 2H); 4,18 (m, 1H); 8,70 (s, 1H); 8,79 (br, 1H); 12,4 (br, 1H) |
| 1295 | [CDCl3] 0,89 (t, 3H); 1,20 (d, 3H); 1,25-1,4 (m, 6H); 1,50 (m, 2H); 4,16 (m, 1H); 8,70 (s, 1H); 8,75 (br, 1H) |
| 1298 | [CDCl3] 0,92 (t, 6H); 1,40 (d, 3H); 1,32 (m, 1H); 1,45 (m, 1H); 1,55 (m, 1H); 4,25 (m, 1H); 8,70 (s, 1H); 8,75 (br, 1H); 12,4 (br, 1H) |
| 1299 | [CDCl3] 0,95 (s, 9H); 1,15 (d, 3H); 4,10 (m, 1H); 8,72 (s, 1H); 8,88 (br, 1H); 11,9 (br, 1H) |
| 1300 | [CDCl3] 0,95 (m, 6H); 1,15 (d, 3H); 1,77 (m, 1H); 4,05 (m, 1H); 8,71 (s, 1H); 8,85 (br, 1H) |
| 1302 | [CDCl3] 0,93 (t, 6H); 1,48 (m, 2H); 1,65 (m, 2H); 3,99 (m, 1H); 8,71 (s, 1H); 8,76 (br, 1H); 12,4 (br, 1H) |
| 1305 | [CDCl3] 3,40 (s, 6H); 3,62 (t, 2H); 4,50 (t, 1H); 8,60 (s, 1H); 8,74 (br, 1H); 11,7 (br, 1H) |
| 1307 | [CDCl3] 3,65 (m, 2H); 3,87 (m, 2H); 4,50 (t, 1H); 8,66 (s, 1H); 9,11 (br, 1H) |
| 1308 | [CDCl3] 3,40 (s, 3H); 3,58 (m, 2H); 3,67 (m, 2H); 8,60 (s, 1H); 8,75 (br, 1H); 11,7 (br, 1H) |
| 1315 | [CDCl3] 1,48 (d, 3H); 3,38 (s, 3H); 3,44 (m, 2H); 4,38 (m, 1H); 8,62 (s, 1H); 8,72 (br, 1H); 12,1 (br, 1H) |
| 1317 | [CDCl3] 1,90 (m, 2H); 3,40 (s, 3H); 3,58 (m, 4H); 8,39 (s, 1H); 8,62 (br, 1H) |
| 1322 | [CDCl3] 3,79 (s, 3H); 4,25 (d, 2H); 8,65 (s, 1H); 9,18 (br, 1H) |
| 1365 | [DMSO] 8,24 (br, 1H), 8,42 (br, 1H); 8,81 (s, 1H); 13,6 (br, 1H) |
| 1366 | [DMSO] 8,19 (br, 1H), 8,34 (br, 1H); 8,78 (s, 1H); 13,7 (br, 1H) |
| 1368 | [DMSO] 2,83 (d, 3H); 6,81 (tt, 1H); 7,22 (br, 1H); 8,41 (d, 1H); 9,00 (br, 1H); 13,4 (br, 1H) |
| 1369 | [DMSO] 1,12 (t, 3H); 3,35 (m, 2H); 6,85 (tt, 1H); 7,20 (br, 1H); 8,41 (d, 1H); 9,20 (br, 1H); 13,4 (br, 1H) |
| 1370 | [DMSO] 0,89 (t, 3H); 1,52 (m, 2H); 3,27 (q, 2H); 6,82 (tt, 1H); 7,20 (br, 1H); 8,40 (d, 1H); 9,20 (br, 1H); 13,4 (br, 1H) |
| 1371 | [DMSO] 1,18 (d, 6H); 4,09 (m, 1H); 6,82 (tt, 1H); 7,20 (br, 1H); 8,42 (d, 1H); 8,95 (br, 1H); 13,4 (br, 1H) |
| 1372 | [DMSO] 0,56 (m, 2H); 0,75 (m, 2H); 2,85 (m, 1H); 6,83 (tt, 1H); 7,21 (br, 1H); 8,40 (d, 1H); 9,05 (br, 1H); 13,4 (br, 1H) |
| 1373 | [DMSO] 0,89 (t, 3H); 1,32 (m, 2H); 1,50 (m, 2H); 3,30 (q, 2H); 6,84 (tt, 1H); 7,20 (br, 1H); 8,41 (d, 1H); 9,10 (br, 1H); 13,4 (br, 1H) |
| 1389 | [DMSO] 0,26 (m, 2H); 0,47 (m, 2H); 1,05 (m, 1H); 3,20 (m, 2H); 6,85 (tt, 1H); 7,25 (br, 1H); 8,45 (d, 1H); 9,15 (br, 1H), 13,4 (br, 1H) |
| 1391 | [DMSO] 0,91 (s, 9H); 3,16 (d, 2H); 6,82 (tt, 1H); 7,10 (br, 1H); 8,41 (d, 1H); 9,20 (br, 1H); 13,3 (br, 1H) |
| 1397 | [DMSO] 0,90 (d, 6H); 1,82 (m, 1H); 3,15 (m, 2H); 6,82 (tt, 1H); 7,20 (br, 1H); 8,40 (d, 1H); 9,15 (br, 1H), 13,4 (br, 1H) |
| 1401 | [DMSO] 3,95 (m, 2H); 5,18 (dd, 2H); 5,92 (m, 1H); 6,82 (tt, 1H); 7,20 (d, 1H); 8,42 (d, 1H); 9,20 (br, 1H); 13,3 (br, 1H) |
| 1406 | [DMSO] 3,15 (t, 1H); 4,13 (m, 2H); 6,82 (tt, 1H); 7,18 (br, 1H); 8,41 (d, 1H); 9,35 (br, 1H); 13,3 (br, 1H) |
| 1408 | [DMSO] 0,87 (t, 3H); 1,14 (d, 3H); 1,51 (m, 2H); 3,92 (m, 1H); 6,82 (tt, 1H); 7,20 (br, 1H); 8,41 (d, 1H); 8,90 (br, 1H), 13,4 (br, 1H) |
| 1410 | [DMSO] 0,86 (t, 3H); 1,15 (d, 3H); 1,30 (m, 2H); 1,48 (m, 2H); 4,02 (m, 1H); 6,83 (tt, 1H); 7,20 (br, 1H); 8,42 (d, 1H); 8,90 (br, 1H), 13,4 (br, 1H) |
| 1411 | [DMSO] 0,88 (t, 3H); 1,15 (d, 3H); 1,27 (m, 6H); 1,49 (m, 2H); 4,00 (m, 1H); 6,82 (tt, 1H); 7,20 (br, 1H); 8,42 (d, 1H); 8,92 (br, 1H), 13,4 (br, 1H) |
| 1414 | [DMSO] 0,88 (d, 6H); 1,15 (d, 3H); 1,28 (m, 1H); 1,46 (m, 1H); 1,60 (m, 1H); 4,10 (m, 1H); 6,81 (tt, 1H); 7,20 (br, 1H); 8,42 (d, 1H); 8,90 (br, 1H), 13,4 (br, 1H) |
| 1418 | [CDCl3] 0,93 (t, 6H); 1,50 (m, 2H); 1,65 (m, 2H); 4,00 (m, 1H); 6,83 (d, 1H); 8,69 (d, 1H); 9,21 (d, br, 1H); 13,0 (br, 1H) |
| 1421 | [CDCl3] 3,41 (s, 6H); 3,52 (t, 2H); 4,50 (t, 1H); 6,12 (tt, 1H); 6,81 (d, 1H); 8,64 (d, 1H); 9,41 (br, 1H); 12,5 (br, 1H) |
| 1424 | [CDCl3] 3,37 (s, 3H); 3,56 (m, 2H); 3,67 (m, 2H); 6,11 (tt, 1H); 6,84 (d, 1H); 8,65 (d, 1H); 9,46 (br, 1H), 12,2 (br, 1H) |
| 1431 | [CDCl3] 1,28 (d, 3H); 3,39 (s, 3H); 3,45 (m, 2H); 4,40 (m, 1H); 6,10 (tt, 1H); 6,83 (d, 1H); 8,67 (d, 1H); 9,39 (d, br, 1H); 13,0 (br, 1H) |
| 1433 | [DMSO] 1,75 (m, 2H); 3,24 (s, 3H); 3,38 (m, 4H); 6,83 (tt, 1H); 7,20 (br, 1H); 8,42 (d, 1H); 9,15 (br, 1H), 13,4 (br, 1H) |
| 1481 | [DMSO] 7,42 (br, 1H); 8,10 (br, 1H); 8,40 (br, 1H); 8,46 (d, 1H), 13,6 (br, 1H) |
| 1482 | [CDCl3] 3,01 (d, 3H); 6,88 (d, 1H); 8,67 (d, 1H); 9,34 (br, 1H); 12,5 (br, 1H) |
| 1483 | [CDCl3] 1,27 (t, 3H); 3,50 (m, 2H); 6,88 (d, 1H); 8,68 (d, 1H); 9,32 (br, 1H); 12,6 (br, 1H) |
| 1484 | [CDCl3] 1,00 (t, 3H); 1,63 (m, 2H); 3,41 (m, 2H); 6,88 (d, 1H); 8,70 (d, 1H); 9,38 (br, 1H); 12,9 (br, 1H) |
| 1485 | [CDCl3] 1,25 (d, 6H); 4,27 (m, 1H); 6,88 (d, 1H); 8,70 (d, 1H); 9,30 (d, br, 1H); 13,4 (br, 1H) |
| 1486 | [CDCl3] 0,62 (m, 2H); 0,86 (m, 2H); 3,01 (m, 1H); 6,88 (d, 1H); 8,70 (d, 1H); 9,41 (br, 1H); 12,6 (br, 1H) |
| 1487 | [CDCl3] 0,95 (t, 3H); 1,44 (m, 2H); 1,61 (m, 2H); 3,45 (m, 2H); 6,88 (d, 1H); 8,70 (d, 1H); 9,35 (t, br, 1H); 12,7 (br, 1H) |
| 1488 | [CDCl3] 1,78 (m, 2H); 2,00 (m, 2H); 2,42 (m, 2H); 4,58 (m, 1H); 6,88 (d, 1 H); 8,68 (d, 1H); 9,60 (d, br, 1H); 13,3 (br, 1H) |
| 1503 | [CDCl3] 0,26 (m, 2H); 0,55 (m, 2H); 1,06 (m, 1H); 3,33 (m, 2H); 6,88 (d, 1H); 8,70 (d, 1H); 9,45 (br, 1H); 12,9 (br, 1H) |
| 1505 | [CDCl3] 0,98 (s, 9H); 3,30 (d, 2H); 6,88 (d, 1H); 8,72 (d, 1H); 9,43 (t, br, 1H); 12,5 (br, 1H) |
| 1511 | [CDCl3] 0,98 (d, 6H); 1,90 (m, 1H); 3,30 (m, 2H); 6,88 (d, 1H); 8,70 (d, 1H); 9,42 (t, br, 1H) |
| 1513 | [CDCl3] 0,85 (d, 6H); 1,51 (m, 2H); 1,70 (m, 1H); 3,47 (m, 2H); 6,88 (d, 1H); 8,70 (d, 1H); 9,32 (t, br, 1H); 12,7 (br, 1H) |
| 1515 | [CDCl3] 4,10 (t, 2H); 5,25 (dd, 2H); 5,91 (m, 1H); 6,88 (d, 1H); 8,70 (d, 1H); 9,47 (br, 1H); 12,8 (br, 1H) |
| 1519 | [DMSO] 1,71 (s, 3H); 3,88 (d, 2H); 4,84 (d, 2H); 7,35 (br, 1H); 8,41 (d, 1H); 9,02 (br, 1H); 13,4 (br, 1H) |
| 1520 | [DMSO] 3,15 (m, 1H); 4,12 (m, 2H); 7,36 (d, br, 1H); 8,42 (d, 1H); 9,15 (br, 1H), 13,4 (br, 1H) |
| 1522 | [CDCl3] 0,95 (t, 3H); 1,22 (d, 3H); 1,59 (m, 2H); 4,11 (m, 1H); 6,88 (d, 1H); 8,71 (d, 1H); 9,28 (d, br, 1H); 13,4 (br, 1H) |
| 1524 | [CDCl3] 0,92 (t, 3H); 1,23 (d, 3H); 1,40 (m, 2H); 1,52 (m, 2H); 4,20 (m, 1H); 6,89 (d, 1H); 8,71 (d, 1H); 9,27 (d, br, 1H); 13,4 (br, 1H) |
| 1525 | [CDCl3] 0,88 (t, 3H); 1,23 (d, 3H); 1,25-1,4 (m, 6H); 1,55 (m, 2H); 4,18 (m, 1H); 7,89 (d, 1H); 8,70 (d, 1H); 9,28 (br, 1H); 13,2 (br, 1H) |
| 1528 | [CDCl3] 0,92 (d, 6H); 1,22 (d, 3H), 1,35 (m, 1 H); 1,50 (m, 1H); 1,68 (m, 1H); 4,28 (m, 1H); 6,89 (d, 1H); 8,71 (d, 1H); 9,26 (d, br, 1H); 13,3 (br, 1H) |
| 1529 | [CDCl3] 0,95 (s, 9H); 1,17 (d, 3H); 4,12 (m, 1H); 6,89 (d, 1H); 8,73 (d, 1H); 9,30 (d, br, 1H), 12,8 (br, 1H) |
| 1530 | [CDCl3] 0,95 (dt, 6H); 1,17 (d, 3H); 1,82 (m, 1H); 4,08 (m, 1H); 6,89 (d, 1H); 8,72 (d, 1H); 9,31 (d, br, 1H); 13,2 (br, 1H) |
| 1532 | [CDCl3] 0,95 (t, 6H); 1,50 (m, 2H); 1,66 (m, 2H); 4,00 (m, 1H); 6,89 (d, 1H); 8,72 (d, 1H); 9,21 (d, br, 1H); 13,3 (br, 1H) |
| 1535 | [DMSO] 3,32 (s, 6H); 3,45 (t, 2H); 4,50 (t, 1H); 7,33 (d, br, 1H); 8,43 (d, 1H); 8,92 (br, 1H); 13,4 (br, 1H) |
| 1537 | [DMSO] 3,38 (m, 2H); 3,52 (m, 2H); 4,80 (br, 1H); 7,37 (br, 1H); 8,45 (d, 1H); 8,96 (br, 1H); 13,4 (br, 1H) |
| 1538 | [DMSO] 3,25 (s, 3H); 3,49 (m, 4H); 7,37 (d, br, 1H); 8,44 (d, 1H); 8,97 (br, 1H); 13,5 (br, 1H) |
| 1545 | [DMSO] 1,15 (d, 3H); 3,27 (s, 3H); 3,40 (m, 2H); 4,19 (m, 1H); 7,35 (d, br, 1H); 8,43 (d, 1H); 8,82 (br, 1H); 13,4 (br, 1H) |
| 1547 | [DMSO] 1,75 (m, 2H); 3,25 (s, 3H); 3,40 (m, 4H); 7,38 (br, 1H); 8,40 (d, 1H); 8,94 (br, 1H); 13,4 (br, 1H) |
| 1595 | [DMSO] 7,45 (d, 1H); 8,15 (br, 1H); 8,40 (br, 1H); 8,50 (d, 1H); 13,7 (br, 1H) |
| 1596 | [DMSO] 2,82 (d, 3H); 7,42 (d, 1H); 8,41 (d, 1H); 8,80 (br, 1H); 13,4 (br, 1H) |
| 1597 | [DMSO] 1,12 (t, 3H); 3,31 (m, 2H); 7,41 (d, 1H); 8,41 (d, 1H); 8,85 (br, 1H); 13,45 (br, 1H) |
| 1598 | [DMSO] 0,90 (t, 3H); 1,54 (m, 2H); 3,25 (q, 2H); 7,41 (d, 1H); 8,40 (d, 1H); 8,85 (br, 1H); 13,45 (br) |
| 1599 | [DMSO] 1,17 (d, 6H); 4,10 (m, 1H); 7,40 (d, 1H); 8,41 (d,1H); 8,67 (d, br, 1H); 13,4 (br, 1H) |
| 1600 | [DMSO] 0,57 (m, 2H); 0,75 (m, 2H); 2,86 (m, 1H); 7,41 (d, 1H); 8,37 (d, 1H); 8,80 (br, 1H); 13,35 (br, 1H) |
| 1601 | [CDCl3] 0,95 (t, 3H); 1,40 (m, 2H); 1,60 (m, 2H); 3,45 (q, 2H); 6,88 (d, 1H); 8,71 (d, 1H); 9,28 (br, 1H); 12,9 (br, 1H) |
| 1602 | [CDCl3] 1,78 (m, 2H); 1,95 (m, 2H); 2,40 (m, 2H); 4,57 (m, 1H); 6,86 (d, 1H); 8,70 (d, 1H); 9,45 (d, br, 1H); 12,9 (br, 1H) |
| 1603 | [CDCl3] 1,45 (s, 9H); 6,85 (d, 1H); 8,67 (d, 1H); 9,15 (br, 1H); 12,6 (br, 1H) |
| 1604 | [CDCl3] 3,05 (s, 6H); 7,07 (d, 1H); 7,80 (d, 1H) |
| 1609 | [CDCl3] 1,22 (t, 6H); 3,40 (m, 4H); 7,02 (d, 1H); 7,72 (d, 1H) |
| 1617 | [DMSO] 0,25 (m, 2H), 0,46 (m, 2H); 1,05 (m, 1H); 3,22 (m, 2H); 7,45 (d, br, 1H); 8,45 (d, 1H); 8,95 (br, 1H); 13,4 (br, 1H) |
| 1619 | [DMSO] 0,90 (s, 9H), 3,15 (d, 2H); 7,38 (d, br, 1H); 8,40 (d, 1H); 8,80 (br, 1H); 13,4 (br, 1H) |
| 1625 | [DMSO] 0,90 (d, 6H); 1,82 (m, 1H); 3,14 (t, 2H); 7,39 (d, 1H); 8,40 (d, 1H); 8,91 (t, br, 1H) |
| 1627 | [CDCl3] 0,92 (d, 6H); 1,49 (m, 2H); 1,68 (m, 1H); 3,46 (m, 2H); 6,87 (d, 1H); 8,70 (d, 1H); 9,22 (br, 1H); 12,8 (br, 1H) |
| 1629 | [DMSO] 3,95 (m, 2H); 5,15 (dd, 2H); 5,90 (m, 1H); 7,40 (d, 1H); 8,40 (d, 1H); 8,95 (br, 1H); 13,4 (br, 1H) |
| 1633 | [CDCl3] 1,78 (s, 3H); 4,02 (d, 2H); 4,88 (d, 2H); 6,87 (d 1H); 8,72 (d, 1H); 9,42 (br, 1H); 12,7 (br, 1H) |
| 1634 | [CDCl3] 2,23 (m, 1H); 4,23 (m, 2H); 6,88 (d, 1H); 8,73 (d, 1H); 9,51 (br, 1H), 12,8 (br, 1H) |
| 1636 | [DMSO] 0,89 (t, 3H); 1,14 (d, 3H); 1,51 (m, 2H); 3,95 (m, 1H); 7,41 (d, 1H); 8,40 (d, 1H); 8,60 (br, 1H); 13,6 (br, 1 H) |
| 1638 | [DMSO] 0,87 (t, 3H); 1,15 (d, 3H); 1,32 (m, 2H); 1,46 (m, 2H); 4,03 (m, 1H); 7,40 (d, 1H); 8,40 (d, 1H); 8,51 (br, 1H); 13,4 (br, 1H) |
| 1639 | [CDCl3] 0,88 (t, 3H); 1,21 (d, 3H); 1,32 (m, 6H); 1,50 (m, 2H); 4,18 (m, 1H); 6,88 (d, 1H); 8,71 (d, 1H); 9,12 (d, br, 1H), 12,9 (br, 1H) |
| 1642 | [CDCl3] 0,92 (t, 6H); 1,20 (d, 3H); 1,32 (m, 1H); 1,46 (m, 1H); 1,67 (m, 1H); 4,27 (m, 1H); 6,88 (d, 1H); 8,71 (d, 1H); 9,12 (d, br, 1H), 12,9 (br, 1H) |
| 1643 | [CDCl3] 0,93 (s, 9H); 1,15 (d, 3H); 4,12 (m, 1H); 6,88 (d, 1H); 8,72 (d, 1H); 9,22 (d, br, 1H) |
| 1644 | [CDCl3] 0,95 (m, 6H); 1,15 (d, 3H); 1,78 (m, 1H); 4,05 (m, 1H); 6,88 (d, 1H); 8,72 (d, 1H); 9,18 (d, br, 1H); 12,7 (br, 1H) |
| 1646 | [DMSO] 0,87 (t, 6H); 1,45 (m, 2H); 1,55 (m, 2H); 3,81 (m, 1H); 7,40 (d, 1H); 8,40 (d, 1H); 8,52 (d, br, 1H); 13,4 (br, 1H) |
| 1649 | [CDCl3] 3,40 (s, 6H); 3,61 (t, 2H); 4,50 (t, 1H); 6,87 (d, 1H); 8,69 (d, 1H); 9,32 (br, 1H); 12,3 (br, 1H) |
| 1651 | [CDCl3] 3,63 (m, 2H); 3,84 (m, 2H); 6,88 (d, 1H); 8,68 (d, 1H); 9,60 (br, 1H) |
| 1652 | [DMSO] 3,25 (s, 3H); 3,50 (m, 4H); 7,40 (d, br, 1H); 8,45 (d, 1H); 8,92 (br, 1H); 13,5 (br, 1H) |
| 1659 | [DMSO] 1,15 (d, 3H); 3,25 (s, 3H); 3,38 (m, 2H); 4,17 (m, 1H); 7,40 (d, br, 1H); 8,43 (d, 1H); 8,75 (br, 1H); 13,5 (br, 1H) |
| 1661 | [DMSO] 1,74 (m, 2H); 3,22 (s, 3H); 3,37 (m, 4H); 7,42 (d, br, 1H); 8,42 (d, 1H); 8,90 (br, 1H); 13,4 (br, 1H) |
| 1666 | [CDCl3] 3,77 (s, 3H); 4,23 (d, 2H); 6,87 (d, 1H); 8,67 (d, 1H); 9,65 (br, 1H); 12,1 (br, 1H) |
| 1711 | [DMSO] 2,83 (d, 3H); 8,39 (s, 1H); 8,72 (d, br, 1H); 13,5 (br) |
| 1712 | [DMSO] 1,11 (t, 3H); 3,30 (m, 2H); 8,40 (s, 1H); 8,75 (t, br, 1H) |
| 1713 | [CDCl3] 0,99 (t, 3H); 1,62 (m, 2H); 3,41 (q, 2H); 8,56 (s, 1H); 9,00 (br, 1H) |
| 1714 | [CDCl3] 1,24 (d, 6H); 4,22 (m, 1H); 8,41 (s, 1H); 9,17 (br, 1H) |
| 1715 | [CDCl3] 0,61 (m, 2H); 0,90 (m, 2H); 2,95 (m, 1H); 8,57 (s, 1H); 9,02 (br, 1H) |
| 1716 | [CDCl3] 0,94 (t, 3H); 1,41 (m, 2H); 1,58 (m, 2H); 3,45 (m, 2H); 8,54 (s, 1H); 9,05 (br, 1H) |
| 1717 | [CDCl3] 1,80 (m, 2H); 1,98 (m, 2H); 2,42 (m, 2H); 4,55 (m, 1H); 8,55 (s, 1H); 9,01 (br, 1H); 12,0 (br, 1H) |
| 1719 | [DMSO] 2,83 (s, 3H); 2,97 (s, 3H); 8,04 (s, 1H); 12,6 (br, 1H) |
| 1720 | [DMSO] 1,03 (t, 3H); 1,15 (t, 3H); 3,12 (q, 2H); 3,45 (q, 2H); 8,05 (s, 1H); 12,5 (br, 1H) |
| 1721 | [CDCl3] 0,25 (m, 2H); 0,55 (m, 2H); 1,05 (m, 1H); 3,32 (m, 2H); 8,57 (s, 1H); 8,93 (br, 1H); 12,1 (br, 1H) |
| 1722 | [CDCl3] 0,96 (s, 9H); 3,28 (d, 2H); 8,59 (s, 1H); 9,05 (br, 1H) |
| 1723 | [CDCl3] 0,95 (d, 6H); 1,90 (m, 1H); 3,29 (t, 2H); 8,59 (s, 1H); 9,00 (br, 1H); 12,0 (br, 1H) |
| 1725 | [CDCl3] 0,94 (d, 6H); 1,49 (q, 2H); 1,65 (m, 1H); 3,45 (m, 2H); 8,59 (s, 1H); 8,93 (br, 1H); 12,1 (br, 1H) |
| 1727 | [CDCl3] 4,08 (m, 2H); 5,21 (dd, 2H); 5,90 (m, 1H); 8,60 (s, 1H); 9,05 (br, 1H); 12,1 (br, 1H) |
| 1728 | [CDCl3] 1,78 (s, 3H); 4,01 (d, 2H); 4,90 (d, 2H); 8,61 (s, 1H); 9,09 (br, 1H); 11,9 (br, 1H) |
| 1729 | [CDCl3] 2,25 (m, 1H); 4,23 (m, 2H); 8,63 (s, 1H); 9,23 (br, 1H); 11,9 (br, 1H) |
| 1731 | [CDCl3] 0,95 (t, 3H); 1,23 (d, 3H); 1,58 (m, 2H); 4,11 (m, 1H); 8,55 (s, 1H); 8,60 (br, 1H) |
| 1733 | [CDCl3] 0,92 (t, 3H); 1,23 (d, 3H); 1,39 (m, 2H); 1,52 (m, 2H); 4,20 (m, 1H); 8,55 (s, 1H); 8,66 (br, 1H) |
| 1734 | [CDCl3] 0,87 (t, 3H); 1,22 (d, 3H); 1,35 (m, 6H); 1,52 (m, 2H); 4,17 (m, 1H); 8,52 (s, 1H); 8,61 (br, 1H) |
| 1735 | [CDCl3] 0,94 (m, 6H); 1,23 (d, 3H); 1,33 (m, 1H); 1,45 (m, 1H); 1,65 (m, 1H); 4,27 (m, 1H); 8,56 (s, 1H); 8,63 (br, 1H); 11,9 (br) |
| 1736 | [CDCl3] 0,96 (s, 9H); 1,17 (d, 3H); 4,08 (m, 1H); 8,57 (s, 1H); 8,80 (d, br, 1H) |
| 1737 | [CDCl3] 0,95 (m, 6H); 1,18 (d, 3H); 1,80 (m, 1H); 4,05 (m, 1H); 8,57 (s, 1H); 8,73 (d, br, 1H) |
| 1738 | [DMSO] 0,87 (t, 6H), 1,45 (m, 2H), 1,55 (m, 2H); 3,80 (m, 1H); 8,40 (s, 1H); 8,42 (d, br, 1H); 13,4 (br, 1H) |
| 1739 | [CDCl3] 3,42 (s, 6H); 3,63 (t, 2H); 4,50 (t, 1H); 8,49 (s, 1H); 8,74 (br, 1H); 11,4 (br, 1H) |
| 1741 | [CDCl3] 3,65 (m, 2H); 3,85 (m, 2H); 8,55 (s, 1 H); 9,16 (br, 1H) |
| 1742 | [CDCl3] 3,40 (s, 3H); 3,57 (m, 2H); 3,67 (m, 2H); 8,47 (s, 1H); 8,75 (br, 1H); 11,5 (br, 1H) |
| 1744 | [CDCl3] 1,27 (d, 3H); 3,39 (s, 3H); 3,45 (m, 2H); 4,37 (m, 1H); 8,45 (s, 1H); 8,57 (br, 1H); 11,6 (br, 1H) |
| 1745 | [CDCl3] 1,90 (m, 2H); 3,40 (s, 3H); 3,56 (m, 4H); 8,27 (s, 1H); 8,65 (br, 1H); 11,8 (br, 1H) |
| 1746 | [DMSO] 3,67 (s, 3H); 4,13 (d, 2H); 8,38 (s, 1H); 9,12 (br, 1H); 13,6 (br, 1H) |
| 1751 | [DMSO] 8,24 (br, 1H); 8,38 (br, 1H); 8,61 (br, 1H); 13,7 (br, 1H) |
| 1752 | [CDCl3] 3,00 (d, 3H); 8,71 (s, 1H); 8,91 (br, 1H); 12,2 (br, 1H) |
| 1753 | [CDCl3] 1,23 (t, 3H); 3,49 (m, 2H); 8,71 (s, 1H); 8,89 (br, 1H); 12,1 (br, 1H) |
| 1754 | [CDCl3] 0,98 (t, 3H); 1,62 (m, 2H); 3,40 (q, 2H); 8,70 (s, 1H); 8,90 (br, 1H) |
| 1755 | [CDCl3] 1,25 (d, 6H); 4,25 (m, 1H); 8,68 (br, 2H); 12,0 (br, 1H) |
| 1756 | [CDCl3] 0,61 (m, 2H); 0,89 (m, 2H); 2,95 (m, 1H); 8,70 (s, 1H); 8,97 (br, 1H) |
| 1757 | [CDCl3] 0,94 (t, 3H); 1,40 (m, 2H); 1,58 (m, 2H); 3,45 (m, 2H); 8,69 (s, 1H); 8,88 (br, 1H); 12,0 (br, 1H) |
| 1758 | [CDCl3] 1,80 (m, 2H); 1,98 (m, 2H); 2,41 (m, 2H); 4,55 (m, 1H); 8,67 (s, 1H); 8,98 (br, 1H); 12,1 (br, 1H) |
| 1759 | [CDCl3] 1,32 (s, 9H); 8,50 (br, 1H); 8,60 (s, 1H) |
| 1760 | [CDCl3] 3,12 (s, 6H); 7,99 (s, 1H) |
| 1761 | [CDCl3] 1,24 (t, 6H); 3,43 (m, 4H); 7,89 (s, 1H) |
| 1762 | [CDCl3] 0,28 (m, 2H); 0,55 (m, 2H); 1,03 (m, 1H); 3,31 (m, 2H); 8,67 (s, 1H); 8,85 (br, 1H); 11,9 (br, 1H) |
| 1763 | [CDCl3] 0,96 (s, 9H); 3,27 (d, 2H); 8,71 (s, 1H); 9,00 (br, 1H) |
| 1764 | [CDCl3] 0,95 (d, 6H); 1,89 (m, 1H); 3,28 (t, 2H); 8,70 (s, 1H); 8,97 (br, 1H) |
| 1766 | [CDCl3] 0,95 (d, 6H); 1,48 (q, 2H); 1,65 (m, 1H); 3,45 (m, 2H); 8,69 (s, 1H); 8,85 (br, 1H); 12,0 (br, 1H) |
| 1768 | [CDCl3] 4,08 (m, 2H); 5,21 (dd, 2H); 5,89 (m, 1H); 8,71 (s, 1H); 9,02 (br, 1H); 12,1 (br, 1H) |
| 1769 | [CDCl3] 1,77 (s, 3H); 4,01 (d, 2H); 4,87 (d, 2H); 8,71 (s, 1H); 9,03 (br, 1H) |
| 1770 | [CDCl3] 2,23 (m, 1H); 4,23 (m, 2H); 8,72 (s, 1H); 9,20 (br, 1H); 12,0 (br, 1H) |
| 1772 | [CDCl3] 0,95 (t, 3H); 1,21 (d, 3H); 1,55 (m, 2H); 4,10 (m, 1H); 8,67 (br, 1H); 8,70 (s, 1H); 12,1 (br, 1H) |
| 1774 | [CDCl3] 0,91 (t, 3H); 1,21 (d, 3H); 1,38 (m, 2H); 1,50 (m, 2H); 4,19 (m, 1H); 8,64 (br, 1H); 8,69 (s, 1H); 12,0 (br, 1H) |
| 1775 | [CDCl3] 0,89 (t, 3H); 1,21 (d, 3H); 1,33 (m, 6H); 1,51 (m, 2H); 4,17 (m, 1H); 8,60 (br, 1H); 8,68 (s, 1H) |
| 1776 | [CDCl3] 0,90 (m, 6H); 1,21 (d, 3H); 1,31 (m, 1H); 1,45 (m, 1H); 1,64 (m, 1H); 4,25 (m, 1H); 8,60 (br, 1H); 8,69 (s, 1H) |
| 1777 | [CDCl3] 0,94 (s, 9H); 1,15 (d, 3H); 4,08 (m, 1H); 8,69 (s, 1H); 8,79 (br, 1H) |
| 1778 | [CDCl3] 0,93 (m, 6H); 1,17 (d, 3H); 1,79 (m, 1H); 4,05 (m, 1H); 8,67 (s, 1H); 8,71 (br, 1H) |
| 1780 | [CDCl3] 0,92 (t, 6H); 1,48 (m, 2H); 1,62 (m, 2H); 3,96 (m, 1H); 8,61 (s, 1H); 9,16 (d, br, 1H) |
| 1781 | [CDCl3] 3,40 (s, 6H); 3,61 (t, 2H); 4,50 (t, 1H); 8,63 (s, 1H); 8,76 (br, 1H) |
| 1783 | [CDCl3] 3,64 (m, 2H); 3,86 (m, 2H); 8,68 (s, 1H); 9,17 (br, 1H) |
| 1784 | [CDCl3] 3,49 (s, 3H); 3,55 (m, 2H); 3,66 (m, 2H); 8,62 (s, 1H); 8,77 (br, 1H) |
| 1785 | [CDCl3] 1,27 (d, 3H); 3,39 (s, 3H); 3,44 (m, 2H); 4,37 (m, 1H); 8,56 (br, 1H); 8,60 (s, 1H); 11,9 (br, 1H) |
| 1786 | [CDCl3] 1,90 (m, 2H); 3,40 (s, 3H); 3,58 (m, 4H); 8,40 (s, 1H); 8,65 (br, 1H) |
| 1787 | [CDCl3] 3,78 (s, 3H); 4,24 (d, 2H); 8,69 (s, 1H); 9,25 (br, 1H); 11,5 (br, 1H) |
| 1792 | [DMSO] 8,22 (br, 1H); 8,37 (br, 1H); 8,81 (br, 1H); 13,7 (br, 1H) |
| 1793 | [CDCl3] 3,00 (d, 3H); 8,80 (s, br, 2H); 12,0 (br, 1H) |
| 1794 | [CDCl3] 1,24 (t, 3H); 3,49 (m, 2H); 8,40 (s, br, 2H); 12,0 (br, 1H) |
| 1795 | [DMSO] 0,89 (t, 3H); 1,53 (m, 2H); 3,26 (q, 2H); 8,72 (s, 1H); 8,78 (t, br, 1H); 13,4 (br, 1H) |
| 1796 | [DMSO] 1,18 (d, 6H); 4,08 (m, 1H); 8,60 (d,br, 2H); 13,4 (br, 1H) |
| 1797 | [DMSO] 0,60 (m, 2H); 0,75 (m, 2H); 2,88 (m, 1H); 8,68 (s, 1H); 8,77 (d, br, 1H); 13,3 (br, 1H) |
| 1798 | [CDCl3] 0,94 (t, 3H); 1,40 (m, 2H); 1,59 (m, 2H); 3,44 (m, 2H); 8,79 (s, br, 2H) |
| 1799 | [DMSO] 1,70 (m, 2H); 2,05 (m, 2H); 2,27 (m, 2H); 4,39 (m, 1H); 8,71 (s, 1H); 8,95 (d, br, 1H); 13,4 (br, 1H) |
| 1800 | [CDCl3] 1,43 (s, 9H); 8,70 (br, 1H); 8,85 (s, 1H); 11,5 (br) |
| 1801 | [CDCl3] 3,10 (s, 6H); 8,20 (s, 1H) |
| 1802 | [CDCl3] 1,23 (t, 6H); 3,43 (m, br, 4H); 8,12 (s, 1H) |
| 1803 | [DMSO] 0,26 (m, 2H); 0,50 (m, 2H); 1,05 (m, 1H); 3,19 (t, 2H); 8,76 (s, 1H); 8,87 (br, 1H); 13,5 (br, 1H) |
| 1804 | [DMSO] 0,91 (s, 9H); 3,13 (d, 2H); 8,61 (t, br, 1H); 8,68 (s, 1H); 13,4 (br, 1H) |
| 1805 | [DMSO] 0,89 (d, 6H); 1,84 (m, 1H); 3,14 (t, 2H); 8,70 (s, 1H); 8,75 (t, br, 1H); 13,4 (br, 1H) |
| 1807 | [DMSO] 0,89 (d, 6H); 1,40 (q, 2H); 1,62 (m, 1H); 3,30 (m, 2H); 8,71 (s, 1H); 8,73 (t, br, 1H); 13,4 (br, 1H) |
| 1809 | [DMSO] 3,93 (m, 2H); 5,18 (dd, 2H); 5,90 (m, 1H); 8,71 (s, 1H); 8,89 (t, br, 1H); 13,4 (br, 1H) |
| 1810 | [DMSO] 1,72 (s, 3H); 3,87 (d, 2H); 4,85 (d, 2H); 8,71 (s, 1H); 8,87 (t, br, 1H); 13,3 (br, 1H) |
| 1811 | [DMSO] 3,18 (m, 1H); 4,10 (m, 2H); 8,69 (s, 1H); 9,01 (t, br, 1H); 13,4 (br, 1H) |
| 1813 | [DMSO] 0,87 (t, 3H); 1,14 (d, 3H); 1,50 (m, 2H); 3,90 (m, 1H); 8,52 (d, br, 1H); 8,72 (s, 1H); 13,4 (br, 1H) |
| 1815 | [DMSO] 0,89 (t, 3H); 1,15 (d, 3H); 1,30 (m, 2H); 1,47 (m, 2H); 4,00 (m, 1H); 8,54 (br, 1H); 8,72 (s, 1H); 13,4 (br, 1H) |
| 1816 | [CDCl3] 0,89 (t, 3H); 1,20 (d, 3H); 1,27-1,37 (m, 6H); 1,51 (m, 2H); 4,16 (m, 1H); 8,75 (br, 1H); 8,90 (s, 1H); 12,1 (br,1H) |
| 1817 | [CDCl3] 0,91 (m, 6H); 1,20 (d, 3H); 1,32 (m, 1H); 1,45 (m, 1H); 1,65 (m, 1H); 4,25 (m, 1H); 8,66 (br, 1H); 8,90 (s, 1H); 11,9 (br, 1H) |
| 1818 | [CDCl3] 0,94 (s, 9H); 1,14 (d, 3H); 4,07 (m, 1H); 8,85 (br, 1H); 8,90 (s, 1H) |
| 1819 | [CDCl3] 0,95 (m, 6H); 1,18 (d, 3H); 1,79 (m, 1H); 4,04 (m, 1H); 8,75 (br, 1H); 8,89 (s, 1H) |
| 1820 | [CDCl3] 0,93 (t, 6H); 1,48 (m, 2H); 1,64 (m, 2H); 3,98 (m, 1H); 8,63 (br, 1H); 8,90 (s, 1H); 11,6 (br, 1H) |
| 1821 | [CDCl3] 3,40 (s, 6H); 3,60 (t, 2H); 4,49 (t, 1H); 8,85 (s, br, 2H); 11,4 (br, 1H) |
| 1823 | [CDCl3] 3,63 (m, 2H); 3,83 (m, 2H); 8,86 (s, 1H); 9,17 (br, 1H) |
| 1824 | [CDCl3] 3,38 (s, 3H); 3,55 (m, 2H); 3,65 (m, 2H); 8,82 (s, 1H); 8,84 (br, 1H); 11,4 (br, 1H) |
| 1826 | [CDCl3] 1,25 (d, 3H); 3,39 (s, 3H); 3,43 (m, 2H); 4,38 (m, 1H); 8,67 (br, 1H); 8,82 (s, 1H); 11,5 (br, 1H) |
| 1827 | [CDCl3] 1,89 (m, 2H); 3,40 (s, 3H); 3,55 (m, 4H); 8,67 (s, 1H); 8,74 (br, 1H); 11,8 (br, 1H) |
| 1828 | [CDCl3] 3,78 (s, 3H); 4,24 (d, 2H); 8,89 (s, 1H); 9,30 (br, 1H) |
| 1833 | [DMSO] 2,85 (d, 3H); 8,69 (s, 1H); 8,71 (br, 1H); 13,5 (br, 1H) |
| 1834 | [DMSO] 1,12 (t, 3H); 3,30 (m, 2H); 8,70 (s, 1H); 8,75 (t, br, 1H); 13,5 (br, 1H) |
| 1835 | [DMSO] 0,89 (t, 3H); 1,52 (m, 2H); 3,25 (q, 2H); 8,70 (s, 1H); 8,75 (t, br, 1H); 13,5 (br, 1H) |
| 1837 | [DMSO] 0,59 (m, 2H); 0,76 (m, 2H); 2,86 (m, 1H); 8,66 (s, 1H); 8,76 (br, 1H); 13,3 (br) |
| 1838 | [CDCl3] 0,95 (t, 3H); 1,42 (m, 2H); 1,60 (m, 2H); 3,45 (m, 2H); 8,86 (s, 1H); 8,95 (br, 1H) |
| 1839 | [CDCl3] 1,81 (m, 2H); 2,00 (m, 2H); 2,42 (m, 2H); 4,54 (m, 1H); 8,84 (s, 1H); 9,17 (d, br, 1H); 12,2 (br, 1H) |
| 1845 | [CDCl3] 0,29 (m, 2H); 0,57 (m, 2H); 1,05 (m, 1H); 3,32 (t, 2H); 8,87 (s, 1H); 9,05 (br, 1H); 12,3 (br, 1H) |
| 1847 | [CDCl3] 1,00 (d, 6H); 1,90 (m, 1H); 3,30 (t, 2H); 8,85 (s, 1H); 9,00 (br, 1H) |
| 1849 | [CDCl3] 0,95 (d, 6H); 1,51 (q, 2H); 1,68 (m, 1H); 3,46 (m, 2H); 8,85 (s, 1H); 8,91 (br, 1H) |
| 1851 | [DMSO] 3,94 (m, 2H); 5,16 (dd, 2H); 5,90 (m, 1H); 8,70 (s, 1H); 8,85 (t, br, 1H); 13,4 (br, 1H) |
| 1852 | [CDCl3] 1,79 (s, 3H); 4,02 (m, 2H); 4,92 (m, 2H); 8,88 (s, 1H); 9,10 (t, br, 1H) |
| 1853 | [CDCl3] 2,26 (m, 1H); 4,22 (m, 2H); 8,86 (s, 1H); 9,25 (br, 1H) |
| 1854 | [DMSO] 0,87 (t, 3H); 1,13 (d, 3H); 1,50 (m, 2H); 3,91 (m, 1H); 8,55 (d, br, 1H); 8,70 (br, 1H); 13,5 (br, 1H) |
| 1856 | [DMSO] 0,89 (t, 3H); 1,15 (d, 3H); 1,30 (m, 2H); 1,49 (m, 2H); 4,01 (m, 1H); 8,51 (d, br, 1H); 8,71 (s, 1H); 13,6 (br, 1H) |
| 1857 | [CDCl3] 0,87 (t, 3H); 1,22 (d, 3H); 1,25-1,35 (m, 6H); 1,53 (m, 2H); 4,14 (m, 1H); 8,81 (d, br, 1H); 8,88 (s, 1H) |
| 1858 | [CDCl3] 0,92 (m, 6H); 1,22 (d, 3H); 1,33 (m, 1H); 1,48 (m, 1H); 1,65 (m, 1H); 4,25 (m, 1H); 8,84 (br, 1H); 8,88 (s, 1H); 12,3 (br, 1 H) |
| 1860 | [CDCl3] 0,95 (m, 6H); 1,18 (d, 3H); 1,81 (m, 1H); 4,05 (m, 1H); 8,90 (s, 1H); 9,01 (d, br, 1H) |
| 1861 | [DMSO] 0,88 (t, 6H); 1,45 (m, 2H); 1,55 (m, 2H); 3,79 (m, 1H); 8,40 (d, br, 1H); 8,70 (s, 1H); 13,5 (br, 1H) |
| 1867 | [DMSO] 3,29 (s, 3H); 3,47 (m, 4H); 8,73 (s, 1H); 8,79 (br, 1H); 13,6 (br, 1H) |
| 1869 | [CDCl3] 1,28 (d, 3H); 3,39 (s, 3H); 3,45 (m, 2H); 4,35 (m, 1H); 8,81 (s, 1H); 8,86 (br, 1H); 12,0 (br, 1H) |
| 1870 | [CDCl3] 1,90 (m, 2H); 3,41 (s, 3H); 3,56 (m, 4H); 8,62 (s, 1H); 8,76 (br, 1H); 11,9 (br, 1H) |
| 1876 | [DMSO] 8,18 (br, 1H); 8,35 (br, 1H); 8,79 (s, 1H); 13,7 (br, 1H) |
| 1877 | [DMSO] 2,83 (d, 3H); 8,70 (br, 2H); 13,5 (br, 1H) |
| 1878 | [DMSO] 1,12 (t, 3H); 3,32 (m, 2H); 8,71 (s, 1H); 8,76 (t, br, 1H); 13,4 (br, 1H) |
| 1879 | [DMSO] 0,90 (t, 3H); 1,51 (m, 2H); 3,25 (q, 2H); 8,70 (s, 1H); 8,78 (t, br, 1H); 13,5 (br, 1H) |
| 1880 | [DMSO] 1,18 (d, 6H); 4,07 (m, 2H); 8,57 (d, br, 1H); 8,72 (s, 1H); 13,4 (br, 1H) |
| 1881 | [DMSO] 0,58 (m, 2H); 0,74 (m, 2H); 2,85 (m, 1H); 8,66 (s, 1H); 8,74 (br, 1H); 13,4 (br, 1H) |
| 1882 | [DMSO] 0,89 (t, 3H); 1,31 (m, 2H); 1,50 (m, 2H); 3,30 (m, 2H); 8,70 (s, 1H); 8,75 (br, 1H) |
| 1883 | [DMSO] 1,69 (m, 2H); 2,02 (m, 2H); 2,25 (m, 2H); 4,39 (m, 1H); 8,70 (s, 1H); 8,95 (br, 1H) |
| 1884 | [CDCl3] 1,47 (s, 9H); 8,87 (s, 1H); 9,02 (br, 1H); 12,2 (br, 1H) |
| 1885 | [CDCl3] 3,12 (s, 6H); 8,16 (s, 1H) |
| 1886 | [DMSO] 0,23 (m, 2H); 0,45 (m, 2H); 1,02 (m, 1H); 3,19 (t, 2H); 8,72 (s, 1H); 8,83 (br, 1H); 13,4 (br, 1H) |
| 1887 | [DMSO] 0,90 (s, 9H); 3,14 (d, 2H); 8,72 (br, 1H); 8,67 (s, 1H); 13,4 (br, 1H) |
| 1888 | [DMSO] 0,90 (d, 6H); 1,84 (m, 1H); 3,12 (t, 2H); 8,68 (s, 1H); 8,73 (t, br, 1H); 13,4 (br, 1H) |
| 1890 | [DMSO] 0,90 (d, 6H); 1,41 (m, 2H); 1,60 (m, 1H); 3,30 (m, 2H); 8,69 (s, 1H); 8,72 (t, br, 1H); 13,5 (br, 1H) |
| 1892 | [DMSO] 3,94 (m, 2H); 5,15 (dd, 2H); 5,89 (m, 1H); 8,70 (s, 1H); 8,85 (t, br, 1H) |
| 1894 | [DMSO] 1,70 (s, 3H); 3,86 (d, 2H); 4,84 (d, 2H); 8,68 (s, 1H); 8,95 (br, 1H); 13,4 (br, 1H) |
| 1895 | [DMSO] 3,10 (t, 1H); 4,05 (m, 2H); 8,25 (br, 1H); 8,30 (s, 1H); 11,5 (br, 1H) |
| 1897 | [CDCl3] 0,95 (t, 3H); 1,24 (d, 3H); 1,60 (m, 2H); 4,10 (m, 1H); 8,87 (br, 2H) |
| 1899 | [CDCl3] 0,92 (t, 3H); 1,23 (d, 3H); 1,40 (m, 2H); 1,52 (m, 2H); 4,17 (m, 1H); 8,88 (s, 1H); 8,91 (br, 1H) |
| 1900 | [DMSO] 0,86 (t, 3H); 1,15 (d, 3H); 1,25 (m, 6H); 1,48 (m, 2H); 4,00 (m, 1H); 8,53 (d, br, 1H); 8,71 (s, 1H); 13,4 (br, 1H) |
| 1903 | [DMSO] 0,89 (d, 6H); 1,13 (d, 3H); 1,27 (m, 1H); 1,48 (m, 1H); 1,62 (m, 1H); 4,09 (m, 1H); 8,50 (d, br, 1H); 8,71 (s, 1H); 13,5 (br, 1H) |
| 1904 | [DMSO] 0,81 (s, 9H); 1,07 (d, 3H); 3,90 (m, 1H); 8,43 (d, br, 1H); 8,67 (s, 1H); 13,4 (br, 1H) |
| 1905 | [DMSO] 0,88 (d, 6H); 1,10 (d, 3H); 1,75 (m, 1H); 1,48 (m, 1H); 3,85 (m, 1H); 8,49 (d, br, 1H); 8,70 (s, 1H); 13,5 (br, 1H) |
| 1907 | [DMSO] 0,87 (t, 6H); 1,43 (m, 2H); 1,55 (m, 2H); 3,78 (m, 1H); 8,55 (br, 1H); 8,67 (s, 1H); 13,5 (br, 1H) |
| 1910 | [CDCl3] 3,41 (s, 6H); 3,62 (t, 2H); 4,50 (t, 1H); 8,82 (s, 1H); 8,95 (br, 1H); 11,2 (br, 1H) |
| 1912 | [DMSO] 3,39 (m, 2H); 3,52 (m, 2H); 4,83 (br, 1H); 8,76 (s, 1H); 8,80 (t, br, 1H); 13,6 (br, 1H) |
| 1913 | [DMSO] 3,26 (s, 3H); 3,47 (m, 4H); 8,71 (s, 1H); 8,79 (br, 1H) |
| 1916 | [CDCl3] 1,27 (d, 3H); 3,39 (s, 3H); 3,44 (m, 2H); 4,38 (m, 1H); 8,83 (s, 1H); 8,95 (br, 1H); 12,1 (br, 1H) |
| 1917 | [DMSO] 1,75 (m, 2H); 3,24 (s, 3H); 3,39 (m, 4H); 8,70 (s, 1H); 8,81 (br, 1H); 13,4 (br, 1H) |
| 1918 | [DMSO] 3,66 (s, 3H); 4,12 (d, 2H); 8,71 (s, 1H); 9,02 (t, br, 1H), 13,5 (br, 1H) |
| 1923 | [DMSO] 2,81 (d, 3H); 8,72 (s, 1H); 8,77 (br, 1H); 13,4 (br) |
| 1924 | [DMSO] 1,14 (t, 3H); 3,35 (m, 2H); 8,74 (s, 1H); 8,82 (t, br, 1H); 13,4 (br) |
| 1925 | [DMSO] 0,89 (t, 3H); 1,53 (m, 2H); 3,28 (q, 2H); 8,73 (s, 1H); 8,80 (t, br, 1H) |
| 1926 | [DMSO] 1,18 (d, 6H); 4,10 (m, 1H); 8,63 (d,br, 1H); 8,75 (s, 1H); 13,4 (br, 1H) |
| 1927 | [DMSO] 0,60 (m, 2H); 0,75 (m, 2H); 2,87 (m, 1H); 8,69 (s, 1H); 8,80 (br, 1H) |
| 1928 | [DMSO] 0,89 (t, 3H); 1,34 (m, 2H); 1,51 (m, 2H); 3,30 (m, 2H); 8,72 (s, 1H); 8,79 (br, 1H) |
| 1929 | [DMSO] 1,72 (m, 2H); 2,05 (m, 2H); 2,27 (m, 2H); 4,38 (m, 1H); 8,73 (s, 1H); 9,00 (d, br, 1H); 13,3 (br) |
| 1930 | [DMSO] 1,37 (s, 9H); 8,30 (br, 1H); 8,66 (s, 1H); 13,4 (br) |
| 1931 | [DMSO] 2,80 (s, 3H); 2,96 (s, 3H); 8,27 (s, 1H); 12,4 (br, 1H) |
| 1932 | [DMSO] 1,01 (t, 3H); 1,11 (t, 3H); 3,10 (q, 2H); 3,42 (q, 2H); 8,25 (s, 1H); 12,3 (br, 1H) |
| 1933 | [DMSO] 0,26 (m, 2H); 0,47 (m, 2H); 1,04 (m, 1H); 3,20 (m, 2H); 8,77 (s, 1H); 8,87 (t, br, 1H); 13,5 (br, 1H) |
| 1934 | [DMSO] 0,90 (s, 9H); 3,13 (d, 2H); 8,62 (t, br, 1H); 8,70 (s, 1H); 13,3 (br, 1H) |
| 1935 | [DMSO] 0,90 (d, 6H); 1,85 (m, 1H); 3,13 (m, 2H); 8,71 (s, 1H); 8,75 (t, br, 1H); 13,4 (br, 1H) |
| 1937 | [CDCl3] 0,94 (d, 6H); 1,49 (q, 2H); 1,66 (m, 1H); 3,46 (m, 2H); 8,75 (br, 1H); 8,85 (s, 1H); 11,5 (br) |
| 1939 | [DMSO] 3,93 (m, 2H); 5,19 (dd, 2H); 5,89 (m, 1H); 8,72 (s, 1H); 8,90 (t, br, 1H); 13,3 (br, 1H) |
| 1941 | [DMSO] 1,72 (s, 3H); 3,87 (d, 2H); 4,87 (d, 2H); 8,73 (s, 1H); 8,89 (t, br, 1H); 13,3 (br) |
| 1942 | [DMSO] 3,18 (m, 1H); 4,10 (m, 2H); 8,70 (s, 1H); 9,04 (t, br, 1H); 13,3 (br, 1H) |
| 1944 | [DMSO] 0,89 (t, 3H); 1,14 (d, 3H); 1,51 (m, 2H); 3,91 (m, 1H); 8,55 (d, br, 1H); 8,75 (s, 1H); 13,4 (br, 1H) |
| 1946 | [DMSO] 0,89 (t, 3H); 1,15 (d, 3H); 1,31 (m, 2H); 1,47 (m, 2H); 4,01 (m, 1H); 8,56 (br, 1H); 8,74 (s, 1H); 13,4 (br, 1H) |
| 1947 | [DMSO] 0,85 (t, 3H); 1,15 (d, 3H); 1,20-1,35 (m, 6H); 1,49 (m, 2H); 4,00 (m, 1H); 8,58 (d, br, 1H); 8,74 (s, 1H); 13,4 (br, 1H) |
| 1948 | [DMSO]- 0,88 (d. 6H); 1,15 (d, 3H); 1,27 (m, 1H); 1,48 (m, 1H); 1,61 (m, 1H); 4,10 (m, 1H); 8,56 (d, br, 1H); 8,74 (s, 1H); 13,5 (br, 1H) |
| 1949 | [CDCl3] 0,94 (s, 9H); 1,15 (d, 3H); 4,09 (m, 1H); 8,89 (br, 1H); 8,91 (s, 1H) |
| 1950 | [CDCl3] 0,94 (m, 6H); 1,17 (d, 3H); 1,80 (m, 1H); 4,05 (m, 1H); 8,88 (br, 1H); 8,92 (s, 1H) |
| 1951 | [CDCl3] 0,92 (t, 6H); 1,49 (m, 2H); 1,65 (m, 2H); 3,99 (m, 1H); 8,78 (br, 1H); 8,92 (s, 1H) |
| 1952 | [CDCl3] 3,41 (s, 6H); 3,61 (t, 2H); 4,50 (t, 1H); 8,84 (s, br, 2H); 11,4 (br, 1 H) |
| 1954 | [DMSO] 3,39 (m, 2H); 3,52 (m, 2H); 4,80 (br, 1H); 8,79 (s, 1H); 8,82 (t, br, 1H); 13,5 (br, 1H) |
| 1955 | [CDCl3] 3,38 (s, 3H); 3,55 (m, 2H); 3,66 (m, 2H); 8,78 (s, br, 1H); 8,82 (s, 1H); 11,5 (br) |
| 1957 | [CDCl3] 1,27 (d, 3H); 3,38 (s, 3H); 3,42 (m, 2H); 4,38 (m, 1H); 8,75 (br, 1H); 8,84 (s, 1H); 12,3 (br, 1H) |
| 1958 | [CDCl3] 1,89 (m, 2H); 3,41 (s, 3H); 3,56 (m, 4H); 8,61 (s, 1H); 8,68 (br, 1H); 11,8 (br, 1H) |
| 1959 | [DMSO] 3,67 (s, 3H); 4,12 (d, 2H); 8,73 (s, 1H); 9,04 (t, br, 1H); 13,5 (br, 1H) |
| 1964 | [DMSO] 8,25 (br, 1H); 8,37 (br, 1H); 8,47 (s, 1H); 13,7 (br, 1H) |
| 1965 | [DMSO] 2,82 (d, 3H); 8,37 (s, 1H); 8,72 (d, br, 1H); 13,5 (br, 1H) |
| 1966 | [DMSO] 1,13 (t, 3H); 3,34 (m, 2H); 8,39 (s, 1H); 8,76 (t, br, 1H); 13,5 (br, 1H) |
| 1967 | [DMSO] 0,90 (t, 3H); 1,53 (m, 2H); 3,26 (q, 2H); 8,39 (s, 1H); 8,75 (t, br, 1H); 13,5 (br, 1H) |
| 1968 | [DMSO] 1,18 (d, 6H); 4,09 (m, 1H); 8,41 (s, 1H); 8,58 (d, br, 1H); 13,5 (br, 1H) |
| 1969 | [DMSO] 0,59 (m, 2H); 0,75 (m, 2H); 2,86 (m, 1H); 8,34 (s, 1H); 8,73 (d, br, 1H); 13,4 (br, 1H) |
| 1970 | [DMSO] 0,89 (t, 3H); 1,33 (m, 2H); 1,51 (m, 2H); 3,31 (m, 2H); 8,39 (s, 1H); 8,73 (t, br, 1H); 13,5 (br, 1H) |
| 1971 | [DMSO] 1,70 (m, 2H); 2,04 (m, 2H); 2,25 (m, 2H); 4,38 (m, 1H); 8,39 (s, 1H); 8,92 (d, br, 1H); 13,4 (br, 1H) |
| 2006 | [DMSO] 8,25 (br, 1H); 8,35 (br, 1H); 8,61 (s, 1H); 13,7 (br, 1H) |
| 2007 | [DMSO] 2,82 (d, 3H); 8,51 (s, 1H); 8,74 (d, br, 1H); 13,5 (br, 1H) |
| 2008 | [DMSO] 1,13 (t, 3H); 3,33 (m, 2H); 8,53 (s, 1H); 8,78 (t, br, 1H); 13,5 (br, 1H) |
| 2009 | [DMSO] 0,89 (t, 3H); 1,53 (m, 2H); 3,26 (q, 2H); 8,51 (s, 1H); 8,76 (t, br, 1H); 13,5 (br, 1H) |
| 2010 | [DMSO] 1,18 (d, 6H); 4,07 (m, 1H); 8,53 (s, 1H); 8,61 (d, br, 1H); 13,5 (br, 1H) |
| 2011 | [DMSO] 0,58 (m, 2H); 0,74 (m, 2H); 2,86 (m, 1H); 8,48 (s, 1H); 8,77 (d, br, 1H); 13,4 (br, 1H) |
| 2012 | [DMSO] 0,90 (t, 3H); 1,33 (m, 2H); 1,51 (m, 2H); 3,30 (m, 2H); 8,52 (s, 1H); 8,77 (t, br, 1H); 13,5 (br, 1H) |
| 2013 | [DMSO] 1,72 (m, 2H); 2,05 (m, 2H); 2,25 (m, 2H); 4,39 (m, 1H); 8,51 (s, 1H); 8,95 (d, br, 1H); 13,4 (br, 1H) |
| 2014 | [DMSO] 1,37 (s, 9H); 8,31 (br, 1H); 8,45 (s, 1H); 13,4 (br, 1H) |
| 2015 | [DMSO] 2,82 (s, 3H); 2,95 (s, 3H); 8,12 (s, 1H); 12,5 (br, 1H) |
| 2017 | [DMSO] 0,26 (m, 2H); 0,47 (m, 2H); 1,04 (m, 1H); 3,30 (m, 2H); 8,56 (s, 1H); 8,86 (t, br, 1H); 13,5 (br, 1H) |
| 2018 | [DMSO] 0,91 (s, 9H); 3,13 (d, 2H); 8,47 (s, 1H); 8,63 (t, br, 1H); 13,5 (br, 1H) |
| 2019 | [DMSO] 0,90 (d, 6H); 1,83 (m, 1H); 3,13 (m, 2H); 8,50 (s, 1H); 8,73 (t, br, 1H); 13,4 (br, 1H) |
| 2021 | [DMSO] 0,89 (d, 6H); 1,42 (q, 2H); 1,62 (m, 1H); 3,31 (m, 2H); 8,51 (s, 1H); 8,75 (t, br, 1 H); 13,5 (br, 1 H) |
| 2023 | [DMSO] 3,94 (m, 2H); 5,18 (dd, 2H); 5,89 (m, 1H); 8,52 (s, 1H); 8,88 (t, br, 1H); 13,4 (br, 1H) |
| 2025 | [DMSO] 1,72 (s, 3H); 3,86 (d, 2H); 4,85 (d, 2H); 8,50 (s, 1H); 8,87 (t, br, 1H); 13,4 (br, 1H) |
| 2026 | [DMSO] 3,18 (m, 1H); 4,10 (m, 2H); 8,48 (s, 1H); 9,02 (t, br, 1H); 13,3 (br, 1H) |
| 2030 | [CDCl3] 0,93 (t, 3H); 1,23 (d, 3H); 1,40 (m, 2H); 1,52 (m, 2H); 4,21 (m, 1H); 8,74 (s, 1H); 8,97 (d, br, 1H); 12,9 (br, 1H) |
| 2031 | [DMSO] 0,85 (t, 3H); 1,15 (d, 3H); 1,20-1,35 (m, 6H); 1,48 (m, 2H); 3,99 (m, 1H); 8,52 (s, 1 H); 8,54 (d, br, 1H); 13,5 (br, 1H) |
| 2032 | [DMSO] 0,88 (d, 6H); 1,14 (d, 3H); 1,27 (m, 1H); 1,48 (m, 1H); 1,62 (m, 1H); 4,10 (m, 1H); 8,51 (s, 1H); 8,53 (d, br, 1H); 13,5 (br, 1 H) |
| 2035 | [DMSO] 0,86 (t, 6H); 1,45 (m, 2H); 1,55 (m, 2H); 3,78 (m, 1H); 8,82 (d, br, 1H); 8,51 (s, 1H); 13,5 (br, 1H) |
| 2036 | [CDCl3] 3,41 (s, 6H); 3,61 (t, 2H); 4,50 (t, 1H); 8,67 (s, 1H); 9,00 (br, 1H); 11,7 (br, 1H) |
| 2038 | [DMSO] 3,35 (m, 2H); 3,53 (m, 2H); 4,81 (br, 1H); 8,56 (s, 1H); 8,80 (t, br, 1H); 13,6 (br, 1H) |
| 2039 | [CDCl3] 3,38 (s, 3H); 3,56 (m, 2H); 3,65 (m, 2H); 8,65 (s, 1H); 9,01 (s, 1H); 11,8 (br, 1H) |
| 2041 | [DMSO] 1,15 (d, 3H); 3,30 (s, 3H); 3,40 (m, 2H); 4,18 (m, 1H); 8,52 (s, 1H); 8,64 (d, br, 1H); 13,4 (br, 1H) |
| 2048 | [DMSO] 8,20 (br, 1H); 8,37 (br, 1H); 8,81 (s, 1H); 13,7 (br, 1H) |
| 2090 | [DMSO] 7,45(d, 1H); 8,14(br, 1H); 8,42 (br, 1H); 8,48 (d, 1H); 13,7 (br, 1H) |
| 2091 | [DMSO] 2,84 (d, 3H); 7,42 (d, 1H); 8,41 (d, 1H); 8,79 (br, 1H); 13,4 (br, 1H) |
| 2092 | [DMSO] 1,12 (t, 3H); 3,32 (m, 2H); 7,42 (d, 1H); 8,42 (d, 1H); 8,83 (br, 1H); 13,4 (br, 1H) |
| 2093 | [DMSO] 0,89 (t, 3H); 1,54 (m, 2H); 3,27 (q, 2H); 7,42 (d, 1H); 8,41 (d, 1H); 8,82 (br,1H); 13,4 (br, 1H) |
| 2094 | [DMSO] 1,18 (d, 6H); 4,11 (m, 1H); 7,41 (d, 1H); 8,41 (d, 1H); 8,63 (br, 1H); 13,4 (br, 1H) |
| 2095 | [CDCl3] 0,61 (m, 2H); 0,88 (m, 2H); 3,00 (m, 1H); 6,89 (d, 1H); 8,68 (d, 1H); 9,35 (br, 1H); 12,5 (br, 1H) |
| 2096 | [DMSO] 0,89 (t, 3H); 1,33 (m, 2H); 1,52 (m, 2H); 3,32 (m, 2H); 7,42 (d, 1H); 8,41 (d, 1H); 8,81 (br, 1H); 13,3 (br, 1H) |
| 2097 | [DMSO] 1,72 (m, 2H); 2,05 (m, 2H); 2,46 (m, 2H); 4,41 (m, 1H); 7,43 (d, 1H); 8,39 (d, 1H); 8,96 (br, 1H); 13,3 (br, 1H) |
| 2101 | [DMSO] 0,26 (m, 2H), 0,47 (m, 2H); 1,06 (m, 1H); 3,22 (m, 2H); 7,45 (d, 1H); 8,45 (d, 1H); 8,91 (br, 1H); 13,4 (br, 1H) |
| 2102 | [DMSO] 0,92 (s, 9H); 3,15 (d, 2H); 7,40 (d, 1H); 8,41 (d, 1H); 8,75 (br, 1H); 13,3 (br, 1H) |
| 2103 | [DMSO] 0,90 (d, 6H); 1,83 (m, 1H); 3,14 (t, 2H); 7,40 (d, 1H); 8,40 (d, 1H); 8,81 (t, br, 1H); 13,3 (br, 1H) |
| 2105 | [DMSO] 0,90 (d, 6H); 1,42 (m, 2H); 1,62 (m, 1H); 3,33 (m, 2H); 7,41 (d, 1H); 8,40 (d, 1H); 8,80 (br, 1H); 13,3 (br, 1H) |
| 2107 | [DMSO] 3,95 (t, 2H); 5,17 (dd, 2H); 5,90 (m, 1H); 7,41 (d, 1H); 8,41 (d, 1H); 8,93 (br, 1H); 13,3 (br, 1H) |
| 2109 | [DMSO] 1,72 (s, 3H); 3,88 (d, 2H); 4,85 (d, 2H); 7,42 (d 1H); 8,42 (d, 1H); 8,94 (br, 1H); 13,3 (br, 1H) |
| 2110 | [DMSO] 3,17 (m, 1H); 4,11 (m, 2H); 7,41 (d, 1H); 8,40 (d, 1H); 9,06 (br, 1H), 13,3 (br, 1H) |
| 2112 | [DMSO] 0,89 (t, 3H); 1,15 (d, 3H); 1,53 (m, 2H); 3,95 (m, 1H); 7,42 (d, 1H); 8,42 (d, 1H); 8,60 (br, 1H); 13,4 (br, 1H) |
| 2114 | [DMSO] 0,88 (t, 3H); 1,15 (d, 3H); 1,32 (m, 2H); 1,48 (m, 2H); 4,04 (m, 1H); 7,42 (d, 1H); 8,41 (d, 1H); 8,59 (br, 1H); 13,4 (br, 1H) |
| 2115 | [DMSO] 0,85 (t, 3H); 1,16 (d, 3H); 1,20-1,35 (m, 6H); 1,50 (m, 2H); 4,02 (m, 1H); 7,42 (d, 1H); 8,44 (d, 1H); 8,62 (d, br, 1H), 13,4 (br, 1H) |
| 2116 | [DMSO] 0,88 (d, 6H); 1,14 (d, 3H); 1,25 (m, 1H); 1,48 (m, 1H); 1,63 (m, 1H); 4,21 (m, 1H); 6,92 (d, 1H); 8,42 (d, 1H); 8,59 (d, br, 1H), 13,4 (br, 1H) |
| 2122 | [DMSO] 3,40 (m, 2H); 3,52 (m, 2H); 4,82 (br, 1H); 7,44 (d, 1H); 8,46 (d, 1H); 8,88 (br, 1H); 13,4 (br, 1H) |
| 2133 | [DMSO] 2,83 (d, 3H); 8,58 (s, 1H); 8,82 (d, br, 1H); 13,4 (br, 1H) |
| 2134 | [DMSO] 1,14 (t, 3H); 3,34 (m, 2H); 8,60 (s, 1H); 8,86 (t, br, 1H); 13,5 (br, 1H) |
| 2135 | [DMSO] 0,89 (t, 3H); 1,54 (m, 2H); 3,26 (q, 2H); 8,59 (s, 1H); 8,85 (t, br, 1H); 13,5 (br, 1H) |
| 2137 | [DMSO] 0,60 (m, 2H); 0,75 (m, 2H); 2,88 (m, 1H); 8,55 (s, 1H); 8,85 (d, br, 1H); 13,4 (br, 1H) |
| 2138 | [DMSO] 0,90 (t, 3H); 1,33 (m, 2H); 1,51 (m, 2H); 3,31 (m, 2H); 8,59 (s, 1H); 8,84 (t, br, 1H); 13,4 (br, 1H) |
| 2139 | [DMSO] 1,71 (m, 2H); 2,05 (m, 2H); 2,25 (m, 2H); 4,40 (m, 1H); 8,62 (s, 1H); 9,04 (d, br, 1H); 13,4 (br, 1H) |
| 2143 | [DMSO] 0,26 (m, 2H); 0,47 (m, 2H); 1,05 (m, 1H); 3,20 (m, 2H); 8,63 (s, 1H); 8,94 (t, br, 1H); 13,4 (br, 1H) |
| 2144 | [DMSO] 0,91 (s, 9H); 3,15 (d, 2H); 8,55 (s, 1H); 8,66 (t, br. 1H); 13,3 (br, 1H) |

### 3. Biologische Beispiele

### 3.1 BONITIERUNG DER SCHADWIRKUNG

Die Schadwirkung an den Pflanzen wird nach einer Skala von 0-100 % optisch im Vergleich zu Kontrollpflanzen bewertet:
- 0% =: keine erkennbare Wirkung im Vergleich zur unbehandelten Pflanze
- 100% =: behandelte Pflanze stirbt ab.

### 3.2 HERBIZIDWIRKUNG UND SAFENERWIRKUNG IM NACHAUFLAUF

Samen bzw. Rhizomstücke von mono- und dikotylen Schadpflanzen und von Kulturpflanzen werden in Plastiktöpfen in sandiger Lehmerde ausgelegt, mit Erde abgedeckt und im Gewächshaus unter guten Wachstumsbedingungen angezogen. Alternativ hierzu werden im Paddy-Reisanbau vorkommende Schadpflanzen in Töpfen kultiviert, in denen Wasser bis zu 2 cm über der Bodenoberfläche steht. Drei Wochen nach der Aussaat werden die Versuchspflanzen im Dreiblattstadium behandelt. Die als Emulsionskonzentrate formulierten erfindungsgemäßen Herbizid-Safener-Wirkstoffkombinationen sowie in parallelen Versuchen die entsprechend formulierten Einzelwirkstoffe werden in verschiedenen Dosierungen mit einer Wasseraufwandmenge von umgerechnet 300 l/ha auf die grünen Pflanzenteile gesprüht und nach 2-3 Wochen Standzeit der Versuchspflanzen im Gewächshaus unter optimalen Wachstumsbedingungen die Wirkung der Präparate optisch im Vergleich zu unbehandelten Kontrollen bonitiert. Bei Reis oder bei Schadpflanzen, die im Reisanbau vorkommen, werden die Wirkstoffe auch direkt ins Bewässerungswasser gegeben (Applikation in Analogie zur sogenannten Granulatanwendung) oder auf Pflanzen und ins Bewässerungswasser gesprüht.

Die Versuche zeigen, dass erfindungsgemäße Safener in Kombination mit Herbiziden, im Verhältnis von Herbizid:Safener von 2:1 bis 1:20, Schäden des Herbizids an Kulturpflanzen wie Mais, Reis, Weizen oder Gerste oder anderem Getreide oder dicotylen Kulturpflanzen wie Soja oder Raps im Vergleich zur Anwendung der einzelnen Herbizide ohne Safener wesentlich reduzieren, sodass um 30% bis zu 100% geringere Schäden an der Kulturpflanze beobachtet werden. Gleichzeitig wird die Wirkung des Herbizids an wirtschaftlich bedeutenden Schadpflanzen nicht oder nicht wesentlich beeinträchtigt, so dass eine gute herbizide Nachauflaufwirkung gegen ein breites Spektrum von Ungräsern und Unkräutern erreicht werden kann.

Beispielsweise konnte in Gerste für das Herbizid Mesosulfuron-methyl mit den Verbindungen Nr. 1, 2, 3, 5, 7, 9, 10, 13, 14, 22, 25, 26, 30, 34, 36, 40, 57, 64, 66, 79, 100, 101, 102, 105, 114, 115, 116, 117, 119, 123, 124, 128, 130, 134, 136, 138, 140, 146, 148, 153, 154, 155, 157, 163, 165, 168, 169, 171, 178, 193, 194, 214, 341, 342, 344, 345, 346, 382, 384, 392, 398, 405, 407, 455, 457, 489, 496, 498, 571, 909, 910, 911, 913, 943, 950, 960, 1023, 1026, 1029, 1030, 1201, 1481, 1487, 1598, 1599, 1600, 1636, 1907 eine gute Safenerwirkung erzielt werden.

Im Mais konnte beispielsweise für das Herbizid Tembotrione eine gute Safeneraktivität bei folgenden Verbindungen aus Tabelle 1 erzielt werden: 4, 6, 9, 10, 14, 24, 32, 34, 36, 43, 64, 66, 71, 75, 79, 105, 114, 118, 140, 146, 148, 153, 169, 178, 210, 214, 345, 51, 95, 341, 456, 457, 489, 496, 498, 909, 911, 913, 943, 960, 1026, 1139, 1368, 1371, 1485, 1488, 1596, 1597, 1598, 1599, 1600, 1604, 1619, 1625, 1629, 1636, 1638, 1646, 1758.

In Reis erzielten beispielsweise folgende Verbindungen der Tabelle 1 eine gute Safeneraktivität bei Fenoxaprop-P-ethyl und Thiencarbazone oder Thiencarbazonemethyl:
1, 2, 3, 4, 9, 16, 20, 22, 34, 41, 47, 54, 57, 75, 76, 79, 80, 81, 101, 103, 105, 114, 115, 117, 118, 120, 127, 128, 130, 153, 154, 157, 162, 165, 169, 171, 178, 218, 271, 341, 342, 344, 345, 346, 350, 355, 392, 398, 405, 407, 456, 973, 1482, 1596, 1597, 1598, 1600, 1603, 1609, 1625, 1638, 1646, 1659.

### 3.3 HERBIZIDWIRKUNG UND SAFENERWIRKUNG IM VORAUFLAUF

Samen bzw. Rhizomstücke von mono- und dikotylen Unkrautpflanzen und Kulturpflanzen wurden in Plastiktöpfen in sandiger Lehmerde ausgelegt und mit Erde abgedeckt. Die als Suspensions- oder Emulsionskonzentrate formulierten erfindungsgemäßen Herbizid-Safener-Wirkstoffkombinationen sowie in parallelen Versuchen die entsprechend formulierten Einzelwirkstoffe wurden dann in verschiedenen Dosierungen mit einer Wasseraufwandmenge von umgerechnet 600 bis 800 l/ha auf die Oberfläche der Abdeckerde appliziert.

Nach der Behandlung wurden die Töpfe im Gewächshaus aufgestellt und unter guten Wachstumsbedingungen für die Unkräuter und die Kulturpflanzen gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigten, wurden durch die erfindungsgemäßen Verbindungen Herbizidschäden an den Kulturpflanzen verhindert oder reduziert, ohne dass die Herbizidwirkung an den Schadpflanzen verringert oder wesentlich verringert wurde.

Beispielsweise zeigten die Beispiele Nr. 2, 19, 39, 72, 104, 122, 155, 193, 194, 217, 232, 271, 341, 380, 392, 1368, 1597, 1625, 1636 der Tabelle 1 im Test in Kombination mit dem Herbizid Isoxaflutol eine gute Safenerwirkung in Mais. Die herbizide Wirkung der verwendeten Herbizidwirkstoffe war dabei nicht beeinträchtigt. Die Safener eignen sich daher in Kombination mit Herbiziden in vielen Fällen für die selektive Kontrolle von Schadpflanzen bei der Vorauflaufbehandlung von Nutzpfanzenkulturen.

### 3.4 SAATGUTBEHANDLUNG

Saatkörner von Kulturpflanzen wurden mit den als Suspensions- oder Emulsionskonzentraten formulierten erfindungsgemäßen Safenern und Wasser in Flaschen gemischt und gut geschüttelt, so dass die Saatkörner gleichmäßig mit der Formulierung des jeweiligen Safeners beschichtet wurden. Die Saatkörner bzw. die aufgelaufenen Pflanzen wurden dann im Vorauflauf- oder Nachauflaufverfahren entsprechend den Versuchen nach Beispielen 3.3 bzw. 3.2 mit Herbiziden getestet. Auch bei der Behandlung von Saatgut zeigten die Safener gute Wirkung. Die herbizide Wirkung der verwendeten Herbizidwirkstoffe war dabei nicht beeinträchtigt.

## Patentansprüche

1. Verwendung von Verbindungen der Formel (I), oder deren Salzen, 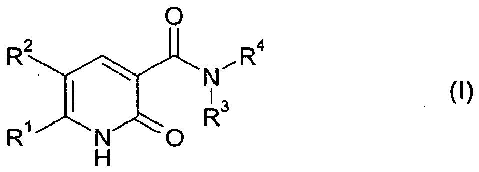 worin
R¹ einen (C₁-C₆)Haloalkylrest bedeutet und
R² Wasserstoff oder Halogen bedeutet und
R³ Wasserstoff, (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet und
R⁴ (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
R³ (C₁-C₄)-Alkoxy, (C₂-C₄)Alkenyloxy, (C₂-C₆)Alkinyloxy oder (C₂-C₄)Haloalkoxy bedeutet und
R⁴ Wasserstoff oder (C₁-C₄)-Alkyl bedeutet oder
R³ und R⁴ zusammmen mit dem direkt gebundenen N-Atom einen vier- bis achtgliedrigen heterocyclischen Ring, der neben dem N-Atom auch weitere Heteroringatome enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet oder
R³ und R⁴ zusammen mit dem direkt gebundenen N-Atom die Gruppe -N=CR⁵-NR⁶R⁷ bedeutet, worin
R⁵ für Wasserstoff oder (C₁-C₆)Alkyl steht, und
R⁶, R⁷ unabhängig voneinander für Wasserstoff oder (C₁-C₄)Alkyl stehen oder
R⁶ und R⁷ zusammen mit dem direkt gebundenen N-Atom einen fünf- bis siebengliedrigen heterocyclischen Ring bilden, oder
R¹ einen (C₁-C₆)Haloalkylrest bedeutet,
R² Halogen bedeutet,
R³ Wasserstoff bedeutet und
R⁴ Wasserstoff bedeutet oder
R¹ einen Rest der Formel CF₂Cl, CF₂H, CF₂CF₃, CF₂CF₂H, CF₂CF₂Cl, CFClCF₃, CFHCF₃, CF(CF₃)₂ , CH(CF₃)₂, CF₂CF₂CF₃, oder C(CH₃)₂F bedeutet,
R² Wasserstoff bedeutet,
R³ Wasserstoff bedeutet und
R⁴ Wasserstoff bedeutet,
als nutzpflanzenschützendes Mittel zum Reduzieren oder Verhindern von Schadwirkungen von Agrochemikalien an den Nutzpflanzen.

2. Verwendung nach Anspruch 1, **dadurch gekennzeichnet, dass**
R³ Wasserstoff, (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, substituiert ist, und
R⁴ (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthlo, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, substituiert ist, bedeuten.

3. Verwendung nach Anspruch 1, **dadurch gekennzeichnet, dass**
R³ Wasserstoff, (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, oder (C₃-C₆)Cycloalkyl oder (C₃-C₆)Cycloalkyl, das an einer Seite des Rings mit einem 4 bis 6-gliedrigen gesättigten oder ungesättigten carbocyclischen Ring kondensiert ist, wobei jeder der letztgenannten 2 Reste unsubstituiert oder durch einen oder mehrere Reste aus der Gruppe (C₁-C₄)Alkyl substituiert ist,
und
R⁴ (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio und Oxo substituiert ist, substituiert ist, oder (C₃-C₆)Cycloalkyl oder (C₃-C₆)Cycloalkyl, das an einer Seite des Rings mit einem 4 bis 6-gliedrigen gesättigten oder ungesättigten carbocyclischen Ring kondensiert ist, wobei jeder der letztgenannten 2 Reste unsubstituiert oder durch einen oder mehrere Reste aus der Gruppe (C₁-C₄)Alkyl substituiert ist, bedeuten.

4. Verwendung nach Anspruch 1, **dadurch gekennzeichnet, dass**
R³ Wasserstoff, (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und [(C₁-C₄)Alkoxy]-carbonyl substituiert ist,
und
R⁴ (C₁-C₁₀)Alkyl, (C₂-C₁₀)Alkenyl oder (C₂-C₁₀)Alkinyl, wobei jeder der letztgenannten 3 Reste unsubstituiert oder durch einen oder mehrere Reste aus der Gruppe (C₁-C₄)Alkoxy, (C₁-C₄)Haloalkoxy, (C₁-C₄)Alkylthio und [(C₁-C₄)Alkoxy]-carbohyl substituiert ist, bedeuten.

5. Verwendung nach einem der Ansprüche 1 bis 4, **dadurch gekennzeichnet, dass**
R¹ CF₃, CF₂Cl, CF₂H, CF₂CF₃, CF₂CF₂H, CF₂CF₂Cl, CF₂CF₂CF₃ oder C(CH₃)₂F und
R² Wasserstoff oder Halogen bedeuten.

6. Verwendung nach Anspruch 1, **dadurch gekennzeichnet**,
R¹ einen (C₁-C₆)Haloalkylrest,
R² Halogen,
R³ Wasserstoff, und
R⁴ Wasserstoff bedeuten.

7. Verwendung nach Anspruch 1, **dadurch gekennzeichnet**,
R¹ einen Rest der Formel CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃, oder C(CH₃)₂F,
R² Wasserstoff,
R³ Wasserstoff und
R⁴ Wasserstoff
bedeuten.

8. Verwendung nach einem der Ansprüche 1 bis 7, **dadurch gekennzeichnet, dass** ein oder mehrere Verbindungen der Formel (I) oder deren Salze zusammen mit einer oder mehreren Agrochemikalien, welche alleine appliziert Schäden an den Nutzpflanzen verursachen, gegebenenfalls in Gegenwart von Formulierungshilfsmitteln, verwendet werden.

9. Verbindungen der Formel (I) oder deren Salze, 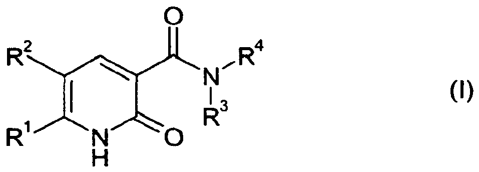 worin
R¹ einen (C₁-C₆)Haloalkylrest bedeutet,
R² Wasserstoff oder Halogen bedeutet und
R³ Wasserstoff, (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthlo, (C₁-C₄)Alkylamino, Di[(C₁-C₄)alkyl]-amino, [(C₁-C₄)Alkoxy]-carbonyl und [(C₁-C₄)Haloalkoxyl-carbonyl, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet und
R⁴ (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl und [(C₁-C₄)Haloalkoxy]-carbonyl, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino; [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
R³ (C₁-C₄)-Alkoxy, (C₂-C₄)Alkenyloxy, (C₂-C₆)Alkinyloxy oder (C₂-C₄)Haloalkoxy bedeutet und
R⁴ Wasserstoff oder (C₁-C₄)-Alkyl bedeutet oder
R³ und R⁴ zusammen mit dem direkt gebundenen N-Atom einen vier- bis achtgliedrigen heterocyclischen Ring, der neben dem N-Atom auch weitere Heteroringatome enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet oder
R³ und R⁴ zusammen mit dem direkt gebundenen N-Atom die Gruppe -N=CR⁵-NR⁶R⁷ bedeutet, worin
R⁵ für Wasserstoff oder (C₁-C₆)Alkyl steht und
R⁶, R⁷ unabhängig voneinander für Wasserstoff oder (C₁-C₄)Alkyl stehen oder R⁶ und R⁷ zusammen mit dem direkt gebundenen N-Atom einen fünfbis siebengliedrigen heterocyclischen Ring bilden, oder
R¹ einen (C₁-C₆)Haloalkylrest bedeutet,
R² Halogen bedeutet,
R³ Wasserstoff bedeutet und
R⁴ Wasserstoff bedeutet oder
R¹ einen Rest der Formel CF₂CF₃, CF₂CF₂H, CF₂CF₂Cl, CFClCF₃, CFHCF₃, CF(CF₃)₂, CH(CF₃)₂, CF₂CF₂CF₃, oder C(CH₃)₂F bedeutet,
R² Wasserstoff bedeutet,
R³ Wasserstoff bedeutet und
R⁴ Wasserstoff bedeutet.

10. Verfahren zur Herstellung von Verbindungen der allgemeinen Formel (I) oder deren Salzen gemäß Anspruch 9, **dadurch gekennzeichnet, dass** man
(a) eine Carbonsäure der allgemeinen Formel (II) 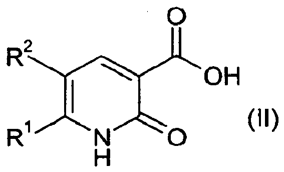 worin R¹ und R² wie in der herzustellenden Verbindung der Formel (I) definiert sind,
mit einem Amin der Formel (III) oder dessen Salz, 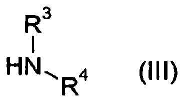 worin R³ und R⁴ wie in der herzustellenden Verbindung der Formel (I) definiert sind,
zur Verbindung der Formel (I) umsetzt oder
(b) einen Carbonsäureester der allgemeinen Formel (IV) 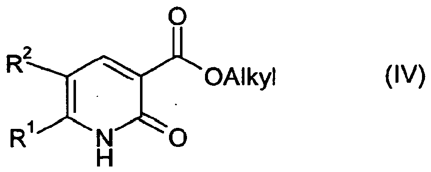 worin R¹ und R² wie in der herzustellenden Verbindung der Formel (I) definiert sind und "Alkyl" einen Alkylrest bedeutet, mit einem Amin der Formel (III) oder dessen Salz, 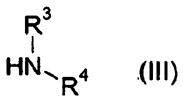 worin R³ und R⁴ wie in der herzustellenden Verbindung der Formel (I) definiert sind, zur Verbindung der Formel (I) umsetzt oder
(c) ein Carbonsäurehalogenid oder -anhydrid der allgemeinen Formel (V), 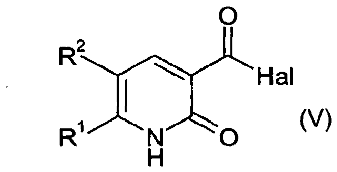 worin R¹ und R² wie in der herzustellenden Verbindung der Formel (I) definiert sind und Hal ein Halogenatom oder einen Acyloxyrest bedeutet, mit einem Amin der Formel (III) oder dessen Salz, 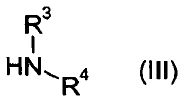 worin R³ und R⁴ wie in der herzustellenden Verbindung der Formel (I) definiert sind, zur Verbindung der Formel (I) umsetzt, oder
(d), im Falle, dass R³ und R⁴ in der herzustellenden Verbindung der Formel (I) jeweils Wasserstoff bedeutet,
eine Verbindung der Formel (VI), 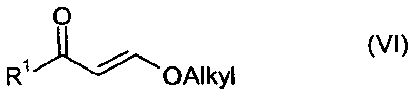 worin R¹ wie in der herzustellenden Verbindung der Formel (I) definiert ist und "Alkyl" einen Alkylrest bedeutet,
mit Malonsäurediamid zur Verbindung der Formel (I) umsetzt.

11. Pflanzenschutzmittel, **dadurch gekennzeichnet, dass** es eine oder mehrere Verbindungen der Formel (I) oder deren Salze, wie sie nach einem der Ansprüche 1 bis 7 und 9 definiert sind, und Formulierungshilfsmittel enthält.

12. Pflanzenschutzmittel, **dadurch gekennzeichnet, dass** es eine oder mehrere Verbindungen der Formel (I) oder deren Salze, wie sie nach einem der Ansprüche 1 bis 7 und 9 definiert sind, und ein oder mehrere Agrochemikalien und gegebenenfalls Formulierungshilfsmittel enthält.

13. Verfahren zum Schützen von Nutzpflanzen vor phytotoxischen Nebenwirkungen von Agrochemikalien, **dadurch gekennzeichnet, dass** eine wirksame Menge einer oder mehrerer Verbindungen der Formel (I) oder deren Salze, wie sie nach einem der Ansprüche 1 bis 7 und 9 definiert sind, vor, nach oder gleichzeitig mit der oder den Agrochemikalien auf die Pflanzen, Pflanzenteile, Pflanzensamen oder das Saatgut appliziert wird.

14. Verfahren nach Anspruch 13, **dadurch gekennzeichnet, dass** die Applikation im Nachauflaufverfahren erfolgt.

15. Verfahren nach Anspruch 13, **dadurch gekennzeichnet, dass** die Applikation mit der Verbindung der Formel (I) oder deren Salz durch Behandlung der Pflanzensamen oder des Saatguts erfolgt.

16. Verfahren nach Anspruch 13, **dadurch gekennzeichnet, dass** die Applikation im Vorauflaufverfahren erfolgt.

17. Verfahren zur selektiven Bekämpfung von Schadpflanzen in Nutzpflanzenkulturen, **dadurch gekennzeichnet, dass** man eine wirksame Menge einer oder mehrerer Verbindungen der Formel (I) oder deren Salze, wie sie nach einem der Ansprüche 1 bis 7 und 9 definiert sind, vor, nach oder gleichzeitig mit einem oder mehreren Herbiziden auf die Pflanzen, Pflanzenteile, Pflanzensamen oder das Saatgut appliziert.

18. Verfahren nach Anspruch 17, **dadurch gekennzeichnet, dass** man das Saatgut mit einer oder mehreren Verbindungen der Formel (I) oder deren Salze behandelt und das Herbizid nach der Einsaat im Vorauflaufverfahren oder im Nachauflaufverfahren appliziert.

## Claims

1. A use of a compound of the formula (I) or a salt
thereof 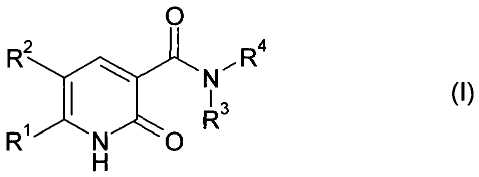 in which
R¹ is a (C₁-C₆)-haloalkyl radical and
R² is hydrogen or halogen and
R³ is hydrogen, (C₁-C₁₆)-alkyl, (C₂-C₁₆)-alkenyl or (C₂-C₁₆)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄)-alkylamino, di [(C₁-C₄)-alkyl]-amino, [(C₁-C₄)-alkoxy]-carbonyl, [(C₁-C₄)-haloalkoxy]-carbonyl, (C₃-C₆)-cycloalkyl, which is unsubstituted or substituted, phenyl, which is unsubstituted or substituted, and heterocyclyl, which is unsubstituted or substituted, or (C₃-C₆)-cycloalkyl, (C₄-C₆)-cycloalkenyl, (C₃-C₆)-cycloalkyl which is condensed at one side of the ring to a 4- to 6-membered saturated or unsaturated carbocyclic ring, or (C₄-C₆)-cycloalkenyl which is condensed at one side of the ring to a 4- to 6-membered saturated or unsaturated carbocyclic ring, where each of the 4 lastmentioned radicals is unsubstituted or substituted by one or more radicals selected from the group consisting of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted, phenyl, which is unsubstituted or substituted, and heterocyclyl, which is unsubstituted or substituted, and
R⁴ is (C₁-C₁₆)-alkyl, (C₂-C₁₆)-alkenyl or (C₂-C₁₆)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted, phenyl, which is unsubstituted or substituted, and heterocyclyl, which is unsubstituted or substituted, or (C₃-C₆)-cycloalkyl, (C₄-C₆)-cycloalkenyl, (C₃-C₆)-cycloalkyl which is condensed at one side of the ring to a 4- to 6-membered saturated or unsaturated carbocyclic ring, or (C₄-C₆)-cycloalkenyl which is condensed at one side of the ring to a 4- to 6-membered saturated or unsaturated carbocyclic ring, where each of the 4 lastmentioned radicals is unsubstituted or substituted by one or more radicals selected from the group consisting of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted, phenyl, which is unsubstituted or substituted, and heterocyclyl, which is unsubstituted or substituted, or
R³ is (C₁-C₄)-alkoxy, (C₂-C₄)-alkenyloxy, (C₂-C₆)-alkynyloxy or (C₂-C₄)-haloalkoxy and
R⁴ is hydrogen or (C₁-C₄)-alkyl or
R³ and R⁴ together with the directly attached nitrogen atom are a four- to eight-membered heterocyclic ring which, in addition to the nitrogen atom, may also comprise further hetero ring atoms and which is unsubstituted or substituted by one or more radicals selected from the group consisting of halogen, cyano, nitro, (C₁-C₄)-alkyl, (C₁-C₄)-haloalkyl, (C₁-C₄)-alkoxy, (C₁-C₄)-haloalkoxy and (C₁-C₄)-alkylthio, or
R³ and R⁴ together with the directly attached nitrogen atom are the group -N=CR⁵-NR⁶R⁷ in which
R⁵ is hydrogen or (C₁-C₆)-alkyl, and
R⁶, R⁷ independently of one another are hydrogen or (C₁-C₄)-alkyl, or
R⁶ and R⁷ together with the directly attached nitrogen atom form a five- to seven-membered, heterocyclic ring, or
R¹ is a (C₁-C₆)-haloalkyl radical,
R² is halogen,
R³ is hydrogen and
R⁴ is hydrogen or
R¹ is a radical of the formula CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃, or C(CH₃)₂F,
R² is hydrogen,
R³ is hydrogen and
R⁴ is hydrogen,
as a useful-plant-protecting agent for reducing or preventing harmful actions of agrochemicals on the useful plants.

2. The use as claimed in claim 1 wherein
R³ is hydrogen, (C₁-C₁₀)-alkyl, (C₂-C₁₀)-alkenyl or (C₂-C₁₀)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted by one or more radicals selected from the group consisting of halogen, (C₁-C₄)-alkyl and (C₁-C₄)-haloalkyl, phenyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy and (C₁-C₄)-alkylthio, and heterocyclyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo, or (C₃-C₆)-cycloalkyl, (C₄-C₆)-cycloalkenyl, (C₃-C₆)-cycloalkyl which is condensed at one side of the ring to a 4- to 6-membered saturated or unsaturated carbocyclic ring, or (C₄-C₆)-cycloalkenyl which is condensed at one side of the ring to a 4- to 6-membered saturated or unsaturated carbocyclic ring, where each of the 4 lastmentioned radicals is unsubstituted or substituted by one or more radicals selected from the group consisting of halogen, hydroxyl, cyano, (C₁-C₄)-alkyl, (C₁-C₄)-haloalkyl, (C₁-C₄)-alkoxy, (C₁-C₄) haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted by one or more radicals selected from the group consisting of halogen, (C₁-C₄)-alkyl and (C₁-C₄)-haloalkyl, phenyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy and (C₁-C₄)-alkylthio, and heterocyclyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo, and
R⁴ is (C₁-C₁₀)-alkyl, (C₂-C₁₀)-alkenyl or (C₂-C₁₀)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted by one or more radicals selected from the group consisting of halogen, (C₁-C₄)-alkyl and (C₁-C₄)-haloalkyl, phenyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy and (C₁-C₄)-alkylthio, and heterocyclyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo, or (C₃-C₆)-cycloalkyl, (C₄-C₆)-cycloalkenyl, (C₃-C₆)-cycloalkyl which is condensed at one side of the ring to a 4- to 6-membered saturated or unsaturated carbocyclic ring, or (C₄-C₆)-cycloalkenyl which is condensed at one side of the ring to a 4- to 6-membered saturated or unsaturated carbocyclic ring, where each of the 4 lastmentioned radicals is unsubstituted or substituted by one or more radicals selected from the group consisting of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted by one or more radicals selected from the group consisting of halogen, (C₁-C₄)-alkyl and (C₁-C₄)-haloalkyl, phenyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy and (C₁-C₄)-alkylthio, and heterocyclyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo.

3. The use as claimed in claim 1 wherein
R³ is hydrogen, (C₁-C₁₀)-alkyl, (C₂-C₁₀)-alkenyl or (C₂-C₁₀)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄)-alkylamino, di[(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted by one or more radicals selected from the group consisting of halogen, (C₁-C₄)-alkyl and (C₁-C₄)-haloalkyl, phenyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy and (C₁-C₄)-alkylthio, and heterocyclyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo, or (C₃-C₆)-cycloalkyl or (C₃-C₆)-cycloalkyl which is condensed at one side of the ring to a 4- to 6-membered saturated or unsaturated carbocyclic ring, where each of the 2 lastmentioned radicals is unsubstituted or substituted by one or more radicals selected from the group consisting of (C₁-C₄)-alkyl, and
R⁴ is (C₁-C₁₀)-alkyl, (C₂-C₁₀)-alkenyl or (C₂-C₁₀)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄) alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted by one or more radicals selected from the group consisting of halogen, (C₁-C₄)-alkyl and (C₁-C₄)-haloalkyl, phenyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy and (C₁-C₄)-alkylthio, and heterocyclyl, which is unsubstituted or substituted by one or more radicals selected from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo, or (C₃-C₆)-cycloalkyl or (C₃-C₆)-cycloalkyl which is condensed at one side of the ring to a 4- to 6-membered saturated or unsaturated carbocyclic ring, where each of the 2 lastmentioned radicals is unsubstituted or substituted by one or more radicals selected from the group consisting of (C₁-C₄)-alkyl.

4. The use as claimed in claim 1 wherein
R³ is hydrogen, (C₁-C₁₀)-alkyl, (C₂-C₁₀)-alkenyl or (C₂-C₁₀)-alkynyl, where each of the 3 lastmentioned radicals is unsubstituted or substituted by one or more radicals selected from the group consisting of (C₁-C₄)-alkoxy, (C₁-C₄)-haloalkoxy, (C₁-C₄)-alkylthio and [(C₁-C₄)-alkoxy]-carbonyl,
and
R⁴ is (C₁-C₁₀)-alkyl, (C₂-C₁₀)-alkenyl or (C₂-C₁₀)-alkynyl, where each of the 3 lastmentioned radicals is unsubstituted or substituted by one or more radicals selected from the group consisting of (C₁-C₄)-alkoxy, (C₁-C₄)-haloalkoxy, (C₁-C₄)-alkylthio and [(C₁-C₄)-alkoxy]-carbonyl.

5. The use as claimed in any of claims 1 to 4 wherein
R¹ is CF₃, CF₂Cl, CF₂H, CF₂CF₃, CF₂CF₂H, CF₂CF₂Cl, CF-₂CF₂CF₃ or C(CH₃)₂F and
R² is hydrogen or halogen.

6. The use as claimed in claim 1 wherein
R¹ is a (C₁-C₆)-haloalkyl radical,
R² is halogen,
R³ is hydrogen, and
R⁴ is hydrogen.

7. The use as claimed in claim 1 wherein
R¹ is a radical of the formula CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃, or C(CH₃)₂F,
R² is hydrogen,
R³ is hydrogen and
R⁴ is hydrogen.

8. The use as claimed in any of claims 1 to 7 wherein one or more compounds of the formula (I) or salts thereof are used together with one or more agrochemicals which, applied on their own, cause damage to the useful plants, optionally in the presence of formulation auxiliaries.

9. A compound of the formula (I) or a salt thereof 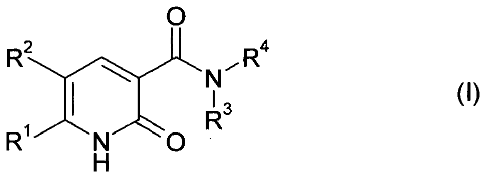 in which
R¹ is a (C₁-C₆)-haloalkyl radical and
R² is hydrogen or halogen and
R³ is hydrogen, (C₁-C₁₆)-alkyl, (C₂-C₁₆)-alkenyl or (C₂-C₁₆)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄)-alkylamino, di[(C₁-C₄)-alkyl] -amino, [(C₁-C₄)-alkoxy]-carbonyl and [(C₁-C₄)-haloalkoxy]-carbonyl or (C₃-C₆)-cycloalkyl, (C₄-C₆)-cycloalkenyl, (C₃-C₆)-cycloalkyl which is condensed at one side of the ring to a 4- to 6-membered saturated or unsaturated carbocyclic ring, or (C₄-C₆)-cycloalkenyl which is condensed at one side of the ring to a 4- to 6-membered saturated or unsaturated carbocyclic ring, where each of the 4 lastmentioned radicals is unsubstituted or substituted by one or more radicals selected from the group consisting of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di[(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted, phenyl, which is unsubstituted or substituted, and heterocyclyl, which is unsubstituted or substituted, and
R⁴ is (C₁-C₁₆)-alkyl, (C₂-C₁₆)-alkenyl or (C₂-C₁₆)-alkynyl, where each of the 3 lastmentioned radicals is unsubstituted or substituted by one or more radicals selected from the group consisting of halogen, hydroxyl, cyano, (C₁-C₄)-alkoxy, (C₁-C₄)-haloalkoxy, (C₁-C₄)-alkylthio, (C₁-C₄)-alkylamino, di[(C₁-C₄)-alkyl]-amino, [(C₁-C₄)-alkoxy]-carbonyl and [(C₁-C₄)-haloalkoxy]-carbonyl, or (C₃-C₆)-cycloalkyl, (C₄-C₆)-cycloalkenyl, (C₃-C₆)-cycloalkyl which is condensed at one side of the ring to a 4- to 6-membered saturated or unsaturated carbocyclic ring, or (C₄-C₆)-cycloalkenyl which is condensed at one side of the ring to a 4- to 6-membered saturated or unsaturated carbocyclic ring, where each of the 4 lastmentioned radicals is unsubstituted or substituted by one or more radicals selected from the group consisting of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di [(C₁-C₄)-alkyl] -amino, [(C₁-C₄)-alkoxy]-carbonyl, [(C₁-C₄)-haloalkoxy]-carbonyl, (C₃-C₆)-cycloalkyl, which is unsubstituted or substituted, phenyl, which is unsubstituted or substituted, and heterocyclyl, which is unsubstituted or substituted, or
R³ is (C₁-C₄)-alkoxy, (C₂-C₄)-alkenyloxy, (C₂-C₆)-alkynyloxy or (C₂-C₄)-haloalkoxy and
R⁴ is hydrogen or (C₁-C₄)-alkyl or
R³ and R⁴ together with the directly attached nitrogen atom are a four- to eight-membered heterocyclic ring which, in addition to the nitrogen atom, may also comprise further hetero ring atoms and which is unsubstituted or substituted by one or more radicals selected from the group consisting of halogen, cyano, nitro, (C₁-C₄)-alkyl, (C₁-C₄)-haloalkyl, (C₁-C₄)-alkoxy, (C₁-C₄)-haloalkoxy and (C₁-C₄)-alkylthio, or
R³ and R⁴ together with the directly attached nitrogen atom are the group -N=CR⁵-NR⁶R⁷ in which
R⁵ is hydrogen or (C₁-C₆)-alkyl, and
R⁶, R⁷ independently of one another are hydrogen or (C₁-C₄)-alkyl, or
R⁶ and R⁷ together with the directly attached nitrogen atom form a five- to seven-membered heterocyclic ring, or
R¹ is a (C₁-C₆)-haloalkyl radical,
R² is halogen,
R³ is hydrogen and
R⁴ is hydrogen or
R¹ is a radical of the formula CF₂CF₃, CF₂CF₂H, CF₂CF₂Cl, CFClCF₃, CFHCF₃, CF(CF₃)₂, CH(CF₃)₂, CF₂CF₂CF₃, or C(CH₃)₂F,
R² is hydrogen,
R³ is hydrogen and
R⁴ is hydrogen.

10. A process for preparing compounds of the general formula (I) or salts thereof as defined in claim 9, which comprises
(a) reacting a carboxylic acid of the general formula (II) 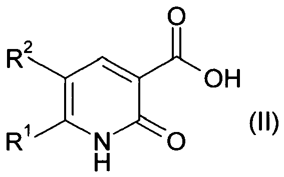 in which R¹ and R² are as defined for the compound of the formula (I) to be prepared,
with an amine of the formula (III) or a salt thereof, 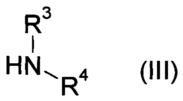 in which R³ and R⁴ are as defined for the compound of the formula (I) to be prepared,
to give the compound of the formula (I) or
(b) reacting a carboxylic ester of the general formula (IV) 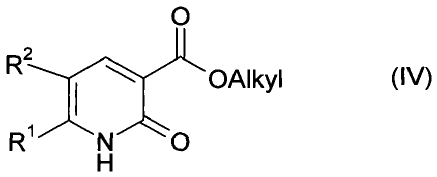 in which R¹ and R² are as defined for the compound of the formula (I) to be prepared and "Alkyl" is an alkyl radical, with an amine of the formula (III) or a salt thereof, 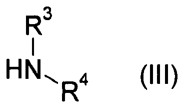 in which R³ and R⁴ are as defined for the compound of the formula (I) to be prepared, to give the compound of the formula (I) or
(c) reacting a carbonyl halide or a carboxylic
anhydride of the general formula (V), 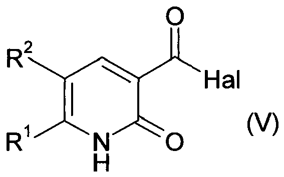 in which R¹ and R² are as defined for the compound of the formula (I) to be prepared and Hal is a halogen atom or an acyloxy radical with an amine of the formula (III) or a salt thereof, 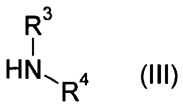 in which R³ and R⁴ are as defined for the compound of the formula (I) to be prepared to give the compound of the formula (I), or
(d) if R³ and R⁴ in the compound of the formula (I) to be prepared are each hydrogen,
reacting a compound of the formula (VI), 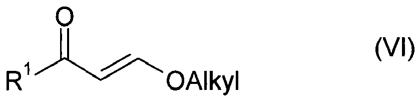 in which R¹ is as defined for the compound of the formula (I) to be prepared, and "Alkyl" is an alkyl radical,
with malonamide to give the compound of the formula (I).

11. A crop protection composition which comprises one or more compounds of the formula (I) or salts thereof as defined in any of claims 1 to 7 and 9 and formulation auxiliaries.

12. A crop protection composition which comprises one or more compounds of the formula (I) or salts thereof as defined in any of claims 1 to 7 and 9 and one or more agrochemicals and, optionally, formulation auxiliaries.

13. A method for protecting useful plants against phytotoxic side effects of agrochemicals, which comprises applying an effective amount of one or more compounds of the formula (I) or salts thereof as defined in any of claims 1 to 7 and 9 before, after or simultaneously with the agrochemical(s) to the plants, parts of plants, plant seeds or seed.

14. The method as claimed in claim 13, wherein the application is by the post-emergence method.

15. The method as claimed in claim 13, wherein the application of the compound of the formula (I) or a salt thereof is by treating the plant seeds or the seed.

16. The method as claimed in claim 13, wherein the application is by the pre-emergence method.

17. A method for the selective control of harmful plants in crops of useful plants, which comprises applying an effective amount of one or more compounds of the formula (I) or salts thereof as defined in any of claims 1 to 7 and 9 before, after or simultaneously with one or more herbicides to the plants, parts of plants, plant seeds or the seed.

18. The method as claimed in claim 17, which comprises treating the seed with one or more compounds of the formula (I) or salts thereof and applying the herbicide after sowing by the pre-emergence method or by the post-emergence method.

## Revendications

1. Utilisation de composés de formule (I) ou de leurs sels, 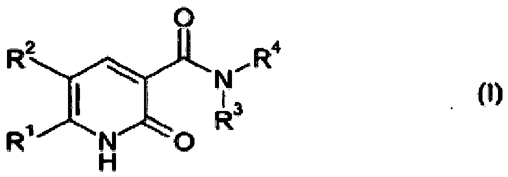 formule dans laquelle
R¹ représente un radical halogénoalkyle(C₁-C₆) et
R² représente un atome d'hydrogène ou d'halogène et
R³ représente un atome d'hydrogène, un groupe alkyle en C₁-C₁₆, alcényle en C₂-C₁₆ ou alcynyle en C₂-C₁₆, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est substitué ou non substitué, phényle qui est substitué ou non substitué, et hétérocyclyle qui est substitué ou non substitué, ou cycloalkyle en C₃-C₆, cycloalcényle en C₄-C₆, cycloalkyle en C₃-C₆ qui est condensé sur un côté du cycle avec un cycle carbocyclique saturé ou insaturé à 4 à 6 chaînons, ou cycloalcényle en C₄-C₆ qui est condensé sur un côté du cycle avec un cycle carbocyclique saturé ou insaturé à 4 à 6 chaînons, chacun des 4 radicaux nommés en dernier étant non substitué ou substitué par un ou plusieurs radicaux choisis dans le groupe constitué par halogéno, hydroxy, cyano, alkyle en C₁-C₄, halogénoalkyle(C₁-C₄), alcoxy en C₁-C₄, halogénoalcoxy(C₁-C₄), alkyl(C₁-C₄)thio, alkyl(C₁-C₄)amino, di [alkyl(C₁-C₄)]-amino, [alcoxy(C₁-C₄)]-carbonyle, [halogénoalcoxy(C₁-C₄)]-carbonyle, cycloalkyle en C₃-C₆ qui est substitué ou non substitué, phényle qui est substitué ou non substitué, et hétérocyclyle qui est substitué ou non substitué, et
R⁴ représente un groupe alkyle en C₁-C₁₆, alcényle en C₂-C₁₆ ou alcynyle en C₂-C₁₆, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est substitué ou non substitué, phényle qui est substitué ou non substitué, et hétérocyclyle qui est substitué ou non substitué, ou cycloalkyle en C₃-C₆, cycloalcényle en C₄-C₆, cycloalkyle en C₃-C₆ qui est condensé sur un côté du cycle avec un cycle carbocyclique saturé ou insaturé à 4 à 6 chaînons, ou cycloalcényle en C₄-C₆ qui est condensé sur un côté du cycle avec un cycle carbocyclique saturé ou insaturé à 4 à 6 chaînons, chacun des 4 radicaux nommés en dernier étant non substitué ou substitué par un ou plusieurs radicaux choisis dans le groupe constitué par halogéno, hydroxy, cyano, alkyle en C₁-C₄, halogénoalkyle(C₁-C₄), alcoxy en C₁-C₄, halogénoalcoxy(C₁-C₄), alkyl (C₁-C₄)thio, alkyl (C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est substitué ou non substitué, phényle qui est substitué ou non substitué, et hétérocyclyle qui est substitué ou non substitué, ou
R³ représente un groupe alcoxy en C₁-C₄, alcényl(C₂-C₄)oxy, alcynyl(C₂-C₆)oxy ou halogéno-alcoxy(C₂-C₄) et
R⁴ représente un atome d'hydrogène ou un groupe alkyle en C₁-C₄ ou
R³ et R⁴ forment ensemble avec l'atome d'azote directement lié un cycle hétérocyclique à quatre à huit chaînons, qui outre l'atome d'azote peut également contenir d'autres hétéroatomes formant le cycle et qui est non substitué ou substitué par un ou plusieurs radicaux choisis dans le groupe constitué par halogéno, cyano, nitro, alkyle en C₁-C₄, halogénoalkyle (C₁-C₄), alcoxy en C₁-C₄, halogénoalcoxy(C₁-C₄) et alkyl(C₁-C₄)thio, ou
R³ et R⁴ forment ensemble avec l'atome d'azote directement lié le groupe -N=CR⁵-NR⁶R⁷, dans lequel
R⁵ représente un atome d'hydrogène ou un groupe alkyle en C₁-C₆, et
R⁶, R⁷ représentent indépendamment l'un de l'autre un atome d'hydrogène ou un groupe alkyle en C₁-C₄ ou
R⁶ et R⁷ forment ensemble avec l'atome d'azote directement lié un cycle hétérocyclique à cinq à sept chaînons, ou
R¹ représente un radical halogénoalkyle(C₁-C₆),
R² représente un atome d'halogène,
R³ représente un atome d'hydrogène et
R⁴ représente un atome d'hydrogène ou
R¹ représente un radical de formule CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃ ou C(CH₃)₂F,
R² représente un atome d'hydrogène,
R³ représente un atome d'hydrogène et
R⁴ représente un atome d'hydrogène,
en tant qu'agent protecteur de plantes utiles pour la réduction ou l'empêchement d'effets nocifs de substances agrochimiques sur les plantes utiles.

2. Utilisation selon la revendication 1, **caractérisée en ce que**
R³ représente un atome d'hydrogène, un groupe alkyle en C₁-C₁₀, alcényle en C₂-C₁₀ ou alcynyle en C₂-C₁₀, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est non substitué ou substitué par un ou plusieurs radicaux choisis dans le groupe constitué par halogéno, alkyle en C₁-C₄ et halogéno-alkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄) et alkyl(C₁-C₄)thio, et hétérocyclyle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo, ou cycloalkyle en C₃-C₆, cycloalcényle en C₄-C₆, cycloalkyle en C₃-C₆ qui est condensé sur un côté du cycle avec un cycle carbocyclique saturé ou insaturé à 4 à 6 chaînons, ou cycloalcényle en C₄-C₆ qui est condensé sur un côté du cycle avec un cycle carbocyclique saturé ou insaturé à 4 à 6 chaînons, chacun des 4 radicaux nommés en dernier étant non substitué ou substitué par un ou plusieurs radicaux choisis dans le groupe constitué par halogéno, hydroxy, cyano, alkyle en C₁-C₄, halogénoalkyle(C₁-C₄), alcoxy en C₁-C₄, halogénoalcoxy(C₁-C₄), alkyl(C₁-C₄)thio, alkyl (C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est non substitué ou substitué par un ou plusieurs radicaux choisis dans le groupe constitué par halogéno, alkyle en C₁-C₄ et halogéno-alkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄) et alkyl(C₁-C₄)thio, et hétérocyclyle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo, et
R⁴ représente un groupe alkyle en C₁-C₁₀, alcényle en C₂-C₁₀ ou alcynyle en C₂-C₁₀, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est non substitué ou substitué par un ou plusieurs radicaux choisis dans le groupe constitué par halogéno, alkyle en C₁-C₄ et halogéno-alkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄) et alkyl(C₁-C₄)thio, et hétérocyclyle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo, ou cycloalkyle en C₃-C₆, cycloalcényle en C₄-C₆, cycloalkyle en C₃-C₆ qui est condensé sur un côté du cycle avec un cycle carbocyclique saturé ou insaturé à 4 à 6 chaînons, ou cycloalcényle en C₄-C₆ qui est condensé sur un côté du cycle avec un cycle carbocyclique saturé ou insaturé à 4 à 6 chaînons, chacun des 4 radicaux nommés en dernier étant non substitué ou substitué par un ou plusieurs radicaux choisis dans le groupe constitué par halogéno, hydroxy, cyano, alkyle en C₁-C₄, halogénoalkyle(C₁-C₄), alcoxy en C₁-C₄, halogénoalcoxy(C₁-C₄), alkyl(C₁-C₄)thio, alkyl(C₁-C₄)amino, di [alkyl (C₁-C₄)]-amino, [alcoxy(C₁-C₄)]-carbonyle, [halogénoalcoxy(C₁-C₄)]-carbonyle, cycloalkyle en C₃-C₆ qui est non substitué ou substitué par un ou plusieurs radicaux choisis dans le groupe constitué par halogéno, alkyle en C₁-C₄ et halogéno-alkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄) et alkyl(C₁-C₄)thio, et hétérocyclyle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo.

3. Utilisation selon la revendication 1, **caractérisée en ce que**
R³ représente un atome d'hydrogène, un groupe alkyle en C₁-C₁₀, alcényle en C₂-C₁₀ ou alcynyle en C₂-C₁₀, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est non substitué ou substitué par un ou plusieurs radicaux choisis dans le groupe constitué par halogéno, alkyle en C₁-C₄ et halogéno-alkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄) et alkyl(C₁-C₄)thio, et hétérocyclyle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo, ou cycloalkyle en C₃-C₆ ou cycloalkyle en C₃-C₆ qui est condensé sur un côté du cycle avec un cycle carbocyclique saturé ou insaturé à 4 à 6 chaînons, et chacun des 2 radicaux nommés en dernier étant non substitué ou substitué par un ou plusieurs radicaux choisis dans le groupe constitué par alkyle en C₁-C₄,
R⁴ représente un groupe alkyle en C₁-C₁₀, alcényle en C₂-C₁₀ ou alcynyle en C₂-C₁₀, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est non substitué ou substitué par un ou plusieurs radicaux choisis dans le groupe constitué par halogéno, alkyle en C₁-C₄ et halogéno-alkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄) et alkyl(C₁-C₄)thio, et hétérocyclyle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo, ou cycloalkyle en C₃-C₆ ou cycloalkyle en C₃-C₆ qui est condensé sur un côté du cycle avec un cycle carbocyclique saturé ou insaturé à 4 à 6 chaînons, chacun des 2 radicaux nommés en dernier étant non substitué ou substitué par un ou plusieurs radicaux choisis dans le groupe constitué par alkyle en C₁-C₄.

4. Utilisation selon la revendication 1, **caractérisée en ce que**
R³ représente un atome d'hydrogène, un groupe alkyle en C₁-C₁₀, alcényle en C₂-C₁₀ ou alcynyle en C₂-C₁₀, chacun des 3 radicaux nommés en dernier étant non substitué ou substitué par un ou plusieurs radicaux choisis dans le groupe constitué par alcoxy en C₁-C₄, halogénoalcoxy(C₁-C₄), alkyl(C₁-C₄)thio et [alcoxy(C₁-C₄)]-carbonyle, et
R⁴ représente un groupe alkyle en C₁-C₁₀, alcényle en C₂-C₁₀ ou alcynyle en C₂-C₁₀, chacun des 3 radicaux nommés en dernier étant non substitué ou substitué par un ou plusieurs radicaux choisis dans le groupe constitué par alcoxy en C₁-C₄, halogénoalcoxy(C₁-C₄), alkyl(C₁-C₄)thio et [alcoxy(C₁-C₄)]-carbonyle.

5. Utilisation selon l'une quelconque des revendications 1 à 4, **caractérisée en ce que**
R¹ représente un radical de formule CF₃, CF₂Cl, CF₂H, CF₂CF₃, CF₂CF₂H, CF₂CF₂Cl, CF₂CF₂CF₃ ou C(CH₃)₂F et
R² représente un atome d'hydrogène ou d'halogène.

6. Utilisation selon la revendication 1, **caractérisée en ce que**
R¹ représente un radical halogénoalcoxy(C₁-C₆),
R² représente un atome d'halogène,
R³ représente un atome d'hydrogène, et
R⁴ représente un atome d'hydrogène.

7. Utilisation selon la revendication 1, **caractérisée en ce que**
R¹ représente un radical de formule CF₂Cl, CF₂H, CF₂CF₃, CF₂CF₂H, CF₂CF₂Cl, CFClCF₃, CFHCF₃, CF(CF₃)₂, CH(CF₃)₂, CF₂CF₂CF₃ ou C(CH₃)₂F,
R² représente un atome d'hydrogène,
R³ représente un atome d'hydrogène et
R⁴ représente un atome d'hydrogène.

8. Utilisation selon l'une quelconque des revendications 1 à 7, **caractérisée en ce qu'**on utilise un ou plusieurs composés de formule (I) ou sels de tels composés conjointement avec une ou plusieurs substances agrochimiques qui, appliquées seules, provoquent des dommages aux plantes utiles, éventuellement en présence d'adjuvants de formulation.

9. Composés de formule (I) ou leurs sels, 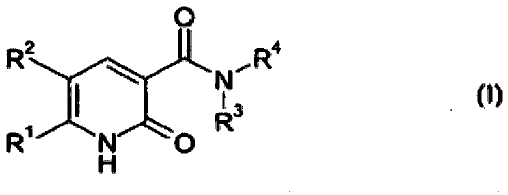 formule dans laquelle
R¹ représente un radical halogénoalkyle(C₁-C₆) et
R² représente un atome d'hydrogène ou d'halogène et
R³ représente un atome d'hydrogène, un groupe alkyle en C₁-C₁₆, alcényle en C₂-C₁₆ ou alcynyle en C₂-C₁₆, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle et [halogénoalcoxy(C₁-C₄)]-carbonyle, ou cycloalkyle en C₃-C₆, cycloalcényle en C₄-C₆, cycloalkyle en C₃-C₆ qui est condensé sur un côté du cycle avec un cycle carbocyclique saturé ou insaturé à 4 à 6 chaînons, ou cycloalcényle en C₄-C₆ qui est condensé sur un côté du cycle avec un cycle carbocyclique saturé ou insaturé à 4 à 6 chaînons, chacun des 4 radicaux nommés en dernier étant non substitué ou substitué par un ou plusieurs radicaux choisis dans le groupe constitué par halogéno, hydroxy, cyano, alkyle en C₁-C₄, halogénoalkyle(C₁-C₄), alcoxy en C₁-C₄, halogénoalcoxy(C₁-C₄), alkyl (C₁-C₄)thio, alkyl (C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est substitué ou non substitué, phényle qui est substitué ou non substitué, et hétérocyclyle qui est substitué ou non substitué, et
R⁴ représente un groupe alkyle en C₁-C₁₆, alcényle en C₂-C₁₆ ou alcynyle en C₂-C₁₆, chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle et [halogénoalcoxy(C₁-C₄)]-carbonyle, ou cycloalkyle en C₃-C₆, cycloalcényle en C₄-C₆, cycloalkyle en C₃-C₆ qui est condensé sur un côté du cycle avec un cycle carbocyclique saturé ou insaturé à 4 à 6 chaînons, ou cycloalcényle en C₄-C₆ qui est condensé sur un côté du cycle avec un cycle carbocyclique saturé ou insaturé à 4 à 6 chaînons, chacun des 4 radicaux nommés en dernier étant non substitué ou substitué par un ou plusieurs radicaux choisis dans le groupe constitué par halogéno, hydroxy, cyano, alkyle en C₁-C₄, halogénoalkyle(C₁-C₄), alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogénoalcoxy(C₁-C₄)]-carbonyle, cycloalkyle en C₃-C₆ qui est substitué ou non substitué, phényle qui est substitué ou non substitué, et hétérocyclyle qui est substitué ou non substitué, ou
R³ représente un groupe alcoxy en C₁-C₄, alcényl(C₂-C₄)oxy, alcynyl(C₂-C₆)oxy ou halogéno-alcoxy(C₂-C₄) et
R⁴ représente un atome d'hydrogène ou un groupe alkyle en C₁-C₄ ou
R³ et R⁴ forment ensemble avec l'atome d'azote directement lié un cycle hétérocyclique à quatre à huit chaînons, qui outre l'atome d'azote peut également contenir d'autres hétéroatomes formant le cycle et qui est non substitué ou substitué par un ou plusieurs radicaux choisis dans le groupe constitué par halogéno, cyano, nitro, alkyle en C₁-C₄, halogénoalkyle(C₁-C₄), alcoxy en C₁-C₄, halogénoalcoxy(C₁-C₄) et alkyl(C₁-C₄)thio, ou
R³ et R⁴ forment ensemble avec l'atome d'azote directement lié le groupe -N=CR⁵-NR⁶R⁷, dans lequel
R⁵ représente un atome d'hydrogène ou un groupe alkyle en C₁-C₆, et
R⁶, R⁷ représentent indépendamment l'un de l'autre un atome d'hydrogène ou un groupe alkyle en C₁-C₄ ou R⁶ et R⁷ forment ensemble avec l'atome d'azote directement lié un cycle hétérocyclique à cinq à sept chaînons, ou
R¹ représente un radical halogénoalkyle(C₁-C₆),
R² représente un atome d'halogène,
R³ représente un atome d'hydrogène et
R⁴ représente un atome d'hydrogène ou
R¹ représente un radical de formule CF₂CF₃, CF₂CF₂H, CF₂CF₂Cl, CFClCF₃, CFHCF₃, CF(CF₃)₂, CH(CF₃)₂, CF₂CF₂CF₃ ou C(CH₃)₂F,
R² représente un atome d'hydrogène,
R³ représente un atome d'hydrogène et
R⁴ représente un atome d'hydrogène.

10. Procédé pour la préparation des composés de formule (I) ou de leurs sels selon la revendication 9, **caractérisé en ce que**
(a) on fait réagir un acide carboxylique de formule
générale (II) 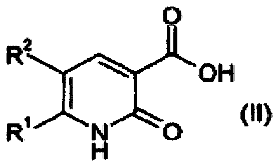 dans laquelle R¹ et R² sont tels que définis dans le composé de formule (I) à préparer,
avec une amine de formule (III) ou un sel d'une telle amine, 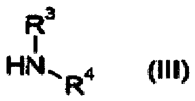 formule dans laquelle R³ et R⁴ sont tels que définis dans le composé de formule (I) à préparer, pour aboutir au composé de formule (I) ou
(b) on fait réagir un ester d'acide carboxylique de
formule générale (IV) 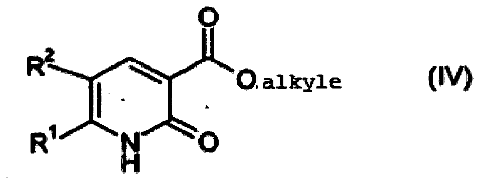 dans laquelle R¹ et R² sont tels que définis dans le composé de formule (I) à préparer et « alkyle » signifie un radical alkyle, avec une amine de formule (III) ou un sel d'une telle amine, 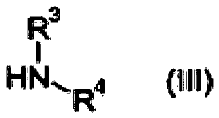 formule dans laquelle R³ et R⁴ sont tels que définis dans le composé de formule (I) à préparer, pour aboutir au composé de formule (I) ou
(c) on fait réagir un halogénure ou anhydride d'acide
carboxylique de formule générale (V), 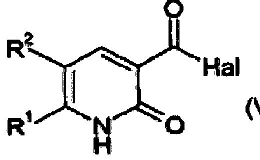 dans laquelle R¹ et R² sont tels que définis dans le composé de formule (I) à préparer et Hal représente un atome d'halogène ou un radical acyloxy, avec une amine de formule (III) ou un sel d'une telle amine, 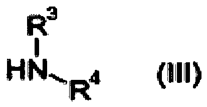 formule dans laquelle R³ et R⁴ sont tels que définis dans le composé de formule (I) à préparer, pour aboutir au composé de formule (I) ou
(d) dans le cas où R³ et R⁴ dans le composé de formule (I) à préparer représentent chacun un atome d'hydrogène,
on fait réagir un composé de formule (VI), 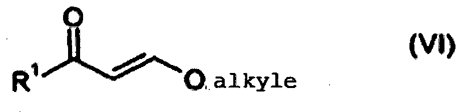 dans laquelle R¹ est tel que défini dans le composé de formule (I) à préparer et « alkyle » signifie un radical alkyle,
avec du malondiamide, pour aboutir au composé de formule (I).

11. Produit phytosanitaire, **caractérisé en ce qu'**il contient un ou plusieurs composés de formule (I) ou sels de tels composés, tels qu'ils sont définis selon l'une quelconque des revendications 1 à 7 et 9, et des adjuvants de formulation.

12. Produit phytosanitaire, **caractérisé en ce qu'**il contient un ou plusieurs composés de formule (I) ou sels de tels composés, tels qu'ils sont définis selon l'une quelconque des revendications 1 à 7 et 9, et une ou plusieurs substances agrochimiques et éventuellement des adjuvants de formulation.

13. Procédé pour la protection de plantes utiles contre les effets secondaires phytotoxiques de substances agrochimiques, **caractérisé en ce qu'**on applique sur les plantes, parties de plantes, graines de plantes ou les semences une quantité efficace d'un ou de plusieurs composés de formule (I) ou sels de tels composés, tels qu'ils sont définis selon l'une quelconque des revendications 1 à 7 et 9, avant, après ou en même temps que les substances agrochimiques.

14. Procédé selon la revendication 13, **caractérisé en ce que** l'application s'effectue dans le procédé en post-levée.

15. Procédé selon la revendication 13, **caractérisé en ce que** l'application avec le composé de formule (I) ou son sel s'effectue par traitement des graines de plantes ou des semences.

16. Procédé selon la revendication 13, **caractérisé en ce que** l'application s'effectue dans le procédé en pré-levée.

17. Procédé pour la lutte sélective contre des plantes nuisibles dans des cultures de plantes utiles, **caractérisé en ce qu'**on applique sur les plantes, parties de plantes, graines de plantes ou les semences une quantité efficace d'un ou de plusieurs composés de formule (I) ou sels de tels composés, tels qu'ils sont définis selon l'une quelconque des revendications 1 à 7 et 9, avant, après ou en même temps qu'un ou plusieurs herbicides.

18. Procédé selon la revendication 17, **caractérisé en ce qu'**on traite les semences par un ou plusieurs composés de formule (I) ou sels de tels composés et on applique l'herbicide après le semis dans le procédé en pré-levée ou dans le procédé en post-levée.
